# EUROPEAN PATENT SPECIFICATION

(11) **EP 3 362 473 B1**
(45) Date of publication and mention of the grant of the patent: **07.04.2021**
(21) Application number: 16784956.1
(22) Date of filing: 14.10.2016
(51) Int. Cl.: A21D 8/04, A61K 38/36, C12N 9/26, G01N 33/50, G01N 33/574, C07K 14/75

(54) **USE OF A FIBRINOGEN CAPTURE AGENT TO DETECT A CIZ1 B-VARIANT**
VERWENDUNG EINES FIBRINOGENEINFANGWIRKSTOFFS ZUR DETEKTION EINER CIZ1-B-VARIANTE
UTILISATION D'UN AGENT DE CAPTURE FIBRINOGÈNE POUR DÉTECTER UN VARIANT B DE CIZ1

(30) Priority: 19.10.2015 GB 201518466
(43) Date of publication of application: 22.08.2018
(73) Proprietor: Cizzle Biotechnology Limited, Heslington, Yorkshire YO10 5DD (GB)
(72) Inventor: COVERLEY, Dawn Alison, Heslington Yorkshire YO10 5DD (GB)
(74) Representative: D Young & Co LLP
(86) International application number: PCT/GB2016/053203
(87) International publication number: WO 2017/068330

(56) References cited:
- WO-A1-2010/089559
- WO-A1-2012/017208
- G. HIGGINS ET AL: "Variant Ciz1 is a circulating biomarker for early-stage lung cancer", PROCEEDINGS OF THE NATIONAL ACADEMY OF SCIENCES, vol. 109, no. 45, 16 October 2012 (2012-10-16), pages E3128-E3135, XP055333175, US ISSN: 0027-8424, DOI: 10.1073/pnas.1210107109 cited in the application & G. HIGGINS ET AL: "Variant Ciz1 is a circulating biomarker for early-stage lung cancer: Supporting information", PROCEEDINGS OF THE NATIONAL ACADEMY OF SCIENCES, vol. 109, no. 45, 16 October 2012 (2012-10-16), pages E3128-E3135, XP055333445, US ISSN: 0027-8424, DOI: 10.1073/pnas.1210107109 cited in the application
- Ahn ET AL: "J Mol Biomark Diagn Protein & Prognostic Biomarkers ISSN: 2155-9929 JMBD, an open access journal Molecular Biomarkers & Diagnosis", J Mol Biomark Diagn, 11 March 2013 (2013-03-11), pages 1-7, XP055107049, DOI: 10.4172/2155-9929.S4-001 Retrieved from the Internet: URL:http://omicsonline.org/current-serum-l ung-cancer-biomarkers-2155-9929.S4-001.pdf [retrieved on 2014-03-11]
- DAWN COVERLEY ET AL: "A quantitative immunoassay for lung cancer biomarker CIZ1b in patient plasma", CLINICAL BIOCHEMISTRY, 17 November 2016 (2016-11-17), XP055333177, US, CA ISSN: 0009-9120, DOI: 10.1016/j.clinbiochem.2016.11.015

### SEQUENCE LISTING

<110> Cizzle Biotechnology Limited
<120> USE
<130> P105215PCT
<150> GB 1518466.6
   <151> 2015-10-19
<160> 1063
<170> PatentIn version 3.5
<210> 1
   <211> 890
   <212> PRT
   <213> Artificial Sequence
<220>
   <223> Full Ciz1 b-variant sequence
<400> 1
   Met Phe Ser Gln Gln Gln Gln Gln Gln Leu Gln Gln Gln Gln Gln Gln
   1               5                   10                  15
   Leu Gln Gln Leu Gln Gln Gln Gln Leu Gln Gln Gln Gln Leu Gln Gln
               20                  25                  30
   Gln Gln Leu Leu Gln Leu Gln Gln Leu Leu Gln Gln Ser Pro Pro Gln
           35                  40                  45
   Ala Pro Leu Pro Met Ala Val Ser Arg Gly Leu Pro Pro Gln Gln Pro
       50                  55                  60
   Gln Gln Pro Leu Leu Asn Leu Gln Gly Thr Asn Ser Ala Ser Leu Leu
   65                  70                  75                  80
   Asn Gly Ser Met Leu Gln Arg Ala Leu Leu Leu Gln Gln Leu Gln Gly
                   85                  90                  95
   Leu Asp Gln Phe Ala Met Pro Pro Ala Thr Tyr Asp Thr Ala Gly Leu
               100                 105                 110
   Thr Met Pro Thr Ala Thr Leu Gly Asn Leu Arg Gly Tyr Gly Met Ala
           115                 120                 125
   Ser Pro Gly Leu Ala Ala Pro Ser Leu Thr Pro Pro Gln Leu Ala Thr
       130                 135                 140
   Pro Asn Leu Gln Gln Phe Phe Pro Gln Ala Thr Arg Gln Ser Leu Leu
   145                 150                 155                 160
   Gly Pro Pro Pro Val Gly Val Pro Met Asn Pro Ser Gln Phe Asn Leu
                   165                 170                 175
   Ser Gly Arg Asn Pro Gln Lys Gln Ala Arg Thr Ser Ser Ser Thr Thr
               180                 185                 190
   Pro Asn Arg Lys Asp Ser Ser Ser Gln Thr Met Pro Val Glu Asp Lys
           195                 200                 205
   Ser Asp Pro Pro Glu Gly Ser Glu Glu Ala Ala Glu Pro Arg Met Asp
       210                 215                 220
   Thr Pro Glu Asp Gln Asp Leu Pro Pro Cys Pro Glu Asp Ile Ala Lys
   225                 230                 235                 240
   Glu Lys Arg Thr Pro Ala Pro Glu Pro Glu Pro Cys Glu Ala Ser Glu
                   245                 250                 255
   Leu Pro Ala Lys Arg Leu Arg Ser Ser Glu Glu Pro Thr Glu Lys Glu
               260                 265                 270
   Pro Pro Gly Gln Leu Gln Val Lys Ala Gln Pro Gln Ala Arg Met Thr
           275                 280                 285
   Val Pro Lys Gln Thr Gln Thr Pro Asp Leu Leu Pro Glu Ala Leu Glu
       290                 295                 300
   Ala Gln Val Leu Pro Arg Phe Gln Pro Arg Val Leu Gln Val Gln Ala
   305                 310                 315                 320
   Gln Val Gln Ser Gln Thr Gln Pro Arg Ile Pro Ser Thr Asp Thr Gln
                   325                 330                 335
   Val Gln Pro Lys Leu Gln Lys Gln Ala Gln Thr Gln Thr Ser Pro Glu
               340                 345                 350
   His Leu Val Leu Gln Gln Lys Gln Val Gln Pro Gln Leu Gln Gln Glu
           355                 360                 365
   Ala Glu Pro Gln Lys Gln Val Gln Pro Gln Val Gln Pro Gln Ala His
       370                 375                 380
   Ser Gln Gly Pro Arg Gln Val Gln Leu Gln Gln Glu Ala Glu Pro Leu
   385                 390                 395                 400
   Lys Gln Val Gln Pro Gln Val Gln Pro Gln Ala His Ser Gln Pro Pro
   405 410 415
   Arg Gln Val Gln Leu Gln Leu Gln Lys Gln Val Gln Thr Gln Thr Tyr
   420 425 430
   Pro Gln Val His Thr Gln Ala Gln Pro Ser Val Gln Pro Gln Glu His
   435 440 445
   Pro Pro Ala Gln Val Ser Val Gln Pro Pro Glu Gln Thr His Glu Gln
   450 455 460
   Pro His Thr Gln Pro Gln Val Ser Leu Leu Ala Pro Glu Gln Thr Pro
   465 470 475 480
   Val Val Val His Val Cys Gly Leu Glu Met Pro Pro Asp Ala Val Glu
   485 490 495
   Ala Gly Gly Gly Met Glu Lys Thr Leu Pro Glu Pro Val Gly Thr Gln
   500 505 510
   Val Ser Met Glu Glu Ile Gln Asn Glu Ser Ala Cys Gly Leu Asp Val
   515 520 525
   Gly Glu Cys Glu Asn Arg Ala Arg Glu Met Pro Gly Val Trp Gly Ala
   530 535 540
   Gly Gly Ser Leu Lys Val Thr Ile Leu Gln Ser Ser Asp Ser Arg Ala
   545 550 555 560
   Phe Ser Thr Val Pro Leu Thr Pro Val Pro Arg Pro Ser Asp Ser Val
   565 570 575
   Ser Ser Thr Pro Ala Ala Thr Ser Thr Pro Ser Lys Gln Ala Leu Gln
   580 585 590
   Phe Phe Cys Tyr Ile Cys Lys Ala Ser Cys Ser Ser Gln Gln Glu Phe
   595 600 605
   Gln Asp His Met Ser Glu Pro Gln His Gln Gln Arg Leu Gly Glu Ile
   610 615 620
   Gln His Met Ser Gln Ala Cys Leu Leu Ser Leu Leu Pro Val Pro Arg
   625 630 635 640
   Asp Val Leu Glu Thr Glu Asp Glu Glu Pro Pro Pro Arg Arg Trp Cys
   645 650 655
   Asn Thr Cys Gln Leu Tyr Tyr Met Gly Asp Leu Ile Gln His Arg Arg
               660                 665                 670
   Thr Gln Asp His Lys Ile Ala Lys Gln Ser Leu Arg Pro Phe Cys Thr
           675                 680                 685
   Val Cys Asn Arg Tyr Phe Lys Thr Pro Arg Lys Phe Val Glu His Val
       690                 695                 700
   Lys Ser Gln Gly His Lys Asp Lys Ala Lys Glu Leu Lys Ser Leu Glu
   705                 710                 715                 720
   Lys Glu Ile Ala Gly Gln Asp Glu Asp His Phe Ile Thr Val Asp Ala
                   725                 730                 735
   Val Gly Cys Phe Glu Gly Asp Glu Glu Glu Glu Glu Asp Asp Glu Asp
               740                 745                 750
   Glu Glu Glu Ile Glu Val Arg Ser Arg Asp Ile Ser Arg Glu Glu Trp
           755                 760                 765
   Lys Gly Ser Glu Thr Tyr Ser Pro Asn Thr Ala Tyr Gly Val Asp Phe
       770                 775                 780
   Leu Val Pro Val Met Gly Tyr Ile Cys Arg Ile Cys His Lys Phe Tyr
   785                 790                 795                 800
   His Ser Asn Ser Gly Ala Gln Leu Ser His Cys Lys Ser Leu Gly His
                   805                 810                 815
   Phe Glu Asn Leu Gln Lys Tyr Lys Ala Ala Lys Asn Pro Ser Pro Thr
               820                 825                 830
   Thr Arg Pro Val Ser Arg Arg Cys Ala Ile Asn Ala Arg Asn Ala Leu
           835                 840                 845
   Thr Ala Leu Phe Thr Ser Ser Gly Arg Pro Pro Ser Gln Pro Asn Thr
       850                 855                 860
   Gln Asp Lys Thr Pro Ser Lys Val Thr Ala Arg Pro Ser Gln Pro Pro
   865                 870                 875                 880
   Leu Pro Arg Arg Ser Thr Arg Leu Lys Thr
                   885                 890
<210> 2
   <211> 13
   <212> PRT
   <213> Artificial Sequence
<220>
   <223> sequence comprising the exon 14b/exon 15 amino acid junction sequence
<400> 2
   Asp Glu Glu Glu Ile Glu Val Arg Ser Arg Asp Ile Ser
   1               5                   10
<210> 3
   <211> 126
   <212> RNA
   <213> Artificial Sequence
<220>
   <223> exon 14b nucleotide sequence
<400> 3
   cugaagucgc uugagaaaga aauugcuggc caagaugagg agcacuucau uacaguggac    60
   gcuguggguu gcuucgaggg ugaugaagaa gaggaagagg augaugagga ugaagaagag   120
   aucgag                                                              126
<210> 4
   <211> 150
   <212> RNA
   <213> Artificial Sequence
<220>
   <223> exon 14a nucleotide sequence
<400> 4
   cugaagucgc uugagaaaga aauugcuggc caagaugagg agcacuucau uacaguggac    60
   gcuguggguu gcuucgaggg ugaugaagaa gaggaagagg augaugagga ugaagaagag   120
   aucgagguug aggaggaacu cugcaagcag                                    150
<210> 5
   <211> 42
   <212> PRT
   <213> Artificial Sequence
<220>
   <223> exon 14b amino acid sequence
<400> 5
   Leu Lys Ser Leu Glu Lys Glu Ile Ala Gly Gln Asp Glu Asp His Phe
   1               5                   10                  15
   Ile Thr Val Asp Ala Val Gly Cys Phe Glu Gly Asp Glu Glu Glu Glu
               20                  25                  30
   Glu Asp Asp Glu Asp Glu Glu Glu Ile Glu
                       35                  40
<210> 6
   <211> 50
   <212> PRT
   <213> Artificial Sequence
<220>
   <223> exon 14a amino acid sequence
<400> 6
   Leu Lys Ser Leu Glu Lys Glu Ile Ala Gly Gln Asp Glu Asp His Phe
   1               5                   10                  15
   Ile Thr Val Asp Ala Val Gly Cys Phe Glu Gly Asp Glu Glu Glu Glu
               20                  25                  30
   Glu Asp Asp Glu Asp Glu Glu Glu Ile Glu Val Glu Glu Glu Leu Cys
           35                  40                  45
   Lys Gln
       50
<210> 7
   <211> 23
   <212> PRT
   <213> Artificial Sequence
<220>
   <223> exon 15 amino acid sequence
<400> 7
   Val Arg Ser Arg Asp Ile Ser Arg Glu Glu Trp Lys Gly Ser Glu Thr
   1               5                   10                  15
   Tyr Ser Pro Asn Thr Ala Tyr
               20
<210> 8
   <211> 898
   <212> PRT
   <213> Homo sapiens
<400> 8
   Met Phe Ser Gln Gln Gln Gln Gln Gln Leu Gln Gln Gln Gln Gln Gln
   1               5                   10                  15
   Leu Gln Gln Leu Gln Gln Gln Gln Leu Gln Gln Gln Gln Leu Gln Gln
               20                  25                  30
   Gln Gln Leu Leu Gln Leu Gln Gln Leu Leu Gln Gln Ser Pro Pro Gln
               35                  40                  45
   Ala Pro Leu Pro Met Ala Val Ser Arg Gly Leu Pro Pro Gln Gln Pro
       50                  55                  60
   Gln Gln Pro Leu Leu Asn Leu Gln Gly Thr Asn Ser Ala Ser Leu Leu
   65                  70                  75                  80
   Asn Gly Ser Met Leu Gln Arg Ala Leu Leu Leu Gln Gln Leu Gln Gly
                   85                  90                  95
   Leu Asp Gln Phe Ala Met Pro Pro Ala Thr Tyr Asp Thr Ala Gly Leu
               100                 105                 110
   Thr Met Pro Thr Ala Thr Leu Gly Asn Leu Arg Gly Tyr Gly Met Ala
           115                 120                 125
   Ser Pro Gly Leu Ala Ala Pro Ser Leu Thr Pro Pro Gln Leu Ala Thr
       130                 135                 140
   Pro Asn Leu Gln Gln Phe Phe Pro Gln Ala Thr Arg Gln Ser Leu Leu
   145                 150                 155                 160
   Gly Pro Pro Pro Val Gly Val Pro Met Asn Pro Ser Gln Phe Asn Leu
                   165                 170                 175
   Ser Gly Arg Asn Pro Gln Lys Gln Ala Arg Thr Ser Ser Ser Thr Thr
               180                 185                 190
   Pro Asn Arg Lys Asp Ser Ser Ser Gln Thr Met Pro Val Glu Asp Lys
           195                 200                 205
   Ser Asp Pro Pro Glu Gly Ser Glu Glu Ala Ala Glu Pro Arg Met Asp
       210                 215                 220
   Thr Pro Glu Asp Gln Asp Leu Pro Pro Cys Pro Glu Asp Ile Ala Lys
   225                 230                 235                 240
   Glu Lys Arg Thr Pro Ala Pro Glu Pro Glu Pro Cys Glu Ala Ser Glu
                   245                 250                 255
   Leu Pro Ala Lys Arg Leu Arg Ser Ser Glu Glu Pro Thr Glu Lys Glu
               260                 265                 270
   Pro Pro Gly Gln Leu Gln Val Lys Ala Gln Pro Gln Ala Arg Met Thr
           275                 280                 285
   Val Pro Lys Gln Thr Gln Thr Pro Asp Leu Leu Pro Glu Ala Leu Glu
   290 295 300
   Ala Gln Val Leu Pro Arg Phe Gln Pro Arg Val Leu Gln Val Gln Ala
   305 310 315 320
   Gln Val Gln Ser Gln Thr Gln Pro Arg Ile Pro Ser Thr Asp Thr Gln
   325 330 335
   Val Gln Pro Lys Leu Gln Lys Gln Ala Gln Thr Gln Thr Ser Pro Glu
   340 345 350
   His Leu Val Leu Gln Gln Lys Gln Val Gln Pro Gln Leu Gln Gln Glu
   355 360 365
   Ala Glu Pro Gln Lys Gln Val Gln Pro Gln Val Gln Pro Gln Ala His
   370 375 380
   Ser Gln Gly Pro Arg Gln Val Gln Leu Gln Gln Glu Ala Glu Pro Leu
   385 390 395 400
   Lys Gln Val Gln Pro Gln Val Gln Pro Gln Ala His Ser Gln Pro Pro
   405 410 415
   Arg Gln Val Gln Leu Gln Leu Gln Lys Gln Val Gln Thr Gln Thr Tyr
   420 425 430
   Pro Gln Val His Thr Gln Ala Gln Pro Ser Val Gln Pro Gln Glu His
   435 440 445
   Pro Pro Ala Gln Val Ser Val Gln Pro Pro Glu Gln Thr His Glu Gln
   450 455 460
   Pro His Thr Gln Pro Gln Val Ser Leu Leu Ala Pro Glu Gln Thr Pro
   465 470 475 480
   Val Val Val His Val Cys Gly Leu Glu Met Pro Pro Asp Ala Val Glu
   485 490 495
   Ala Gly Gly Gly Met Glu Lys Thr Leu Pro Glu Pro Val Gly Thr Gln
   500 505 510
   Val Ser Met Glu Glu Ile Gln Asn Glu Ser Ala Cys Gly Leu Asp Val
   515 520 525
   Gly Glu Cys Glu Asn Arg Ala Arg Glu Met Pro Gly Val Trp Gly Ala
   530 535 540
   Gly Gly Ser Leu Lys Val Thr Ile Leu Gln Ser Ser Asp Ser Arg Ala
   545                 550                 555                 560
   Phe Ser Thr Val Pro Leu Thr Pro Val Pro Arg Pro Ser Asp Ser Val
                   565                 570                 575
   Ser Ser Thr Pro Ala Ala Thr Ser Thr Pro Ser Lys Gln Ala Leu Gln
               580                 585                 590
   Phe Phe Cys Tyr Ile Cys Lys Ala Ser Cys Ser Ser Gln Gln Glu Phe
           595                 600                 605
   Gln Asp His Met Ser Glu Pro Gln His Gln Gln Arg Leu Gly Glu Ile
       610                 615                 620
   Gln His Met Ser Gln Ala Cys Leu Leu Ser Leu Leu Pro Val Pro Arg
   625                 630                 635                 640
   Asp Val Leu Glu Thr Glu Asp Glu Glu Pro Pro Pro Arg Arg Trp Cys
                   645                 650                 655
   Asn Thr Cys Gln Leu Tyr Tyr Met Gly Asp Leu Ile Gln His Arg Arg
               660                 665                 670
   Thr Gln Asp His Lys Ile Ala Lys Gln Ser Leu Arg Pro Phe Cys Thr
           675                 680                 685
   Val Cys Asn Arg Tyr Phe Lys Thr Pro Arg Lys Phe Val Glu His Val
       690                 695                 700
   Lys Ser Gln Gly His Lys Asp Lys Ala Lys Glu Leu Lys Ser Leu Glu
   705                 710                 715                 720
   Lys Glu Ile Ala Gly Gln Asp Glu Asp His Phe Ile Thr Val Asp Ala
                   725                 730                 735
   Val Gly Cys Phe Glu Gly Asp Glu Glu Glu Glu Glu Asp Asp Glu Asp
               740                 745                 750
   Glu Glu Glu Ile Glu Val Glu Glu Glu Leu Cys Lys Gln Val Arg Ser
           755                 760                 765
   Arg Asp Ile Ser Arg Glu Glu Trp Lys Gly Ser Glu Thr Tyr Ser Pro
       770                 775                 780
   Asn Thr Ala Tyr Gly Val Asp Phe Leu Val Pro Val Met Gly Tyr Ile
   785                 790                 795                 800
   Cys Arg Ile Cys His Lys Phe Tyr His Ser Asn Ser Gly Ala Gln Leu
   805 810 815
   Ser His Cys Lys Ser Leu Gly His Phe Glu Asn Leu Gln Lys Tyr Lys
   820 825 830
   Ala Ala Lys Asn Pro Ser Pro Thr Thr Arg Pro Val Ser Arg Arg Cys
   835 840 845
   Ala Ile Asn Ala Arg Asn Ala Leu Thr Ala Leu Phe Thr Ser Ser Gly
   850 855 860
   Arg Pro Pro Ser Gln Pro Asn Thr Gln Asp Lys Thr Pro Ser Lys Val
   865 870 875 880
   Thr Ala Arg Pro Ser Gln Pro Pro Leu Pro Arg Arg Ser Thr Arg Leu
   885 890 895
   Lys Thr
<210> 9
   <211> 21
   <212> PRT
   <213> Artificial Sequence
<220>
   <223> sequence comprising the exon 14a/exon 15 splice junction
<400> 9
   Asp Glu Glu Glu Ile Glu Val Glu Glu Glu Leu Cys Lys Gln Val Arg
   1 5 10 15
   Ser Arg Asp Ile Ser
   20
<210> 10
   <211> 8
   <212> PRT
   <213> Artificial Sequence
<220>
   <223> excluded sequence from Ciz1 exon 14/exon 15 junction which specifies a Ciz1 b-variant
<400> 10
   Val Glu Glu Glu Leu Cys Lys Gln
   1 5
<210> 11
   <211> 66
   <212> PRT
   <213> Artificial Sequence
<220>
   <223> Ciz1 b-variant peptide
<400> 11
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr Gly Val Asp Phe Leu
       50                  55                  60
   Val Pro
   65
<210> 12
   <211> 65
   <212> PRT
   <213> Artificial Sequence
<220>
   <223> long Ciz1 b-variant peptide
<400> 12
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr Gly Val Asp Phe Leu
       50                  55                  60
   Val
   65
<210> 13
   <211> 72
   <212> PRT
   <213> Artificial Sequence
<220>
   <223> long Ciz1 exon 14a peptide
<400> 13
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Glu Glu Glu Leu Cys Lys Gln Arg Ser Arg Asp
           35                  40                  45
   Ile Ser Arg Glu Glu Trp Lys Gly Ser Glu Thr Tyr Ser Pro Asn Thr
       50                  55                  60
   Ala Tyr Gly Val Asp Phe Leu Val
   65                  70
<210> 14
   <211> 14
   <212> PRT
   <213> Artificial Sequence
<220>
   <223> GLA3 sequence
<220>
   <221> MOD_RES
   <222> (5)..(5)
   <223> carboxylated glutamic acid
<220>
   <221> MOD_RES
   <222> (14)..(14)
   <223> AMIDATION
<400> 14
   Cys Asp Glu Glu Glu Ile Glu Val Arg Ser Arg Asp Ile Ser
   1               5                   10
<210> 15
   <211> 14
   <212> PRT
   <213> Artificial Sequence
<220>
   <223> GLA6 sequence
<220>
   <221> MOD_RES
   <222> (3)..(5)
   <223> carboxylated glutamic acid
<220>
   <221> MOD_RES
   <222> (7)..(7)
   <223> carboxylated glutamic acid
<220>
   <221> MOD_RES
   <222> (14)..(14)
   <223> AMIDATION
<400> 15
   Cys Asp Glu Glu Glu Ile Glu Val Arg Ser Arg Asp Ile Ser
   1               5                   10
<210> 16
   <211> 10
   <212> PRT
   <213> Artificial Sequence
<220>
   <223> diagnostic fragment of CIZ1b
<400> 16
   Asp Glu Glu Glu Ile Glu Val Arg Ser Arg
   1               5                   10
<210> 17
   <211> 8
   <212> PRT
   <213> Artificial Sequence
<220>
   <223> super short CIZ1b peptide (b7)
<220>
   <221> MOD_RES
   <222> (8)..(8)
   <223> AMIDATION
<400> 17
   Cys Glu Ile Glu Val Arg Ser Arg
   1               5
<210> 18
   <211> 23
   <212> PRT
   <213> Artificial Sequence
<220>
   <223> short CIZ1a peptide (a22)
<220>
   <221> MOD_RES
   <222> (23)..(23)
   <223> AMIDATION
<400> 18
   Cys Asp Glu Glu Glu Ile Glu Val Glu Glu Glu Leu Cys Lys Gln Val
   1               5                   10                  15
   Arg Ser Arg Asp Ile Ser Arg
               20
<210> 19
   <211> 74
   <212> PRT
   <213> Artificial Sequence
<220>
   <223> long CIZ1a peptide (a74)
<400> 19
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Glu Glu Glu Leu Cys Lys Gln Val Arg Ser Arg
           35                  40                  45
   Asp Ile Ser Arg Glu Glu Trp Lys Gly Ser Glu Thr Tyr Ser Pro Asn
       50                  55                  60
   Thr Ala Tyr Gly Val Asp Phe Leu Val Pro
   65                  70
<210> 20
   <211> 20
   <212> PRT
   <213> Artificial Sequence
<220>
   <223> exon 17 peptide sequence
<400> 20
   Cys Thr Ser Ser Gly Arg Pro Pro Ser Gln Pro Asn Thr Gln Asp Lys
   1               5                   10                  15
   Thr Pro Ser Lys
               20
<211> 19
   <212> PRT
   <213> Artificial Sequence
<220>
   <223> exon 17 peptide sequence
<400> 21 Leu Lys Thr
<210> 22
   <211> 14
   <212> PRT
   <213> Artificial Sequence
<220>
   <223> short CIZ1b peptide (b13)
<220>
   <221> MOD_RES
   <222> (14)..(14)
   <223> AMIDATION
<400> 22
   Cys Asp Glu Glu Glu Ile Glu Val Arg Ser Arg Asp Ile Ser
   1 5 10
<210> 23
   <211> 14
   <212> PRT
   <213> Artificial Sequence
<220>
   <223> GLA4 peptide
<220>
   <221> MOD_RES
   <222> (4)..(4)
   <223> carboxylated glutamic acid
<220>
   <221> MOD_RES
   <222> (14)..(14)
   <223> AMIDATION
<400> 23
   Cys Asp Glu Glu Glu Ile Glu Val Arg Ser Arg Asp Ile Ser
   1 5 10
<210> 24
   <211> 38
   <212> PRT
   <213> Artificial Sequence
<220>
   <223> Ciz1 b-variant peptide comprising an exon 14b/exon 15 junction
<400> 24
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg
           35
<210> 25
   <211> 37
   <212> PRT
   <213> Artificial Sequence
<220>
   <223> Ciz1 b-variant peptide comprising an exon 14b/exon 15 junction
<400> 25
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg
           35
<210> 26
   <211> 36
   <212> PRT
   <213> Artificial Sequence
<220>
   <223> Ciz1 b-variant peptide comprising an exon 14b/exon 15 junction
<400> 26
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg
           35
<210> 27
   <211> 35
   <212> PRT
   <213> Artificial Sequence
<220>
   <223> Ciz1 b-variant peptide comprising an exon 14b/exon 15 junction
<400> 27
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg
           35
<210> 28
   <211> 34
   <212> PRT
   <213> Artificial Sequence
<220>
   <223> Ciz1 b-variant peptide comprising an exon 14b/exon 15 junction
<400> 28
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg
<210> 29
   <211> 33
   <212> PRT
   <213> Artificial Sequence
<220>
   <223> Ciz1 b-variant peptide comprising an exon 14b/exon 15 junction
<400> 29
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg
<210> 30
   <211> 32
   <212> PRT
   <213> Artificial Sequence
<220>
   <223> Ciz1 b-variant peptide comprising an exon 14b/exon 15 junction
<400> 30
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
<210> 31
   <211> 31
   <212> PRT
   <213> Artificial Sequence
<220>
   <223> Ciz1 b-variant peptide comprising an exon 14b/exon 15 junction
<400> 31
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg
               20                  25                  30
<210> 32
   <211> 30
   <212> PRT
   <213> Artificial Sequence
<220>
   <223> Ciz1 b-variant peptide comprising an exon 14b/exon 15 junction
<400> 32
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg
               20                  25                  30
<210> 33
   <211> 29
   <212> PRT
   <213> Artificial Sequence
<220>
   <223> Ciz1 b-variant peptide comprising an exon 14b/exon 15 junction
<400> 33
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg
               20                  25
<210> 34
   <211> 28
   <212> PRT
   <213> Artificial Sequence
<220>
   <223> Ciz1 b-variant peptide comprising an exon 14b/exon 15 junction
<400> 34
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg
               20                  25
<210> 35
   <211> 27
   <212> PRT
   <213> Artificial Sequence
<220>
   <223> Ciz1 b-variant peptide comprising an exon 14b/exon 15 junction
<400> 35
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg
               20                  25
<210> 36
   <211> 26
   <212> PRT
   <213> Artificial Sequence
<220>
   <223> Ciz1 b-variant peptide comprising an exon 14b/exon 15 junction
<400> 36
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg
               20                  25
<210> 37
   <211> 25
   <212> PRT
   <213> Artificial Sequence
<220>
   <223> Ciz1 b-variant peptide comprising an exon 14b/exon 15 junction
<400> 37
   Asp Ala Val Gly Cys Phe Glu Gly Asp Glu Glu Glu Glu Glu Asp Asp
   1               5                   10                  15
   Glu Asp Glu Glu Glu Ile Glu Val Arg
               20                  25
<210> 38
   <211> 24
   <212> PRT
   <213> Artificial Sequence
<220>
   <223> Ciz1 b-variant peptide comprising an exon 14b/exon 15 junction
<400> 38
   Ala Val Gly Cys Phe Glu Gly Asp Glu Glu Glu Glu Glu Asp Asp Glu
   1               5                   10                  15
   Asp Glu Glu Glu Ile Glu Val Arg
               20
<210> 39
   <211> 23
   <212> PRT
   <213> Artificial Sequence
<220>
   <223> Ciz1 b-variant peptide comprising an exon 14b/exon 15 junction
<400> 39
   Val Gly Cys Phe Glu Gly Asp Glu Glu Glu Glu Glu Asp Asp Glu Asp
   1               5                   10                  15
   Glu Glu Glu Ile Glu Val Arg
               20
<210> 40
   <211> 22
   <212> PRT
   <213> Artificial Sequence
<220>
   <223> Ciz1 b-variant peptide comprising an exon 14b/exon 15 junction
<400> 40
   Gly Cys Phe Glu Gly Asp Glu Glu Glu Glu Glu Asp Asp Glu Asp Glu
   1               5                   10                  15
   Glu Glu Ile Glu Val Arg
               20
<210> 41
   <211> 21
   <212> PRT
   <213> Artificial Sequence
<220>
   <223> Ciz1 b-variant peptide comprising an exon 14b/exon 15 junction
<400> 41
   Cys Phe Glu Gly Asp Glu Glu Glu Glu Glu Asp Asp Glu Asp Glu Glu
   1               5                   10                  15
   Glu Ile Glu Val Arg
               20
<210> 42
   <211> 20
   <212> PRT
   <213> Artificial Sequence
<220>
   <223> Ciz1 b-variant peptide comprising an exon 14b/exon 15 junction
<400> 42
   Phe Glu Gly Asp Glu Glu Glu Glu Glu Asp Asp Glu Asp Glu Glu Glu
   1               5                   10                  15
   Ile Glu Val Arg
               20
<210> 43
   <211> 19
   <212> PRT
   <213> Artificial Sequence
<220>
   <223> Ciz1 b-variant peptide comprising an exon 14b/exon 15 junction
<400> 43
   Glu Gly Asp Glu Glu Glu Glu Glu Asp Asp Glu Asp Glu Glu Glu Ile
   1               5                   10                  15
   Glu Val Arg
<210> 44
   <211> 18
   <212> PRT
   <213> Artificial Sequence
<220>
   <223> Ciz1 b-variant peptide comprising an exon 14b/exon 15 junction
<400> 44
   Gly Asp Glu Glu Glu Glu Glu Asp Asp Glu Asp Glu Glu Glu Ile Glu
   1               5                   10                  15
   Val Arg
<210> 45
   <211> 17
   <212> PRT
   <213> Artificial Sequence
<220>
   <223> Ciz1 b-variant peptide comprising an exon 14b/exon 15 junction
<400> 45
   Asp Glu Glu Glu Glu Glu Asp Asp Glu Asp Glu Glu Glu Ile Glu Val
   1               5                   10                  15
   Arg
<210> 46
   <211> 16
   <212> PRT
   <213> Artificial Sequence
<220>
   <223> Ciz1 b-variant peptide comprising an exon 14b/exon 15 junction
<400> 46
   Glu Glu Glu Glu Glu Asp Asp Glu Asp Glu Glu Glu Ile Glu Val Arg
   1               5                   10                  15
<210> 47
   <211> 15
   <212> PRT
   <213> Artificial Sequence
<220>
   <223> Ciz1 b-variant peptide comprising an exon 14b/exon 15 junction
<400> 47
   Glu Glu Glu Glu Asp Asp Glu Asp Glu Glu Glu Ile Glu Val Arg
   1               5                   10                  15
<210> 48
   <211> 14
   <212> PRT
   <213> Artificial Sequence
<220>
   <223> Ciz1 b-variant peptide comprising an exon 14b/exon 15 junction
<400> 48
   Glu Glu Glu Asp Asp Glu Asp Glu Glu Glu Ile Glu Val Arg
   1               5                   10
<210> 49
   <211> 13
   <212> PRT
   <213> Artificial Sequence
<220>
   <223> Ciz1 b-variant peptide comprising an exon 14b/exon 15 junction
<400> 49
   Glu Glu Asp Asp Glu Asp Glu Glu Glu Ile Glu Val Arg
   1               5                   10
<210> 50
   <211> 12
   <212> PRT
   <213> Artificial Sequence
<220>
   <223> Ciz1 b-variant peptide comprising an exon 14b/exon 15 junction
<400> 50
   Glu Asp Asp Glu Asp Glu Glu Glu Ile Glu Val Arg
   1               5                   10
<210> 51
   <211> 11
   <212> PRT
   <213> Artificial Sequence
<220>
   <223> Ciz1 b-variant peptide comprising an exon 14b/exon 15 junction
<400> 51
   Asp Asp Glu Asp Glu Glu Glu Ile Glu Val Arg
   1 5 10
<210> 52
   <211> 10
   <212> PRT
   <213> Artificial Sequence
<220>
   <223> Ciz1 b-variant peptide comprising an exon 14b/exon 15 junction
<400> 52
   Asp Glu Asp Glu Glu Glu Ile Glu Val Arg
   1 5 10
<210> 53
   <211> 9
   <212> PRT
   <213> Artificial Sequence
<220>
   <223> Ciz1 b-variant peptide comprising an exon 14b/exon 15 junction
<400> 53
   Glu Asp Glu Glu Glu Ile Glu Val Arg
   1 5
<210> 54
   <211> 8
   <212> PRT
   <213> Artificial Sequence
<220>
   <223> Ciz1 b-variant peptide comprising an exon 14b/exon 15 junction
<400> 54
   Asp Glu Glu Glu Ile Glu Val Arg
   1 5
<210> 55
   <211> 7
   <212> PRT
   <213> Artificial Sequence
<220>
   <223> Ciz1 b-variant peptide comprising an exon 14b/exon 15 junction
<400> 55
   Glu Glu Glu Ile Glu Val Arg
   1 5
<210> 56
   <211> 6
   <212> PRT
   <213> Artificial Sequence
<220>
   <223> Ciz1 b-variant peptide comprising an exon 14b/exon 15 junction
<400> 56
   Glu Glu Ile Glu Val Arg
   1               5
<210> 57
   <211> 5
   <212> PRT
   <213> Artificial Sequence
<220>
   <223> Ciz1 b-variant peptide comprising an exon 14b/exon 15 junction
<400> 57
   Glu Ile Glu Val Arg
   1               5
<210> 58
   <211> 4
   <212> PRT
   <213> Artificial Sequence
<220>
   <223> Ciz1 b-variant peptide comprising an exon 14b/exon 15 junction
<400> 58
   Ile Glu Val Arg
   1
<210> 59
   <211> 39
   <212> PRT
   <213> Artificial Sequence
<220>
   <223> Ciz1 b-variant peptide comprising an exon 14b/exon 15 junction
<400> 59
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser
           35
<210> 60
   <211> 38
   <212> PRT
   <213> Artificial Sequence
<220>
   <223> Ciz1 b-variant peptide comprising an exon 14b/exon 15 junction
<400> 60
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser
           35
<210> 61
   <211> 37
   <212> PRT
   <213> Artificial Sequence
<220>
   <223> Ciz1 b-variant peptide comprising an exon 14b/exon 15 junction
<400> 61
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser
           35
<210> 62
   <211> 36
   <212> PRT
   <213> Artificial Sequence
<220>
   <223> Ciz1 b-variant peptide comprising an exon 14b/exon 15 junction
<400> 62
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser
           35
<210> 63
   <211> 35
   <212> PRT
   <213> Artificial Sequence
<220>
   <223> ciz1 b-variant peptide comprising an exon 14b/exon 15 junction
<400> 63
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser
           35
<210> 64
   <211> 34
   <212> PRT
   <213> Artificial Sequence
<220>
   <223> Ciz1 b-variant peptide comprising an exon 14b/exon 15 junction
<400> 64
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser
<210> 65
   <211> 33
   <212> PRT
   <213> Artificial Sequence
<220>
   <223> Ciz1 b-variant peptide comprising an exon 14b/exon 15 junction
<400> 65
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser
<210> 66
   <211> 32
   <212> PRT
   <213> Artificial Sequence
<220>
   <223> Ciz1 b-variant peptide comprising an exon 14b/exon 15 junction
<400> 66
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
<210> 67
   <211> 31
   <212> PRT
   <213> Artificial Sequence
<220>
   <223> Ciz1 b-variant peptide comprising an exon 14b/exon 15 junction
<400> 67
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser
               20                  25                  30
<210> 68
   <211> 30
   <212> PRT
   <213> Artificial Sequence
<220>
   <223> Ciz1 b-variant peptide comprising an exon 14b/exon 15 junction
<400> 68
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser
               20                  25                  30
<210> 69
   <211> 29
   <212> PRT
   <213> Artificial Sequence
<220>
   <223> Ciz1 b-variant peptide comprising an exon 14b/exon 15 junction
<400> 69
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser
               20                  25
<210> 70
   <211> 28
   <212> PRT
   <213> Artificial Sequence
<220>
   <223> Ciz1 b-variant peptide comprising an exon 14b/exon 15 junction
<400> 70
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser
               20                  25
<210> 71
   <211> 27
   <212> PRT
   <213> Artificial Sequence
<220>
   <223> Ciz1 b-variant peptide comprising an exon 14b/exon 15 junction
<400> 71
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser
               20                  25
<210> 72
   <211> 26
   <212> PRT
   <213> Artificial Sequence
<220>
   <223> Ciz1 b-variant peptide comprising an exon 14b/exon 15 junction
<400> 72
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser
               20                  25
<210> 73
   <211> 25
   <212> PRT
   <213> Artificial Sequence
<220>
   <223> Ciz1 b-variant peptide comprising an exon 14b/exon 15 junction
<400> 73
   Ala Val Gly Cys Phe Glu Gly Asp Glu Glu Glu Glu Glu Asp Asp Glu
   1               5                   10                  15
   Asp Glu Glu Glu Ile Glu Val Arg Ser
               20                  25
<210> 74
   <211> 24
   <212> PRT
   <213> Artificial Sequence
<220>
   <223> Ciz1 b-variant peptide comprising an exon 14b/exon 15 junction
<400> 74
   Val Gly Cys Phe Glu Gly Asp Glu Glu Glu Glu Glu Asp Asp Glu Asp
   1               5                   10                  15
   Glu Glu Glu Ile Glu Val Arg Ser
               20
<210> 75
   <211> 23
   <212> PRT
   <213> Artificial Sequence
<220>
   <223> Ciz1 b-variant peptide comprising an exon 14b/exon 15 junction
<400> 75
   Gly Cys Phe Glu Gly Asp Glu Glu Glu Glu Glu Asp Asp Glu Asp Glu
   1               5                   10                  15
   Glu Glu Ile Glu Val Arg Ser
               20
<210> 76
   <211> 22
   <212> PRT
   <213> Artificial Sequence
<220>
   <223> Ciz1 b-variant peptide comprising an exon 14b/exon 15 junction
<400> 76
   Cys Phe Glu Gly Asp Glu Glu Glu Glu Glu Asp Asp Glu Asp Glu Glu
   1               5                   10                  15
   Glu Ile Glu Val Arg Ser
               20
<210> 77
   <211> 21
   <212> PRT
   <213> Artificial Sequence
<220>
   <223> Ciz1 b-variant peptide comprising an exon 14b/exon 15 junction
<400> 77
   Phe Glu Gly Asp Glu Glu Glu Glu Glu Asp Asp Glu Asp Glu Glu Glu
   1               5                   10                  15
   Ile Glu Val Arg Ser
               20
<210> 78
   <211> 20
   <212> PRT
   <213> Artificial Sequence
<220>
   <223> Ciz1 b-variant peptide comprising an exon 14b/exon 15 junction
<400> 78
   Glu Gly Asp Glu Glu Glu Glu Glu Asp Asp Glu Asp Glu Glu Glu Ile
   1               5                   10                  15
   Glu Val Arg Ser
               20
<210> 79
   <211> 19
   <212> PRT
   <213> Artificial Sequence
<220>
   <223> Ciz1 b-variant peptide comprising an exon 14b/exon 15 junction
<400> 79
   Gly Asp Glu Glu Glu Glu Glu Asp Asp Glu Asp Glu Glu Glu Ile Glu
   1               5                   10                  15
   Val Arg Ser
<210> 80
   <211> 18
   <212> PRT
   <213> Artificial Sequence
<220>
   <223> Ciz1 b-variant peptide comprising an exon 14b/exon 15 junction
<400> 80
   Asp Glu Glu Glu Glu Glu Asp Asp Glu Asp Glu Glu Glu Ile Glu Val
   1               5                   10                  15
   Arg Ser
<210> 81
   <211> 17
   <212> PRT
   <213> Artificial Sequence
<220>
   <223> Ciz1 b-variant peptide comprising an exon 14b/exon 15 junction
<400> 81
   Glu Glu Glu Glu Glu Asp Asp Glu Asp Glu Glu Glu Ile Glu Val Arg
   1               5                   10                  15
   Ser
<210> 82
   <211> 16
   <212> PRT
   <213> Artificial Sequence
<220>
   <223> Ciz1 b-variant peptide comprising an exon 14b/exon 15 junction
<400> 82
   Glu Glu Glu Glu Asp Asp Glu Asp Glu Glu Glu Ile Glu Val Arg Ser
   1 5 10 15
<210> 83
   <211> 15
   <212> PRT
   <213> Artificial Sequence
<220>
   <223> Ciz1 b-variant peptide comprising an exon 14b/exon 15 junction
<400> 83
   Glu Glu Glu Asp Asp Glu Asp Glu Glu Glu Ile Glu Val Arg Ser
   1 5 10 15
<210> 84
   <211> 14
   <212> PRT
   <213> Artificial Sequence
<220>
   <223> Ciz1 b-variant peptide comprising an exon 14b/exon 15 junction
<400> 84
   Glu Glu Asp Asp Glu Asp Glu Glu Glu Ile Glu Val Arg Ser
   1 5 10
<210> 85
   <211> 13
   <212> PRT
   <213> Artificial Sequence
<220>
   <223> Ciz1 b-variant peptide comprising an exon 14b/exon 15 junction
<400> 85
   Glu Asp Asp Glu Asp Glu Glu Glu Ile Glu Val Arg Ser
   1 5 10
<210> 86
   <211> 12
   <212> PRT
   <213> Artificial Sequence
<220>
   <223> Ciz1 b-variant peptide comprising an exon 14b/exon 15 junction
<400> 86
   Asp Asp Glu Asp Glu Glu Glu Ile Glu Val Arg Ser
   1               5                   10
<210> 87
   <211> 11
   <212> PRT
   <213> Artificial Sequence
<220>
   <223> Ciz1 b-variant peptide comprising an exon 14b/exon 15 junction
<400> 87
   Asp Glu Asp Glu Glu Glu Ile Glu Val Arg Ser
   1               5                   10
<210> 88
   <211> 10
   <212> PRT
   <213> Artificial Sequence
<220>
   <223> Ciz1 b-variant peptide comprising an exon 14b/exon 15 junction
<400> 88
   Glu Asp Glu Glu Glu Ile Glu Val Arg Ser
   1               5                   10
<210> 89
   <211> 9
   <212> PRT
   <213> Artificial Sequence
<220>
   <223> Ciz1 b-variant peptide comprising an exon 14b/exon 15 junction
<400> 89
   Asp Glu Glu Glu Ile Glu Val Arg Ser
   1               5
<210> 90
   <211> 8
   <212> PRT
   <213> Artificial Sequence
<220>
   <223> Ciz1 b-variant peptide comprising an exon 14b/exon 15 junction
<400> 90
   Glu Glu Glu Ile Glu Val Arg Ser
   1               5
<210> 91
   <211> 7
   <212> PRT
   <213> Artificial Sequence
<220>
   <223> Ciz1 b-variant peptide comprising an exon 14b/exon 15 junction
<400> 91
   Glu Glu Ile Glu Val Arg Ser
   1 5
<210> 92
   <211> 6
   <212> PRT
   <213> Artificial Sequence
<220>
   <223> Ciz1 b-variant peptide comprising an exon 14b/exon 15 junction
<400> 92
   Glu Ile Glu Val Arg Ser
   1 5
<210> 93
   <211> 5
   <212> PRT
   <213> Artificial Sequence
<220>
   <223> Ciz1 b-variant peptide comprising an exon 14b/exon 15 junction
<400> 93
   Ile Glu Val Arg Ser
   1 5
<210> 94
   <211> 4
   <212> PRT
   <213> Artificial Sequence
<220>
   <223> Ciz1 b-variant peptide comprising an exon 14b/exon 15 junction
<400> 94
   Glu Val Arg Ser
   1
<210> 95
   <211> 40
   <212> PRT
   <213> Artificial Sequence
<220>
   <223> Ciz1 b-variant peptide comprising an exon 14b/exon 15 junction
<400> 95
   Glu Ile Ala Gly Gln Asp Glu Asp His Phe Ile Thr Val Asp Ala Val
   1 5 10 15
   Gly Cys Phe Glu Gly Asp Glu Glu Glu Glu Glu Asp Asp Glu Asp Glu
   20 25 30
   Glu Glu Ile Glu Val Arg Ser Arg
   35 40
<210> 96
   <211> 39
   <212> PRT
   <213> Artificial Sequence
<220>
   <223> Ciz1 b-variant peptide comprising an exon 14b/exon 15 junction
<400> 96
   Ile Ala Gly Gln Asp Glu Asp His Phe Ile Thr Val Asp Ala Val Gly
   1 5 10 15
   Cys Phe Glu Gly Asp Glu Glu Glu Glu Glu Asp Asp Glu Asp Glu Glu
   20 25 30
   Glu Ile Glu Val Arg Ser Arg
   35
<210> 97
   <211> 38
   <212> PRT
   <213> Artificial Sequence
<220>
   <223> Ciz1 b-variant peptide comprising an exon 14b/exon 15 junction
<400> 97
   Ala Gly Gln Asp Glu Asp His Phe Ile Thr Val Asp Ala Val Gly Cys
   1 5 10 15
   Phe Glu Gly Asp Glu Glu Glu Glu Glu Asp Asp Glu Asp Glu Glu Glu
   20 25 30
   Ile Glu Val Arg Ser Arg
   35
<210> 98
   <211> 37
   <212> PRT
   <213> Artificial Sequence
<220>
   <223> Ciz1 b-variant peptide comprising an exon 14b/exon 15 junction
<400> 98
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg
           35
<210> 99
   <211> 36
   <212> PRT
   <213> Artificial Sequence
<220>
   <223> Ciz1 b-variant peptide comprising an exon 14b/exon 15 junction
<400> 99
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg
           35
<210> 100
   <211> 35
   <212> PRT
   <213> Artificial Sequence
<220>
   <223> Ciz1 b-variant peptide comprising an exon 14b/exon 15 junction
<400> 100
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg
           35
<210> 101
   <211> 34
   <212> PRT
   <213> Artificial Sequence
<220>
   <223> Ciz1 b-variant peptide comprising an exon 14b/exon 15 junction
<400> 101
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg
<210> 102
   <211> 33
   <212> PRT
   <213> Artificial Sequence
<220>
   <223> Ciz1 b-variant peptide comprising an exon 14b/exon 15 junction
<400> 102
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg
<210> 103
   <211> 32
   <212> PRT
   <213> Artificial Sequence
<220>
   <223> Ciz1 b-variant peptide comprising an exon 14b/exon 15 junction
<400> 103
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
<210> 104
   <211> 31
   <212> PRT
   <213> Artificial Sequence
<220>
   <223> Ciz1 b-variant peptide comprising an exon 14b/exon 15 junction
<400> 104
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg
               20                  25                  30
<210> 105
   <211> 30
   <212> PRT
   <213> Artificial Sequence
<220>
   <223> Ciz1 b-variant peptide comprising an exon 14b/exon 15 junction
<400> 105
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg
               20                  25                  30
<210> 106
   <211> 29
   <212> PRT
   <213> Artificial Sequence
<220>
   <223> Ciz1 b-variant peptide comprising an exon 14b/exon 15 junction
<400> 106
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg
               20                  25
<210> 107
   <211> 28
   <212> PRT
   <213> Artificial Sequence
<220>
   <223> Ciz1 b-variant peptide comprising an exon 14b/exon 15 junction
<400> 107
   Val Asp Ala Val Gly Cys Phe Glu Gly Asp Glu Glu Glu Glu Glu Asp
   1 5 10 15
   Asp Glu Asp Glu Glu Glu Ile Glu Val Arg Ser Arg
   20 25
<210> 108
   <211> 27
   <212> PRT
   <213> Artificial Sequence
<220>
   <223> Ciz1 b-variant peptide comprising an exon 14b/exon 15 junction
<400> 108
   Asp Ala Val Gly Cys Phe Glu Gly Asp Glu Glu Glu Glu Glu Asp Asp
   1 5 10 15
   Glu Asp Glu Glu Glu Ile Glu Val Arg Ser Arg
   20 25
<210> 109
   <211> 26
   <212> PRT
   <213> Artificial Sequence
<220>
   <223> Ciz1 b-variant peptide comprising an exon 14b/exon 15 junction
<400> 109
   Ala Val Gly Cys Phe Glu Gly Asp Glu Glu Glu Glu Glu Asp Asp Glu
   1 5 10 15
   Asp Glu Glu Glu Ile Glu Val Arg Ser Arg
   20 25
<210> 110
   <211> 25
   <212> PRT
   <213> Artificial Sequence
<220>
   <223> Ciz1 b-variant peptide comprising an exon 14b/exon 15 junction
<400> 110
   Val Gly Cys Phe Glu Gly Asp Glu Glu Glu Glu Glu Asp Asp Glu Asp
   1 5 10 15
   Glu Glu Glu Ile Glu Val Arg Ser Arg
   20 25
<210> 111
   <211> 24
   <212> PRT
   <213> Artificial Sequence
<220>
   <223> Ciz1 b-variant peptide comprising an exon 14b/exon 15 junction
<400> 111
   Gly Cys Phe Glu Gly Asp Glu Glu Glu Glu Glu Asp Asp Glu Asp Glu
   1               5                   10                  15
   Glu Glu Ile Glu Val Arg Ser Arg
               20
<210> 112
   <211> 23
   <212> PRT
   <213> Artificial Sequence
<220>
   <223> Ciz1 b-variant peptide comprising an exon 14b/exon 15 junction
<400> 112
   Cys Phe Glu Gly Asp Glu Glu Glu Glu Glu Asp Asp Glu Asp Glu Glu
   1               5                   10                  15
   Glu Ile Glu Val Arg Ser Arg
               20
<210> 113
   <211> 22
   <212> PRT
   <213> Artificial Sequence
<220>
   <223> Ciz1 b-variant peptide comprising an exon 14b/exon 15 junction
<400> 113
   Phe Glu Gly Asp Glu Glu Glu Glu Glu Asp Asp Glu Asp Glu Glu Glu
   1               5                   10                  15
   Ile Glu Val Arg Ser Arg
               20
<210> 114
   <211> 21
   <212> PRT
   <213> Artificial Sequence
<220>
   <223> Ciz1 b-variant peptide comprising an exon 14b/exon 15 junction
<400> 114
   Glu Gly Asp Glu Glu Glu Glu Glu Asp Asp Glu Asp Glu Glu Glu Ile
   1               5                   10                  15
   Glu Val Arg Ser Arg
               20
<210> 115
   <211> 20
   <212> PRT
   <213> Artificial Sequence
<220>
   <223> Ciz1 b-variant peptide comprising an exon 14b/exon 15 junction
<400> 115
   Gly Asp Glu Glu Glu Glu Glu Asp Asp Glu Asp Glu Glu Glu Ile Glu
   1               5                   10                  15
   Val Arg Ser Arg
               20
<210> 116
   <211> 19
   <212> PRT
   <213> Artificial Sequence
<220>
   <223> Ciz1 b-variant peptide comprising an exon 14b/exon 15 junction
<400> 116
   Asp Glu Glu Glu Glu Glu Asp Asp Glu Asp Glu Glu Glu Ile Glu Val
   1               5                   10                  15
   Arg Ser Arg
<210> 117
   <211> 18
   <212> PRT
   <213> Artificial Sequence
<220>
   <223> Ciz1 b-variant peptide comprising an exon 14b/exon 15 junction
<400> 117
   Glu Glu Glu Glu Glu Asp Asp Glu Asp Glu Glu Glu Ile Glu Val Arg
   1               5                   10                  15
   Ser Arg
<210> 118
   <211> 17
   <212> PRT
   <213> Artificial Sequence
<220>
   <223> Ciz1 b-variant peptide comprising an exon 14b/exon 15 junction
<400> 118
   Glu Glu Glu Glu Asp Asp Glu Asp Glu Glu Glu Ile Glu Val Arg Ser
   1 5 10 15
   Arg
<210> 119
   <211> 16
   <212> PRT
   <213> Artificial Sequence
<220>
   <223> Ciz1 b-variant peptide comprising an exon 14b/exon 15 junction
<400> 119
   Glu Glu Glu Asp Asp Glu Asp Glu Glu Glu Ile Glu Val Arg Ser Arg
   1 5 10 15
<210> 120
   <211> 15
   <212> PRT
   <213> Artificial Sequence
<220>
   <223> Ciz1 b-variant peptide comprising an exon 14b/exon 15 junction
<400> 120
   Glu Glu Asp Asp Glu Asp Glu Glu Glu Ile Glu Val Arg Ser Arg
   1 5 10 15
<210> 121
   <211> 14
   <212> PRT
   <213> Artificial Sequence
<220>
   <223> Ciz1 b-variant peptide comprising an exon 14b/exon 15 junction
<400> 121
   Glu Asp Asp Glu Asp Glu Glu Glu Ile Glu Val Arg Ser Arg
   1 5 10
<210> 122
   <211> 13
   <212> PRT
   <213> Artificial Sequence
<220>
   <223> Ciz1 b-variant peptide comprising an exon 14b/exon 15 junction
<400> 122
   Asp Asp Glu Asp Glu Glu Glu Ile Glu Val Arg Ser Arg
   1 5 10
<210> 123
   <211> 12
   <212> PRT
   <213> Artificial Sequence
<220>
   <223> Ciz1 b-variant peptide comprising an exon 14b/exon 15 junction
<400> 123
   Asp Glu Asp Glu Glu Glu Ile Glu Val Arg Ser Arg
   1 5 10
<210> 124
   <211> 11
   <212> PRT
   <213> Artificial Sequence
<220>
   <223> Ciz1 b-variant peptide comprising an exon 14b/exon 15 junction
<400> 124
   Glu Asp Glu Glu Glu Ile Glu Val Arg Ser Arg
   1 5 10
<210> 125
   <211> 9
   <212> PRT
   <213> Artificial Sequence
<220>
   <223> Ciz1 b-variant peptide comprising an exon 14b/exon 15 junction
<400> 125
   Glu Glu Glu Ile Glu Val Arg Ser Arg
   1 5
<210> 126
   <211> 8
   <212> PRT
   <213> Artificial Sequence
<220>
   <223> Ciz1 b-variant peptide comprising an exon 14b/exon 15 junction
<400> 126
   Glu Glu Ile Glu Val Arg Ser Arg
   1 5
<210> 127
   <211> 7
   <212> PRT
   <213> Artificial Sequence
<220>
   <223> Ciz1 b-variant peptide comprising an exon 14b/exon 15 junction
<400> 127
   Glu Ile Glu Val Arg Ser Arg
   1 5
<210> 128
   <211> 6
   <212> PRT
   <213> Artificial Sequence
<220>
   <223> Ciz1 b-variant peptide comprising an exon 14b/exon 15 junction
<400> 128
   Ile Glu Val Arg Ser Arg
   1 5
<210> 129
   <211> 5
   <212> PRT
   <213> Artificial Sequence
<220>
   <223> Ciz1 b-variant peptide comprising an exon 14b/exon 15 junction
<400> 129
   Glu Val Arg Ser Arg
   1 5
<210> 130
   <211> 41
   <212> PRT
   <213> Artificial Sequence
<220>
   <223> Ciz1 b-variant peptide comprising an exon 14b/exon 15 junction
<400> 130
   Glu Ile Ala Gly Gln Asp Glu Asp His Phe Ile Thr Val Asp Ala Val
   1 5 10 15
   Gly Cys Phe Glu Gly Asp Glu Glu Glu Glu Glu Asp Asp Glu Asp Glu
   20 25 30
   Glu Glu Ile Glu Val Arg Ser Arg Asp
   35 40
<210> 131
   <211> 40
   <212> PRT
   <213> Artificial Sequence
<220>
   <223> Ciz1 b-variant peptide comprising an exon 14b/exon 15 junction
<400> 131
   Ile Ala Gly Gln Asp Glu Asp His Phe Ile Thr Val Asp Ala Val Gly
   1 5 10 15
   Cys Phe Glu Gly Asp Glu Glu Glu Glu Glu Asp Asp Glu Asp Glu Glu
   20 25 30
   Glu Ile Glu Val Arg Ser Arg Asp
   35 40
<210> 132
   <211> 39
   <212> PRT
   <213> Artificial Sequence
<220>
   <223> Ciz1 b-variant peptide comprising an exon 14b/exon 15 junction
<400> 132
   Ala Gly Gln Asp Glu Asp His Phe Ile Thr Val Asp Ala Val Gly Cys
   1 5 10 15
   Phe Glu Gly Asp Glu Glu Glu Glu Glu Asp Asp Glu Asp Glu Glu Glu
   20 25 30
   Ile Glu Val Arg Ser Arg Asp
   35
<210> 133
   <211> 38
   <212> PRT
   <213> Artificial Sequence
<220>
   <223> Ciz1 b-variant peptide comprising an exon 14b/exon 15 junction
<400> 133
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp
           35
<210> 134
   <211> 37
   <212> PRT
   <213> Artificial Sequence
<220>
   <223> Ciz1 b-variant peptide comprising an exon 14b/exon 15 junction
<400> 134
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp
           35
<210> 135
   <211> 36
   <212> PRT
   <213> Artificial Sequence
<220>
   <223> Ciz1 b-variant peptide comprising an exon 14b/exon 15 junction
<400> 135
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp
           35
<210> 136
   <211> 35
   <212> PRT
   <213> Artificial Sequence
<220>
   <223> Ciz1 b-variant peptide comprising an exon 14b/exon 15 junction
<400> 136
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp
           35
<210> 137
   <211> 34
   <212> PRT
   <213> Artificial Sequence
<220>
   <223> Ciz1 b-variant peptide comprising an exon 14b/exon 15 junction
<400> 137
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp
<210> 138
   <211> 33
   <212> PRT
   <213> Artificial Sequence
<220>
   <223> Ciz1 b-variant peptide comprising an exon 14b/exon 15 junction
<400> 138
   His Phe Ile Thr Val Asp Ala Val Gly Cys Phe Glu Gly Asp Glu Glu
   1 5 10 15
   Glu Glu Glu Asp Asp Glu Asp Glu Glu Glu Ile Glu Val Arg Ser Arg
   20 25 30
   Asp
<210> 139
   <211> 32
   <212> PRT
   <213> Artificial Sequence
<220>
   <223> Ciz1 b-variant peptide comprising an exon 14b/exon 15 junction
<400> 139
   Phe Ile Thr Val Asp Ala Val Gly Cys Phe Glu Gly Asp Glu Glu Glu
   1 5 10 15
   Glu Glu Asp Asp Glu Asp Glu Glu Glu Ile Glu Val Arg Ser Arg Asp
   20 25 30
<210> 140
   <211> 31
   <212> PRT
   <213> Artificial Sequence
<220>
   <223> Ciz1 b-variant peptide comprising an exon 14b/exon 15 junction
<400> 140
   Ile Thr Val Asp Ala Val Gly Cys Phe Glu Gly Asp Glu Glu Glu Glu
   1 5 10 15
   Glu Asp Asp Glu Asp Glu Glu Glu Ile Glu Val Arg Ser Arg Asp
   20 25 30
<210> 141
   <211> 30
   <212> PRT
   <213> Artificial Sequence
<220>
   <223> Ciz1 b-variant peptide comprising an exon 14b/exon 15 junction
<400> 141
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp
               20                  25                  30
<210> 142
   <211> 29
   <212> PRT
   <213> Artificial Sequence
<220>
   <223> Ciz1 b-variant peptide comprising an exon 14b/exon 15 junction
<400> 142
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp
               20                  25
<210> 143
   <211> 28
   <212> PRT
   <213> Artificial Sequence
<220>
   <223> Ciz1 b-variant peptide comprising an exon 14b/exon 15 junction
<400> 143
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp
               20                  25
<210> 144
   <211> 27
   <212> PRT
   <213> Artificial Sequence
<220>
   <223> Ciz1 b-variant peptide comprising an exon 14b/exon 15 junction
<400> 144
   Ala Val Gly Cys Phe Glu Gly Asp Glu Glu Glu Glu Glu Asp Asp Glu
   1 5 10 15
   Asp Glu Glu Glu Ile Glu Val Arg Ser Arg Asp
   20 25
<210> 145
   <211> 26
   <212> PRT
   <213> Artificial Sequence
<220>
   <223> Ciz1 b-variant peptide comprising an exon 14b/exon 15 junction
<400> 145
   Val Gly Cys Phe Glu Gly Asp Glu Glu Glu Glu Glu Asp Asp Glu Asp
   1 5 10 15
   Glu Glu Glu Ile Glu Val Arg Ser Arg Asp
   20 25
<210> 146
   <211> 25
   <212> PRT
   <213> Artificial Sequence
<220>
   <223> Ciz1 b-variant peptide comprising an exon 14b/exon 15 junction
<400> 146
   Gly Cys Phe Glu Gly Asp Glu Glu Glu Glu Glu Asp Asp Glu Asp Glu
   1 5 10 15
   Glu Glu Ile Glu Val Arg Ser Arg Asp
   20 25
<210> 147
   <211> 24
   <212> PRT
   <213> Artificial Sequence
<220>
   <223> Ciz1 b-variant peptide comprising an exon 14b/exon 15 junction
<400> 147
   Cys Phe Glu Gly Asp Glu Glu Glu Glu Glu Asp Asp Glu Asp Glu Glu
   1 5 10 15
   Glu Ile Glu Val Arg Ser Arg Asp
   20
<210> 148
   <211> 23
   <212> PRT
   <213> Artificial Sequence
<220>
   <223> Ciz1 b-variant peptide comprising an exon 14b/exon 15 junction
<400> 148
   Phe Glu Gly Asp Glu Glu Glu Glu Glu Asp Asp Glu Asp Glu Glu Glu
   1               5                   10                  15
   Ile Glu Val Arg Ser Arg Asp
               20
<210> 149
   <211> 22
   <212> PRT
   <213> Artificial Sequence
<220>
   <223> Ciz1 b-variant peptide comprising an exon 14b/exon 15 junction
<400> 149
   Glu Gly Asp Glu Glu Glu Glu Glu Asp Asp Glu Asp Glu Glu Glu Ile
   1               5                   10                  15
   Glu Val Arg Ser Arg Asp
               20
<210> 150
   <211> 21
   <212> PRT
   <213> Artificial Sequence
<220>
   <223> Ciz1 b-variant peptide comprising an exon 14b/exon 15 junction
<400> 150
   Gly Asp Glu Glu Glu Glu Glu Asp Asp Glu Asp Glu Glu Glu Ile Glu
   1               5                   10                  15
   Val Arg Ser Arg Asp
               20
<210> 151
   <211> 20
   <212> PRT
   <213> Artificial Sequence
<220>
   <223> Ciz1 b-variant peptide comprising an exon 14b/exon 15 junction
<400> 151
   Asp Glu Glu Glu Glu Glu Asp Asp Glu Asp Glu Glu Glu Ile Glu Val
   1 5 10 15
   Arg Ser Arg Asp
   20
<210> 152
   <211> 19
   <212> PRT
   <213> Artificial Sequence
<220>
   <223> Ciz1 b-variant peptide comprising an exon 14b/exon 15 junction
<400> 152
   Glu Glu Glu Glu Glu Asp Asp Glu Asp Glu Glu Glu Ile Glu Val Arg
   1 5 10 15
   Ser Arg Asp
<210> 153
   <211> 18
   <212> PRT
   <213> Artificial Sequence
<220>
   <223> Ciz1 b-variant peptide comprising an exon 14b/exon 15 junction
<400> 153
   Glu Glu Glu Glu Asp Asp Glu Asp Glu Glu Glu Ile Glu Val Arg Ser
   1 5 10 15
   Arg Asp
<210> 154
   <211> 17
   <212> PRT
   <213> Artificial Sequence
<220>
   <223> Ciz1 b-variant peptide comprising an exon 14b/exon 15 junction
<400> 154
   Glu Glu Glu Asp Asp Glu Asp Glu Glu Glu Ile Glu Val Arg Ser Arg
   1 5 10 15
   Asp
<210> 155
   <211> 16
   <212> PRT
   <213> Artificial Sequence
<220>
   <223> Ciz1 b-variant peptide comprising an exon 14b/exon 15 junction
<400> 155
   Glu Glu Asp Asp Glu Asp Glu Glu Glu Ile Glu Val Arg Ser Arg Asp
   1               5                   10                  15
<210> 156
   <211> 15
   <212> PRT
   <213> Artificial Sequence
<220>
   <223> Ciz1 b-variant peptide comprising an exon 14b/exon 15 junction
<400> 156
   Glu Asp Asp Glu Asp Glu Glu Glu Ile Glu Val Arg Ser Arg Asp
   1               5                   10                  15
<210> 157
   <211> 14
   <212> PRT
   <213> Artificial Sequence
<220>
   <223> Ciz1 b-variant peptide comprising an exon 14b/exon 15 junction
<400> 157
   Asp Asp Glu Asp Glu Glu Glu Ile Glu Val Arg Ser Arg Asp
   1               5                   10
<210> 158
   <211> 13
   <212> PRT
   <213> Artificial Sequence
<220>
   <223> Ciz1 b-variant peptide comprising an exon 14b/exon 15 junction
<400> 158
   Asp Glu Asp Glu Glu Glu Ile Glu Val Arg Ser Arg Asp
   1               5                   10
<210> 159
   <211> 12
   <212> PRT
   <213> Artificial Sequence
<220>
   <223> Ciz1 b-variant peptide comprising an exon 14b/exon 15 junction
<400> 159
   Glu Asp Glu Glu Glu Ile Glu Val Arg Ser Arg Asp
   1 5 10
<210> 160
   <211> 11
   <212> PRT
   <213> Artificial Sequence
<220>
   <223> Ciz1 b-variant peptide comprising an exon 14b/exon 15 junction
<400> 160
   Asp Glu Glu Glu Ile Glu Val Arg Ser Arg Asp
   1 5 10
<210> 161
   <211> 10
   <212> PRT
   <213> Artificial Sequence
<220>
   <223> Ciz1 b-variant peptide comprising an exon 14b/exon 15 junction
<400> 161
   Glu Glu Glu Ile Glu Val Arg Ser Arg Asp
   1 5 10
<210> 162
   <211> 9
   <212> PRT
   <213> Artificial Sequence
<220>
   <223> Ciz1 b-variant peptide comprising an exon 14b/exon 15 junction
<400> 162
   Glu Glu Ile Glu Val Arg Ser Arg Asp
   1 5
<210> 163
   <211> 8
   <212> PRT
   <213> Artificial Sequence
<220>
   <223> Ciz1 b-variant peptide comprising an exon 14b/exon 15 junction
<400> 163
   Glu Ile Glu Val Arg Ser Arg Asp
   1 5
<210> 164
   <211> 7
   <212> PRT
   <213> Artificial Sequence
<220>
   <223> Ciz1 b-variant peptide comprising an exon 14b/exon 15 junction
<400> 164
   Ile Glu Val Arg Ser Arg Asp
   1               5
<210> 165
   <211> 6
   <212> PRT
   <213> Artificial Sequence
<220>
   <223> Ciz1 b-variant peptide comprising an exon 14b/exon 15 junction
<400> 165
   Glu Val Arg Ser Arg Asp
   1               5
<210> 166
   <211> 42
   <212> PRT
   <213> Artificial Sequence
<220>
   <223> Ciz1 b-variant peptide comprising an exon 14b/exon 15 junction
<400> 166
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile
           35                  40
<210> 167
   <211> 41
   <212> PRT
   <213> Artificial Sequence
<220>
   <223> Ciz1 b-variant peptide comprising an exon 14b/exon 15 junction
<400> 167
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
                   20                  25                  30
   Glu Ile Glu Val Arg Ser Arg Asp Ile
           35                  40
<210> 168
   <211> 40
   <212> PRT
   <213> Artificial Sequence
<220>
   <223> Ciz1 b-variant peptide comprising an exon 14b/exon 15 junction
<400> 168
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile
           35                  40
<210> 169
   <211> 39
   <212> PRT
   <213> Artificial Sequence
<220>
   <223> Ciz1 b-variant peptide comprising an exon 14b/exon 15 junction
<400> 169
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile
           35
<210> 170
   <211> 38
   <212> PRT
   <213> Artificial Sequence
<220>
   <223> Ciz1 b-variant peptide comprising an exon 14b/exon 15 junction
<400> 170
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile
           35
<210> 171
   <211> 37
   <212> PRT
   <213> Artificial Sequence
<220>
   <223> Ciz1 b-variant peptide comprising an exon 14b/exon 15 junction
<400> 171
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile
           35
<210> 172
   <211> 36
   <212> PRT
   <213> Artificial Sequence
<220>
   <223> Ciz1 b-variant peptide comprising an exon 14b/exon 15 junction
<400> 172
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile
           35
<210> 173
   <211> 35
   <212> PRT
   <213> Artificial Sequence
<220>
   <223> Ciz1 b-variant peptide comprising an exon 14b/exon 15 junction
<400> 173
   Asp His Phe Ile Thr Val Asp Ala Val Gly Cys Phe Glu Gly Asp Glu
   1 5 10 15
   Glu Glu Glu Glu Asp Asp Glu Asp Glu Glu Glu Ile Glu Val Arg Ser
   20 25 30
   Arg Asp Ile
   35
<210> 174
   <211> 34
   <212> PRT
   <213> Artificial Sequence
<220>
   <223> Ciz1 b-variant peptide comprising an exon 14b/exon 15 junction
<400> 174
   His Phe Ile Thr Val Asp Ala Val Gly Cys Phe Glu Gly Asp Glu Glu
   1 5 10 15
   Glu Glu Glu Asp Asp Glu Asp Glu Glu Glu Ile Glu Val Arg Ser Arg
   20 25 30
   Asp Ile
<210> 175
   <211> 33
   <212> PRT
   <213> Artificial Sequence
<220>
   <223> Ciz1 b-variant peptide comprising an exon 14b/exon 15 junction
<400> 175
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile
<210> 176
   <211> 32
   <212> PRT
   <213> Artificial Sequence
<220>
   <223> Ciz1 b-variant peptide comprising an exon 14b/exon 15 junction
<400> 176
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
<210> 177
   <211> 31
   <212> PRT
   <213> Artificial Sequence
<220>
   <223> Ciz1 b-variant peptide comprising an exon 14b/exon 15 junction
<400> 177
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile
               20                  25                  30
<210> 178
   <211> 30
   <212> PRT
   <213> Artificial Sequence
<220>
   <223> Ciz1 b-variant peptide comprising an exon 14b/exon 15 junction
<400> 178
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile
               20                  25                  30
<210> 179
   <211> 29
   <212> PRT
   <213> Artificial Sequence
<220>
   <223> Ciz1 b-variant peptide comprising an exon 14b/exon 15 junction
<400> 179
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile
                       20                  25
<210> 180
   <211> 28
   <212> PRT
   <213> Artificial Sequence
<220>
   <223> Ciz1 b-variant peptide comprising an exon 14b/exon 15 junction
<400> 180
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile
               20                  25
<210> 181
   <211> 27
   <212> PRT
   <213> Artificial Sequence
<220>
   <223> Ciz1 b-variant peptide comprising an exon 14b/exon 15 junction
<400> 181
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile
               20                  25
<210> 182
   <211> 26
   <212> PRT
   <213> Artificial Sequence
<220>
   <223> Ciz1 b-variant peptide comprising an exon 14b/exon 15 junction
<400> 182
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile
               20                  25
<210> 183
   <211> 25
   <212> PRT
   <213> Artificial Sequence
<220>
   <223> Ciz1 b-variant peptide comprising an exon 14b/exon 15 junction
<400> 183
   Cys Phe Glu Gly Asp Glu Glu Glu Glu Glu Asp Asp Glu Asp Glu Glu
   1               5                   10                  15
   Glu Ile Glu Val Arg Ser Arg Asp Ile
               20                  25
<210> 184
   <211> 24
   <212> PRT
   <213> Artificial Sequence
<220>
   <223> Ciz1 b-variant peptide comprising an exon 14b/exon 15 junction
<400> 184
   Phe Glu Gly Asp Glu Glu Glu Glu Glu Asp Asp Glu Asp Glu Glu Glu
   1               5                   10                  15
   Ile Glu Val Arg Ser Arg Asp Ile
               20
<210> 185
   <211> 23
   <212> PRT
   <213> Artificial Sequence
<220>
   <223> Ciz1 b-variant peptide comprising an exon 14b/exon 15 junction
<400> 185
   Glu Gly Asp Glu Glu Glu Glu Glu Asp Asp Glu Asp Glu Glu Glu Ile
   1               5                   10                  15
   Glu Val Arg Ser Arg Asp Ile
               20
<210> 186
   <211> 22
   <212> PRT
   <213> Artificial Sequence
<220>
   <223> Ciz1 b-variant peptide comprising an exon 14b/exon 15 junction
<400> 186
   Gly Asp Glu Glu Glu Glu Glu Asp Asp Glu Asp Glu Glu Glu Ile Glu
   1               5                   10                  15
   Val Arg Ser Arg Asp Ile
                                       20
<210> 187
   <211> 21
   <212> PRT
   <213> Artificial Sequence
<220>
   <223> Ciz1 b-variant peptide comprising an exon 14b/exon 15 junction
<400> 187
   Asp Glu Glu Glu Glu Glu Asp Asp Glu Asp Glu Glu Glu Ile Glu Val
   1               5                   10                  15
   Arg Ser Arg Asp Ile
               20
<210> 188
   <211> 20
   <212> PRT
   <213> Artificial Sequence
<220>
   <223> Ciz1 b-variant peptide comprising an exon 14b/exon 15 junction
<400> 188
   Glu Glu Glu Glu Glu Asp Asp Glu Asp Glu Glu Glu Ile Glu Val Arg
   1               5                   10                  15
   Ser Arg Asp Ile
               20
<210> 189
   <211> 19
   <212> PRT
   <213> Artificial Sequence
<220>
   <223> Ciz1 b-variant peptide comprising an exon 14b/exon 15 junction
<400> 189
   Glu Glu Glu Glu Asp Asp Glu Asp Glu Glu Glu Ile Glu Val Arg Ser
   1               5                   10                  15
   Arg Asp Ile
<210> 190
   <211> 18
   <212> PRT
   <213> Artificial Sequence
<220>
   <223> Ciz1 b-variant peptide comprising an exon 14b/exon 15 junction
<400> 190
   Glu Glu Glu Asp Asp Glu Asp Glu Glu Glu Ile Glu Val Arg Ser Arg
   1               5                   10                  15
   Asp Ile
<210> 191
   <211> 17
   <212> PRT
   <213> Artificial Sequence
<220>
   <223> Ciz1 b-variant peptide comprising an exon 14b/exon 15 junction
<400> 191
   Glu Glu Asp Asp Glu Asp Glu Glu Glu Ile Glu Val Arg Ser Arg Asp
   1 5 10 15
   Ile
<210> 192
   <211> 16
   <212> PRT
   <213> Artificial Sequence
<220>
   <223> Ciz1 b-variant peptide comprising an exon 14b/exon 15 junction
<400> 192
   Glu Asp Asp Glu Asp Glu Glu Glu Ile Glu Val Arg Ser Arg Asp Ile
   1 5 10 15
<210> 193
   <211> 15
   <212> PRT
   <213> Artificial Sequence
<220>
   <223> Ciz1 b-variant peptide comprising an exon 14b/exon 15 junction
<400> 193
   Asp Asp Glu Asp Glu Glu Glu Ile Glu Val Arg Ser Arg Asp Ile
   1 5 10 15
<210> 194
   <211> 14
   <212> PRT
   <213> Artificial Sequence
<220>
   <223> Ciz1 b-variant peptide comprising an exon 14b/exon 15 junction
<400> 194
   Asp Glu Asp Glu Glu Glu Ile Glu Val Arg Ser Arg Asp Ile
   1 5 10
<210> 195
   <211> 13
   <212> PRT
   <213> Artificial Sequence
<220>
   <223> Ciz1 b-variant peptide comprising an exon 14b/exon 15 junction
<400> 195
   Glu Asp Glu Glu Glu Ile Glu Val Arg Ser Arg Asp Ile
   1 5 10
<210> 196
   <211> 12
   <212> PRT
   <213> Artificial Sequence
<220>
   <223> Ciz1 b-variant peptide comprising an exon 14b/exon 15 junction
<400> 196
   Asp Glu Glu Glu Ile Glu Val Arg Ser Arg Asp Ile
   1               5                   10
<210> 197
   <211> 11
   <212> PRT
   <213> Artificial Sequence
<220>
   <223> Ciz1 b-variant peptide comprising an exon 14b/exon 15 junction
<400> 197
   Glu Glu Glu Ile Glu Val Arg Ser Arg Asp Ile
   1               5                   10
<210> 198
   <211> 10
   <212> PRT
   <213> Artificial Sequence
<220>
   <223> Ciz1 b-variant peptide comprising an exon 14b/exon 15 junction
<400> 198
   Glu Glu Ile Glu Val Arg Ser Arg Asp Ile
   1               5                   10
<210> 199
   <211> 9
   <212> PRT
   <213> Artificial Sequence
<220>
   <223> Ciz1 b-variant peptide comprising an exon 14b/exon 15 junction
<400> 199
   Glu Ile Glu Val Arg Ser Arg Asp Ile
   1               5
<210> 200
   <211> 8
   <212> PRT
   <213> Artificial Sequence
<220>
   <223> Ciz1 b-variant peptide comprising an exon 14b/exon 15 junction
<400> 200
   Ile Glu Val Arg Ser Arg Asp Ile
   1 5
<210> 201
   <211> 7
   <212> PRT
   <213> Artificial Sequence
<220>
   <223> Ciz1 b-variant peptide comprising an exon 14b/exon 15 junction
<400> 201
   Glu Val Arg Ser Arg Asp Ile
   1 5
<210> 202
   <211> 43
   <212> PRT
   <213> Artificial Sequence
<220>
   <223> Ciz1 b-variant peptide comprising an exon 14b/exon 15 junction
<400> 202
   Glu Ile Ala Gly Gln Asp Glu Asp His Phe Ile Thr Val Asp Ala Val
   1 5 10 15
   Gly Cys Phe Glu Gly Asp Glu Glu Glu Glu Glu Asp Asp Glu Asp Glu
   20 25 30
   Glu Glu Ile Glu Val Arg Ser Arg Asp Ile Ser
   35 40
<210> 203
   <211> 42
   <212> PRT
   <213> Artificial Sequence
<220>
   <223> Ciz1 b-variant peptide comprising an exon 14b/exon 15 junction
<400> 203
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser
           35                  40
<210> 204
   <211> 41
   <212> PRT
   <213> Artificial Sequence
<220>
   <223> Ciz1 b-variant peptide comprising an exon 14b/exon 15 junction
<400> 204
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser
           35                  40
<210> 205
   <211> 40
   <212> PRT
   <213> Artificial Sequence
<220>
   <223> Ciz1 b-variant peptide comprising an exon 14b/exon 15 junction
<400> 205
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser
           35                  40
<210> 206
   <211> 39
   <212> PRT
   <213> Artificial Sequence
<220>
   <223> Ciz1 b-variant peptide comprising an exon 14b/exon 15 junction
<400> 206
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser
           35
<210> 207
   <211> 38
   <212> PRT
   <213> Artificial Sequence
<220>
   <223> Ciz1 b-variant peptide comprising an exon 14b/exon 15 junction
<400> 207
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser
           35
<210> 208
   <211> 37
   <212> PRT
   <213> Artificial Sequence
<220>
   <223> Ciz1 b-variant peptide comprising an exon 14b/exon 15 junction
<400> 208
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser
           35
<210> 209
   <211> 36
   <212> PRT
   <213> Artificial Sequence
<220>
   <223> Ciz1 b-variant peptide comprising an exon 14b/exon 15 junction
<400> 209
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser
           35
<210> 210
   <211> 35
   <212> PRT
   <213> Artificial Sequence
<220>
   <223> Ciz1 b-variant peptide comprising an exon 14b/exon 15 junction
<400> 210
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser
           35
<210> 211
   <211> 34
   <212> PRT
   <213> Artificial Sequence
<220>
   <223> Ciz1 b-variant peptide comprising an exon 14b/exon 15 junction
<400> 211
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser
<210> 212
   <211> 33
   <212> PRT
   <213> Artificial Sequence
<220>
   <223> Ciz1 b-variant peptide comprising an exon 14b/exon 15 junction
<400> 212
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser
<210> 213
   <211> 32
   <212> PRT
   <213> Artificial Sequence
<220>
   <223> Ciz1 b-variant peptide comprising an exon 14b/exon 15 junction
<400> 213
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
<210> 214
   <211> 31
   <212> PRT
   <213> Artificial Sequence
<220>
   <223> Ciz1 b-variant peptide comprising an exon 14b/exon 15 junction
<400> 214
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser
               20                  25                  30
<210> 215
   <211> 30
   <212> PRT
   <213> Artificial Sequence
<220>
   <223> Ciz1 b-variant peptide comprising an exon 14b/exon 15 junction
<400> 215
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser
               20                  25                  30
<210> 216
   <211> 29
   <212> PRT
   <213> Artificial Sequence
<220>
   <223> Ciz1 b-variant peptide comprising an exon 14b/exon 15 junction
<400> 216
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser
               20                  25
<210> 217
   <211> 28
   <212> PRT
   <213> Artificial Sequence
<220>
   <223> Ciz1 b-variant peptide comprising an exon 14b/exon 15 junction
<400> 217
   Val Gly Cys Phe Glu Gly Asp Glu Glu Glu Glu Glu Asp Asp Glu Asp
   1 5 10 15
   Glu Glu Glu Ile Glu Val Arg Ser Arg Asp Ile Ser
   20 25
<210> 218
   <211> 27
   <212> PRT
   <213> Artificial Sequence
<220>
   <223> Ciz1 b-variant peptide comprising an exon 14b/exon 15 junction
<400> 218
   Gly Cys Phe Glu Gly Asp Glu Glu Glu Glu Glu Asp Asp Glu Asp Glu
   1 5 10 15
   Glu Glu Ile Glu Val Arg Ser Arg Asp Ile Ser
   20 25
<210> 219
   <211> 26
   <212> PRT
   <213> Artificial Sequence
<220>
   <223> Ciz1 b-variant peptide comprising an exon 14b/exon 15 junction
<400> 219
   Cys Phe Glu Gly Asp Glu Glu Glu Glu Glu Asp Asp Glu Asp Glu Glu
   1 5 10 15
   Glu Ile Glu Val Arg Ser Arg Asp Ile Ser
   20 25
<210> 220
   <211> 25
   <212> PRT
   <213> Artificial Sequence
<220>
   <223> Ciz1 b-variant peptide comprising an exon 14b/exon 15 junction
<400> 220
   Phe Glu Gly Asp Glu Glu Glu Glu Glu Asp Asp Glu Asp Glu Glu Glu
   1 5 10 15
   Ile Glu Val Arg Ser Arg Asp Ile Ser
   20 25
<210> 221
   <211> 24
   <212> PRT
   <213> Artificial Sequence
<220>
   <223> Ciz1 b-variant peptide comprising an exon 14b/exon 15 junction
<400> 221
   Glu Gly Asp Glu Glu Glu Glu Glu Asp Asp Glu Asp Glu Glu Glu Ile
   1               5                   10                  15
   Glu Val Arg Ser Arg Asp Ile Ser
               20
<210> 222
   <211> 23
   <212> PRT
   <213> Artificial Sequence
<220>
   <223> Ciz1 b-variant peptide comprising an exon 14b/exon 15 junction
<400> 222
   Gly Asp Glu Glu Glu Glu Glu Asp Asp Glu Asp Glu Glu Glu Ile Glu
   1               5                   10                  15
   Val Arg Ser Arg Asp Ile Ser
               20
<210> 223
   <211> 22
   <212> PRT
   <213> Artificial Sequence
<220>
   <223> Ciz1 b-variant peptide comprising an exon 14b/exon 15 junction
<400> 223
   Asp Glu Glu Glu Glu Glu Asp Asp Glu Asp Glu Glu Glu Ile Glu Val
   1               5                   10                  15
   Arg Ser Arg Asp Ile Ser
               20
<210> 224
   <211> 21
   <212> PRT
   <213> Artificial Sequence
<220>
   <223> Ciz1 b-variant peptide comprising an exon 14b/exon 15 junction
<400> 224
   Glu Glu Glu Glu Glu Asp Asp Glu Asp Glu Glu Glu Ile Glu Val Arg
   1               5                   10                  15
   Ser Arg Asp Ile Ser
               20
<210> 225
   <211> 20
   <212> PRT
   <213> Artificial Sequence
<220>
   <223> Ciz1 b-variant peptide comprising an exon 14b/exon 15 junction
<400> 225
   Glu Glu Glu Glu Asp Asp Glu Asp Glu Glu Glu Ile Glu Val Arg Ser
   1               5                   10                  15
   Arg Asp Ile Ser
               20
<210> 226
   <211> 19
   <212> PRT
   <213> Artificial Sequence
<220>
   <223> Ciz1 b-variant peptide comprising an exon 14b/exon 15 junction
<400> 226
   Glu Glu Glu Asp Asp Glu Asp Glu Glu Glu Ile Glu Val Arg Ser Arg
   1               5                   10                  15
   Asp Ile Ser
<210> 227
   <211> 18
   <212> PRT
   <213> Artificial Sequence
<220>
   <223> Ciz1 b-variant peptide comprising an exon 14b/exon 15 junction
<400> 227
   Glu Glu Asp Asp Glu Asp Glu Glu Glu Ile Glu Val Arg Ser Arg Asp
   1               5                   10                  15
   Ile Ser
<210> 228
   <211> 17
   <212> PRT
   <213> Artificial Sequence
<220>
   <223> Ciz1 b-variant peptide comprising an exon 14b/exon 15 junction
<400> 228
   Glu Asp Asp Glu Asp Glu Glu Glu Ile Glu Val Arg Ser Arg Asp Ile
   1               5                   10                  15
   Ser
<210> 229
   <211> 16
   <212> PRT
   <213> Artificial Sequence
<220>
   <223> Ciz1 b-variant peptide comprising an exon 14b/exon 15 junction
<400> 229
   Asp Asp Glu Asp Glu Glu Glu Ile Glu Val Arg Ser Arg Asp Ile Ser
   1               5                   10                  15
<210> 230
   <211> 15
   <212> PRT
   <213> Artificial Sequence
<220>
   <223> Ciz1 b-variant peptide comprising an exon 14b/exon 15 junction
<400> 230
   Asp Glu Asp Glu Glu Glu Ile Glu Val Arg Ser Arg Asp Ile Ser
   1               5                   10                  15
<210> 231
   <211> 14
   <212> PRT
   <213> Artificial Sequence
<220>
   <223> Ciz1 b-variant peptide comprising an exon 14b/exon 15 junction
<400> 231
   Glu Asp Glu Glu Glu Ile Glu Val Arg Ser Arg Asp Ile Ser
   1               5                   10
<210> 232
   <211> 12
   <212> PRT
   <213> Artificial Sequence
<220>
   <223> Ciz1 b-variant peptide comprising an exon 14b/exon 15 junction
<400> 232
   Glu Glu Glu Ile Glu Val Arg Ser Arg Asp Ile Ser
   1               5                   10
<210> 233
   <211> 11
   <212> **PRT**
   <213> **Artificial** Sequence
<220>
   <223> Ciz1 b-variant peptide comprising an exon 14b/exon 15 junction
<400> 233
   Glu Glu Ile Glu Val Arg Ser Arg Asp Ile Ser
   1               5                   10
<210> 234
   <211> 10
   <212> PRT
   <213> Artificial Sequence
<220>
   <223> Ciz1 b-variant peptide comprising an exon 14b/exon 15 junction
<400> 234
   Glu Ile Glu Val Arg Ser Arg Asp Ile Ser
   1               5                   10
<210> 235
   <211> 9
   <212> PRT
   <213> Artificial Sequence
<220>
   <223> Ciz1 b-variant peptide comprising an exon 14b/exon 15 junction
<400> 235
   Ile Glu Val Arg Ser Arg Asp Ile Ser
   1               5
<210> 236
   <211> 8
   <212> PRT
   <213> Artificial Sequence
<220>
   <223> Ciz1 b-variant peptide comprising an exon 14b/exon 15 junction
<400> 236
   Glu Val Arg Ser Arg Asp Ile Ser
   1               5
<210> 237
   <211> 44
   <212> PRT
   <213> Artificial Sequence
<220>
   <223> Ciz1 b-variant peptide comprising an exon 14b/exon 15 junction
<400> 237
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg
           35                  40
<210> 238
   <211> 43
   <212> PRT
   <213> Artificial Sequence
<220>
   <223> Ciz1 b-variant peptide comprising an exon 14b/exon 15 junction
<400> 238
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg
           35                  40
<210> 239
   <211> 42
   <212> PRT
   <213> Artificial Sequence
<220>
   <223> Ciz1 b-variant peptide comprising an exon 14b/exon 15 junction
<400> 239
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg
           35                  40
<210> 240
   <211> 41
   <212> PRT
   <213> Artificial Sequence
<220>
   <223> Ciz1 b-variant peptide comprising an exon 14b/exon 15 junction
<400> 240
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg
           35                  40
<210> 241
   <211> 40
   <212> PRT
   <213> Artificial Sequence
<220>
   <223> Ciz1 b-variant peptide comprising an exon 14b/exon 15 junction
<400> 241
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg
           35                  40
<210> 242
   <211> 39
   <212> PRT
   <213> Artificial Sequence
<220>
   <223> Ciz1 b-variant peptide comprising an exon 14b/exon 15 junction
<400> 242
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg
           35
<210> 243
   <211> 38
   <212> PRT
   <213> Artificial Sequence
<220>
   <223> Ciz1 b-variant peptide comprising an exon 14b/exon 15 junction
<400> 243
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg
           35
<210> 244
   <211> 37
   <212> PRT
   <213> Artificial Sequence
<220>
   <223> Ciz1 b-variant peptide comprising an exon 14b/exon 15 junction
<400> 244
   Asp His Phe Ile Thr Val Asp Ala Val Gly Cys Phe Glu Gly Asp Glu
   1 5 10 15
   Glu Glu Glu Glu Asp Asp Glu Asp Glu Glu Glu Ile Glu Val Arg Ser
   20 25 30
   Arg Asp Ile Ser Arg
   35
<210> 245
   <211> 36
   <212> PRT
   <213> Artificial Sequence
<220>
   <223> Ciz1 b-variant peptide comprising an exon 14b/exon 15 junction
<400> 245
   His Phe Ile Thr Val Asp Ala Val Gly Cys Phe Glu Gly Asp Glu Glu
   1 5 10 15
   Glu Glu Glu Asp Asp Glu Asp Glu Glu Glu Ile Glu Val Arg Ser Arg
   20 25 30
   Asp Ile Ser Arg
   35
<210> 246
   <211> 35
   <212> PRT
   <213> Artificial Sequence
<220>
   <223> Ciz1 b-variant peptide comprising an exon 14b/exon 15 junction
<400> 246
   Phe Ile Thr Val Asp Ala Val Gly Cys Phe Glu Gly Asp Glu Glu Glu
   1 5 10 15
   Glu Glu Asp Asp Glu Asp Glu Glu Glu Ile Glu Val Arg Ser Arg Asp
   20 25 30
   Ile Ser Arg
   35
<210> 247
   <211> 34
   <212> PRT
   <213> Artificial Sequence
<220>
   <223> Ciz1 b-variant peptide comprising an exon 14b/exon 15 junction
<400> 247
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg
<210> 248
   <211> 33
   <212> PRT
   <213> Artificial Sequence
<220>
   <223> Ciz1 b-variant peptide comprising an exon 14b/exon 15 junction
<400> 248
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg
<210> 249
   <211> 32
   <212> PRT
   <213> Artificial Sequence
<220>
   <223> Ciz1 b-variant peptide comprising an exon 14b/exon 15 junction
<400> 249
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
<210> 250
   <211> 31
   <212> PRT
   <213> Artificial Sequence
<220>
   <223> Ciz1 b-variant peptide comprising an exon 14b/exon 15 junction
<400> 250
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg
               20                  25                  30
<210> 251
   <211> 30
   <212> PRT
   <213> Artificial Sequence
<220>
   <223> Ciz1 b-variant peptide comprising an exon 14b/exon 15 junction
<400> 251
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg
               20                  25                  30
<210> 252
   <211> 29
   <212> PRT
   <213> Artificial Sequence
<220>
   <223> Ciz1 b-variant peptide comprising an exon 14b/exon 15 junction
<400> 252
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg
               20                  25
<210> 253
   <211> 28
   <212> PRT
   <213> Artificial Sequence
<220>
   <223> Ciz1 b-variant peptide comprising an exon 14b/exon 15 junction
<400> 253
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg
               20                  25
<210> 254
   <211> 27
   <212> PRT
   <213> Artificial Sequence
<220>
   <223> Ciz1 b-variant peptide comprising an exon 14b/exon 15 junction
<400> 254
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg
               20                  25
<210> 255
   <211> 26
   <212> PRT
   <213> Artificial Sequence
<220>
   <223> Ciz1 b-variant peptide comprising an exon 14b/exon 15 junction
<400> 255
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg
               20                  25
<210> 256
   <211> 25
   <212> PRT
   <213> Artificial Sequence
<220>
   <223> Ciz1 b-variant peptide comprising an exon 14b/exon 15 junction
<400> 256
   Glu Gly Asp Glu Glu Glu Glu Glu Asp Asp Glu Asp Glu Glu Glu Ile
   1               5                   10                  15
   Glu Val Arg Ser Arg Asp Ile Ser Arg
               20                  25
<210> 257
   <211> 24
   <212> PRT
   <213> Artificial Sequence
<220>
   <223> Ciz1 b-variant peptide comprising an exon 14b/exon 15 junction
<400> 257
   Gly Asp Glu Glu Glu Glu Glu Asp Asp Glu Asp Glu Glu Glu Ile Glu
   1               5                   10                  15
   Val Arg Ser Arg Asp Ile Ser Arg
               20
<210> 258
   <211> 23
   <212> PRT
   <213> Artificial Sequence
<220>
   <223> Ciz1 b-variant peptide comprising an exon 14b/exon 15 junction
<400> 258
   Asp Glu Glu Glu Glu Glu Asp Asp Glu Asp Glu Glu Glu Ile Glu Val
   1               5                   10                  15
   Arg Ser Arg Asp Ile Ser Arg
               20
<210> 259
   <211> 22
   <212> PRT
   <213> Artificial Sequence
<220>
   <223> Ciz1 b-variant peptide comprising an exon 14b/exon 15 junction
<400> 259
   Glu Glu Glu Glu Glu Asp Asp Glu Asp Glu Glu Glu Ile Glu Val Arg
   1               5                   10                  15
   Ser Arg Asp Ile Ser Arg
               20
<210> 260
   <211> 21
   <212> PRT
   <213> Artificial Sequence
<220>
   <223> Ciz1 b-variant peptide comprising an exon 14b/exon 15 junction
<400> 260
   Glu Glu Glu Glu Asp Asp Glu Asp Glu Glu Glu Ile Glu Val Arg Ser
   1 5 10 15
   Arg Asp Ile Ser Arg
   20
<210> 261
   <211> 20
   <212> PRT
   <213> Artificial Sequence
<220>
   <223> Ciz1 b-variant peptide comprising an exon 14b/exon 15 junction
<400> 261
   Glu Glu Glu Asp Asp Glu Asp Glu Glu Glu Ile Glu Val Arg Ser Arg
   1 5 10 15
   Asp Ile Ser Arg
   20
<210> 262
   <211> 19
   <212> PRT
   <213> Artificial Sequence
<220>
   <223> Ciz1 b-variant peptide comprising an exon 14b/exon 15 junction
<400> 262 Ile Ser Arg
<210> 263
   <211> 18
   <212> PRT
   <213> Artificial Sequence
<220>
   <223> Ciz1 b-variant peptide comprising an exon 14b/exon 15 junction
<400> 263
   Glu Asp Asp Glu Asp Glu Glu Glu Ile Glu Val Arg Ser Arg Asp Ile
   1 5 10 15
   Ser Arg
<210> 264
   <211> 17
   <212> PRT
   <213> Artificial Sequence
<220>
   <223> Ciz1 b-variant peptide comprising an exon 14b/exon 15 junction
<400> 264
   Asp Asp Glu Asp Glu Glu Glu Ile Glu Val Arg Ser Arg Asp Ile Ser
   1 5 10 15
   Arg
<210> 265
   <211> 16
   <212> PRT
   <213> Artificial Sequence
<220>
   <223> Ciz1 b-variant peptide comprising an exon 14b/exon 15 junction
<400> 265
   Asp Glu Asp Glu Glu Glu Ile Glu Val Arg Ser Arg Asp Ile Ser Arg
   1 5 10 15
<210> 266
   <211> 15
   <212> PRT
   <213> Artificial Sequence
<220>
   <223> Ciz1 b-variant peptide comprising an exon 14b/exon 15 junction
<400> 266
   Glu Asp Glu Glu Glu Ile Glu Val Arg Ser Arg Asp Ile Ser Arg
   1 5 10 15
<210> 267
   <211> 14
   <212> PRT
   <213> Artificial Sequence
<220>
   <223> Ciz1 b-variant peptide comprising an exon 14b/exon 15 junction
<400> 267
   Asp Glu Glu Glu Ile Glu Val Arg Ser Arg Asp Ile Ser Arg
   1 5 10
<210> 268
   <211> 13
   <212> PRT
   <213> Artificial Sequence
<220>
   <223> Ciz1 b-variant peptide comprising an exon 14b/exon 15 junction
<400> 268
   Glu Glu Glu Ile Glu Val Arg Ser Arg Asp Ile Ser Arg
   1 5 10
<210> 269
   <211> 12
   <212> PRT
   <213> Artificial Sequence
<220>
   <223> Ciz1 b-variant peptide comprising an exon 14b/exon 15 junction
<400> 269
   Glu Glu Ile Glu Val Arg Ser Arg Asp Ile Ser Arg
   1 5 10
<210> 270
   <211> 11
   <212> PRT
   <213> Artificial Sequence
<220>
   <223> Ciz1 b-variant peptide comprising an exon 14b/exon 15 junction
<400> 270
   Glu Ile Glu Val Arg Ser Arg Asp Ile Ser Arg
   1 5 10
<210> 271
   <211> 10
   <212> PRT
   <213> Artificial Sequence
<220>
   <223> Ciz1 b-variant peptide comprising an exon 14b/exon 15 junction
<400> 271
   Ile Glu Val Arg Ser Arg Asp Ile Ser Arg
   1 5 10
<210> 272
   <211> 9
   <212> PRT
   <213> Artificial Sequence
<220>
   <223> Ciz1 b-variant peptide comprising an exon 14b/exon 15 junction
<400> 272
   Glu Val Arg Ser Arg Asp Ile Ser Arg
   1 5
<210> 273
   <211> 45
   <212> PRT
   <213> Artificial Sequence
<220>
   <223> Ciz1 b-variant peptide comprising an exon 14b/exon 15 junction
<400> 273
   Glu Ile Ala Gly Gln Asp Glu Asp His Phe Ile Thr Val Asp Ala Val
   1 5 10 15
   Gly Cys Phe Glu Gly Asp Glu Glu Glu Glu Glu Asp Asp Glu Asp Glu
   20 25 30
   Glu Glu Ile Glu Val Arg Ser Arg Asp Ile Ser Arg Glu
   35 40 45
<210> 274
   <211> 44
   <212> PRT
   <213> Artificial Sequence
<220>
   <223> Ciz1 b-variant peptide comprising an exon 14b/exon 15 junction
<400> 274
   Ile Ala Gly Gln Asp Glu Asp His Phe Ile Thr Val Asp Ala Val Gly
   1 5 10 15
   Cys Phe Glu Gly Asp Glu Glu Glu Glu Glu Asp Asp Glu Asp Glu Glu
   20 25 30
   Glu Ile Glu Val Arg Ser Arg Asp Ile Ser Arg Glu
   35 40
<210> 275
   <211> 43
   <212> PRT
   <213> Artificial Sequence
<220>
   <223> Ciz1 b-variant peptide comprising an exon 14b/exon 15 junction
<400> 275
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu
           35                  40
<210> 276
   <211> 42
   <212> PRT
   <213> Artificial Sequence
<220>
   <223> Ciz1 b-variant peptide comprising an exon 14b/exon 15 junction
<400> 276
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu
           35                  40
<210> 277
   <211> 41
   <212> PRT
   <213> Artificial Sequence
<220>
   <223> Ciz1 b-variant peptide comprising an exon 14b/exon 15 junction
<400> 277
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu
           35                  40
<210> 278
   <211> 40
   <212> PRT
   <213> Artificial Sequence
<220>
   <223> Ciz1 b-variant peptide comprising an exon 14b/exon 15 junction
<400> 278
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu
           35                  40
<210> 279
   <211> 39
   <212> PRT
   <213> Artificial Sequence
<220>
   <223> Ciz1 b-variant peptide comprising an exon 14b/exon 15 junction
<400> 279
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu
           35
<210> 280
   <211> 38
   <212> PRT
   <213> Artificial Sequence
<220>
   <223> Ciz1 b-variant peptide comprising an exon 14b/exon 15 junction
<400> 280
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu
           35
<210> 281
   <211> 37
   <212> PRT
   <213> Artificial Sequence
<220>
   <223> Ciz1 b-variant peptide comprising an exon 14b/exon 15 junction
<400> 281
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu
           35
<210> 282
   <211> 36
   <212> PRT
   <213> Artificial Sequence
<220>
   <223> Ciz1 b-variant peptide comprising an exon 14b/exon 15 junction
<400> 282
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu
           35
<210> 283
   <211> 35
   <212> PRT
   <213> Artificial Sequence
<220>
   <223> Ciz1 b-variant peptide comprising an exon 14b/exon 15 junction
<400> 283
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu
           35
<210> 284
   <211> 34
   <212> PRT
   <213> Artificial Sequence
<220>
   <223> Ciz1 b-variant peptide comprising an exon 14b/exon 15 junction
<400> 284
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu
<210> 285
   <211> 33
   <212> PRT
   <213> Artificial Sequence
<220>
   <223> Ciz1 b-variant peptide comprising an exon 14b/exon 15 junction
<400> 285
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu
<210> 286
   <211> 32
   <212> PRT
   <213> Artificial Sequence
<220>
   <223> Ciz1 b-variant peptide comprising an exon 14b/exon 15 junction
<400> 286
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
<210> 287
   <211> 31
   <212> PRT
   <213> Artificial Sequence
<220>
   <223> Ciz1 b-variant peptide comprising an exon 14b/exon 15 junction
<400> 287
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu
               20                  25                  30
<210> 288
   <211> 30
   <212> PRT
   <213> Artificial Sequence
<220>
   <223> Ciz1 b-variant peptide comprising an exon 14b/exon 15 junction
<400> 288
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu
               20                  25                  30
<210> 289
   <211> 29
   <212> PRT
   <213> Artificial Sequence
<220>
   <223> Ciz1 b-variant peptide comprising an exon 14b/exon 15 junction
<400> 289
   Gly Cys Phe Glu Gly Asp Glu Glu Glu Glu Glu Asp Asp Glu Asp Glu
     1               5                   10                  15
   Glu Glu Ile Glu Val Arg Ser Arg Asp Ile Ser Arg Glu
               20                  25
<210> 290
   <211> 28
   <212> PRT
   <213> Artificial Sequence
<220>
   <223> Ciz1 b-variant peptide comprising an exon 14b/exon 15 junction
<400> 290
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu
               20                  25
<210> 291
   <211> 27
   <212> PRT
   <213> Artificial Sequence
<220>
   <223> Ciz1 b-variant peptide comprising an exon 14b/exon 15 junction
<400> 291
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu
               20                  25
<210> 292
   <211> 26
   <212> PRT
   <213> Artificial Sequence
<220>
   <223> Ciz1 b-variant peptide comprising an exon 14b/exon 15 junction
<400> 292
   Glu Gly Asp Glu Glu Glu Glu Glu Asp Asp Glu Asp Glu Glu Glu Ile
   1 5 10 15
   Glu Val Arg Ser Arg Asp Ile Ser Arg Glu
   20 25
<210> 293
   <211> 25
   <212> PRT
   <213> Artificial Sequence
<220>
   <223> Ciz1 b-variant peptide comprising an exon 14b/exon 15 junction
<400> 293
   Gly Asp Glu Glu Glu Glu Glu Asp Asp Glu Asp Glu Glu Glu Ile Glu
   1 5 10 15
   Val Arg Ser Arg Asp Ile Ser Arg Glu
   20 25
<210> 294
   <211> 24
   <212> PRT
   <213> Artificial Sequence
<220>
   <223> Ciz1 b-variant peptide comprising an exon 14b/exon 15 junction
<400> 294
   Asp Glu Glu Glu Glu Glu Asp Asp Glu Asp Glu Glu Glu Ile Glu Val
   1 5 10 15
   Arg Ser Arg Asp Ile Ser Arg Glu
   20
<210> 295
   <211> 23
   <212> PRT
   <213> Artificial Sequence
<220>
   <223> Ciz1 b-variant peptide comprising an exon 14b/exon 15 junction
<400> 295
   Glu Glu Glu Glu Glu Asp Asp Glu Asp Glu Glu Glu Ile Glu Val Arg
   1 5 10 15
   Ser Arg Asp Ile Ser Arg Glu
   20
<210> 296
   <211> 22
   <212> PRT
   <213> Artificial Sequence
<220>
   <223> Ciz1 b-variant peptide comprising an exon 14b/exon 15 junction
<400> 296
   Glu Glu Glu Glu Asp Asp Glu Asp Glu Glu Glu Ile Glu Val Arg Ser
   1               5                   10                  15
   Arg Asp Ile Ser Arg Glu
               20
<210> 297
   <211> 21
   <212> PRT
   <213> Artificial Sequence
<220>
   <223> Ciz1 b-variant peptide comprising an exon 14b/exon 15 junction
<400> 297
   Glu Glu Glu Asp Asp Glu Asp Glu Glu Glu Ile Glu Val Arg Ser Arg
   1               5                   10                  15
   Asp Ile Ser Arg Glu
               20
<210> 298
   <211> 20
   <212> PRT
   <213> Artificial Sequence
<220>
   <223> Ciz1 b-variant peptide comprising an exon 14b/exon 15 junction
<400> 298
   Glu Glu Asp Asp Glu Asp Glu Glu Glu Ile Glu Val Arg Ser Arg Asp
   1               5                   10                  15
   Ile Ser Arg Glu
               20
<210> 299
   <211> 19
   <212> PRT
   <213> Artificial Sequence
<220>
   <223> Ciz1 b-variant peptide comprising an exon 14b/exon 15 junction
<400> 299
   Glu Asp Asp Glu Asp Glu Glu Glu Ile Glu Val Arg Ser Arg Asp Ile
   1 5 10 15
   Ser Arg Glu
<210> 300
   <211> 18
   <212> PRT
   <213> Artificial Sequence
<220>
   <223> Ciz1 b-variant peptide comprising an exon 14b/exon 15 junction
<400> 300
   Asp Asp Glu Asp Glu Glu Glu Ile Glu Val Arg Ser Arg Asp Ile Ser
   1 5 10 15
   Arg Glu
<210> 301
   <211> 17
   <212> PRT
   <213> Artificial Sequence
<220>
   <223> Ciz1 b-variant peptide comprising an exon 14b/exon 15 junction
<400> 301
   Asp Glu Asp Glu Glu Glu Ile Glu Val Arg Ser Arg Asp Ile Ser Arg
   1 5 10 15
   Glu
<210> 302
   <211> 16
   <212> PRT
   <213> Artificial Sequence
<220>
   <223> Ciz1 b-variant peptide comprising an exon 14b/exon 15 junction
<400> 302
   Glu Asp Glu Glu Glu Ile Glu Val Arg Ser Arg Asp Ile Ser Arg Glu
   1 5 10 15
<210> 303
   <211> 15
   <212> PRT
   <213> Artificial Sequence
<220>
   <223> Ciz1 b-variant peptide comprising an exon 14b/exon 15 junction
<400> 303
   Asp Glu Glu Glu Ile Glu Val Arg Ser Arg Asp Ile Ser Arg Glu
   1               5                   10                  15
<210> 304
   <211> 14
   <212> PRT
   <213> Artificial Sequence
<220>
   <223> Ciz1 b-variant peptide comprising an exon 14b/exon 15 junction
<400> 304
   Glu Glu Glu Ile Glu Val Arg Ser Arg Asp Ile Ser Arg Glu
   1               5                   10
<210> 305
   <211> 13
   <212> PRT
   <213> Artificial Sequence
<220>
   <223> Ciz1 b-variant peptide comprising an exon 14b/exon 15 junction
<400> 305
   Glu Glu Ile Glu Val Arg Ser Arg Asp Ile Ser Arg Glu
   1               5                   10
<210> 306
   <211> 12
   <212> PRT
   <213> Artificial Sequence
<220>
   <223> Ciz1 b-variant peptide comprising an exon 14b/exon 15 junction
<400> 306
   Glu Ile Glu Val Arg Ser Arg Asp Ile Ser Arg Glu
   1               5                   10
<210> 307
   <211> 11
   <212> PRT
   <213> Artificial Sequence
<220>
   <223> Ciz1 b-variant peptide comprising an exon 14b/exon 15 junction
<400> 307
   Ile Glu Val Arg Ser Arg Asp Ile Ser Arg Glu
   1               5                   10
<210> 308
   <211> 10
   <212> PRT
   <213> Artificial Sequence
<220>
   <223> Ciz1 b-variant peptide comprising an exon 14b/exon 15 junction
<400> 308
   Glu Val Arg Ser Arg Asp Ile Ser Arg Glu
   1               5                   10
<210> 309
   <211> 46
   <212> PRT
   <213> Artificial Sequence
<220>
   <223> Ciz1 b-variant peptide comprising an exon 14b/exon 15 junction
<400> 309
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu
           35                  40                  45
<210> 310
   <211> 45
   <212> PRT
   <213> Artificial Sequence
<220>
   <223> Ciz1 b-variant peptide comprising an exon 14b/exon 15 junction
<400> 310
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu
           35                  40                  45
<210> 311
   <211> 44
   <212> PRT
   <213> Artificial Sequence
<220>
   <223> Ciz1 b-variant peptide comprising an exon 14b/exon 15 junction
<400> 311
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu
           35                  40
<210> 312
   <211> 43
   <212> PRT
   <213> Artificial Sequence
<220>
   <223> Ciz1 b-variant peptide comprising an exon 14b/exon 15 junction
<400> 312
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu
           35                  40
<210> 313
   <211> 42
   <212> PRT
   <213> Artificial Sequence
<220>
   <223> Ciz1 b-variant peptide comprising an exon 14b/exon 15 junction
<400> 313
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu
           35                  40
<210> 314
   <211> 41
   <212> PRT
   <213> Artificial Sequence
<220>
   <223> Ciz1 b-variant peptide comprising an exon 14b/exon 15 junction
<400> 314
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu
           35                  40
<210> 315
   <211> 40
   <212> PRT
   <213> Artificial Sequence
<220>
   <223> Ciz1 b-variant peptide comprising an exon 14b/exon 15 junction
<400> 315
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu
           35                  40
<210> 316
   <211> 39
   <212> PRT
   <213> Artificial Sequence
<220>
   <223> Ciz1 b-variant peptide comprising an exon 14b/exon 15 junction
<400> 316
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu
           35
<210> 317
   <211> 38
   <212> PRT
   <213> Artificial Sequence
<220>
   <223> Ciz1 b-variant peptide comprising an exon 14b/exon 15 junction
<400> 317
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu
           35
<210> 318
   <211> 37
   <212> PRT
   <213> Artificial Sequence
<220>
   <223> Ciz1 b-variant peptide comprising an exon 14b/exon 15 junction
<400> 318
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu
           35
<210> 319
   <211> 36
   <212> PRT
   <213> Artificial Sequence
<220>
   <223> Ciz1 b-variant peptide comprising an exon 14b/exon 15 junction
<400> 319
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu
           35
<210> 320
   <211> 35
   <212> PRT
   <213> Artificial Sequence
<220>
   <223> Ciz1 b-variant peptide comprising an exon 14b/exon 15 junction
<400> 320
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu
           35
<210> 321
   <211> 34
   <212> PRT
   <213> Artificial Sequence
<220>
   <223> Ciz1 b-variant peptide comprising an exon 14b/exon 15 junction
<400> 321
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu
<210> 322
   <211> 33
   <212> PRT
   <213> Artificial Sequence
<220>
   <223> Ciz1 b-variant peptide comprising an exon 14b/exon 15 junction
<400> 322
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu
<210> 323
   <211> 32
   <212> PRT
   <213> Artificial Sequence
<220>
   <223> Ciz1 b-variant peptide comprising an exon 14b/exon 15 junction
<400> 323
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
<210> 324
   <211> 31
   <212> PRT
   <213> Artificial Sequence
<220>
   <223> Ciz1 b-variant peptide comprising an exon 14b/exon 15 junction
<400> 324
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu
               20                  25                  30
<210> 325
   <211> 30
   <212> PRT
   <213> Artificial Sequence
<220>
   <223> Ciz1 b-variant peptide comprising an exon 14b/exon 15 junction
<400> 325
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu
               20                  25                  30
<210> 326
   <211> 29
   <212> PRT
   <213> Artificial Sequence
<220>
   <223> Ciz1 b-variant peptide comprising an exon 14b/exon 15 junction
<400> 326
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu
               20                  25
<210> 327
   <211> 28
   <212> PRT
   <213> Artificial Sequence
<220>
   <223> Ciz1 b-variant peptide comprising an exon 14b/exon 15 junction
<400> 327
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu
               20                  25
<210> 328
   <211> 27
   <212> PRT
   <213> Artificial Sequence
<220>
   <223> Ciz1 b-variant peptide comprising an exon 14b/exon 15 junction
<400> 328
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu
               20                  25
<210> 329
   <211> 26
   <212> PRT
   <213> Artificial Sequence
<220>
   <223> Ciz1 b-variant peptide comprising an exon 14b/exon 15 junction
<400> 329
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu
               20                  25
<210> 330
   <211> 25
   <212> PRT
   <213> Artificial Sequence
<220>
   <223> Ciz1 b-variant peptide comprising an exon 14b/exon 15 junction
<400> 330
   Asp Glu Glu Glu Glu Glu Asp Asp Glu Asp Glu Glu Glu Ile Glu Val
   1               5                   10                  15
   Arg Ser Arg Asp Ile Ser Arg Glu Glu
               20                  25
<210> 331
   <211> 24
   <212> PRT
   <213> Artificial Sequence
<220>
   <223> Ciz1 b-variant peptide comprising an exon 14b/exon 15 junction
<400> 331
   Glu Glu Glu Glu Glu Asp Asp Glu Asp Glu Glu Glu Ile Glu Val Arg
   1               5                   10                  15
   Ser Arg Asp Ile Ser Arg Glu Glu
               20
<210> 332
   <211> 23
   <212> PRT
   <213> Artificial Sequence
<220>
   <223> Ciz1 b-variant peptide comprising an exon 14b/exon 15 junction
<400> 332
   Glu Glu Glu Glu Asp Asp Glu Asp Glu Glu Glu Ile Glu Val Arg Ser
   1               5                   10                  15
   Arg Asp Ile Ser Arg Glu Glu
               20
<210> 333
   <211> 22
   <212> PRT
   <213> Artificial Sequence
<220>
   <223> Ciz1 b-variant peptide comprising an exon 14b/exon 15 junction
<400> 333
   Glu Glu Glu Asp Asp Glu Asp Glu Glu Glu Ile Glu Val Arg Ser Arg
   1               5                   10                  15
   Asp Ile Ser Arg Glu Glu
               20
<210> 334
   <211> 21
   <212> PRT
   <213> Artificial Sequence
<220>
   <223> Ciz1 b-variant peptide comprising an exon 14b/exon 15 junction
<400> 334
   Glu Glu Asp Asp Glu Asp Glu Glu Glu Ile Glu Val Arg Ser Arg Asp
   1               5                   10                  15
   Ile Ser Arg Glu Glu
               20
<210> 335
   <211> 20
   <212> PRT
   <213> Artificial Sequence
<220>
   <223> Ciz1 b-variant peptide comprising an exon 14b/exon 15 junction
<400> 335
   Glu Asp Asp Glu Asp Glu Glu Glu Ile Glu Val Arg Ser Arg Asp Ile
   1               5                   10                  15
   Ser Arg Glu Glu
               20
<210> 336
   <211> 19
   <212> PRT
   <213> Artificial Sequence
<220>
   <223> Ciz1 b-variant peptide comprising an exon 14b/exon 15 junction
<400> 336
   Asp Asp Glu Asp Glu Glu Glu Ile Glu Val Arg Ser Arg Asp Ile Ser
   1               5                   10                  15
   Arg Glu Glu
<210> 337
   <211> 18
   <212> PRT
   <213> Artificial Sequence
<220>
   <223> Ciz1 b-variant peptide comprising an exon 14b/exon 15 junction
<400> 337
   Asp Glu Asp Glu Glu Glu Ile Glu Val Arg Ser Arg Asp Ile Ser Arg
   1               5                   10                  15
   Glu Glu
<210> 338
   <211> 17
   <212> **PRT**
   <213> **Artificial** Sequence
<220>
   <223> Ciz1 b-variant peptide comprising an exon 14b/exon 15 junction
<400> 338
   Glu Asp Glu Glu Glu Ile Glu Val Arg Ser Arg Asp Ile Ser Arg Glu
   1               5                   10                  15
   Glu
<210> 339
   <211> 16
   <212> PRT
   <213> Artificial Sequence
<220>
   <223> Ciz1 b-variant peptide comprising an exon 14b/exon 15 junction
<400> 339
   Asp Glu Glu Glu Ile Glu Val Arg Ser Arg Asp Ile Ser Arg Glu Glu
   1               5                   10                  15
<210> 340
   <211> 15
   <212> PRT
   <213> Artificial Sequence
<220>
   <223> Ciz1 b-variant peptide comprising an exon 14b/exon 15 junction
<400> 340
   Glu Glu Glu Ile Glu Val Arg Ser Arg Asp Ile Ser Arg Glu Glu
   1               5                   10                  15
<210> 341
   <211> 14
   <212> PRT
   <213> Artificial Sequence
<220>
   <223> Ciz1 b-variant peptide comprising an exon 14b/exon 15 junction
<400> 341
   Glu Glu Ile Glu Val Arg Ser Arg Asp Ile Ser Arg Glu Glu
   1               5                   10
<210> 342
   <211> 13
   <212> PRT
   <213> Artificial Sequence
<220>
   <223> Ciz1 b-variant peptide comprising an exon 14b/exon 15 junction
<400> 342
   Glu Ile Glu Val Arg Ser Arg Asp Ile Ser Arg Glu Glu
   1               5                   10
<210> 343
   <211> 12
   <212> PRT
   <213> Artificial Sequence
<220>
   <223> Ciz1 b-variant peptide comprising an exon 14b/exon 15 junction
<400> 343
   Ile Glu Val Arg Ser Arg Asp Ile Ser Arg Glu Glu
   1               5                   10
<210> 344
   <211> 11
   <212> PRT
   <213> Artificial Sequence
<220>
   <223> Ciz1 b-variant peptide comprising an exon 14b/exon 15 junction
<400> 344
   Glu Val Arg Ser Arg Asp Ile Ser Arg Glu Glu
   1               5                   10
<210> 345
   <211> 47
   <212> PRT
   <213> Artificial Sequence
<220>
   <223> Ciz1 b-variant peptide comprising an exon 14b/exon 15 junction
<400> 345
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp
           35                  40                  45
<210> 346
   <211> 46
   <212> PRT
   <213> Artificial Sequence
<220>
   <223> Ciz1 b-variant peptide comprising an exon 14b/exon 15 junction
<400> 346
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp
           35                  40                  45
<210> 347
   <211> 45
   <212> PRT
   <213> Artificial Sequence
<220>
   <223> Ciz1 b-variant peptide comprising an exon 14b/exon 15 junction
<400> 347
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp
           35                  40                  45
<210> 348
   <211> 44
   <212> PRT
   <213> Artificial Sequence
<220>
   <223> Ciz1 b-variant peptide comprising an exon 14b/exon 15 junction
<400> 348
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp
           35                  40
<210> 349
   <211> 43
   <212> PRT
   <213> Artificial Sequence
<220>
   <223> Ciz1 b-variant peptide comprising an exon 14b/exon 15 junction
<400> 349
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp
           35                  40
<210> 350
   <211> 42
   <212> PRT
   <213> Artificial Sequence
<220>
   <223> Ciz1 b-variant peptide comprising an exon 14b/exon 15 junction
<400> 350
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp
           35                  40
<210> 351
   <211> 41
   <212> PRT
   <213> Artificial Sequence
<220>
   <223> Ciz1 b-variant peptide comprising an exon 14b/exon 15 junction
<400> 351
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp
           35                  40
<210> 352
   <211> 40
   <212> PRT
   <213> Artificial Sequence
<220>
   <223> Ciz1 b-variant peptide comprising an exon 14b/exon 15 junction
<400> 352
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp
           35                  40
<210> 353
   <211> 39
   <212> PRT
   <213> Artificial Sequence
<220>
   <223> Ciz1 b-variant peptide comprising an exon 14b/exon 15 junction
<400> 353
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp
           35
<210> 354
   <211> 38
   <212> PRT
   <213> Artificial Sequence
<220>
   <223> Ciz1 b-variant peptide comprising an exon 14b/exon 15 junction
<400> 354
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp
           35
<210> 355
   <211> 37
   <212> PRT
   <213> Artificial Sequence
<220>
   <223> Ciz1 b-variant peptide comprising an exon 14b/exon 15 junction
<400> 355
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp
           35
<210> 356
   <211> 36
   <212> PRT
   <213> Artificial Sequence
<220>
   <223> Ciz1 b-variant peptide comprising an exon 14b/exon 15 junction
<400> 356
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp
   35
<210> 357
   <211> 35
   <212> PRT
   <213> Artificial Sequence
<220>
   <223> Ciz1 b-variant peptide comprising an exon 14b/exon 15 junction
<400> 357
   Val Asp Ala Val Gly Cys Phe Glu Gly Asp Glu Glu Glu Glu Glu Asp
   1 5 10 15
   Asp Glu Asp Glu Glu Glu Ile Glu Val Arg Ser Arg Asp Ile Ser Arg
   20 25 30
   Glu Glu Trp
   35
<210> 358
   <211> 34
   <212> PRT
   <213> Artificial Sequence
<220>
   <223> Ciz1 b-variant peptide comprising an exon 14b/exon 15 junction
<400> 358
   Asp Ala Val Gly Cys Phe Glu Gly Asp Glu Glu Glu Glu Glu Asp Asp
   1 5 10 15
   Glu Asp Glu Glu Glu Ile Glu Val Arg Ser Arg Asp Ile Ser Arg Glu
   20 25 30
   Glu Trp
<210> 359
   <211> 33
   <212> PRT
   <213> Artificial Sequence
<220>
   <223> Ciz1 b-variant peptide comprising an exon 14b/exon 15 junction
<400> 359
   Ala Val Gly Cys Phe Glu Gly Asp Glu Glu Glu Glu Glu Asp Asp Glu
   1 5 10 15
   Asp Glu Glu Glu Ile Glu Val Arg Ser Arg Asp Ile Ser Arg Glu Glu
   20 25 30
   Trp
<210> 360
   <211> 32
   <212> PRT
   <213> Artificial Sequence
<220>
   <223> Ciz1 b-variant peptide comprising an exon 14b/exon 15 junction
<400> 360
   Val Gly Cys Phe Glu Gly Asp Glu Glu Glu Glu Glu Asp Asp Glu Asp
   1 5 10 15
   Glu Glu Glu Ile Glu Val Arg Ser Arg Asp Ile Ser Arg Glu Glu Trp
   20 25 30
<210> 361
   <211> 31
   <212> PRT
   <213> Artificial Sequence
<220>
   <223> Ciz1 b-variant peptide comprising an exon 14b/exon 15 junction
<400> 361
   Gly Cys Phe Glu Gly Asp Glu Glu Glu Glu Glu Asp Asp Glu Asp Glu
   1 5 10 15
   Glu Glu Ile Glu Val Arg Ser Arg Asp Ile Ser Arg Glu Glu Trp
   20 25 30
<210> 362
   <211> 30
   <212> PRT
   <213> Artificial Sequence
<220>
   <223> Ciz1 b-variant peptide comprising an exon 14b/exon 15 junction
<400> 362
   Cys Phe Glu Gly Asp Glu Glu Glu Glu Glu Asp Asp Glu Asp Glu Glu
   1 5 10 15
   Glu Ile Glu Val Arg Ser Arg Asp Ile Ser Arg Glu Glu Trp
   20 25 30
<210> 363
   <211> 29
   <212> PRT
   <213> Artificial Sequence
<220>
   <223> Ciz1 b-variant peptide comprising an exon 14b/exon 15 junction
<400> 363
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp
               20                  25
<210> 364
   <211> 28
   <212> PRT
   <213> Artificial Sequence
<220>
   <223> Ciz1 b-variant peptide comprising an exon 14b/exon 15 junction
<400> 364
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp
               20                  25
<210> 365
   <211> 27
   <212> PRT
   <213> Artificial Sequence
<220>
   <223> Ciz1 b-variant peptide comprising an exon 14b/exon 15 junction
<400> 365
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp
               20                  25
<210> 366
   <211> 26
   <212> PRT
   <213> Artificial Sequence
<220>
   <223> Ciz1 b-variant peptide comprising an exon 14b/exon 15 junction
<400> 366
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp
               20                  25
<210> 367
   <211> 25
   <212> PRT
   <213> Artificial Sequence
<220>
   <223> Ciz1 b-variant peptide comprising an exon 14b/exon 15 junction
<400> 367
   Glu Glu Glu Glu Glu Asp Asp Glu Asp Glu Glu Glu Ile Glu Val Arg
   1               5                   10                  15
   Ser Arg Asp Ile Ser Arg Glu Glu Trp
               20                  25
<210> 368
   <211> 24
   <212> PRT
   <213> Artificial Sequence
<220>
   <223> Ciz1 b-variant peptide comprising an exon 14b/exon 15 junction
<400> 368
   Glu Glu Glu Glu Asp Asp Glu Asp Glu Glu Glu Ile Glu Val Arg Ser
   1               5                   10                  15
   Arg Asp Ile Ser Arg Glu Glu Trp
               20
<210> 369
   <211> 23
   <212> PRT
   <213> Artificial Sequence
<220>
   <223> Ciz1 b-variant peptide comprising an exon 14b/exon 15 junction
<400> 369
   Glu Glu Glu Asp Asp Glu Asp Glu Glu Glu Ile Glu Val Arg Ser Arg
   1 5 10 15
   Asp Ile Ser Arg Glu Glu Trp
   20
<210> 370
   <211> 22
   <212> PRT
   <213> Artificial Sequence
<220>
   <223> Ciz1 b-variant peptide comprising an exon 14b/exon 15 junction
<400> 370
   Glu Glu Asp Asp Glu Asp Glu Glu Glu Ile Glu Val Arg Ser Arg Asp
   1 5 10 15
   Ile Ser Arg Glu Glu Trp
   20
<210> 371
   <211> 21
   <212> PRT
   <213> Artificial Sequence
<220>
   <223> Ciz1 b-variant peptide comprising an exon 14b/exon 15 junction
<400> 371
   Glu Asp Asp Glu Asp Glu Glu Glu Ile Glu Val Arg Ser Arg Asp Ile
   1 5 10 15
   Ser Arg Glu Glu Trp
   20
<210> 372
   <211> 20
   <212> PRT
   <213> Artificial Sequence
<220>
   <223> Ciz1 b-variant peptide comprising an exon 14b/exon 15 junction
<400> 372
   Asp Asp Glu Asp Glu Glu Glu Ile Glu Val Arg Ser Arg Asp Ile Ser
   1 5 10 15
   Arg Glu Glu Trp
   20
<210> 373
   <211> 19
   <212> PRT
   <213> Artificial Sequence
<220>
   <223> Ciz1 b-variant peptide comprising an exon 14b/exon 15 junction
<400> 373
   Asp Glu Asp Glu Glu Glu Ile Glu Val Arg Ser Arg Asp Ile Ser Arg
   1               5                   10                  15
   Glu Glu Trp
<210> 374
   <211> 18
   <212> PRT
   <213> Artificial Sequence
<220>
   <223> Ciz1 b-variant peptide comprising an exon 14b/exon 15 junction
<400> 374
   Glu Asp Glu Glu Glu Ile Glu Val Arg Ser Arg Asp Ile Ser Arg Glu
   1               5                   10                  15
   Glu Trp
<210> 375
   <211> 17
   <212> PRT
   <213> Artificial Sequence
<220>
   <223> Ciz1 b-variant peptide comprising an exon 14b/exon 15 junction
<400> 375
   Asp Glu Glu Glu Ile Glu Val Arg Ser Arg Asp Ile Ser Arg Glu Glu
   1               5                   10                  15
   Trp
<210> 376
   <211> 16
   <212> PRT
   <213> Artificial Sequence
<220>
   <223> Ciz1 b-variant peptide comprising an exon 14b/exon 15 junction
<400> 376
   Glu Glu Glu Ile Glu Val Arg Ser Arg Asp Ile Ser Arg Glu Glu Trp
   1 5 10 15
<210> 377
   <211> 15
   <212> PRT
   <213> Artificial Sequence
<220>
   <223> Ciz1 b-variant peptide comprising an exon 14b/exon 15 junction
<400> 377
   Glu Glu Ile Glu Val Arg Ser Arg Asp Ile Ser Arg Glu Glu Trp
   1 5 10 15
<210> 378
   <211> 14
   <212> PRT
   <213> Artificial Sequence
<220>
   <223> Ciz1 b-variant peptide comprising an exon 14b/exon 15 junction
<400> 378
   Glu Ile Glu Val Arg Ser Arg Asp Ile Ser Arg Glu Glu Trp
   1 5 10
<210> 379
   <211> 13
   <212> PRT
   <213> Artificial Sequence
<220>
   <223> Ciz1 b-variant peptide comprising an exon 14b/exon 15 junction
<400> 379
   Ile Glu Val Arg Ser Arg Asp Ile Ser Arg Glu Glu Trp
   1 5 10
<210> 380
   <211> 12
   <212> PRT
   <213> Artificial Sequence
<220>
   <223> Ciz1 b-variant peptide comprising an exon 14b/exon 15 junction
<400> 380
   Glu Val Arg Ser Arg Asp Ile Ser Arg Glu Glu Trp
   1 5 10
<210> 381
   <211> 48
   <212> PRT
   <213> Artificial Sequence
<220>
   <223> Ciz1 b-variant peptide comprising an exon 14b/exon 15 junction
<400> 381
   Glu Ile Ala Gly Gln Asp Glu Asp His Phe Ile Thr Val Asp Ala Val
   1 5 10 15
   Gly Cys Phe Glu Gly Asp Glu Glu Glu Glu Glu Asp Asp Glu Asp Glu
   20 25 30
   Glu Glu Ile Glu Val Arg Ser Arg Asp Ile Ser Arg Glu Glu Trp Lys
   35 40 45
<210> 382
   <211> 47
   <212> PRT
   <213> Artificial Sequence
<220>
   <223> Ciz1 b-variant peptide comprising an exon 14b/exon 15 junction
<400> 382
   Ile Ala Gly Gln Asp Glu Asp His Phe Ile Thr Val Asp Ala Val Gly
   1 5 10 15
   Cys Phe Glu Gly Asp Glu Glu Glu Glu Glu Asp Asp Glu Asp Glu Glu
   20 25 30
   Glu Ile Glu Val Arg Ser Arg Asp Ile Ser Arg Glu Glu Trp Lys
   35 40 45
<210> 383
   <211> 46
   <212> PRT
   <213> Artificial Sequence
<220>
   <223> Ciz1 b-variant peptide comprising an exon 14b/exon 15 junction
<400> 383
   Ala Gly Gln Asp Glu Asp His Phe Ile Thr Val Asp Ala Val Gly Cys
   1 5 10 15
   Phe Glu Gly Asp Glu Glu Glu Glu Glu Asp Asp Glu Asp Glu Glu Glu
   20 25 30
   Ile Glu Val Arg Ser Arg Asp Ile Ser Arg Glu Glu Trp Lys
   35 40 45
<210> 384
   <211> 45
   <212> PRT
   <213> Artificial Sequence
<220>
   <223> Ciz1 b-variant peptide comprising an exon 14b/exon 15 junction
<400> 384
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys
           35                  40                  45
<210> 385
   <211> 44
   <212> PRT
   <213> Artificial Sequence
<220>
   <223> Ciz1 b-variant peptide comprising an exon 14b/exon 15 junction
<400> 385
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys
           35                  40
<210> 386
   <211> 43
   <212> PRT
   <213> Artificial Sequence
<220>
   <223> Ciz1 b-variant peptide comprising an exon 14b/exon 15 junction
<400> 386
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys
           35                  40
<210> 387
   <211> 42
   <212> PRT
   <213> Artificial Sequence
<220>
<210> 388
   <211> 41
   <212> PRT
   <213> Artificial Sequence
<220>
   <223> Ciz1 b-variant peptide comprising an exon 14b/exon 15 junction
<400> 388
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys
           35                  40
<210> 389
   <211> 40
   <212> PRT
   <213> Artificial Sequence
<220>
   <223> Ciz1 b-variant peptide comprising an exon 14b/exon 15 junction
<400> 389
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys
           35                  40
<210> 390
   <211> 39
   <212> PRT
   <213> Artificial Sequence
<220>
   <223> Ciz1 b-variant peptide comprising an exon 14b/exon 15 junction
<400> 390
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys
           35
<210> 391
   <211> 38
   <212> PRT
   <213> Artificial Sequence
<220>
   <223> Ciz1 b-variant peptide comprising an exon 14b/exon 15 junction
<400> 391
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys
           35
<210> 392
   <211> 37
   <212> PRT
   <213> Artificial Sequence
<220>
   <223> Ciz1 b-variant peptide comprising an exon 14b/exon 15 junction
<400> 392
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys
           35
<210> 393
   <211> 36
   <212> PRT
   <213> Artificial Sequence
<220>
   <223> Ciz1 b-variant peptide comprising an exon 14b/exon 15 junction
<400> 393
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys
           35
<210> 394
   <211> 35
   <212> PRT
   <213> Artificial Sequence
<220>
   <223> Ciz1 b-variant peptide comprising an exon 14b/exon 15 junction
<400> 394
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys
           35
<210> 395
   <211> 34
   <212> PRT
   <213> Artificial Sequence
<220>
   <223> Ciz1 b-variant peptide comprising an exon 14b/exon 15 junction
<400> 395
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys
<210> 396
   <211> 33
   <212> PRT
   <213> Artificial Sequence
<220>
   <223> Ciz1 b-variant peptide comprising an exon 14b/exon 15 junction
<400> 396
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys
<210> 397
   <211> 32
   <212> PRT
   <213> Artificial Sequence
<220>
   <223> Ciz1 b-variant peptide comprising an exon 14b/exon 15 junction
<400> 397
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
<210> 398
   <211> 31
   <212> PRT
   <213> Artificial Sequence
<220>
   <223> Ciz1 b-variant peptide comprising an exon 14b/exon 15 junction
<400> 398
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys
               20                  25                  30
<210> 399
   <211> 30
   <212> PRT
   <213> Artificial Sequence
<220>
   <223> Ciz1 b-variant peptide comprising an exon 14b/exon 15 junction
<400> 399
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys
               20                  25                  30
<210> 400
   <211> 29
   <212> PRT
   <213> Artificial Sequence
<220>
   <223> Ciz1 b-variant peptide comprising an exon 14b/exon 15 junction
<400> 400
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys
               20                  25
<210> 401
   <211> 28
   <212> PRT
   <213> Artificial Sequence
<220>
   <223> Ciz1 b-variant peptide comprising an exon 14b/exon 15 junction
<400> 401
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys
               20                  25
<210> 402
   <211> 27
   <212> PRT
   <213> Artificial Sequence
<220>
   <223> Ciz1 b-variant peptide comprising an exon 14b/exon 15 junction
<400> 402
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys
               20                  25
<210> 403
   <211> 26
   <212> PRT
   <213> Artificial Sequence
<220>
   <223> Ciz1 b-variant peptide comprising an exon 14b/exon 15 junction
<400> 403
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys
               20                  25
<210> 404
   <211> 25
   <212> PRT
   <213> Artificial Sequence
<220>
   <223> Ciz1 b-variant peptide comprising an exon 14b/exon 15 junction
<400> 404
   Glu Glu Glu Glu Asp Asp Glu Asp Glu Glu Glu Ile Glu Val Arg Ser
   1               5                   10                  15
   Arg Asp Ile Ser Arg Glu Glu Trp Lys
               20                  25
<210> 405
   <211> 24
   <212> PRT
   <213> Artificial Sequence
<220>
   <223> Ciz1 b-variant peptide comprising an exon 14b/exon 15 junction
<400> 405
   Glu Glu Glu Asp Asp Glu Asp Glu Glu Glu Ile Glu Val Arg Ser Arg
   1               5                   10                  15
   Asp Ile Ser Arg Glu Glu Trp Lys
               20
<210> 406
   <211> 23
   <212> PRT
   <213> Artificial Sequence
<220>
   <223> Ciz1 b-variant peptide comprising an exon 14b/exon 15 junction
<400> 406
   Glu Glu Asp Asp Glu Asp Glu Glu Glu Ile Glu Val Arg Ser Arg Asp
   1               5                   10                  15
   Ile Ser Arg Glu Glu Trp Lys
               20
<210> 407
   <211> 22
   <212> PRT
   <213> Artificial Sequence
<220>
   <223> Ciz1 b-variant peptide comprising an exon 14b/exon 15 junction
<400> 407
   Glu Asp Asp Glu Asp Glu Glu Glu Ile Glu Val Arg Ser Arg Asp Ile
   1 5 10 15
   Ser Arg Glu Glu Trp Lys
   20
<210> 408
   <211> 21
   <212> PRT
   <213> Artificial Sequence
<220>
   <223> Ciz1 b-variant peptide comprising an exon 14b/exon 15 junction
<400> 408
   Asp Asp Glu Asp Glu Glu Glu Ile Glu Val Arg Ser Arg Asp Ile Ser
   1 5 10 15
   Arg Glu Glu Trp Lys
   20
<210> 409
   <211> 20
   <212> PRT
   <213> Artificial Sequence
<220>
   <223> Ciz1 b-variant peptide comprising an exon 14b/exon 15 junction
<400> 409
   Asp Glu Asp Glu Glu Glu Ile Glu Val Arg Ser Arg Asp Ile Ser Arg
   1 5 10 15
   Glu Glu Trp Lys
   20
<210> 410
   <211> 19
   <212> PRT
   <213> Artificial Sequence
<220>
   <223> Ciz1 b-variant peptide comprising an exon 14b/exon 15 junction
<400> 410
   Glu Asp Glu Glu Glu Ile Glu Val Arg Ser Arg Asp Ile Ser Arg Glu
   1 5 10 15
   Glu Trp Lys
<210> 411
   <211> 18
   <212> PRT
   <213> Artificial Sequence
<220>
   <223> Ciz1 b-variant peptide comprising an exon 14b/exon 15 junction
<400> 411 Trp Lys
<210> 412
   <211> 17
   <212> PRT
   <213> Artificial Sequence
<220>
   <223> Ciz1 b-variant peptide comprising an exon 14b/exon 15 junction
<400> 412
   Glu Glu Glu Ile Glu Val Arg Ser Arg Asp Ile Ser Arg Glu Glu Trp
   1 5 10 15
   Lys
<210> 413
   <211> 16
   <212> PRT
   <213> Artificial Sequence
<220>
   <223> Ciz1 b-variant peptide comprising an exon 14b/exon 15 junction
<400> 413
   Glu Glu Ile Glu Val Arg Ser Arg Asp Ile Ser Arg Glu Glu Trp Lys
   1 5 10 15
<210> 414
   <211> 15
   <212> PRT
   <213> Artificial Sequence
<220>
   <223> Ciz1 b-variant peptide comprising an exon 14b/exon 15 junction
<400> 414
   Glu Ile Glu Val Arg Ser Arg Asp Ile Ser Arg Glu Glu Trp Lys
   1               5                   10                  15
<210> 415
   <211> 14
   <212> PRT
   <213> Artificial Sequence
<220>
   <223> Ciz1 b-variant peptide comprising an exon 14b/exon 15 junction
<400> 415
   Ile Glu Val Arg Ser Arg Asp Ile Ser Arg Glu Glu Trp Lys
   1               5                   10
<210> 416
   <211> 13
   <212> PRT
   <213> Artificial Sequence
<220>
   <223> Ciz1 b-variant peptide comprising an exon 14b/exon 15 junction
<400> 416
   Glu Val Arg Ser Arg Asp Ile Ser Arg Glu Glu Trp Lys
   1               5                   10
<210> 417
   <211> 49
   <212> PRT
   <213> Artificial Sequence
<220>
   <223> Ciz1 b-variant peptide comprising an exon 14b/exon 15 junction
<400> 417
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly
<210> 418
   <211> 48
   <212> PRT
   <213> Artificial Sequence
<220>
   <223> Ciz1 b-variant peptide comprising an exon 14b/exon 15 junction
<400> 418
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
<210> 419
   <211> 47
   <212> PRT
   <213> Artificial Sequence
<220>
   <223> Ciz1 b-variant peptide comprising an exon 14b/exon 15 junction
<400> 419
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly
           35                  40                  45
<210> 420
   <211> 46
   <212> PRT
   <213> Artificial Sequence
<220>
   <223> Ciz1 b-variant peptide comprising an exon 14b/exon 15 junction
<400> 420
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly
           35                  40                  45
<210> 421
   <211> 45
   <212> PRT
   <213> Artificial Sequence
<220>
   <223> Ciz1 b-variant peptide comprising an exon 14b/exon 15 junction
<400> 421
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly
           35                  40                  45
<210> 422
   <211> 44
   <212> PRT
   <213> Artificial Sequence
<220>
   <223> Ciz1 b-variant peptide comprising an exon 14b/exon 15 junction
<400> 422
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly
           35                  40
<210> 423
   <211> 43
   <212> PRT
   <213> Artificial Sequence
<220>
   <223> Ciz1 b-variant peptide comprising an exon 14b/exon 15 junction
<400> 423
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly
           35                  40
<210> 424
   <211> 42
   <212> PRT
   <213> Artificial Sequence
<220>
   <223> Ciz1 b-variant peptide comprising an exon 14b/exon 15 junction
<400> 424
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly
           35                  40
<210> 425
   <211> 41
   <212> PRT
   <213> Artificial Sequence
<220>
   <223> Ciz1 b-variant peptide comprising an exon 14b/exon 15 junction
<400> 425
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly
           35                  40
<210> 426
   <211> 40
   <212> PRT
   <213> Artificial Sequence
<220>
   <223> Ciz1 b-variant peptide comprising an exon 14b/exon 15 junction
<400> 426
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly
           35                  40
<210> 427
   <211> 39
   <212> PRT
   <213> Artificial Sequence
<220>
   <223> Ciz1 b-variant peptide comprising an exon 14b/exon 15 junction
<400> 427
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly
           35
<210> 428
   <211> 38
   <212> PRT
   <213> Artificial Sequence
<220>
   <223> Ciz1 b-variant peptide comprising an exon 14b/exon 15 junction
<400> 428
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
                   20                  25                  30
   Arg Glu Glu Trp Lys Gly
           35
<210> 429
   <211> 37
   <212> PRT
   <213> Artificial Sequence
<220>
   <223> Ciz1 b-variant peptide comprising an exon 14b/exon 15 junction
<400> 429
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly
           35
<210> 430
   <211> 36
   <212> PRT
   <213> Artificial Sequence
<220>
   <223> Ciz1 b-variant peptide comprising an exon 14b/exon 15 junction
<400> 430
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly
           35
<210> 431
   <211> 35
   <212> PRT
   <213> Artificial Sequence
<220>
   <223> Ciz1 b-variant peptide comprising an exon 14b/exon 15 junction
<400> 431
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly
           35
<210> 432
   <211> 34
   <212> PRT
   <213> Artificial Sequence
<220>
   <223> Ciz1 b-variant peptide comprising an exon 14b/exon 15 junction
<400> 432
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly
<210> 433
   <211> 33
   <212> PRT
   <213> Artificial Sequence
<220>
   <223> Ciz1 b-variant peptide comprising an exon 14b/exon 15 junction
<400> 433
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly
<210> 434
   <211> 32
   <212> PRT
   <213> Artificial Sequence
<220>
   <223> Ciz1 b-variant peptide comprising an exon 14b/exon 15 junction
<400> 434
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
<210> 435
   <211> 31
   <212> PRT
   <213> Artificial Sequence
<220>
   <223> Ciz1 b-variant peptide comprising an exon 14b/exon 15 junction
<400> 435
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly
               20                  25                  30
<210> 436
   <211> 30
   <212> PRT
   <213> Artificial Sequence
<220>
   <223> Ciz1 b-variant peptide comprising an exon 14b/exon 15 junction
<400> 436
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly
               20                  25                  30
<210> 437
   <211> 29
   <212> PRT
   <213> Artificial Sequence
<220>
   <223> Ciz1 b-variant peptide comprising an exon 14b/exon 15 junction
<400> 437
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly
               20                  25
<210> 438
   <211> 28
   <212> PRT
   <213> Artificial Sequence
<220>
   <223> Ciz1 b-variant peptide comprising an exon 14b/exon 15 junction
<400> 438
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly
               20                  25
<210> 439
   <211> 27
   <212> PRT
   <213> Artificial Sequence
<220>
   <223> Ciz1 b-variant peptide comprising an exon 14b/exon 15 junction
<400> 439
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly
               20                  25
<210> 440
   <211> 26
   <212> PRT
   <213> Artificial Sequence
<220>
   <223> Ciz1 b-variant peptide comprising an exon 14b/exon 15 junction
<400> 440
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly
               20                  25
<210> 441
   <211> 25
   <212> PRT
   <213> Artificial Sequence
<220>
   <223> Ciz1 b-variant peptide comprising an exon 14b/exon 15 junction
<400> 441
   Glu Glu Glu Asp Asp Glu Asp Glu Glu Glu Ile Glu Val Arg Ser Arg
   1               5                   10                  15
   Asp Ile Ser Arg Glu Glu Trp Lys Gly
               20                  25
<210> 442
   <211> 24
   <212> PRT
   <213> Artificial Sequence
<220>
   <223> Ciz1 b-variant peptide comprising an exon 14b/exon 15 junction
<400> 442
   Glu Glu Asp Asp Glu Asp Glu Glu Glu Ile Glu Val Arg Ser Arg Asp
   1               5                   10                  15
   Ile Ser Arg Glu Glu Trp Lys Gly
               20
<210> 443
   <211> 23
   <212> PRT
   <213> Artificial Sequence
<220>
   <223> Ciz1 b-variant peptide comprising an exon 14b/exon 15 junction
<400> 443
   Glu Asp Asp Glu Asp Glu Glu Glu Ile Glu Val Arg Ser Arg Asp Ile
   1               5                   10                  15
   Ser Arg Glu Glu Trp Lys Gly
               20
<210> 444
   <211> 22
   <212> PRT
   <213> Artificial Sequence
<220>
   <223> Ciz1 b-variant peptide comprising an exon 14b/exon 15 junction
<400> 444
   Asp Asp Glu Asp Glu Glu Glu Ile Glu Val Arg Ser Arg Asp Ile Ser
   1               5                   10                  15
   Arg Glu Glu Trp Lys Gly
               20
<210> 445
   <211> 21
   <212> PRT
   <213> Artificial Sequence
<220>
   <223> Ciz1 b-variant peptide comprising an exon 14b/exon 15 junction
<400> 445
   Asp Glu Asp Glu Glu Glu Ile Glu Val Arg Ser Arg Asp Ile Ser Arg
   1               5                   10                  15
   Glu Glu Trp Lys Gly
               20
<210> 446
   <211> 20
   <212> PRT
   <213> Artificial Sequence
<220>
   <223> Ciz1 b-variant peptide comprising an exon 14b/exon 15 junction
<400> 446
   Glu Asp Glu Glu Glu Ile Glu Val Arg Ser Arg Asp Ile Ser Arg Glu
   1               5                   10                  15
   Glu Trp Lys Gly
               20
<210> 447
   <211> 19
   <212> PRT
   <213> Artificial Sequence
<220>
   <223> Ciz1 b-variant peptide comprising an exon 14b/exon 15 junction
<400> 447
   Asp Glu Glu Glu Ile Glu Val Arg Ser Arg Asp Ile Ser Arg Glu Glu
   1               5                   10                  15
   Trp Lys Gly
<210> 448
   <211> 18
   <212> PRT
   <213> Artificial Sequence
<220>
   <223> Ciz1 b-variant peptide comprising an exon 14b/exon 15 junction
<400> 448
   Glu Glu Glu Ile Glu Val Arg Ser Arg Asp Ile Ser Arg Glu Glu Trp
   1               5                   10                  15
   Lys Gly
<210> 449
   <211> 17
   <212> PRT
   <213> Artificial Sequence
<220>
   <223> Ciz1 b-variant peptide comprising an exon 14b/exon 15 junction
<400> 449
   Glu Glu Ile Glu Val Arg Ser Arg Asp Ile Ser Arg Glu Glu Trp Lys
   1               5                   10                  15
   Gly
<210> 450
   <211> 16
   <212> PRT
   <213> Artificial Sequence
<220>
   <223> Ciz1 b-variant peptide comprising an exon 14b/exon 15 junction
<400> 450
   Glu Ile Glu Val Arg Ser Arg Asp Ile Ser Arg Glu Glu Trp Lys Gly
   1               5                   10                  15
<210> 451
   <211> 15
   <212> PRT
   <213> Artificial Sequence
<220>
   <223> Ciz1 b-variant peptide comprising an exon 14b/exon 15 junction
<400> 451
   Ile Glu Val Arg Ser Arg Asp Ile Ser Arg Glu Glu Trp Lys Gly
   1               5                   10                  15
<210> 452
   <211> 14
   <212> PRT
   <213> Artificial Sequence
<220>
   <223> Ciz1 b-variant peptide comprising an exon 14b/exon 15 junction
<400> 452
   Glu Val Arg Ser Arg Asp Ile Ser Arg Glu Glu Trp Lys Gly
   1               5                   10
<210> 453
   <211> 50
   <212> PRT
   <213> Artificial Sequence
<220>
   <223> Ciz1 b-variant peptide comprising an exon 14b/exon 15 junction
<400> 453
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser
       50
<210> 454
   <211> 49
   <212> PRT
   <213> Artificial Sequence
<220>
   <223> Ciz1 b-variant peptide comprising an exon 14b/exon 15 junction
<400> 454
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser
<210> 455
   <211> 48
   <212> PRT
   <213> Artificial Sequence
<220>
   <223> Ciz1 b-variant peptide comprising an exon 14b/exon 15 junction
<400> 455
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
<210> 456
   <211> 47
   <212> PRT
   <213> Artificial Sequence
<220>
   <223> Ciz1 b-variant peptide comprising an exon 14b/exon 15 junction
<400> 456
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser
           35                  40                  45
<210> 457
   <211> 46
   <212> PRT
   <213> Artificial Sequence
<220>
   <223> Ciz1 b-variant peptide comprising an exon 14b/exon 15 junction
<400> 457
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser
           35                  40                  45
<210> 458
   <211> 45
   <212> PRT
   <213> Artificial Sequence
<220>
   <223> Ciz1 b-variant peptide comprising an exon 14b/exon 15 junction
<400> 458
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser
           35                  40                  45
<210> 459
   <211> 44
   <212> PRT
   <213> Artificial Sequence
<220>
   <223> Ciz1 b-variant peptide comprising an exon 14b/exon 15 junction
<400> 459
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser
           35                  40
<210> 460
   <211> 43
   <212> PRT
   <213> Artificial Sequence
<220>
   <223> Ciz1 b-variant peptide comprising an exon 14b/exon 15 junction
<400> 460
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser
           35                  40
<210> 461
   <211> 42
   <212> PRT
   <213> Artificial Sequence
<220>
   <223> Ciz1 b-variant peptide comprising an exon 14b/exon 15 junction
<400> 461
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser
           35                  40
<210> 462
   <211> 41
   <212> PRT
   <213> Artificial Sequence
<220>
   <223> Ciz1 b-variant peptide comprising an exon 14b/exon 15 junction
<400> 462
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser
           35                  40
<210> 463
   <211> 40
   <212> PRT
   <213> Artificial Sequence
<220>
   <223> Ciz1 b-variant peptide comprising an exon 14b/exon 15 junction
<400> 463
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser
   35 40
<210> 464
   <211> 39
   <212> PRT
   <213> Artificial Sequence
<220>
   <223> Ciz1 b-variant peptide comprising an exon 14b/exon 15 junction
<400> 464
   Thr Val Asp Ala Val Gly Cys Phe Glu Gly Asp Glu Glu Glu Glu Glu
   1 5 10 15
   Asp Asp Glu Asp Glu Glu Glu Ile Glu Val Arg Ser Arg Asp Ile Ser
   20 25 30
   Arg Glu Glu Trp Lys Gly Ser
   35
<210> 465
   <211> 38
   <212> PRT
   <213> Artificial Sequence
<220>
   <223> Ciz1 b-variant peptide comprising an exon 14b/exon 15 junction
<400> 465
   Val Asp Ala Val Gly Cys Phe Glu Gly Asp Glu Glu Glu Glu Glu Asp
   1 5 10 15
   Asp Glu Asp Glu Glu Glu Ile Glu Val Arg Ser Arg Asp Ile Ser Arg
   20 25 30
   Glu Glu Trp Lys Gly Ser
   35
<210> 466
   <211> 37
   <212> PRT
   <213> Artificial Sequence
<220>
   <223> Ciz1 b-variant peptide comprising an exon 14b/exon 15 junction
<400> 466
   Asp Ala Val Gly Cys Phe Glu Gly Asp Glu Glu Glu Glu Glu Asp Asp
   1 5 10 15
   Glu Asp Glu Glu Glu Ile Glu Val Arg Ser Arg Asp Ile Ser Arg Glu
   20 25 30
   Glu Trp Lys Gly Ser
   35
<210> 467
   <211> 36
   <212> PRT
   <213> Artificial Sequence
<220>
   <223> Ciz1 b-variant peptide comprising an exon 14b/exon 15 junction
<400> 467
   Ala Val Gly Cys Phe Glu Gly Asp Glu Glu Glu Glu Glu Asp Asp Glu
   1 5 10 15
   Asp Glu Glu Glu Ile Glu Val Arg Ser Arg Asp Ile Ser Arg Glu Glu
   20 25 30
   Trp Lys Gly Ser
   35
<210> 468
   <211> 35
   <212> PRT
   <213> Artificial Sequence
<220>
   <223> Ciz1 b-variant peptide comprising an exon 14b/exon 15 junction
<400> 468
   Val Gly Cys Phe Glu Gly Asp Glu Glu Glu Glu Glu Asp Asp Glu Asp
   1 5 10 15
   Glu Glu Glu Ile Glu Val Arg Ser Arg Asp Ile Ser Arg Glu Glu Trp
   20 25 30
   Lys Gly Ser
   35
<210> 469
   <211> 34
   <212> PRT
   <213> Artificial Sequence
<220>
   <223> Ciz1 b-variant peptide comprising an exon 14b/exon 15 junction
<400> 469
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser
<210> 470
   <211> 33
   <212> PRT
   <213> Artificial Sequence
<220>
   <223> Ciz1 b-variant peptide comprising an exon 14b/exon 15 junction
<400> 470
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser
<210> 471
   <211> 32
   <212> PRT
   <213> Artificial Sequence
<220>
   <223> Ciz1 b-variant peptide comprising an exon 14b/exon 15 junction
<400> 471
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
<210> 472
   <211> 31
   <212> PRT
   <213> Artificial Sequence
<220>
   <223> Ciz1 b-variant peptide comprising an exon 14b/exon 15 junction
<400> 472
   Glu Gly Asp Glu Glu Glu Glu Glu Asp Asp Glu Asp Glu Glu Glu Ile
   1 5 10 15
   Glu Val Arg Ser Arg Asp Ile Ser Arg Glu Glu Trp Lys Gly Ser
   20 25 30
<210> 473
   <211> 30
   <212> PRT
   <213> Artificial Sequence
<220>
   <223> Ciz1 b-variant peptide comprising an exon 14b/exon 15 junction
<400> 473
   Gly Asp Glu Glu Glu Glu Glu Asp Asp Glu Asp Glu Glu Glu Ile Glu
   1 5 10 15
   Val Arg Ser Arg Asp Ile Ser Arg Glu Glu Trp Lys Gly Ser
   20 25 30
<210> 474
   <211> 29
   <212> PRT
   <213> Artificial Sequence
<220>
   <223> Ciz1 b-variant peptide comprising an exon 14b/exon 15 junction
<400> 474
   Asp Glu Glu Glu Glu Glu Asp Asp Glu Asp Glu Glu Glu Ile Glu Val
   1 5 10 15
   Arg Ser Arg Asp Ile Ser Arg Glu Glu Trp Lys Gly Ser
   20 25
<210> 475
   <211> 28
   <212> PRT
   <213> Artificial Sequence
<220>
   <223> Ciz1 b-variant peptide comprising an exon 14b/exon 15 junction
<400> 475
   Glu Glu Glu Glu Glu Asp Asp Glu Asp Glu Glu Glu Ile Glu Val Arg
   1 5 10 15
   Ser Arg Asp Ile Ser Arg Glu Glu Trp Lys Gly Ser
   20 25
<210> 476
   <211> 27
   <212> PRT
   <213> Artificial Sequence
<220>
   <223> Ciz1 b-variant peptide comprising an exon 14b/exon 15 junction
<400> 476
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser
               20                  25
<210> 477
   <211> 26
   <212> PRT
   <213> Artificial Sequence
<220>
   <223> Ciz1 b-variant peptide comprising an exon 14b/exon 15 junction
<400> 477
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser
               20                  25
<210> 478
   <211> 25
   <212> PRT
   <213> Artificial Sequence
<220>
   <223> Ciz1 b-variant peptide comprising an exon 14b/exon 15 junction
<400> 478
   Glu Glu Asp Asp Glu Asp Glu Glu Glu Ile Glu Val Arg Ser Arg Asp
   1               5                   10                  15
   Ile Ser Arg Glu Glu Trp Lys Gly Ser
               20                  25
<210> 479
   <211> 24
   <212> PRT
   <213> Artificial Sequence
<220>
   <223> Ciz1 b-variant peptide comprising an exon 14b/exon 15 junction
<400> 479
   Glu Asp Asp Glu Asp Glu Glu Glu Ile Glu Val Arg Ser Arg Asp Ile
   1               5                   10                  15
   Ser Arg Glu Glu Trp Lys Gly Ser
               20
<210> 480
   <211> 23
   <212> PRT
   <213> Artificial Sequence
<220>
   <223> Ciz1 b-variant peptide comprising an exon 14b/exon 15 junction
<400> 480
   Asp Asp Glu Asp Glu Glu Glu Ile Glu Val Arg Ser Arg Asp Ile Ser
   1               5                   10                  15
   Arg Glu Glu Trp Lys Gly Ser
               20
<210> 481
   <211> 22
   <212> PRT
   <213> Artificial Sequence
<220>
   <223> Ciz1 b-variant peptide comprising an exon 14b/exon 15 junction
<400> 481
   Asp Glu Asp Glu Glu Glu Ile Glu Val Arg Ser Arg Asp Ile Ser Arg
   1               5                   10                  15
   Glu Glu Trp Lys Gly Ser
               20
<210> 482
   <211> 21
   <212> PRT
   <213> Artificial Sequence
<220>
   <223> Ciz1 b-variant peptide comprising an exon 14b/exon 15 junction
<400> 482
   Glu Asp Glu Glu Glu Ile Glu Val Arg Ser Arg Asp Ile Ser Arg Glu
   1               5                   10                  15
   Glu Trp Lys Gly Ser
               20
<210> 483
   <211> 20
   <212> PRT
   <213> Artificial Sequence
<220>
   <223> Ciz1 b-variant peptide comprising an exon 14b/exon 15 junction
<400> 483
   Asp Glu Glu Glu Ile Glu Val Arg Ser Arg Asp Ile Ser Arg Glu Glu
   1               5                   10                  15
   Trp Lys Gly Ser
               20
<210> 484
   <211> 19
   <212> PRT
   <213> Artificial Sequence
<220>
   <223> Ciz1 b-variant peptide comprising an exon 14b/exon 15 junction
<400> 484
   Glu Glu Glu Ile Glu Val Arg Ser Arg Asp Ile Ser Arg Glu Glu Trp
   1               5                   10                  15
   Lys Gly Ser
<210> 485
   <211> 18
   <212> PRT
   <213> Artificial Sequence
<220>
   <223> Ciz1 b-variant peptide comprising an exon 14b/exon 15 junction
<400> 485
   Glu Glu Ile Glu Val Arg Ser Arg Asp Ile Ser Arg Glu Glu Trp Lys
   1               5                   10                  15
   Gly Ser
<210> 486
   <211> 17
   <212> PRT
   <213> Artificial Sequence
<220>
   <223> Ciz1 b-variant peptide comprising an exon 14b/exon 15 junction
<400> 486
   Glu Ile Glu Val Arg Ser Arg Asp Ile Ser Arg Glu Glu Trp Lys Gly
   1               5                   10                  15
   Ser
<210> 487
   <211> 16
   <212> PRT
   <213> Artificial Sequence
<220>
   <223> Ciz1 b-variant peptide comprising an exon 14b/exon 15 junction
<400> 487
   Ile Glu Val Arg Ser Arg Asp Ile Ser Arg Glu Glu Trp Lys Gly Ser
   1               5                   10                  15
<210> 488
   <211> 15
   <212> PRT
   <213> Artificial Sequence
<220>
   <223> Ciz1 b-variant peptide comprising an exon 14b/exon 15 junction
<400> 488
   Glu Val Arg Ser Arg Asp Ile Ser Arg Glu Glu Trp Lys Gly Ser
   1               5                   10                  15
<210> 489
   <211> 51
   <212> PRT
   <213> Artificial Sequence
<220>
   <223> Ciz1 b-variant peptide comprising an exon 14b/exon 15 junction
<400> 489
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu
       50
<210> 490
   <211> 50
   <212> PRT
   <213> Artificial Sequence
<220>
   <223> Ciz1 b-variant peptide comprising an exon 14b/exon 15 junction
<400> 490
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu
       50
<210> 491
   <211> 49
   <212> PRT
   <213> Artificial Sequence
<220>
   <223> Ciz1 b-variant peptide comprising an exon 14b/exon 15 junction
<400> 491
   Ala Gly Gln Asp Glu Asp His Phe Ile Thr Val Asp Ala Val Gly Cys
   1 5 10 15
   Phe Glu Gly Asp Glu Glu Glu Glu Glu Asp Asp Glu Asp Glu Glu Glu
   20 25 30
   Ile Glu Val Arg Ser Arg Asp Ile Ser Arg Glu Glu Trp Lys Gly Ser
   35 40 45
   Glu
<210> 492
   <211> 48
   <212> PRT
   <213> Artificial Sequence
<220>
   <223> Ciz1 b-variant peptide comprising an exon 14b/exon 15 junction
<400> 492
   Gly Gln Asp Glu Asp His Phe Ile Thr Val Asp Ala Val Gly Cys Phe
   1 5 10 15
   Glu Gly Asp Glu Glu Glu Glu Glu Asp Asp Glu Asp Glu Glu Glu Ile
   20 25 30
   Glu Val Arg Ser Arg Asp Ile Ser Arg Glu Glu Trp Lys Gly Ser Glu
   35 40 45
<210> 493
   <211> 47
   <212> PRT
   <213> Artificial Sequence
<220>
   <223> Ciz1 b-variant peptide comprising an exon 14b/exon 15 junction
<400> 493
   Gln Asp Glu Asp His Phe Ile Thr Val Asp Ala Val Gly Cys Phe Glu
   1 5 10 15
   Gly Asp Glu Glu Glu Glu Glu Asp Asp Glu Asp Glu Glu Glu Ile Glu
   20 25 30
   Val Arg Ser Arg Asp Ile Ser Arg Glu Glu Trp Lys Gly Ser Glu
   35 40 45
<210> 494
   <211> 46
   <212> PRT
   <213> Artificial Sequence
<220>
   <223> Ciz1 b-variant peptide comprising an exon 14b/exon 15 junction
<400> 494
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu
           35                  40                  45
<210> 495
   <211> 45
   <212> PRT
   <213> Artificial Sequence
<220>
   <223> Ciz1 b-variant peptide comprising an exon 14b/exon 15 junction
<400> 495
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu
           35                  40                  45
<210> 496
   <211> 44
   <212> PRT
   <213> Artificial Sequence
<220>
   <223> Ciz1 b-variant peptide comprising an exon 14b/exon 15 junction
<400> 496
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu
           35                  40
<210> 497
   <211> 43
   <212> PRT
   <213> Artificial Sequence
<220>
   <223> Ciz1 b-variant peptide comprising an exon 14b/exon 15 junction
<400> 497
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu
           35                  40
<210> 498
   <211> 42
   <212> PRT
   <213> Artificial Sequence
<220>
   <223> Ciz1 b-variant peptide comprising an exon 14b/exon 15 junction
<400> 498
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu
           35                  40
<210> 499
   <211> 41
   <212> PRT
   <213> Artificial Sequence
<220>
   <223> Ciz1 b-variant peptide comprising an exon 14b/exon 15 junction
<400> 499
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu
           35                  40
<210> 500
   <211> 40
   <212> PRT
   <213> Artificial Sequence
<220>
   <223> Ciz1 b-variant peptide comprising an exon 14b/exon 15 junction
<400> 500
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu
           35                  40
<210> 501
   <211> 39
   <212> PRT
   <213> Artificial Sequence
<220>
   <223> Ciz1 b-variant peptide comprising an exon 14b/exon 15 junction
<400> 501
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu
           35
<210> 502
   <211> 38
   <212> PRT
   <213> Artificial Sequence
<220>
   <223> Ciz1 b-variant peptide comprising an exon 14b/exon 15 junction
<400> 502
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu
           35
<210> 503
   <211> 37
   <212> PRT
   <213> Artificial Sequence
<220>
   <223> Ciz1 b-variant peptide comprising an exon 14b/exon 15 junction
<400> 503
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu
           35
<210> 504
   <211> 36
   <212> PRT
   <213> Artificial Sequence
<220>
   <223> Ciz1 b-variant peptide comprising an exon 14b/exon 15 junction
<400> 504
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu
           35
<210> 505
   <211> 35
   <212> PRT
   <213> Artificial Sequence
<220>
   <223> Ciz1 b-variant peptide comprising an exon 14b/exon 15 junction
<400> 505
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu
           35
<210> 506
   <211> 34
   <212> PRT
   <213> Artificial Sequence
<220>
   <223> Ciz1 b-variant peptide comprising an exon 14b/exon 15 junction
<400> 506
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu
<210> 507
   <211> 33
   <212> PRT
   <213> Artificial Sequence
<220>
   <223> Ciz1 b-variant peptide comprising an exon 14b/exon 15 junction
<400> 507
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu
<210> 508
   <211> 32
   <212> PRT
   <213> Artificial Sequence
<220>
   <223> Ciz1 b-variant peptide comprising an exon 14b/exon 15 junction
<400> 508
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
<210> 509
   <211> 31
   <212> PRT
   <213> Artificial Sequence
<220>
   <223> Ciz1 b-variant peptide comprising an exon 14b/exon 15 junction
<400> 509
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu
               20                  25                  30
<210> 510
   <211> 30
   <212> PRT
   <213> Artificial Sequence
<220>
   <223> Ciz1 b-variant peptide comprising an exon 14b/exon 15 junction
<400> 510
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu
               20                  25                  30
<210> 511
   <211> 29
   <212> PRT
   <213> Artificial Sequence
<220>
   <223> Ciz1 b-variant peptide comprising an exon 14b/exon 15 junction
<400> 511
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu
               20                  25
<210> 512
   <211> 28
   <212> PRT
   <213> Artificial Sequence
<220>
   <223> Ciz1 b-variant peptide comprising an exon 14b/exon 15 junction
<400> 512
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu
               20                  25
<210> 513
   <211> 27
   <212> PRT
   <213> Artificial Sequence
<220>
   <223> Ciz1 b-variant peptide comprising an exon 14b/exon 15 junction
<400> 513
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu
               20                  25
<210> 514
   <211> 26
   <212> PRT
   <213> Artificial Sequence
<220>
   <223> Ciz1 b-variant peptide comprising an exon 14b/exon 15 junction
<400> 514
   Glu Glu Asp Asp Glu Asp Glu Glu Glu Ile Glu Val Arg Ser Arg Asp
   1 5 10 15
   Ile Ser Arg Glu Glu Trp Lys Gly Ser Glu
   20 25
<210> 515
   <211> 25
   <212> PRT
   <213> Artificial Sequence
<220>
   <223> Ciz1 b-variant peptide comprising an exon 14b/exon 15 junction
<400> 515
   Glu Asp Asp Glu Asp Glu Glu Glu Ile Glu Val Arg Ser Arg Asp Ile
   1 5 10 15
   Ser Arg Glu Glu Trp Lys Gly Ser Glu
   20 25
<210> 516
   <211> 24
   <212> PRT
   <213> Artificial Sequence
<220>
   <223> Ciz1 b-variant peptide comprising an exon 14b/exon 15 junction
<400> 516
   Asp Asp Glu Asp Glu Glu Glu Ile Glu Val Arg Ser Arg Asp Ile Ser
   1 5 10 15
   Arg Glu Glu Trp Lys Gly Ser Glu
   20
<210> 517
   <211> 23
   <212> PRT
   <213> Artificial Sequence
<220>
   <223> Ciz1 b-variant peptide comprising an exon 14b/exon 15 junction
<400> 517
   Asp Glu Asp Glu Glu Glu Ile Glu Val Arg Ser Arg Asp Ile Ser Arg
   1               5                   10                  15
   Glu Glu Trp Lys Gly Ser Glu
               20
<210> 518
   <211> 22
   <212> PRT
   <213> Artificial Sequence
<220>
   <223> Ciz1 b-variant peptide comprising an exon 14b/exon 15 junction
<400> 518
   Glu Asp Glu Glu Glu Ile Glu Val Arg Ser Arg Asp Ile Ser Arg Glu
   1               5                   10                  15
   Glu Trp Lys Gly Ser Glu
               20
<210> 519
   <211> 21
   <212> PRT
   <213> Artificial Sequence
<220>
   <223> Ciz1 b-variant peptide comprising an exon 14b/exon 15 junction
<400> 519
   Asp Glu Glu Glu Ile Glu Val Arg Ser Arg Asp Ile Ser Arg Glu Glu
   1               5                   10                  15
   Trp Lys Gly Ser Glu
               20
<210> 520
   <211> 20
   <212> PRT
   <213> Artificial Sequence
<220>
   <223> Ciz1 b-variant peptide comprising an exon 14b/exon 15 junction
<400> 520
   Glu Glu Glu Ile Glu Val Arg Ser Arg Asp Ile Ser Arg Glu Glu Trp
   1               5                   10                  15
   Lys Gly Ser Glu
       20
<210> 521
   <211> 19
   <212> PRT
   <213> Artificial Sequence
<220>
   <223> Ciz1 b-variant peptide comprising an exon 14b/exon 15 junction
<400> 521
   Glu Glu Ile Glu Val Arg Ser Arg Asp Ile Ser Arg Glu Glu Trp Lys
   1               5                   10                  15
   Gly Ser Glu
<210> 522
   <211> 18
   <212> PRT
   <213> Artificial Sequence
<220>
   <223> Ciz1 b-variant peptide comprising an exon 14b/exon 15 junction
<400> 522
   Glu Ile Glu Val Arg Ser Arg Asp Ile Ser Arg Glu Glu Trp Lys Gly
   1               5                   10                  15
   Ser Glu
<210> 523
   <211> 17
   <212> PRT
   <213> Artificial Sequence
<220>
   <223> Ciz1 b-variant peptide comprising an exon 14b/exon 15 junction
<400> 523
   Ile Glu Val Arg Ser Arg Asp Ile Ser Arg Glu Glu Trp Lys Gly Ser
   1               5                   10                  15
   Glu
<210> 524
   <211> 16
   <212> PRT
   <213> Artificial Sequence
<220>
   <223> Ciz1 b-variant peptide comprising an exon 14b/exon 15 junction
<400> 524
   Glu Val Arg Ser Arg Asp Ile Ser Arg Glu Glu Trp Lys Gly Ser Glu
   1               5                   10                  15
<210> 525
   <211> 52
   <212> PRT
   <213> Artificial Sequence
<220>
   <223> Ciz1 b-variant peptide comprising an exon 14b/exon 15 junction
<400> 525
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr
       50
<210> 526
   <211> 51
   <212> PRT
   <213> Artificial Sequence
<220>
   <223> Ciz1 b-variant peptide comprising an exon 14b/exon 15 junction
<400> 526
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr
       50
<210> 527
   <211> 50
   <212> PRT
   <213> Artificial Sequence
<220>
   <223> Ciz1 b-variant peptide comprising an exon 14b/exon 15 junction
<400> 527
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr
       50
<210> 528
   <211> 49
   <212> PRT
   <213> Artificial Sequence
<220>
   <223> Ciz1 b-variant peptide comprising an exon 14b/exon 15 junction
<400> 528
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr
<210> 529
   <211> 48
   <212> PRT
   <213> Artificial Sequence
<220>
   <223> Ciz1 b-variant peptide comprising an exon 14b/exon 15 junction
<400> 529
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
   20 25 30
   Val Arg Ser Arg Asp Ile Ser Arg Glu Glu Trp Lys Gly Ser Glu Thr
   35 40 45
<210> 530
   <211> 47
   <212> PRT
   <213> Artificial Sequence
<220>
   <223> Ciz1 b-variant peptide comprising an exon 14b/exon 15 junction
<400> 530
   Asp Glu Asp His Phe Ile Thr Val Asp Ala Val Gly Cys Phe Glu Gly
   1 5 10 15
   Asp Glu Glu Glu Glu Glu Asp Asp Glu Asp Glu Glu Glu Ile Glu Val
   20 25 30
   Arg Ser Arg Asp Ile Ser Arg Glu Glu Trp Lys Gly Ser Glu Thr
   35 40 45
<210> 531
   <211> 46
   <212> PRT
   <213> Artificial Sequence
<220>
   <223> Ciz1 b-variant peptide comprising an exon 14b/exon 15 junction
<400> 531
   Glu Asp His Phe Ile Thr Val Asp Ala Val Gly Cys Phe Glu Gly Asp
   1 5 10 15
   Glu Glu Glu Glu Glu Asp Asp Glu Asp Glu Glu Glu Ile Glu Val Arg
   20 25 30
   Ser Arg Asp Ile Ser Arg Glu Glu Trp Lys Gly Ser Glu Thr
   35 40 45
<210> 532
   <211> 45
   <212> PRT
   <213> Artificial Sequence
<220>
   <223> Ciz1 b-variant peptide comprising an exon 14b/exon 15 junction
<400> 532
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr
           35                  40                  45
<210> 533
   <211> 44
   <212> PRT
   <213> Artificial Sequence
<220>
   <223> Ciz1 b-variant peptide comprising an exon 14b/exon 15 junction
<400> 533
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr
           35                  40
<210> 534
   <211> 43
   <212> PRT
   <213> Artificial Sequence
<220>
   <223> Ciz1 b-variant peptide comprising an exon 14b/exon 15 junction
<400> 534
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr
           35                  40
<210> 535
   <211> 42
   <212> PRT
   <213> Artificial Sequence
<220>
   <223> Ciz1 b-variant peptide comprising an exon 14b/exon 15 junction
<400> 535
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr
           35                  40
<210> 536
   <211> 41
   <212> PRT
   <213> Artificial Sequence
<220>
   <223> Ciz1 b-variant peptide comprising an exon 14b/exon 15 junction
<400> 536
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr
           35                  40
<210> 537
   <211> 40
   <212> PRT
   <213> Artificial Sequence
<220>
   <223> Ciz1 b-variant peptide comprising an exon 14b/exon 15 junction
<400> 537
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr
           35                  40
<210> 538
   <211> 39
   <212> PRT
   <213> Artificial Sequence
<220>
   <223> Ciz1 b-variant peptide comprising an exon 14b/exon 15 junction
<400> 538
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr
           35
<210> 539
   <211> 38
   <212> PRT
   <213> Artificial Sequence
<220>
   <223> Ciz1 b-variant peptide comprising an exon 14b/exon 15 junction
<400> 539
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr
           35
<210> 540
   <211> 37
   <212> PRT
   <213> Artificial Sequence
<220>
   <223> Ciz1 b-variant peptide comprising an exon 14b/exon 15 junction
<400> 540
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr
           35
<210> 541
   <211> 36
   <212> PRT
   <213> Artificial Sequence
<220>
   <223> Ciz1 b-variant peptide comprising an exon 14b/exon 15 junction
<400> 541
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr
           35
<210> 542
   <211> 35
   <212> PRT
   <213> Artificial Sequence
<220>
   <223> Ciz1 b-variant peptide comprising an exon 14b/exon 15 junction
<400> 542
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr
           35
<210> 543
   <211> 34
   <212> PRT
   <213> Artificial Sequence
<220>
   <223> Ciz1 b-variant peptide comprising an exon 14b/exon 15 junction
<400> 543
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr
<210> 544
   <211> 33
   <212> PRT
   <213> Artificial Sequence
<220>
   <223> Ciz1 b-variant peptide comprising an exon 14b/exon 15 junction
<400> 544
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr
<210> 545
   <211> 32
   <212> PRT
   <213> Artificial Sequence
<220>
   <223> Ciz1 b-variant peptide comprising an exon 14b/exon 15 junction
<400> 545
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
<210> 546
   <211> 31
   <212> PRT
   <213> Artificial Sequence
<220>
   <223> Ciz1 b-variant peptide comprising an exon 14b/exon 15 junction
<400> 546
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr
               20                  25                  30
<210> 547
   <211> 30
   <212> PRT
   <213> Artificial Sequence
<220>
   <223> Ciz1 b-variant peptide comprising an exon 14b/exon 15 junction
<400> 547
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr
               20                  25                  30
<210> 548
   <211> 29
   <212> PRT
   <213> Artificial Sequence
<220>
   <223> Ciz1 b-variant peptide comprising an exon 14b/exon 15 junction
<400> 548
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr
               20                  25
<210> 549
   <211> 28
   <212> PRT
   <213> Artificial Sequence
<220>
   <223> Ciz1 b-variant peptide comprising an exon 14b/exon 15 junction
<400> 549
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr
               20                  25
<210> 550
   <211> 27
   <212> PRT
   <213> Artificial Sequence
<220>
   <223> Ciz1 b-variant peptide comprising an exon 14b/exon 15 junction
<400> 550
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr
               20                  25
<210> 551
   <211> 26
   <212> PRT
   <213> Artificial Sequence
<220>
   <223> Ciz1 b-variant peptide comprising an exon 14b/exon 15 junction
<400> 551
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr
               20                  25
<210> 552
   <211> 25
   <212> PRT
   <213> Artificial Sequence
<220>
   <223> Ciz1 b-variant peptide comprising an exon 14b/exon 15 junction
<400> 552
   Asp Asp Glu Asp Glu Glu Glu Ile Glu Val Arg Ser Arg Asp Ile Ser
   1               5                   10                  15
   Arg Glu Glu Trp Lys Gly Ser Glu Thr
               20                  25
<210> 553
   <211> 24
   <212> PRT
   <213> Artificial Sequence
<220>
   <223> Ciz1 b-variant peptide comprising an exon 14b/exon 15 junction
<400> 553
   Asp Glu Asp Glu Glu Glu Ile Glu Val Arg Ser Arg Asp Ile Ser Arg
   1 5 10 15
   Glu Glu Trp Lys Gly Ser Glu Thr
   20
<210> 554
   <211> 23
   <212> PRT
   <213> Artificial Sequence
<220>
   <223> Ciz1 b-variant peptide comprising an exon 14b/exon 15 junction
<400> 554
   Glu Asp Glu Glu Glu Ile Glu Val Arg Ser Arg Asp Ile Ser Arg Glu
   1 5 10 15
   Glu Trp Lys Gly Ser Glu Thr
   20
<210> 555
   <211> 22
   <212> PRT
   <213> Artificial Sequence
<220>
   <223> Ciz1 b-variant peptide comprising an exon 14b/exon 15 junction
<400> 555
   Asp Glu Glu Glu Ile Glu Val Arg Ser Arg Asp Ile Ser Arg Glu Glu
   1 5 10 15
   Trp Lys Gly Ser Glu Thr
   20
<210> 556
   <211> 21
   <212> PRT
   <213> Artificial Sequence
<220>
   <223> Ciz1 b-variant peptide comprising an exon 14b/exon 15 junction
<400> 556
   Glu Glu Glu Ile Glu Val Arg Ser Arg Asp Ile Ser Arg Glu Glu Trp
   1               5                   10                  15
   Lys Gly Ser Glu Thr
               20
<210> 557
   <211> 20
   <212> PRT
   <213> Artificial Sequence
<220>
   <223> Ciz1 b-variant peptide comprising an exon 14b/exon 15 junction
<400> 557
   Glu Glu Ile Glu Val Arg Ser Arg Asp Ile Ser Arg Glu Glu Trp Lys
   1               5                   10                  15
   Gly Ser Glu Thr
               20
<210> 558
   <211> 19
   <212> PRT
   <213> Artificial Sequence
<220>
   <223> Ciz1 b-variant peptide comprising an exon 14b/exon 15 junction
<400> 558
   Glu Ile Glu Val Arg Ser Arg Asp Ile Ser Arg Glu Glu Trp Lys Gly
   1               5                   10                  15
   Ser Glu Thr
<210> 559
   <211> 18
   <212> PRT
   <213> Artificial Sequence
<220>
   <223> Ciz1 b-variant peptide comprising an exon 14b/exon 15 junction
<400> 559
   Ile Glu Val Arg Ser Arg Asp Ile Ser Arg Glu Glu Trp Lys Gly Ser
   1               5                   10                  15
   Glu Thr
<210> 560
   <211> 17
   <212> PRT
   <213> Artificial Sequence
<220>
   <223> Ciz1 b-variant peptide comprising an exon 14b/exon 15 junction
<400> 560
   Glu Val Arg Ser Arg Asp Ile Ser Arg Glu Glu Trp Lys Gly Ser Glu
   1               5                   10                  15
   Thr
<210> 561
   <211> 53
   <212> PRT
   <213> Artificial Sequence
<220>
   <223> Ciz1 b-variant peptide comprising an exon 14b/exon 15 junction
<400> 561
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr
       50
<210> 562
   <211> 52
   <212> PRT
   <213> Artificial Sequence
<220>
   <223> Ciz1 b-variant peptide comprising an exon 14b/exon 15 junction
<400> 562
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr
       50
<210> 563
   <211> 51
   <212> PRT
   <213> Artificial Sequence
<220>
   <223> Ciz1 b-variant peptide comprising an exon 14b/exon 15 junction
<400> 563
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr
       50
<210> 564
   <211> 50
   <212> PRT
   <213> Artificial Sequence
<220>
   <223> Ciz1 b-variant peptide comprising an exon 14b/exon 15 junction
<400> 564
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr
       50
<210> 565
   <211> 49
   <212> PRT
   <213> Artificial Sequence
<220>
   <223> Ciz1 b-variant peptide comprising an exon 14b/exon 15 junction
<400> 565
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr
<210> 566
   <211> 48
   <212> PRT
   <213> Artificial Sequence
<220>
   <223> Ciz1 b-variant peptide comprising an exon 14b/exon 15 junction
<400> 566
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu Thr Tyr
           35                  40                  45
<210> 567
   <211> 47
   <212> PRT
   <213> Artificial Sequence
<220>
   <223> Ciz1 b-variant peptide comprising an exon 14b/exon 15 junction
<400> 567
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu Thr Tyr
           35                  40                  45
<210> 568
   <211> 46
   <212> PRT
   <213> Artificial Sequence
<220>
   <223> Ciz1 b-variant peptide comprising an exon 14b/exon 15 junction
<400> 568
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr
           35                  40                  45
<210> 569
   <211> 45
   <212> PRT
   <213> Artificial Sequence
<220>
   <223> Ciz1 b-variant peptide comprising an exon 14b/exon 15 junction
<400> 569
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr
           35                  40                  45
<210> 570
   <211> 44
   <212> PRT
   <213> Artificial Sequence
<220>
   <223> Ciz1 b-variant peptide comprising an exon 14b/exon 15 junction
<400> 570
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr
           35                  40
<210> 571
   <211> 43
   <212> PRT
   <213> Artificial Sequence
<220>
   <223> Ciz1 b-variant peptide comprising an exon 14b/exon 15 junction
<400> 571
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
                       20                  25                  30
   Ser Arg Glu Glu Trp Lys Gly Ser Glu Thr Tyr
           35                  40
<210> 572
   <211> 42
   <212> PRT
   <213> Artificial Sequence
<220>
   <223> Ciz1 b-variant peptide comprising an exon 14b/exon 15 junction
<400> 572
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr
           35                  40
<210> 573
   <211> 41
   <212> PRT
   <213> Artificial Sequence
<220>
   <223> Ciz1 b-variant peptide comprising an exon 14b/exon 15 junction
<400> 573
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr
           35                  40
<210> 574
   <211> 40
   <212> PRT
   <213> Artificial Sequence
<220>
   <223> Ciz1 b-variant peptide comprising an exon 14b/exon 15 junction
<400> 574
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
   20 25 30
   Glu Trp Lys Gly Ser Glu Thr Tyr
   35 40
<210> 575
   <211> 39
   <212> PRT
   <213> Artificial Sequence
<220>
   <223> Ciz1 b-variant peptide comprising an exon 14b/exon 15 junction
<400> 575
   Ala Val Gly Cys Phe Glu Gly Asp Glu Glu Glu Glu Glu Asp Asp Glu
   1 5 10 15
   Asp Glu Glu Glu Ile Glu Val Arg Ser Arg Asp Ile Ser Arg Glu Glu
   20 25 30
   Trp Lys Gly Ser Glu Thr Tyr
   35
<210> 576
   <211> 38
   <212> PRT
   <213> Artificial Sequence
<220>
   <223> Ciz1 b-variant peptide comprising an exon 14b/exon 15 junction
<400> 576
   Val Gly Cys Phe Glu Gly Asp Glu Glu Glu Glu Glu Asp Asp Glu Asp
   1 5 10 15
   Glu Glu Glu Ile Glu Val Arg Ser Arg Asp Ile Ser Arg Glu Glu Trp
   20 25 30
   Lys Gly Ser Glu Thr Tyr
   35
<210> 577
   <211> 37
   <212> PRT
   <213> Artificial Sequence
<220>
   <223> Ciz1 b-variant peptide comprising an exon 14b/exon 15 junction
<400> 577
   Gly Cys Phe Glu Gly Asp Glu Glu Glu Glu Glu Asp Asp Glu Asp Glu
   1 5 10 15
   Glu Glu Ile Glu Val Arg Ser Arg Asp Ile Ser Arg Glu Glu Trp Lys
   20 25 30
   Gly Ser Glu Thr Tyr
   35
<210> 578
   <211> 36
   <212> PRT
   <213> Artificial Sequence
<220>
   <223> Ciz1 b-variant peptide comprising an exon 14b/exon 15 junction
<400> 578
   Cys Phe Glu Gly Asp Glu Glu Glu Glu Glu Asp Asp Glu Asp Glu Glu
   1 5 10 15
   Glu Ile Glu Val Arg Ser Arg Asp Ile Ser Arg Glu Glu Trp Lys Gly
   20 25 30
   Ser Glu Thr Tyr
   35
<210> 579
   <211> 35
   <212> PRT
   <213> Artificial Sequence
<220>
   <223> Ciz1 b-variant peptide comprising an exon 14b/exon 15 junction
<400> 579
   Phe Glu Gly Asp Glu Glu Glu Glu Glu Asp Asp Glu Asp Glu Glu Glu
   1 5 10 15
   Ile Glu Val Arg Ser Arg Asp Ile Ser Arg Glu Glu Trp Lys Gly Ser
   20 25 30
   Glu Thr Tyr
   35
<210> 580
   <211> 34
   <212> PRT
   <213> Artificial Sequence
<220>
<210> 581
   <211> 33
   <212> PRT
   <213> Artificial Sequence
<220>
   <223> Ciz1 b-variant peptide comprising an exon 14b/exon 15 junction
<400> 581
   Gly Asp Glu Glu Glu Glu Glu Asp Asp Glu Asp Glu Glu Glu Ile Glu
   1 5 10 15
   Val Arg Ser Arg Asp Ile Ser Arg Glu Glu Trp Lys Gly Ser Glu Thr
   20 25 30
   Tyr
<210> 582
   <211> 32
   <212> PRT
   <213> Artificial Sequence
<220>
   <223> Ciz1 b-variant peptide comprising an exon 14b/exon 15 junction
<400> 582
   Asp Glu Glu Glu Glu Glu Asp Asp Glu Asp Glu Glu Glu Ile Glu Val
   1 5 10 15
   Arg Ser Arg Asp Ile Ser Arg Glu Glu Trp Lys Gly Ser Glu Thr Tyr
   20 25 30
<210> 583
   <211> 31
   <212> PRT
   <213> Artificial Sequence
<220>
   <223> Ciz1 b-variant peptide comprising an exon 14b/exon 15 junction
<400> 583
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr Tyr
               20                  25                  30
<210> 584
   <211> 30
   <212> PRT
   <213> Artificial Sequence
<220>
   <223> Ciz1 b-variant peptide comprising an exon 14b/exon 15 junction
<400> 584
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr
               20                  25                  30
<210> 585
   <211> 29
   <212> PRT
   <213> Artificial Sequence
<220>
   <223> Ciz1 b-variant peptide comprising an exon 14b/exon 15 junction
<400> 585
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr
               20                  25
<210> 586
   <211> 28
   <212> PRT
   <213> Artificial Sequence
<220>
   <223> Ciz1 b-variant peptide comprising an exon 14b/exon 15 junction
<400> 586
   Glu Glu Asp Asp Glu Asp Glu Glu Glu Ile Glu Val Arg Ser Arg Asp
   1 5 10 15
   Ile Ser Arg Glu Glu Trp Lys Gly Ser Glu Thr Tyr
   20 25
<210> 587
   <211> 27
   <212> PRT
   <213> Artificial Sequence
<220>
   <223> Ciz1 b-variant peptide comprising an exon 14b/exon 15 junction
<400> 587
   Glu Asp Asp Glu Asp Glu Glu Glu Ile Glu Val Arg Ser Arg Asp Ile
   1 5 10 15
   Ser Arg Glu Glu Trp Lys Gly Ser Glu Thr Tyr
   20 25
<210> 588
   <211> 26
   <212> PRT
   <213> Artificial Sequence
<220>
   <223> Ciz1 b-variant peptide comprising an exon 14b/exon 15 junction
<400> 588
   Asp Asp Glu Asp Glu Glu Glu Ile Glu Val Arg Ser Arg Asp Ile Ser
   1 5 10 15
   Arg Glu Glu Trp Lys Gly Ser Glu Thr Tyr
   20 25
<210> 589
   <211> 25
   <212> PRT
   <213> Artificial Sequence
<220>
   <223> Ciz1 b-variant peptide comprising an exon 14b/exon 15 junction
<400> 589
   Asp Glu Asp Glu Glu Glu Ile Glu Val Arg Ser Arg Asp Ile Ser Arg
   1 5 10 15
   Glu Glu Trp Lys Gly Ser Glu Thr Tyr
   20 25
<210> 590
   <211> 24
   <212> PRT
   <213> Artificial Sequence
<220>
   <223> Ciz1 b-variant peptide comprising an exon 14b/exon 15 junction
<400> 590
   Glu Asp Glu Glu Glu Ile Glu Val Arg Ser Arg Asp Ile Ser Arg Glu
   1               5                   10                  15
   Glu Trp Lys Gly Ser Glu Thr Tyr
               20
<210> 591
   <211> 23
   <212> PRT
   <213> Artificial Sequence
<220>
   <223> Ciz1 b-variant peptide comprising an exon 14b/exon 15 junction
<400> 591
   Asp Glu Glu Glu Ile Glu Val Arg Ser Arg Asp Ile Ser Arg Glu Glu
   1               5                   10                  15
   Trp Lys Gly Ser Glu Thr Tyr
               20
<210> 592
   <211> 22
   <212> PRT
   <213> Artificial Sequence
<220>
   <223> Ciz1 b-variant peptide comprising an exon 14b/exon 15 junction
<400> 592
   Glu Glu Glu Ile Glu Val Arg Ser Arg Asp Ile Ser Arg Glu Glu Trp
   1               5                   10                  15
   Lys Gly Ser Glu Thr Tyr
               20
<210> 593
   <211> 21
   <212> PRT
   <213> Artificial Sequence
<220>
   <223> Ciz1 b-variant peptide comprising an exon 14b/exon 15 junction
<400> 593
   Glu Glu Ile Glu Val Arg Ser Arg Asp Ile Ser Arg Glu Glu Trp Lys
   1               5                   10                  15
   Gly Ser Glu Thr Tyr
               20
<210> 594
   <211> 20
   <212> PRT
   <213> Artificial Sequence
<220>
   <223> Ciz1 b-variant peptide comprising an exon 14b/exon 15 junction
<400> 594
   Glu Ile Glu Val Arg Ser Arg Asp Ile Ser Arg Glu Glu Trp Lys Gly
   1               5                   10                  15
   Ser Glu Thr Tyr
               20
<210> 595
   <211> 19
   <212> PRT
   <213> Artificial Sequence
<220>
   <223> Ciz1 b-variant peptide comprising an exon 14b/exon 15 junction
<400> 595
   Ile Glu Val Arg Ser Arg Asp Ile Ser Arg Glu Glu Trp Lys Gly Ser
   1               5                   10                  15
   Glu Thr Tyr
<210> 596
   <211> 18
   <212> PRT
   <213> Artificial Sequence
<220>
   <223> Ciz1 b-variant peptide comprising an exon 14b/exon 15 junction
<400> 596
   Glu Val Arg Ser Arg Asp Ile Ser Arg Glu Glu Trp Lys Gly Ser Glu
   1               5                   10                  15
   Thr Tyr
<210> 597
   <211> 54
   <212> PRT
   <213> Artificial Sequence
<220>
   <223> Ciz1 b-variant peptide comprising an exon 14b/exon 15 junction
<400> 597
   Glu Ile Ala Gly Gln Asp Glu Asp His Phe Ile Thr Val Asp Ala Val
   1 5 10 15
   Gly Cys Phe Glu Gly Asp Glu Glu Glu Glu Glu Asp Asp Glu Asp Glu
   20 25 30
   Glu Glu Ile Glu Val Arg Ser Arg Asp Ile Ser Arg Glu Glu Trp Lys
   35 40 45
   Gly Ser Glu Thr Tyr Ser
   50
<210> 598
   <211> 53
   <212> PRT
   <213> Artificial Sequence
<220>
   <223> Ciz1 b-variant peptide comprising an exon 14b/exon 15 junction
<400> 598
   Ile Ala Gly Gln Asp Glu Asp His Phe Ile Thr Val Asp Ala Val Gly
   1 5 10 15
   Cys Phe Glu Gly Asp Glu Glu Glu Glu Glu Asp Asp Glu Asp Glu Glu
   20 25 30
   Glu Ile Glu Val Arg Ser Arg Asp Ile Ser Arg Glu Glu Trp Lys Gly
   35 40 45
   Ser Glu Thr Tyr Ser
   50
<210> 599
   <211> 52
   <212> PRT
   <213> Artificial Sequence
<220>
   <223> Ciz1 b-variant peptide comprising an exon 14b/exon 15 junction
<400> 599
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser
       50
<210> 600
   <211> 51
   <212> PRT
   <213> Artificial Sequence
<220>
   <223> Ciz1 b-variant peptide comprising an exon 14b/exon 15 junction
<400> 600
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr Ser
       50
<210> 601
   <211> 50
   <212> PRT
   <213> Artificial Sequence
<220>
   <223> Ciz1 b-variant peptide comprising an exon 14b/exon 15 junction
<400> 601
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr Ser
       50
<210> 602
   <211> 49
   <212> PRT
   <213> Artificial Sequence
<220>
   <223> Ciz1 b-variant peptide comprising an exon 14b/exon 15 junction
<400> 602
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu Thr Tyr
           35                  40                  45
   Ser
<210> 603
   <211> 48
   <212> PRT
   <213> Artificial Sequence
<220>
   <223> Ciz1 b-variant peptide comprising an exon 14b/exon 15 junction
<400> 603
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu Thr Tyr Ser
           35                  40                  45
<210> 604
   <211> 47
   <212> PRT
   <213> Artificial Sequence
<220>
   <223> Ciz1 b-variant peptide comprising an exon 14b/exon 15 junction
<400> 604
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr Ser
           35                  40                  45
<210> 605
   <211> 46
   <212> PRT
   <213> Artificial Sequence
<220>
   <223> Ciz1 b-variant peptide comprising an exon 14b/exon 15 junction
<400> 605
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser
           35                  40                  45
<210> 606
   <211> 45
   <212> PRT
   <213> Artificial Sequence
<220>
   <223> Ciz1 b-variant peptide comprising an exon 14b/exon 15 junction
<400> 606
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser
           35                  40                  45
<210> 607
   <211> 44
   <212> PRT
   <213> Artificial Sequence
<220>
   <223> Ciz1 b-variant peptide comprising an exon 14b/exon 15 junction
<400> 607
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
                   20                  25                  30
   Ser Arg Glu Glu Trp Lys Gly Ser Glu Thr Tyr Ser
           35                  40
<210> 608
   <211> 43
   <212> PRT
   <213> Artificial Sequence
<220>
   <223> Ciz1 b-variant peptide comprising an exon 14b/exon 15 junction
<400> 608
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser
           35                  40
<210> 609
   <211> 42
   <212> PRT
   <213> Artificial Sequence
<220>
   <223> Ciz1 b-variant peptide comprising an exon 14b/exon 15 junction
<400> 609
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser
           35                  40
<210> 610
   <211> 41
   <212> PRT
   <213> Artificial Sequence
<220>
   <223> Ciz1 b-variant peptide comprising an exon 14b/exon 15 junction
<400> 610
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser
           35                  40
<210> 611
   <211> 40
   <212> PRT
   <213> Artificial Sequence
<220>
   <223> Ciz1 b-variant peptide comprising an exon 14b/exon 15 junction
<400> 611
   Ala Val Gly Cys Phe Glu Gly Asp Glu Glu Glu Glu Glu Asp Asp Glu
   1 5 10 15
   Asp Glu Glu Glu Ile Glu Val Arg Ser Arg Asp Ile Ser Arg Glu Glu
   20 25 30
   Trp Lys Gly Ser Glu Thr Tyr Ser
   35 40
<210> 612
   <211> 39
   <212> PRT
   <213> Artificial Sequence
<220>
   <223> Ciz1 b-variant peptide comprising an exon 14b/exon 15 junction
<400> 612
   Val Gly Cys Phe Glu Gly Asp Glu Glu Glu Glu Glu Asp Asp Glu Asp
   1 5 10 15
   Glu Glu Glu Ile Glu Val Arg Ser Arg Asp Ile Ser Arg Glu Glu Trp
   20 25 30
   Lys Gly Ser Glu Thr Tyr Ser
   35
<210> 613
   <211> 38
   <212> PRT
   <213> Artificial Sequence
<220>
   <223> Ciz1 b-variant peptide comprising an exon 14b/exon 15 junction
<400> 613
   Gly Cys Phe Glu Gly Asp Glu Glu Glu Glu Glu Asp Asp Glu Asp Glu
   1 5 10 15
   Glu Glu Ile Glu Val Arg Ser Arg Asp Ile Ser Arg Glu Glu Trp Lys
   20 25 30
   Gly Ser Glu Thr Tyr Ser
   35
<210> 614
   <211> 37
   <212> PRT
   <213> Artificial Sequence
<220>
   <223> Ciz1 b-variant peptide comprising an exon 14b/exon 15 junction
<400> 614
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser
           35
<210> 615
   <211> 36
   <212> PRT
   <213> Artificial Sequence
<220>
   <223> Ciz1 b-variant peptide comprising an exon 14b/exon 15 junction
<400> 615
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser
           35
<210> 616
   <211> 35
   <212> PRT
   <213> Artificial Sequence
<220>
   <223> Ciz1 b-variant peptide comprising an exon 14b/exon 15 junction
<400> 616
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser
           35
<210> 617
   <211> 34
   <212> PRT
   <213> Artificial Sequence
<220>
   <223> Ciz1 b-variant peptide comprising an exon 14b/exon 15 junction
<400> 617
   Gly Asp Glu Glu Glu Glu Glu Asp Asp Glu Asp Glu Glu Glu Ile Glu
   1 5 10 15
   Val Arg Ser Arg Asp Ile Ser Arg Glu Glu Trp Lys Gly Ser Glu Thr
   20 25 30
   Tyr Ser
<210> 618
   <211> 33
   <212> PRT
   <213> Artificial Sequence
<220>
   <223> Ciz1 b-variant peptide comprising an exon 14b/exon 15 junction
<400> 618
   Asp Glu Glu Glu Glu Glu Asp Asp Glu Asp Glu Glu Glu Ile Glu Val
   1 5 10 15
   Arg Ser Arg Asp Ile Ser Arg Glu Glu Trp Lys Gly Ser Glu Thr Tyr
   20 25 30
   Ser
<210> 619
   <211> 32
   <212> PRT
   <213> Artificial Sequence
<220>
   <223> Ciz1 b-variant peptide comprising an exon 14b/exon 15 junction
<400> 619
   Glu Glu Glu Glu Glu Asp Asp Glu Asp Glu Glu Glu Ile Glu Val Arg
   1 5 10 15
   Ser Arg Asp Ile Ser Arg Glu Glu Trp Lys Gly Ser Glu Thr Tyr Ser
   20 25 30
<210> 620
   <211> 31
   <212> PRT
   <213> Artificial Sequence
<220>
   <223> Ciz1 b-variant peptide comprising an exon 14b/exon 15 junction
<400> 620
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser
               20                  25                  30
<210> 621
   <211> 30
   <212> PRT
   <213> Artificial Sequence
<220>
   <223> Ciz1 b-variant peptide comprising an exon 14b/exon 15 junction
<400> 621
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser
               20                  25                  30
<210> 622
   <211> 29
   <212> PRT
   <213> Artificial Sequence
<220>
   <223> Ciz1 b-variant peptide comprising an exon 14b/exon 15 junction
<400> 622
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser
               20                  25
<210> 623
   <211> 28
   <212> PRT
   <213> Artificial Sequence
<220>
   <223> Ciz1 b-variant peptide comprising an exon 14b/exon 15 junction
<400> 623
   Glu Asp Asp Glu Asp Glu Glu Glu Ile Glu Val Arg Ser Arg Asp Ile
   1 5 10 15
   Ser Arg Glu Glu Trp Lys Gly Ser Glu Thr Tyr Ser
   20 25
<210> 624
   <211> 27
   <212> PRT
   <213> Artificial Sequence
<220>
   <223> Ciz1 b-variant peptide comprising an exon 14b/exon 15 junction
<400> 624
   Asp Asp Glu Asp Glu Glu Glu Ile Glu Val Arg Ser Arg Asp Ile Ser
   1 5 10 15
   Arg Glu Glu Trp Lys Gly Ser Glu Thr Tyr Ser
   20 25
<210> 625
   <211> 26
   <212> PRT
   <213> Artificial Sequence
<220>
   <223> Ciz1 b-variant peptide comprising an exon 14b/exon 15 junction
<400> 625
   Asp Glu Asp Glu Glu Glu Ile Glu Val Arg Ser Arg Asp Ile Ser Arg
   1 5 10 15
   Glu Glu Trp Lys Gly Ser Glu Thr Tyr Ser
   20 25
<210> 626
   <211> 25
   <212> PRT
   <213> Artificial Sequence
<220>
   <223> Ciz1 b-variant peptide comprising an exon 14b/exon 15 junction
<400> 626
   Glu Asp Glu Glu Glu Ile Glu Val Arg Ser Arg Asp Ile Ser Arg Glu
   1 5 10 15
   Glu Trp Lys Gly Ser Glu Thr Tyr Ser
   20 25
<210> 627
   <211> 24
   <212> PRT
   <213> Artificial Sequence
<220>
   <223> Ciz1 b-variant peptide comprising an exon 14b/exon 15 junction
<400> 627
   Asp Glu Glu Glu Ile Glu Val Arg Ser Arg Asp Ile Ser Arg Glu Glu
   1               5                   10                  15
   Trp Lys Gly Ser Glu Thr Tyr Ser
               20
<210> 628
   <211> 23
   <212> PRT
   <213> Artificial Sequence
<220>
   <223> Ciz1 b-variant peptide comprising an exon 14b/exon 15 junction
<400> 628
   Glu Glu Glu Ile Glu Val Arg Ser Arg Asp Ile Ser Arg Glu Glu Trp
   1               5                   10                  15
   Lys Gly Ser Glu Thr Tyr Ser
               20
<210> 629
   <211> 22
   <212> PRT
   <213> Artificial Sequence
<220>
   <223> Ciz1 b-variant peptide comprising an exon 14b/exon 15 junction
<400> 629
   Glu Glu Ile Glu Val Arg Ser Arg Asp Ile Ser Arg Glu Glu Trp Lys
   1               5                   10                  15
   Gly Ser Glu Thr Tyr Ser
               20
<210> 630
   <211> 21
   <212> PRT
   <213> Artificial Sequence
<220>
   <223> Ciz1 b-variant peptide comprising an exon 14b/exon 15 junction
<400> 630
   Glu Ile Glu Val Arg Ser Arg Asp Ile Ser Arg Glu Glu Trp Lys Gly
   1               5                   10                  15
   Ser Glu Thr Tyr Ser
               20
<210> 631
   <211> 20
   <212> PRT
   <213> Artificial Sequence
<220>
   <223> Ciz1 b-variant peptide comprising an exon 14b/exon 15 junction
<400> 631
   Ile Glu Val Arg Ser Arg Asp Ile Ser Arg Glu Glu Trp Lys Gly Ser
   1               5                   10                  15
   Glu Thr Tyr Ser
               20
<210> 632
   <211> 19
   <212> PRT
   <213> Artificial Sequence
<220>
   <223> Ciz1 b-variant peptide comprising an exon 14b/exon 15 junction
<400> 632
   Glu Val Arg Ser Arg Asp Ile Ser Arg Glu Glu Trp Lys Gly Ser Glu
   1               5                   10                  15
   Thr Tyr Ser
<210> 633
   <211> 55
   <212> PRT
   <213> Artificial Sequence
<220>
   <223> Ciz1 b-variant peptide comprising an exon 14b/exon 15 junction
<400> 633
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro
       50                  55
<210> 634
   <211> 54
   <212> PRT
   <213> Artificial Sequence
<220>
   <223> Ciz1 b-variant peptide comprising an exon 14b/exon 15 junction
<400> 634
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro
       50
<210> 635
   <211> 53
   <212> PRT
   <213> Artificial Sequence
<220>
   <223> Ciz1 b-variant peptide comprising an exon 14b/exon 15 junction
<400> 635
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
   20 25 30
   Ile Glu Val Arg Ser Arg Asp Ile Ser Arg Glu Glu Trp Lys Gly Ser
   35 40 45
   Glu Thr Tyr Ser Pro
   50
<210> 636
   <211> 52
   <212> PRT
   <213> Artificial Sequence
<220>
   <223> Ciz1 b-variant peptide comprising an exon 14b/exon 15 junction
<400> 636
   Gly Gln Asp Glu Asp His Phe Ile Thr Val Asp Ala Val Gly Cys Phe
   1 5 10 15
   Glu Gly Asp Glu Glu Glu Glu Glu Asp Asp Glu Asp Glu Glu Glu Ile
   20 25 30
   Glu Val Arg Ser Arg Asp Ile Ser Arg Glu Glu Trp Lys Gly Ser Glu
   35 40 45
   Thr Tyr Ser Pro
   50
<210> 637
   <211> 51
   <212> PRT
   <213> Artificial Sequence
<220>
   <223> Ciz1 b-variant peptide comprising an exon 14b/exon 15 junction
<400> 637
   Gln Asp Glu Asp His Phe Ile Thr Val Asp Ala Val Gly Cys Phe Glu
   1 5 10 15
   Gly Asp Glu Glu Glu Glu Glu Asp Asp Glu Asp Glu Glu Glu Ile Glu
   20 25 30
   Val Arg Ser Arg Asp Ile Ser Arg Glu Glu Trp Lys Gly Ser Glu Thr
   35 40 45
   Tyr Ser Pro
   50
<210> 638
   <211> 50
   <212> PRT
   <213> Artificial Sequence
<220>
   <223> Ciz1 b-variant peptide comprising an exon 14b/exon 15 junction
<400> 638
   Asp Glu Asp His Phe Ile Thr Val Asp Ala Val Gly Cys Phe Glu Gly
   1 5 10 15
   Asp Glu Glu Glu Glu Glu Asp Asp Glu Asp Glu Glu Glu Ile Glu Val
   20 25 30
   Arg Ser Arg Asp Ile Ser Arg Glu Glu Trp Lys Gly Ser Glu Thr Tyr
   35 40 45
   Ser Pro
   50
<210> 639
   <211> 49
   <212> PRT
   <213> Artificial Sequence
<220>
   <223> Ciz1 b-variant peptide comprising an exon 14b/exon 15 junction
<400> 639
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu Thr Tyr Ser
           35                  40                  45
   Pro
<210> 640
   <211> 48
   <212> PRT
   <213> Artificial Sequence
<220>
   <223> Ciz1 b-variant peptide comprising an exon 14b/exon 15 junction
<400> 640
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr Ser Pro
           35                  40                  45
<210> 641
   <211> 47
   <212> PRT
   <213> Artificial Sequence
<220>
   <223> Ciz1 b-variant peptide comprising an exon 14b/exon 15 junction
<400> 641
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser Pro
           35                  40                  45
<210> 642
   <211> 46
   <212> PRT
   <213> Artificial Sequence
<220>
   <223> Ciz1 b-variant peptide comprising an exon 14b/exon 15 junction
<400> 642
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro
           35                  40                  45
<210> 643
   <211> 45
   <212> PRT
   <213> Artificial Sequence
<220>
   <223> Ciz1 b-variant peptide comprising an exon 14b/exon 15 junction
<400> 643
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro
           35                  40                  45
<210> 644
   <211> 44
   <212> PRT
   <213> Artificial Sequence
<220>
   <223> Ciz1 b-variant peptide comprising an exon 14b/exon 15 junction
<400> 644
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro
           35                  40
<210> 645
   <211> 43
   <212> PRT
   <213> Artificial Sequence
<220>
   <223> Ciz1 b-variant peptide comprising an exon 14b/exon 15 junction
<400> 645
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro
           35                  40
<210> 646
   <211> 42
   <212> PRT
   <213> Artificial Sequence
<220>
   <223> Ciz1 b-variant peptide comprising an exon 14b/exon 15 junction
<400> 646
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro
           35                  40
<210> 647
   <211> 41
   <212> PRT
   <213> Artificial Sequence
<220>
   <223> Ciz1 b-variant peptide comprising an exon 14b/exon 15 junction
<400> 647
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr Tyr Ser Pro
           35                  40
<210> 648
   <211> 40
   <212> PRT
   <213> Artificial Sequence
<220>
   <223> Ciz1 b-variant peptide comprising an exon 14b/exon 15 junction
<400> 648
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro
           35                  40
<210> 649
   <211> 39
   <212> PRT
   <213> Artificial Sequence
<220>
   <223> Ciz1 b-variant peptide comprising an exon 14b/exon 15 junction
<400> 649
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro
           35
<210> 650
   <211> 38
   <212> PRT
   <213> Artificial Sequence
<220>
   <223> Ciz1 b-variant peptide comprising an exon 14b/exon 15 junction
<400> 650
   Cys Phe Glu Gly Asp Glu Glu Glu Glu Glu Asp Asp Glu Asp Glu Glu
   1 5 10 15
   Glu Ile Glu Val Arg Ser Arg Asp Ile Ser Arg Glu Glu Trp Lys Gly
   20 25 30
   Ser Glu Thr Tyr Ser Pro
   35
<210> 651
   <211> 37
   <212> PRT
   <213> Artificial Sequence
<220>
   <223> Ciz1 b-variant peptide comprising an exon 14b/exon 15 junction
<400> 651
   Phe Glu Gly Asp Glu Glu Glu Glu Glu Asp Asp Glu Asp Glu Glu Glu
   1 5 10 15
   Ile Glu Val Arg Ser Arg Asp Ile Ser Arg Glu Glu Trp Lys Gly Ser
   20 25 30
   Glu Thr Tyr Ser Pro
   35
<210> 652
   <211> 36
   <212> PRT
   <213> Artificial Sequence
<220>
   <223> Ciz1 b-variant peptide comprising an exon 14b/exon 15 junction
<400> 652
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro
           35
<210> 653
   <211> 35
   <212> PRT
   <213> Artificial Sequence
<220>
   <223> Ciz1 b-variant peptide comprising an exon 14b/exon 15 junction
<400> 653
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
   Tyr Ser Pro
           35
<210> 654
   <211> 34
   <212> PRT
   <213> Artificial Sequence
<220>
   <223> Ciz1 b-variant peptide comprising an exon 14b/exon 15 junction
<400> 654
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro
<210> 655
   <211> 33
   <212> PRT
   <213> Artificial Sequence
<220>
   <223> Ciz1 b-variant peptide comprising an exon 14b/exon 15 junction
<400> 655
   Glu Glu Glu Glu Glu Asp Asp Glu Asp Glu Glu Glu Ile Glu Val Arg
   1 5 10 15
   Ser Arg Asp Ile Ser Arg Glu Glu Trp Lys Gly Ser Glu Thr Tyr Ser
   20 25 30
   Pro
<210> 656
   <211> 32
   <212> PRT
   <213> Artificial Sequence
<220>
   <223> Ciz1 b-variant peptide comprising an exon 14b/exon 15 junction
<400> 656
   Glu Glu Glu Glu Asp Asp Glu Asp Glu Glu Glu Ile Glu Val Arg Ser
   1 5 10 15
   Arg Asp Ile Ser Arg Glu Glu Trp Lys Gly Ser Glu Thr Tyr Ser Pro
   20 25 30
<210> 657
   <211> 31
   <212> PRT
   <213> Artificial Sequence
<220>
   <223> Ciz1 b-variant peptide comprising an exon 14b/exon 15 junction
<400> 657
   Glu Glu Glu Asp Asp Glu Asp Glu Glu Glu Ile Glu Val Arg Ser Arg
   1 5 10 15
   Asp Ile Ser Arg Glu Glu Trp Lys Gly Ser Glu Thr Tyr Ser Pro
   20 25 30
<210> 658
   <211> 30
   <212> PRT
   <213> Artificial Sequence
<220>
   <223> Ciz1 b-variant peptide comprising an exon 14b/exon 15 junction
<400> 658
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro
               20                  25                  30
<210> 659
   <211> 29
   <212> PRT
   <213> Artificial Sequence
<220>
   <223> Ciz1 b-variant peptide comprising an exon 14b/exon 15 junction
<400> 659
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro
               20                  25
<210> 660
   <211> 28
   <212> PRT
   <213> Artificial Sequence
<220>
   <223> Ciz1 b-variant peptide comprising an exon 14b/exon 15 junction
<400> 660
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro
               20                  25
<210> 661
   <211> 27
   <212> PRT
   <213> Artificial Sequence
<220>
   <223> Ciz1 b-variant peptide comprising an exon 14b/exon 15 junction
<400> 661
   Asp Glu Asp Glu Glu Glu Ile Glu Val Arg Ser Arg Asp Ile Ser Arg
   1               5                   10                  15
   Glu Glu Trp Lys Gly Ser Glu Thr Tyr Ser Pro
               20                  25
<210> 662
   <211> 26
   <212> PRT
   <213> Artificial Sequence
<220>
   <223> Ciz1 b-variant peptide comprising an exon 14b/exon 15 junction
<400> 662
   Glu Asp Glu Glu Glu Ile Glu Val Arg Ser Arg Asp Ile Ser Arg Glu
   1               5                   10                  15
   Glu Trp Lys Gly Ser Glu Thr Tyr Ser Pro
               20                  25
<210> 663
   <211> 25
   <212> PRT
   <213> Artificial Sequence
<220>
   <223> Ciz1 b-variant peptide comprising an exon 14b/exon 15 junction
<400> 663
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro
               20                  25
<210> 664
   <211> 24
   <212> PRT
   <213> Artificial Sequence
<220>
   <223> Ciz1 b-variant peptide comprising an exon 14b/exon 15 junction
<400> 664
   Glu Glu Glu Ile Glu Val Arg Ser Arg Asp Ile Ser Arg Glu Glu Trp
   1               5                   10                  15
   Lys Gly Ser Glu Thr Tyr Ser Pro
               20
<210> 665
   <211> 23
   <212> PRT
   <213> Artificial Sequence
<220>
   <223> Ciz1 b-variant peptide comprising an exon 14b/exon 15 junction
<400> 665
   Glu Glu Ile Glu Val Arg Ser Arg Asp Ile Ser Arg Glu Glu Trp Lys
   1               5                   10                  15
   Gly Ser Glu Thr Tyr Ser Pro
               20
<210> 666
   <211> 22
   <212> PRT
   <213> Artificial Sequence
<220>
   <223> Ciz1 b-variant peptide comprising an exon 14b/exon 15 junction
<400> 666
   Glu Ile Glu Val Arg Ser Arg Asp Ile Ser Arg Glu Glu Trp Lys Gly
   1               5                   10                  15
   Ser Glu Thr Tyr Ser Pro
               20
<210> 667
   <211> 21
   <212> PRT
   <213> Artificial Sequence
<220>
   <223> Ciz1 b-variant peptide comprising an exon 14b/exon 15 junction
<400> 667
   Ile Glu Val Arg Ser Arg Asp Ile Ser Arg Glu Glu Trp Lys Gly Ser
   1               5                   10                  15
   Glu Thr Tyr Ser Pro
               20
<210> 668
   <211> 20
   <212> PRT
   <213> Artificial Sequence
<220>
   <223> Ciz1 b-variant peptide comprising an exon 14b/exon 15 junction
<400> 668
   Glu Val Arg Ser Arg Asp Ile Ser Arg Glu Glu Trp Lys Gly Ser Glu
   1 5 10 15
   Thr Tyr Ser Pro
   20
<210> 669
   <211> 56
   <212> PRT
   <213> Artificial Sequence
<220>
   <223> Ciz1 b-variant peptide comprising an exon 14b/exon 15 junction
<400> 669
   Glu Ile Ala Gly Gln Asp Glu Asp His Phe Ile Thr Val Asp Ala Val
   1 5 10 15
   Gly Cys Phe Glu Gly Asp Glu Glu Glu Glu Glu Asp Asp Glu Asp Glu
   20 25 30
   Glu Glu Ile Glu Val Arg Ser Arg Asp Ile Ser Arg Glu Glu Trp Lys
   35 40 45
   Gly Ser Glu Thr Tyr Ser Pro Asn
   50 55
<210> 670
   <211> 55
   <212> PRT
   <213> Artificial Sequence
<220>
   <223> Ciz1 b-variant peptide comprising an exon 14b/exon 15 junction
<400> 670
   Ile Ala Gly Gln Asp Glu Asp His Phe Ile Thr Val Asp Ala Val Gly
   1 5 10 15
   Cys Phe Glu Gly Asp Glu Glu Glu Glu Glu Asp Asp Glu Asp Glu Glu
   20 25 30
   Glu Ile Glu Val Arg Ser Arg Asp Ile Ser Arg Glu Glu Trp Lys Gly
   35 40 45
   Ser Glu Thr Tyr Ser Pro Asn
   50 55
<210> 671
   <211> 54
   <212> PRT
   <213> Artificial Sequence
<220>
   <223> Ciz1 b-variant peptide comprising an exon 14b/exon 15 junction
<400> 671
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser Pro Asn
       50
<210> 672
   <211> 53
   <212> PRT
   <213> Artificial Sequence
<220>
   <223> Ciz1 b-variant peptide comprising an exon 14b/exon 15 junction
<400> 672
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr Ser Pro Asn
       50
<210> 673
   <211> 52
   <212> PRT
   <213> Artificial Sequence
<220>
   <223> Ciz1 b-variant peptide comprising an exon 14b/exon 15 junction
<400> 673
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr Ser Pro Asn
       50
<210> 674
   <211> 51
   <212> PRT
   <213> Artificial Sequence
<220>
   <223> Ciz1 b-variant peptide comprising an exon 14b/exon 15 junction
<400> 674
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu Thr Tyr
           35                  40                  45
   Ser Pro Asn
       50
<210> 675
   <211> 50
   <212> PRT
   <213> Artificial Sequence
<220>
   <223> Ciz1 b-variant peptide comprising an exon 14b/exon 15 junction
<400> 675
   Glu Asp His Phe Ile Thr Val Asp Ala Val Gly Cys Phe Glu Gly Asp
   1 5 10 15
   Glu Glu Glu Glu Glu Asp Asp Glu Asp Glu Glu Glu Ile Glu Val Arg
   20 25 30
   Ser Arg Asp Ile Ser Arg Glu Glu Trp Lys Gly Ser Glu Thr Tyr Ser
   35 40 45
   Pro Asn
   50
<210> 676
   <211> 49
   <212> PRT
   <213> Artificial Sequence
<220>
   <223> Ciz1 b-variant peptide comprising an exon 14b/exon 15 junction
<400> 676
   Asp His Phe Ile Thr Val Asp Ala Val Gly Cys Phe Glu Gly Asp Glu
   1 5 10 15
   Glu Glu Glu Glu Asp Asp Glu Asp Glu Glu Glu Ile Glu Val Arg Ser
   20 25 30
   Arg Asp Ile Ser Arg Glu Glu Trp Lys Gly Ser Glu Thr Tyr Ser Pro
   35 40 45
   Asn
<210> 677
   <211> 48
   <212> PRT
   <213> Artificial Sequence
<220>
   <223> Ciz1 b-variant peptide comprising an exon 14b/exon 15 junction
<400> 677
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser Pro Asn
           35                  40                  45
<210> 678
   <211> 47
   <212> PRT
   <213> Artificial Sequence
<220>
   <223> Ciz1 b-variant peptide comprising an exon 14b/exon 15 junction
<400> 678
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro Asn
           35                  40                  45
<210> 679
   <211> 46
   <212> PRT
   <213> Artificial Sequence
<220>
   <223> Ciz1 b-variant peptide comprising an exon 14b/exon 15 junction
<400> 679
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn
           35                  40                  45
<210> 680
   <211> 45
   <212> PRT
   <213> Artificial Sequence
<220>
   <223> Ciz1 b-variant peptide comprising an exon 14b/exon 15 junction
<400> 680
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn
           35                  40                  45
<210> 681
   <211> 44
   <212> PRT
   <213> Artificial Sequence
<220>
   <223> Ciz1 b-variant peptide comprising an exon 14b/exon 15 junction
<400> 681
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn
           35                  40
<210> 682
   <211> 43
   <212> PRT
   <213> Artificial Sequence
<220>
   <223> Ciz1 b-variant peptide comprising an exon 14b/exon 15 junction
<400> 682
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro Asn
           35                  40
<210> 683
   <211> 42
   <212> PRT
   <213> Artificial Sequence
<220>
   <223> Ciz1 b-variant peptide comprising an exon 14b/exon 15 junction
<400> 683
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr Tyr Ser Pro Asn
           35                  40
<210> 684
   <211> 41
   <212> PRT
   <213> Artificial Sequence
<220>
   <223> Ciz1 b-variant peptide comprising an exon 14b/exon 15 junction
<400> 684
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro Asn
           35                  40
<210> 685
   <211> 40
   <212> PRT
   <213> Artificial Sequence
<220>
   <223> Ciz1 b-variant peptide comprising an exon 14b/exon 15 junction
<400> 685
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro Asn
           35                  40
<210> 686
   <211> 39
   <212> PRT
   <213> Artificial Sequence
<220>
   <223> Ciz1 b-variant peptide comprising an exon 14b/exon 15 junction
<400> 686
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn
           35
<210> 687
   <211> 38
   <212> PRT
   <213> Artificial Sequence
<220>
   <223> Ciz1 b-variant peptide comprising an exon 14b/exon 15 junction
<400> 687
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn
           35
<210> 688
   <211> 37
   <212> PRT
   <213> Artificial Sequence
<220>
   <223> Ciz1 b-variant peptide comprising an exon 14b/exon 15 junction
<400> 688
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn
           35
<210> 689
   <211> 36
   <212> PRT
   <213> Artificial Sequence
<220>
   <223> Ciz1 b-variant peptide comprising an exon 14b/exon 15 junction
<400> 689
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
   Tyr Ser Pro Asn
           35
<210> 690
   <211> 35
   <212> PRT
   <213> Artificial Sequence
<220>
   <223> Ciz1 b-variant peptide comprising an exon 14b/exon 15 junction
<400> 690
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro Asn
           35
<210> 691
   <211> 34
   <212> PRT
   <213> Artificial Sequence
<220>
   <223> Ciz1 b-variant peptide comprising an exon 14b/exon 15 junction
<400> 691
   Glu Glu Glu Glu Glu Asp Asp Glu Asp Glu Glu Glu Ile Glu Val Arg
   1 5 10 15
   Ser Arg Asp Ile Ser Arg Glu Glu Trp Lys Gly Ser Glu Thr Tyr Ser
   20 25 30
   Pro Asn
<210> 692
   <211> 33
   <212> PRT
   <213> Artificial Sequence
<220>
   <223> Ciz1 b-variant peptide comprising an exon 14b/exon 15 junction
<400> 692
   Glu Glu Glu Glu Asp Asp Glu Asp Glu Glu Glu Ile Glu Val Arg Ser
   1 5 10 15
   Arg Asp Ile Ser Arg Glu Glu Trp Lys Gly Ser Glu Thr Tyr Ser Pro
   20 25 30
   Asn
<210> 693
   <211> 32
   <212> PRT
   <213> Artificial Sequence
<220>
   <223> Ciz1 b-variant peptide comprising an exon 14b/exon 15 junction
<400> 693
   Glu Glu Glu Asp Asp Glu Asp Glu Glu Glu Ile Glu Val Arg Ser Arg
   1 5 10 15
   Asp Ile Ser Arg Glu Glu Trp Lys Gly Ser Glu Thr Tyr Ser Pro Asn
   20 25 30
<210> 694
   <211> 31
   <212> PRT
   <213> Artificial Sequence
<220>
   <223> Ciz1 b-variant peptide comprising an exon 14b/exon 15 junction
<400> 694
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn
               20                  25                  30
<210> 695
   <211> 30
   <212> PRT
   <213> Artificial Sequence
<220>
   <223> Ciz1 b-variant peptide comprising an exon 14b/exon 15 junction
<400> 695
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn
               20                  25                  30
<210> 696
   <211> 29
   <212> PRT
   <213> Artificial Sequence
<220>
   <223> Ciz1 b-variant peptide comprising an exon 14b/exon 15 junction
<400> 696
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn
               20                  25
<210> 697
   <211> 28
   <212> PRT
   <213> Artificial Sequence
<220>
   <223> Ciz1 b-variant peptide comprising an exon 14b/exon 15 junction
<400> 697
   Asp Glu Asp Glu Glu Glu Ile Glu Val Arg Ser Arg Asp Ile Ser Arg
   1               5                   10                  15
   Glu Glu Trp Lys Gly Ser Glu Thr Tyr Ser Pro Asn
               20                  25
<210> 698
   <211> 27
   <212> PRT
   <213> Artificial Sequence
<220>
   <223> Ciz1 b-variant peptide comprising an exon 14b/exon 15 junction
<400> 698
   Glu Asp Glu Glu Glu Ile Glu Val Arg Ser Arg Asp Ile Ser Arg Glu
   1               5                   10                  15
   Glu Trp Lys Gly Ser Glu Thr Tyr Ser Pro Asn
               20                  25
<210> 699
   <211> 26
   <212> PRT
   <213> Artificial Sequence
<220>
   <223> Ciz1 b-variant peptide comprising an exon 14b/exon 15 junction
<400> 699
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro Asn
               20                  25
<210> 700
   <211> 25
   <212> PRT
   <213> Artificial Sequence
<220>
   <223> Ciz1 b-variant peptide comprising an exon 14b/exon 15 junction
<400> 700
   Glu Glu Glu Ile Glu Val Arg Ser Arg Asp Ile Ser Arg Glu Glu Trp
   1               5                   10                  15
   Lys Gly Ser Glu Thr Tyr Ser Pro Asn
               20                  25
<210> 701
   <211> 24
   <212> PRT
   <213> Artificial Sequence
<220>
   <223> Ciz1 b-variant peptide comprising an exon 14b/exon 15 junction
<400> 701
   Glu Glu Ile Glu Val Arg Ser Arg Asp Ile Ser Arg Glu Glu Trp Lys
   1               5                   10                  15
   Gly Ser Glu Thr Tyr Ser Pro Asn
               20
<210> 702
   <211> 23
   <212> PRT
   <213> Artificial Sequence
<220>
   <223> Ciz1 b-variant peptide comprising an exon 14b/exon 15 junction
<400> 702
   Glu Ile Glu Val Arg Ser Arg Asp Ile Ser Arg Glu Glu Trp Lys Gly
   1               5                   10                  15
   Ser Glu Thr Tyr Ser Pro Asn
               20
<210> 703
   <211> 22
   <212> PRT
   <213> Artificial Sequence
<220>
   <223> Ciz1 b-variant peptide comprising an exon 14b/exon 15 junction
<400> 703
   Ile Glu Val Arg Ser Arg Asp Ile Ser Arg Glu Glu Trp Lys Gly Ser
   1               5                   10                  15
   Glu Thr Tyr Ser Pro Asn
               20
<210> 704
   <211> 21
   <212> PRT
   <213> Artificial Sequence
<220>
   <223> Ciz1 b-variant peptide comprising an exon 14b/exon 15 junction
<400> 704
   Glu Val Arg Ser Arg Asp Ile Ser Arg Glu Glu Trp Lys Gly Ser Glu
   1               5                   10                  15
   Thr Tyr Ser Pro Asn
               20
<210> 705
   <211> 57
   <212> PRT
   <213> Artificial Sequence
<220>
   <223> Ciz1 b-variant peptide comprising an exon 14b/exon 15 junction
<400> 705
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr
       50                  55
<210> 706
   <211> 56
   <212> PRT
   <213> Artificial Sequence
<220>
   <223> Ciz1 b-variant peptide comprising an exon 14b/exon 15 junction
<400> 706
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr
       50                  55
<210> 707
   <211> 55
   <212> PRT
   <213> Artificial Sequence
<220>
   <223> Ciz1 b-variant peptide comprising an exon 14b/exon 15 junction
<400> 707
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser Pro Asn Thr
       50                  55
<210> 708
   <211> 54
   <212> PRT
   <213> Artificial Sequence
<220>
   <223> Ciz1 b-variant peptide comprising an exon 14b/exon 15 junction
<400> 708
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
   35                  40                  45
   Thr Tyr Ser Pro Asn Thr
       50
<210> 709
   <211> 53
   <212> PRT
   <213> Artificial Sequence
<220>
   <223> Ciz1 b-variant peptide comprising an exon 14b/exon 15 junction
<400> 709
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr Ser Pro Asn Thr
       50
<210> 710
   <211> 52
   <212> PRT
   <213> Artificial Sequence
<220>
   <223> Ciz1 b-variant peptide comprising an exon 14b/exon 15 junction
<400> 710
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu Thr Tyr
           35                  40                  45
   Ser Pro Asn Thr
       50
<210> 711
   <211> 51
   <212> PRT
   <213> Artificial Sequence
<220>
   <223> Ciz1 b-variant peptide comprising an exon 14b/exon 15 junction
<400> 711
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu Thr Tyr Ser
           35                  40                  45
   Pro Asn Thr
       50
<210> 712
   <211> 50
   <212> PRT
   <213> Artificial Sequence
<220>
   <223> Ciz1 b-variant peptide comprising an exon 14b/exon 15 junction
<400> 712
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr Ser Pro
           35                  40                  45
   Asn Thr
       50
<210> 713
   <211> 49
   <212> PRT
   <213> Artificial Sequence
<220>
   <223> Ciz1 b-variant peptide comprising an exon 14b/exon 15 junction
<400> 713
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser Pro Asn
           35                  40                  45
   Thr
<210> 714
   <211> 48
   <212> PRT
   <213> Artificial Sequence
<220>
   <223> Ciz1 b-variant peptide comprising an exon 14b/exon 15 junction
<400> 714
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro Asn Thr
           35                  40                  45
<210> 715
   <211> 47
   <212> PRT
   <213> Artificial Sequence
<220>
   <223> Ciz1 b-variant peptide comprising an exon 14b/exon 15 junction
<400> 715
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr
           35                  40                  45
<210> 716
   <211> 46
   <212> PRT
   <213> Artificial Sequence
<220>
   <223> Ciz1 b-variant peptide comprising an exon 14b/exon 15 junction
<210> 717
   <211> 45
   <212> PRT
   <213> Artificial Sequence
<220>
   <223> Ciz1 b-variant peptide comprising an exon 14b/exon 15 junction
<400> 717
   Val Asp Ala Val Gly Cys Phe Glu Gly Asp Glu Glu Glu Glu Glu Asp
   1 5 10 15
   Asp Glu Asp Glu Glu Glu Ile Glu Val Arg Ser Arg Asp Ile Ser Arg
   20 25 30
   Glu Glu Trp Lys Gly Ser Glu Thr Tyr Ser Pro Asn Thr
   35 40 45
<210> 718
   <211> 44
   <212> PRT
   <213> Artificial Sequence
<220>
   <223> Ciz1 b-variant peptide comprising an exon 14b/exon 15 junction
<400> 718
   Asp Ala Val Gly Cys Phe Glu Gly Asp Glu Glu Glu Glu Glu Asp Asp
   1 5 10 15
   Glu Asp Glu Glu Glu Ile Glu Val Arg Ser Arg Asp Ile Ser Arg Glu
   20 25 30
   Glu Trp Lys Gly Ser Glu Thr Tyr Ser Pro Asn Thr
   35 40
<210> 719
   <211> 43
   <212> PRT
   <213> Artificial Sequence
<220>
   <223> Ciz1 b-variant peptide comprising an exon 14b/exon 15 junction
<400> 719
   Ala Val Gly Cys Phe Glu Gly Asp Glu Glu Glu Glu Glu Asp Asp Glu
   1 5 10 15
   Asp Glu Glu Glu Ile Glu Val Arg Ser Arg Asp Ile Ser Arg Glu Glu
   20 25 30
   Trp Lys Gly Ser Glu Thr Tyr Ser Pro Asn Thr
   35 40
<210> 720
   <211> 42
   <212> PRT
   <213> Artificial Sequence
<220>
   <223> Ciz1 b-variant peptide comprising an exon 14b/exon 15 junction
<400> 720
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro Asn Thr
           35                  40
<210> 721
   <211> 41
   <212> PRT
   <213> Artificial Sequence
<220>
   <223> Ciz1 b-variant peptide comprising an exon 14b/exon 15 junction
<400> 721
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro Asn Thr
           35                  40
<210> 722
   <211> 40
   <212> PRT
   <213> Artificial Sequence
<220>
   <223> Ciz1 b-variant peptide comprising an exon 14b/exon 15 junction
<400> 722
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn Thr
           35                  40
<210> 723
   <211> 39
   <212> PRT
   <213> Artificial Sequence
<220>
   <223> Ciz1 b-variant peptide comprising an exon 14b/exon 15 junction
<400> 723
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn Thr
           35
<210> 724
   <211> 38
   <212> PRT
   <213> Artificial Sequence
<220>
   <223> Ciz1 b-variant peptide comprising an exon 14b/exon 15 junction
<400> 724
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn Thr
           35
<210> 725
   <211> 37
   <212> PRT
   <213> Artificial Sequence
<220>
   <223> Ciz1 b-variant peptide comprising an exon 14b/exon 15 junction
<400> 725
   Gly Asp Glu Glu Glu Glu Glu Asp Asp Glu Asp Glu Glu Glu Ile Glu
   1 5 10 15
   Val Arg Ser Arg Asp Ile Ser Arg Glu Glu Trp Lys Gly Ser Glu Thr
   20 25 30
   Tyr Ser Pro Asn Thr
   35
<210> 726
   <211> 36
   <212> PRT
   <213> Artificial Sequence
<220>
   <223> Ciz1 b-variant peptide comprising an exon 14b/exon 15 junction
<400> 726
   Asp Glu Glu Glu Glu Glu Asp Asp Glu Asp Glu Glu Glu Ile Glu Val
   1 5 10 15
   Arg Ser Arg Asp Ile Ser Arg Glu Glu Trp Lys Gly Ser Glu Thr Tyr
   20 25 30
   Ser Pro Asn Thr
   35
<210> 727
   <211> 35
   <212> PRT
   <213> Artificial Sequence
<220>
   <223> Ciz1 b-variant peptide comprising an exon 14b/exon 15 junction
<400> 727
   Glu Glu Glu Glu Glu Asp Asp Glu Asp Glu Glu Glu Ile Glu Val Arg
   1 5 10 15
   Ser Arg Asp Ile Ser Arg Glu Glu Trp Lys Gly Ser Glu Thr Tyr Ser
   20 25 30
   Pro Asn Thr
   35
<210> 728
   <211> 34
   <212> PRT
   <213> Artificial Sequence
<220>
   <223> Ciz1 b-variant peptide comprising an exon 14b/exon 15 junction
<400> 728
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr
<210> 729
   <211> 33
   <212> PRT
   <213> Artificial Sequence
<220>
   <223> Ciz1 b-variant peptide comprising an exon 14b/exon 15 junction
<400> 729
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr
<210> 730
   <211> 32
   <212> PRT
   <213> Artificial Sequence
<220>
   <223> Ciz1 b-variant peptide comprising an exon 14b/exon 15 junction
<400> 730
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
<210> 731
   <211> 31
   <212> PRT
   <213> Artificial Sequence
<220>
   <223> Ciz1 b-variant peptide comprising an exon 14b/exon 15 junction
<400> 731
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr
               20                  25                  30
<210> 732
   <211> 30
   <212> PRT
   <213> Artificial Sequence
<220>
   <223> Ciz1 b-variant peptide comprising an exon 14b/exon 15 junction
<400> 732
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn Thr
               20                  25                  30
<210> 733
   <211> 29
   <212> PRT
   <213> Artificial Sequence
<220>
   <223> Ciz1 b-variant peptide comprising an exon 14b/exon 15 junction
<400> 733
   Asp Glu Asp Glu Glu Glu Ile Glu Val Arg Ser Arg Asp Ile Ser Arg
   1               5                   10                  15
   Glu Glu Trp Lys Gly Ser Glu Thr Tyr Ser Pro Asn Thr
               20                  25
<210> 734
   <211> 28
   <212> PRT
   <213> Artificial Sequence
<220>
   <223> Ciz1 b-variant peptide comprising an exon 14b/exon 15 junction
<400> 734
   Glu Asp Glu Glu Glu Ile Glu Val Arg Ser Arg Asp Ile Ser Arg Glu
   1               5                   10                  15
   Glu Trp Lys Gly Ser Glu Thr Tyr Ser Pro Asn Thr
               20                  25
<210> 735
   <211> 27
   <212> PRT
   <213> Artificial Sequence
<220>
   <223> Ciz1 b-variant peptide comprising an exon 14b/exon 15 junction
<400> 735
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro Asn Thr
               20                  25
<210> 736
   <211> 26
   <212> PRT
   <213> Artificial Sequence
<220>
   <223> Ciz1 b-variant peptide comprising an exon 14b/exon 15 junction
<400> 736
   Glu Glu Glu Ile Glu Val Arg Ser Arg Asp Ile Ser Arg Glu Glu Trp
   1               5                   10                  15
   Lys Gly Ser Glu Thr Tyr Ser Pro Asn Thr
               20                  25
<210> 737
   <211> 25
   <212> PRT
   <213> Artificial Sequence
<220>
   <223> Ciz1 b-variant peptide comprising an exon 14b/exon 15 junction
<400> 737
   Glu Glu Ile Glu Val Arg Ser Arg Asp Ile Ser Arg Glu Glu Trp Lys
   1               5                   10                  15
   Gly Ser Glu Thr Tyr Ser Pro Asn Thr
   20 25
<210> 738
   <211> 24
   <212> PRT
   <213> Artificial Sequence
<220>
   <223> Ciz1 b-variant peptide comprising an exon 14b/exon 15 junction
<400> 738
   Glu Ile Glu Val Arg Ser Arg Asp Ile Ser Arg Glu Glu Trp Lys Gly
   1               5                   10                  15
   Ser Glu Thr Tyr Ser Pro Asn Thr
               20
<210> 739
   <211> 23
   <212> PRT
   <213> Artificial Sequence
<220>
   <223> Ciz1 b-variant peptide comprising an exon 14b/exon 15 junction
<400> 739
   Ile Glu Val Arg Ser Arg Asp Ile Ser Arg Glu Glu Trp Lys Gly Ser
   1               5                   10                  15
   Glu Thr Tyr Ser Pro Asn Thr
               20
<210> 740
   <211> 22
   <212> PRT
   <213> Artificial Sequence
<220>
   <223> Ciz1 b-variant peptide comprising an exon 14b/exon 15 junction
<400> 740
   Glu Val Arg Ser Arg Asp Ile Ser Arg Glu Glu Trp Lys Gly Ser Glu
   1               5                   10                  15
   Thr Tyr Ser Pro Asn Thr
               20
<210> 741
   <211> 58
   <212> PRT
   <213> Artificial Sequence
<220>
   <223> Ciz1 b-variant peptide comprising an exon 14b/exon 15 junction
<400> 741
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala
       50                  55
<210> 742
   <211> 57
   <212> PRT
   <213> Artificial Sequence
<220>
   <223> Ciz1 b-variant peptide comprising an exon 14b/exon 15 junction
<400> 742
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala
       50                  55
<210> 743
   <211> 56
   <212> PRT
   <213> Artificial Sequence
<220>
   <223> Ciz1 b-variant peptide comprising an exon 14b/exon 15 junction
<400> 743
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser Pro Asn Thr Ala
       50                  55
<210> 744
   <211> 55
   <212> PRT
   <213> Artificial Sequence
<220>
   <223> Ciz1 b-variant peptide comprising an exon 14b/exon 15 junction
<400> 744
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr Ser Pro Asn Thr Ala
       50                  55
<210> 745
   <211> 54
   <212> PRT
   <213> Artificial Sequence
<220>
   <223> Ciz1 b-variant peptide comprising an exon 14b/exon 15 junction
<400> 745
   Gln Asp Glu Asp His Phe Ile Thr Val Asp Ala Val Gly Cys Phe Glu
   1 5 10 15
   Gly Asp Glu Glu Glu Glu Glu Asp Asp Glu Asp Glu Glu Glu Ile Glu
   20 25 30
   Val Arg Ser Arg Asp Ile Ser Arg Glu Glu Trp Lys Gly Ser Glu Thr
   35 40 45
   Tyr Ser Pro Asn Thr Ala
   50
<210> 746
   <211> 53
   <212> PRT
   <213> Artificial Sequence
<220>
   <223> Ciz1 b-variant peptide comprising an exon 14b/exon 15 junction
<400> 746
   Asp Glu Asp His Phe Ile Thr Val Asp Ala Val Gly Cys Phe Glu Gly
   1 5 10 15
   Asp Glu Glu Glu Glu Glu Asp Asp Glu Asp Glu Glu Glu Ile Glu Val
   20 25 30
   Arg Ser Arg Asp Ile Ser Arg Glu Glu Trp Lys Gly Ser Glu Thr Tyr
   35 40 45
   Ser Pro Asn Thr Ala
   50
<210> 747
   <211> 52
   <212> PRT
   <213> Artificial Sequence
<220>
   <223> Ciz1 b-variant peptide comprising an exon 14b/exon 15 junction
<400> 747
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu Thr Tyr Ser
           35                  40                  45
   Pro Asn Thr Ala
       50
<210> 748
   <211> 51
   <212> PRT
   <213> Artificial Sequence
<220>
   <223> Ciz1 b-variant peptide comprising an exon 14b/exon 15 junction
<400> 748
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr Ser Pro
           35                  40                  45
   Asn Thr Ala
       50
<210> 749
   <211> 50
   <212> PRT
   <213> Artificial Sequence
<220>
   <223> Ciz1 b-variant peptide comprising an exon 14b/exon 15 junction
<400> 749
   His Phe Ile Thr Val Asp Ala Val Gly Cys Phe Glu Gly Asp Glu Glu
   1 5 10 15
   Glu Glu Glu Asp Asp Glu Asp Glu Glu Glu Ile Glu Val Arg Ser Arg
   20 25 30
   Asp Ile Ser Arg Glu Glu Trp Lys Gly Ser Glu Thr Tyr Ser Pro Asn
   35 40 45
   Thr Ala
   50
<210> 750
   <211> 49
   <212> PRT
   <213> Artificial Sequence
<220>
   <223> Ciz1 b-variant peptide comprising an exon 14b/exon 15 junction
<400> 750
   Phe Ile Thr Val Asp Ala Val Gly Cys Phe Glu Gly Asp Glu Glu Glu
   1 5 10 15
   Glu Glu Asp Asp Glu Asp Glu Glu Glu Ile Glu Val Arg Ser Arg Asp
   20 25 30
   Ile Ser Arg Glu Glu Trp Lys Gly Ser Glu Thr Tyr Ser Pro Asn Thr
   35 40 45
   Ala
<210> 751
   <211> 48
   <212> PRT
   <213> Artificial Sequence
<220>
   <223> Ciz1 b-variant peptide comprising an exon 14b/exon 15 junction
<400> 751
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr Ala
           35                  40                  45
<210> 752
   <211> 47
   <212> PRT
   <213> Artificial Sequence
<220>
   <223> Ciz1 b-variant peptide comprising an exon 14b/exon 15 junction
<400> 752
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn Thr Ala
           35                  40                  45
<210> 753
   <211> 46
   <212> PRT
   <213> Artificial Sequence
<220>
   <223> Ciz1 b-variant peptide comprising an exon 14b/exon 15 junction
<400> 753
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala
           35                  40                  45
<210> 754
   <211> 45
   <212> PRT
   <213> Artificial Sequence
<220>
   <223> Ciz1 b-variant peptide comprising an exon 14b/exon 15 junction
<400> 754
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro Asn Thr Ala
           35                  40                  45
<210> 755
   <211> 44
   <212> PRT
   <213> Artificial Sequence
<220>
   <223> Ciz1 b-variant peptide comprising an exon 14b/exon 15 junction
<400> 755
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr Tyr Ser Pro Asn Thr Ala
           35                  40
<210> 756
   <211> 43
   <212> PRT
   <213> Artificial Sequence
<220>
   <223> Ciz1 b-variant peptide comprising an exon 14b/exon 15 junction
<400> 756
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro Asn Thr Ala
           35                  40
<210> 757
   <211> 42
   <212> PRT
   <213> Artificial Sequence
<220>
   <223> Ciz1 b-variant peptide comprising an exon 14b/exon 15 junction
<400> 757
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro Asn Thr Ala
           35                  40
<210> 758
   <211> 41
   <212> PRT
   <213> Artificial Sequence
<220>
   <223> Ciz1 b-variant peptide comprising an exon 14b/exon 15 junction
<400> 758
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn Thr Ala
           35                  40
<210> 759
   <211> 40
   <212> PRT
   <213> Artificial Sequence
<220>
   <223> Ciz1 b-variant peptide comprising an exon 14b/exon 15 junction
<400> 759
   Phe Glu Gly Asp Glu Glu Glu Glu Glu Asp Asp Glu Asp Glu Glu Glu
   1 5 10 15
   Ile Glu Val Arg Ser Arg Asp Ile Ser Arg Glu Glu Trp Lys Gly Ser
   20 25 30
   Glu Thr Tyr Ser Pro Asn Thr Ala
   35 40
<210> 760
   <211> 39
   <212> PRT
   <213> Artificial Sequence
<220>
   <223> Ciz1 b-variant peptide comprising an exon 14b/exon 15 junction
<400> 760
   Glu Gly Asp Glu Glu Glu Glu Glu Asp Asp Glu Asp Glu Glu Glu Ile
   1 5 10 15
   Glu Val Arg Ser Arg Asp Ile Ser Arg Glu Glu Trp Lys Gly Ser Glu
   20 25 30
   Thr Tyr Ser Pro Asn Thr Ala
   35
<210> 761
   <211> 38
   <212> PRT
   <213> Artificial Sequence
<220>
   <223> Ciz1 b-variant peptide comprising an exon 14b/exon 15 junction
<400> 761
   Gly Asp Glu Glu Glu Glu Glu Asp Asp Glu Asp Glu Glu Glu Ile Glu
   1 5 10 15
   Val Arg Ser Arg Asp Ile Ser Arg Glu Glu Trp Lys Gly Ser Glu Thr
   20 25 30
   Tyr Ser Pro Asn Thr Ala
   35
<210> 762
   <211> 37
   <212> PRT
   <213> Artificial Sequence
<220>
   <223> Ciz1 b-variant peptide comprising an exon 14b/exon 15 junction
<400> 762
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro Asn Thr Ala
           35
<210> 763
   <211> 36
   <212> PRT
   <213> Artificial Sequence
<220>
   <223> Ciz1 b-variant peptide comprising an exon 14b/exon 15 junction
<400> 763
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr Tyr Ser
               20                  25                  30
   Pro Asn Thr Ala
           35
<210> 764
   <211> 35
   <212> PRT
   <213> Artificial Sequence
<220>
   <223> Ciz1 b-variant peptide comprising an exon 14b/exon 15 junction
<400> 764
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr Ala
           35
<210> 765
   <211> 34
   <212> PRT
   <213> Artificial Sequence
<220>
   <223> Ciz1 b-variant peptide comprising an exon 14b/exon 15 junction
<400> 765
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr Ala
<210> 766
   <211> 33
   <212> PRT
   <213> Artificial Sequence
<220>
   <223> Ciz1 b-variant peptide comprising an exon 14b/exon 15 junction
<400> 766
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
   Ala
<210> 767
   <211> 32
   <212> PRT
   <213> Artificial Sequence
<220>
   <223> Ciz1 b-variant peptide comprising an exon 14b/exon 15 junction
<400> 767
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr Ala
               20                  25                  30
<210> 768
   <211> 31
   <212> PRT
   <213> Artificial Sequence
<220>
   <223> Ciz1 b-variant peptide comprising an exon 14b/exon 15 junction
<400> 768
   Asp Asp Glu Asp Glu Glu Glu Ile Glu Val Arg Ser Arg Asp Ile Ser
   1 5 10 15
   Arg Glu Glu Trp Lys Gly Ser Glu Thr Tyr Ser Pro Asn Thr Ala
   20 25 30
<210> 769
   <211> 30
   <212> PRT
   <213> Artificial Sequence
<220>
   <223> Ciz1 b-variant peptide comprising an exon 14b/exon 15 junction
<400> 769
   Asp Glu Asp Glu Glu Glu Ile Glu Val Arg Ser Arg Asp Ile Ser Arg
   1 5 10 15
   Glu Glu Trp Lys Gly Ser Glu Thr Tyr Ser Pro Asn Thr Ala
   20 25 30
<210> 770
   <211> 29
   <212> PRT
   <213> Artificial Sequence
<220>
   <223> Ciz1 b-variant peptide comprising an exon 14b/exon 15 junction
<400> 770
   Glu Asp Glu Glu Glu Ile Glu Val Arg Ser Arg Asp Ile Ser Arg Glu
   1 5 10 15
   Glu Trp Lys Gly Ser Glu Thr Tyr Ser Pro Asn Thr Ala
   20 25
<210> 771
   <211> 28
   <212> PRT
   <213> Artificial Sequence
<220>
   <223> Ciz1 b-variant peptide comprising an exon 14b/exon 15 junction
<400> 771
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro Asn Thr Ala
                       20                  25
<210> 772
   <211> 27
   <212> PRT
   <213> Artificial Sequence
<220>
   <223> Ciz1 b-variant peptide comprising an exon 14b/exon 15 junction
<400> 772
   Glu Glu Glu Ile Glu Val Arg Ser Arg Asp Ile Ser Arg Glu Glu Trp
   1               5                   10                  15
   Lys Gly Ser Glu Thr Tyr Ser Pro Asn Thr Ala
               20                  25
<210> 773
   <211> 26
   <212> PRT
   <213> Artificial Sequence
<220>
   <223> Ciz1 b-variant peptide comprising an exon 14b/exon 15 junction
<400> 773
   Glu Glu Ile Glu Val Arg Ser Arg Asp Ile Ser Arg Glu Glu Trp Lys
   1               5                   10                  15
   Gly Ser Glu Thr Tyr Ser Pro Asn Thr Ala
               20                  25
<210> 774
   <211> 25
   <212> PRT
   <213> Artificial Sequence
<220>
   <223> Ciz1 b-variant peptide comprising an exon 14b/exon 15 junction
<400> 774
   Glu Ile Glu Val Arg Ser Arg Asp Ile Ser Arg Glu Glu Trp Lys Gly
   1 5 10 15
   Ser Glu Thr Tyr Ser Pro Asn Thr Ala
   20 25
<210> 775
   <211> 24
   <212> PRT
   <213> Artificial Sequence
<220>
   <223> Ciz1 b-variant peptide comprising an exon 14b/exon 15 junction
<400> 775
   Ile Glu Val Arg Ser Arg Asp Ile Ser Arg Glu Glu Trp Lys Gly Ser
   1 5 10 15
   Glu Thr Tyr Ser Pro Asn Thr Ala
   20
<210> 776
   <211> 23
   <212> PRT
   <213> Artificial Sequence
<220>
   <223> Ciz1 b-variant peptide comprising an exon 14b/exon 15 junction
<400> 776
   Glu Val Arg Ser Arg Asp Ile Ser Arg Glu Glu Trp Lys Gly Ser Glu
   1 5 10 15
   Thr Tyr Ser Pro Asn Thr Ala
   20
<210> 777
   <211> 59
   <212> PRT
   <213> Artificial Sequence
<220>
   <223> Ciz1 b-variant peptide comprising an exon 14b/exon 15 junction
<400> 777
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr
       50                  55
<210> 778
   <211> 58
   <212> PRT
   <213> Artificial Sequence
<220>
   <223> Ciz1 b-variant peptide comprising an exon 14b/exon 15 junction
<400> 778
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala Tyr
       50                  55
<210> 779
   <211> 57
   <212> PRT
   <213> Artificial Sequence
<220>
   <223> Ciz1 b-variant peptide comprising an exon 14b/exon 15 junction
<400> 779
   Ala Gly Gln Asp Glu Asp His Phe Ile Thr Val Asp Ala Val Gly Cys
   1 5 10 15
   Phe Glu Gly Asp Glu Glu Glu Glu Glu Asp Asp Glu Asp Glu Glu Glu
   20 25 30
   Ile Glu Val Arg Ser Arg Asp Ile Ser Arg Glu Glu Trp Lys Gly Ser
   35 40 45
   Glu Thr Tyr Ser Pro Asn Thr Ala Tyr
   50 55
<210> 780
   <211> 56
   <212> PRT
   <213> Artificial Sequence
<220>
   <223> Ciz1 b-variant peptide comprising an exon 14b/exon 15 junction
<400> 780
   Gly Gln Asp Glu Asp His Phe Ile Thr Val Asp Ala Val Gly Cys Phe
   1 5 10 15
   Glu Gly Asp Glu Glu Glu Glu Glu Asp Asp Glu Asp Glu Glu Glu Ile
   20 25 30
   Glu Val Arg Ser Arg Asp Ile Ser Arg Glu Glu Trp Lys Gly Ser Glu
   35 40 45
   Thr Tyr Ser Pro Asn Thr Ala Tyr
   50 55
<210> 781
   <211> 55
   <212> PRT
   <213> Artificial Sequence
<220>
   <223> Ciz1 b-variant peptide comprising an exon 14b/exon 15 junction
<400> 781
   Gln Asp Glu Asp His Phe Ile Thr Val Asp Ala Val Gly Cys Phe Glu
   1 5 10 15
   Gly Asp Glu Glu Glu Glu Glu Asp Asp Glu Asp Glu Glu Glu Ile Glu
   20 25 30
   Val Arg Ser Arg Asp Ile Ser Arg Glu Glu Trp Lys Gly Ser Glu Thr
   35 40 45
   Tyr Ser Pro Asn Thr Ala Tyr
   50 55
<210> 782
   <211> 54
   <212> PRT
   <213> Artificial Sequence
<220>
   <223> Ciz1 b-variant peptide comprising an exon 14b/exon 15 junction
<400> 782
   Asp Glu Asp His Phe Ile Thr Val Asp Ala Val Gly Cys Phe Glu Gly
   1 5 10 15
   Asp Glu Glu Glu Glu Glu Asp Asp Glu Asp Glu Glu Glu Ile Glu Val
   20 25 30
   Arg Ser Arg Asp Ile Ser Arg Glu Glu Trp Lys Gly Ser Glu Thr Tyr
   35 40 45
   Ser Pro Asn Thr Ala Tyr
   50
<210> 783
   <211> 53
   <212> PRT
   <213> Artificial Sequence
<220>
   <223> Ciz1 b-variant peptide comprising an exon 14b/exon 15 junction
<400> 783
   Glu Asp His Phe Ile Thr Val Asp Ala Val Gly Cys Phe Glu Gly Asp
   1 5 10 15
   Glu Glu Glu Glu Glu Asp Asp Glu Asp Glu Glu Glu Ile Glu Val Arg
   20 25 30
   Ser Arg Asp Ile Ser Arg Glu Glu Trp Lys Gly Ser Glu Thr Tyr Ser
   35 40 45
   Pro Asn Thr Ala Tyr
   50
<210> 784
   <211> 52
   <212> PRT
   <213> Artificial Sequence
<220>
   <223> Ciz1 b-variant peptide comprising an exon 14b/exon 15 junction
<400> 784
   Asp His Phe Ile Thr Val Asp Ala Val Gly Cys Phe Glu Gly Asp Glu
   1 5 10 15
   Glu Glu Glu Glu Asp Asp Glu Asp Glu Glu Glu Ile Glu Val Arg Ser
   20 25 30
   Arg Asp Ile Ser Arg Glu Glu Trp Lys Gly Ser Glu Thr Tyr Ser Pro
   35 40 45
   Asn Thr Ala Tyr
   50
<210> 785
   <211> 51
   <212> PRT
   <213> Artificial Sequence
<220>
   <223> Ciz1 b-variant peptide comprising an exon 14b/exon 15 junction
<400> 785
   His Phe Ile Thr Val Asp Ala Val Gly Cys Phe Glu Gly Asp Glu Glu
   1 5 10 15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser Pro Asn
           35                  40                  45
   Thr Ala Tyr
       50
<210> 786
   <211> 50
   <212> PRT
   <213> Artificial Sequence
<220>
   <223> Ciz1 b-variant peptide comprising an exon 14b/exon 15 junction
<400> 786
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro Asn Thr
           35                  40                  45
   Ala Tyr
       50
<210> 787
   <211> 49
   <212> PRT
   <213> Artificial Sequence
<220>
   <223> Ciz1 b-variant peptide comprising an exon 14b/exon 15 junction
<400> 787
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr Ala
           35                  40                  45
   Tyr
<210> 788
   <211> 48
   <212> PRT
   <213> Artificial Sequence
<220>
   <223> Ciz1 b-variant peptide comprising an exon 14b/exon 15 junction
<400> 788
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn Thr Ala Tyr
           35                  40                  45
<210> 789
   <211> 47
   <212> PRT
   <213> Artificial Sequence
<220>
   <223> Ciz1 b-variant peptide comprising an exon 14b/exon 15 junction
<400> 789
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala Tyr
           35                  40                  45
<210> 790
   <211> 46
   <212> PRT
   <213> Artificial Sequence
<220>
   <223> Ciz1 b-variant peptide comprising an exon 14b/exon 15 junction
<400> 790
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro Asn Thr Ala Tyr
           35                  40                  45
<210> 791
   <211> 45
   <212> PRT
   <213> Artificial Sequence
<220>
   <223> Ciz1 b-variant peptide comprising an exon 14b/exon 15 junction
<400> 791
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr Tyr Ser Pro Asn Thr Ala Tyr
           35                  40                  45
<210> 792
   <211> 44
   <212> PRT
   <213> Artificial Sequence
<220>
   <223> Ciz1 b-variant peptide comprising an exon 14b/exon 15 junction
<400> 792
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro Asn Thr Ala Tyr
           35                  40
<210> 793
   <211> 43
   <212> PRT
   <213> Artificial Sequence
<220>
   <223> Ciz1 b-variant peptide comprising an exon 14b/exon 15 junction
<400> 793
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
                    20                  25                  30
   Gly Ser Glu Thr Tyr Ser Pro Asn Thr Ala Tyr
           35                  40
<210> 794
   <211> 42
   <212> PRT
   <213> Artificial Sequence
<220>
   <223> Ciz1 b-variant peptide comprising an exon 14b/exon 15 junction
<400> 794
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn Thr Ala Tyr
           35                  40
<210> 795
   <211> 41
   <212> PRT
   <213> Artificial Sequence
<220>
   <223> Ciz1 b-variant peptide comprising an exon 14b/exon 15 junction
<400> 795
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn Thr Ala Tyr
           35                  40
<210> 796
   <211> 40
   <212> PRT
   <213> Artificial Sequence
<220>
   <223> Ciz1 b-variant peptide comprising an exon 14b/exon 15 junction
<400> 796
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn Thr Ala Tyr
           35                  40
<210> 797
   <211> 39
   <212> PRT
   <213> Artificial Sequence
<220>
   <223> Ciz1 b-variant peptide comprising an exon 14b/exon 15 junction
<400> 797
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
   Tyr Ser Pro Asn Thr Ala Tyr
           35
<210> 798
   <211> 38
   <212> PRT
   <213> Artificial Sequence
<220>
   <223> Ciz1 b-variant peptide comprising an exon 14b/exon 15 junction
<400> 798
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro Asn Thr Ala Tyr
           35
<210> 799
   <211> 37
   <212> PRT
   <213> Artificial Sequence
<220>
   <223> Ciz1 b-variant peptide comprising an exon 14b/exon 15 junction
<400> 799
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr Tyr Ser
               20                  25                  30
   Pro Asn Thr Ala Tyr
           35
<210> 800
   <211> 36
   <212> PRT
   <213> Artificial Sequence
<220>
   <223> Ciz1 b-variant peptide comprising an exon 14b/exon 15 junction
<400> 800
   Glu Glu Glu Glu Asp Asp Glu Asp Glu Glu Glu Ile Glu Val Arg Ser
   1 5 10 15
   Arg Asp Ile Ser Arg Glu Glu Trp Lys Gly Ser Glu Thr Tyr Ser Pro
   20 25 30
   Asn Thr Ala Tyr
   35
<210> 801
   <211> 35
   <212> PRT
   <213> Artificial Sequence
<220>
   <223> Ciz1 b-variant peptide comprising an exon 14b/exon 15 junction
<400> 801
   Glu Glu Glu Asp Asp Glu Asp Glu Glu Glu Ile Glu Val Arg Ser Arg
   1 5 10 15
   Asp Ile Ser Arg Glu Glu Trp Lys Gly Ser Glu Thr Tyr Ser Pro Asn
   20 25 30
   Thr Ala Tyr
   35
<210> 802
   <211> 34
   <212> PRT
   <213> Artificial Sequence
<220>
   <223> Ciz1 b-variant peptide comprising an exon 14b/exon 15 junction
<400> 802
   Glu Glu Asp Asp Glu Asp Glu Glu Glu Ile Glu Val Arg Ser Arg Asp
   1 5 10 15
   Ile Ser Arg Glu Glu Trp Lys Gly Ser Glu Thr Tyr Ser Pro Asn Thr
   20 25 30
   Ala Tyr
<210> 803
   <211> 33
   <212> PRT
   <213> Artificial Sequence
<220>
   <223> Ciz1 b-variant peptide comprising an exon 14b/exon 15 junction
<400> 803
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr Ala
               20                  25                  30
   Tyr
<210> 804
   <211> 32
   <212> PRT
   <213> Artificial Sequence
<220>
   <223> Ciz1 b-variant peptide comprising an exon 14b/exon 15 junction
<400> 804
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn Thr Ala Tyr
               20                  25                  30
<210> 805
   <211> 31
   <212> PRT
   <213> Artificial Sequence
<220>
   <223> Ciz1 b-variant peptide comprising an exon 14b/exon 15 junction
<400> 805
   Asp Glu Asp Glu Glu Glu Ile Glu Val Arg Ser Arg Asp Ile Ser Arg
   1               5                   10                  15
   Glu Glu Trp Lys Gly Ser Glu Thr Tyr Ser Pro Asn Thr Ala Tyr
               20                  25                  30
<210> 806
   <211> 30
   <212> PRT
   <213> Artificial Sequence
<220>
   <223> Ciz1 b-variant peptide comprising an exon 14b/exon 15 junction
<400> 806
   Glu Asp Glu Glu Glu Ile Glu Val Arg Ser Arg Asp Ile Ser Arg Glu
   1               5                   10                  15
   Glu Trp Lys Gly Ser Glu Thr Tyr Ser Pro Asn Thr Ala Tyr
               20                  25                  30
<210> 807
   <211> 29
   <212> PRT
   <213> Artificial Sequence
<220>
   <223> Ciz1 b-variant peptide comprising an exon 14b/exon 15 junction
<400> 807
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro Asn Thr Ala Tyr
               20                  25
<210> 808
   <211> 28
   <212> PRT
   <213> Artificial Sequence
<220>
   <223> Ciz1 b-variant peptide comprising an exon 14b/exon 15 junction
<400> 808
   Glu Glu Glu Ile Glu Val Arg Ser Arg Asp Ile Ser Arg Glu Glu Trp
   1               5                   10                  15
   Lys Gly Ser Glu Thr Tyr Ser Pro Asn Thr Ala Tyr
               20                  25
<210> 809
   <211> 27
   <212> PRT
   <213> Artificial Sequence
<220>
   <223> Ciz1 b-variant peptide comprising an exon 14b/exon 15 junction
<400> 809
   Glu Glu Ile Glu Val Arg Ser Arg Asp Ile Ser Arg Glu Glu Trp Lys
   1               5                   10                  15
   Gly Ser Glu Thr Tyr Ser Pro Asn Thr Ala Tyr
               20                  25
<210> 810
   <211> 26
   <212> PRT
   <213> Artificial Sequence
<220>
   <223> Ciz1 b-variant peptide comprising an exon 14b/exon 15 junction
<400> 810
   Glu Ile Glu Val Arg Ser Arg Asp Ile Ser Arg Glu Glu Trp Lys Gly
   1               5                   10                  15
   Ser Glu Thr Tyr Ser Pro Asn Thr Ala Tyr
               20                  25
<210> 811
   <211> 25
   <212> PRT
   <213> Artificial Sequence
<220>
   <223> Ciz1 b-variant peptide comprising an exon 14b/exon 15 junction
<400> 811
   Ile Glu Val Arg Ser Arg Asp Ile Ser Arg Glu Glu Trp Lys Gly Ser
   1               5                   10                  15
   Glu Thr Tyr Ser Pro Asn Thr Ala Tyr
               20                  25
<210> 812
   <211> 24
   <212> PRT
   <213> Artificial Sequence
<220>
   <223> Ciz1 b-variant peptide comprising an exon 14b/exon 15 junction
<400> 812
   Glu Val Arg Ser Arg Asp Ile Ser Arg Glu Glu Trp Lys Gly Ser Glu
   1               5                   10                  15
   Thr Tyr Ser Pro Asn Thr Ala Tyr
               20
<210> 813
   <211> 60
   <212> PRT
   <213> Artificial Sequence
<220>
   <223> Ciz1 b-variant peptide comprising an exon 14b/exon 15 junction
<400> 813
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr Gly
       50                  55                  60
<210> 814
   <211> 59
   <212> PRT
   <213> Artificial Sequence
<220>
   <223> Ciz1 b-variant peptide comprising an exon 14b/exon 15 junction
<400> 814
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala Tyr Gly
       50                  55
<210> 815
   <211> 58
   <212> PRT
   <213> Artificial Sequence
<220>
   <223> Ciz1 b-variant peptide comprising an exon 14b/exon 15 junction
<400> 815
   Ala Gly Gln Asp Glu Asp His Phe Ile Thr Val Asp Ala Val Gly Cys
   1 5 10 15
   Phe Glu Gly Asp Glu Glu Glu Glu Glu Asp Asp Glu Asp Glu Glu Glu
   20 25 30
   Ile Glu Val Arg Ser Arg Asp Ile Ser Arg Glu Glu Trp Lys Gly Ser
   35 40 45
   Glu Thr Tyr Ser Pro Asn Thr Ala Tyr Gly
   50 55
<210> 816
   <211> 57
   <212> PRT
   <213> Artificial Sequence
<220>
   <223> Ciz1 b-variant peptide comprising an exon 14b/exon 15 junction
<400> 816
   Gly Gln Asp Glu Asp His Phe Ile Thr Val Asp Ala Val Gly Cys Phe
   1 5 10 15
   Glu Gly Asp Glu Glu Glu Glu Glu Asp Asp Glu Asp Glu Glu Glu Ile
   20 25 30
   Glu Val Arg Ser Arg Asp Ile Ser Arg Glu Glu Trp Lys Gly Ser Glu
   35 40 45
   Thr Tyr Ser Pro Asn Thr Ala Tyr Gly
   50 55
<210> 817
   <211> 56
   <212> PRT
   <213> Artificial Sequence
<220>
   <223> Ciz1 b-variant peptide comprising an exon 14b/exon 15 junction
<400> 817
   Gln Asp Glu Asp His Phe Ile Thr Val Asp Ala Val Gly Cys Phe Glu
   1 5 10 15
   Gly Asp Glu Glu Glu Glu Glu Asp Asp Glu Asp Glu Glu Glu Ile Glu
   20 25 30
   Val Arg Ser Arg Asp Ile Ser Arg Glu Glu Trp Lys Gly Ser Glu Thr
   35 40 45
   Tyr Ser Pro Asn Thr Ala Tyr Gly
   50 55
<210> 818
   <211> 55
   <212> PRT
   <213> Artificial Sequence
<220>
   <223> Ciz1 b-variant peptide comprising an exon 14b/exon 15 junction
<400> 818
   Asp Glu Asp His Phe Ile Thr Val Asp Ala Val Gly Cys Phe Glu Gly
   1 5 10 15
   Asp Glu Glu Glu Glu Glu Asp Asp Glu Asp Glu Glu Glu Ile Glu Val
   20 25 30
   Arg Ser Arg Asp Ile Ser Arg Glu Glu Trp Lys Gly Ser Glu Thr Tyr
   35 40 45
   Ser Pro Asn Thr Ala Tyr Gly
   50 55
<210> 819
   <211> 54
   <212> PRT
   <213> Artificial Sequence
<220>
   <223> Ciz1 b-variant peptide comprising an exon 14b/exon 15 junction
<400> 819
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu Thr Tyr Ser
           35                  40                  45
   Pro Asn Thr Ala Tyr Gly
       50
<210> 820
   <211> 53
   <212> PRT
   <213> Artificial Sequence
<220>
   <223> Ciz1 b-variant peptide comprising an exon 14b/exon 15 junction
<400> 820
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr Ser Pro
           35                  40                  45
   Asn Thr Ala Tyr Gly
       50
<210> 821
   <211> 52
   <212> PRT
   <213> Artificial Sequence
<220>
   <223> Ciz1 b-variant peptide comprising an exon 14b/exon 15 junction
<400> 821
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser Pro Asn
           35                  40                  45
   Thr Ala Tyr Gly
       50
<210> 822
   <211> 51
   <212> PRT
   <213> Artificial Sequence
<220>
   <223> Ciz1 b-variant peptide comprising an exon 14b/exon 15 junction
<400> 822
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro Asn Thr
           35                  40                  45
   Ala Tyr Gly
       50
<210> 823
   <211> 50
   <212> PRT
   <213> Artificial Sequence
<220>
   <223> Ciz1 b-variant peptide comprising an exon 14b/exon 15 junction
<400> 823
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr Ala
           35                  40                  45
   Tyr Gly
       50
<210> 824
   <211> 49
   <212> PRT
   <213> Artificial Sequence
<220>
   <223> Ciz1 b-variant peptide comprising an exon 14b/exon 15 junction
<400> 824
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn Thr Ala Tyr
           35                  40                  45
   Gly
<210> 825
   <211> 48
   <212> PRT
   <213> Artificial Sequence
<220>
   <223> Ciz1 b-variant peptide comprising an exon 14b/exon 15 junction
<400> 825
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala Tyr Gly
           35                  40                  45
<210> 826
   <211> 47
   <212> PRT
   <213> Artificial Sequence
<220>
   <223> Ciz1 b-variant peptide comprising an exon 14b/exon 15 junction
<400> 826
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro Asn Thr Ala Tyr Gly
           35                  40                  45
<210> 827
   <211> 46
   <212> PRT
   <213> Artificial Sequence
<220>
   <223> Ciz1 b-variant peptide comprising an exon 14b/exon 15 junction
<400> 827
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr Tyr Ser Pro Asn Thr Ala Tyr Gly
           35                  40                  45
<210> 828
   <211> 45
   <212> PRT
   <213> Artificial Sequence
<220>
   <223> Ciz1 b-variant peptide comprising an exon 14b/exon 15 junction
<400> 828
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro Asn Thr Ala Tyr Gly
           35                  40                  45
<210> 829
   <211> 44
   <212> PRT
   <213> Artificial Sequence
<220>
   <223> Ciz1 b-variant peptide comprising an exon 14b/exon 15 junction
<400> 829
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro Asn Thr Ala Tyr Gly
           35                  40
<210> 830
   <211> 43
   <212> PRT
   <213> Artificial Sequence
<220>
   <223> Ciz1 b-variant peptide comprising an exon 14b/exon 15 junction
<400> 830
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn Thr Ala Tyr Gly
           35                  40
<210> 831
   <211> 42
   <212> PRT
   <213> Artificial Sequence
<220>
   <223> Ciz1 b-variant peptide comprising an exon 14b/exon 15 junction
<400> 831
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn Thr Ala Tyr Gly
           35                  40
<210> 832
   <211> 41
   <212> PRT
   <213> Artificial Sequence
<220>
   <223> Ciz1 b-variant peptide comprising an exon 14b/exon 15 junction
<400> 832
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn Thr Ala Tyr Gly
           35                  40
<210> 833
   <211> 40
   <212> PRT
   <213> Artificial Sequence
<220>
   <223> Ciz1 b-variant peptide comprising an exon 14b/exon 15 junction
<400> 833
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
   Tyr Ser Pro Asn Thr Ala Tyr Gly
           35                  40
<210> 834
   <211> 39
   <212> PRT
   <213> Artificial Sequence
<220>
   <223> Ciz1 b-variant peptide comprising an exon 14b/exon 15 junction
<400> 834
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro Asn Thr Ala Tyr Gly
           35
<210> 835
   <211> 38
   <212> PRT
   <213> Artificial Sequence
<220>
   <223> Ciz1 b-variant peptide comprising an exon 14b/exon 15 junction
<400> 835
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr Tyr Ser
               20                  25                  30
   Pro Asn Thr Ala Tyr Gly
           35
<210> 836
   <211> 37
   <212> PRT
   <213> Artificial Sequence
<220>
   <223> Ciz1 b-variant peptide comprising an exon 14b/exon 15 junction
<400> 836
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr Ala Tyr Gly
           35
<210> 837
   <211> 36
   <212> PRT
   <213> Artificial Sequence
<220>
   <223> Ciz1 b-variant peptide comprising an exon 14b/exon 15 junction
<400> 837
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr Ala Tyr Gly
           35
<210> 838
   <211> 35
   <212> PRT
   <213> Artificial Sequence
<220>
   <223> Ciz1 b-variant peptide comprising an exon 14b/exon 15 junction
<400> 838
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
   Ala Tyr Gly
           35
<210> 839
   <211> 34
   <212> PRT
   <213> Artificial Sequence
<220>
   <223> Ciz1 b-variant peptide comprising an exon 14b/exon 15 junction
<400> 839
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr Ala
               20                  25                  30
   Tyr Gly
<210> 840
   <211> 33
   <212> PRT
   <213> Artificial Sequence
<220>
   <223> Ciz1 b-variant peptide comprising an exon 14b/exon 15 junction
<400> 840
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn Thr Ala Tyr
               20                  25                  30
   Gly
<210> 841
   <211> 32
   <212> PRT
   <213> Artificial Sequence
<220>
   <223> Ciz1 b-variant peptide comprising an exon 14b/exon 15 junction
<400> 841
   Asp Glu Asp Glu Glu Glu Ile Glu Val Arg Ser Arg Asp Ile Ser Arg
   1               5                   10                  15
   Glu Glu Trp Lys Gly Ser Glu Thr Tyr Ser Pro Asn Thr Ala Tyr Gly
               20                  25                  30
<210> 842
   <211> 31
   <212> PRT
   <213> Artificial Sequence
<220>
   <223> Ciz1 b-variant peptide comprising an exon 14b/exon 15 junction
<400> 842
   Glu Asp Glu Glu Glu Ile Glu Val Arg Ser Arg Asp Ile Ser Arg Glu
   1               5                   10                  15
   Glu Trp Lys Gly Ser Glu Thr Tyr Ser Pro Asn Thr Ala Tyr Gly
               20                  25                  30
<210> 843
   <211> 30
   <212> PRT
   <213> Artificial Sequence
<220>
   <223> Ciz1 b-variant peptide comprising an exon 14b/exon 15 junction
<400> 843
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro Asn Thr Ala Tyr Gly
               20                  25                  30
<210> 844
   <211> 29
   <212> PRT
   <213> Artificial Sequence
<220>
   <223> Ciz1 b-variant peptide comprising an exon 14b/exon 15 junction
<400> 844
   Glu Glu Glu Ile Glu Val Arg Ser Arg Asp Ile Ser Arg Glu Glu Trp
   1               5                   10                  15
   Lys Gly Ser Glu Thr Tyr Ser Pro Asn Thr Ala Tyr Gly
               20                  25
<210> 845
   <211> 28
   <212> PRT
   <213> Artificial Sequence
<220>
   <223> Ciz1 b-variant peptide comprising an exon 14b/exon 15 junction
<400> 845
   Glu Glu Ile Glu Val Arg Ser Arg Asp Ile Ser Arg Glu Glu Trp Lys
   1               5                   10                  15
   Gly Ser Glu Thr Tyr Ser Pro Asn Thr Ala Tyr Gly
               20                  25
<210> 846
   <211> 27
   <212> PRT
   <213> Artificial Sequence
<220>
   <223> Ciz1 b-variant peptide comprising an exon 14b/exon 15 junction
<400> 846
   Glu Ile Glu Val Arg Ser Arg Asp Ile Ser Arg Glu Glu Trp Lys Gly
   1               5                   10                  15
   Ser Glu Thr Tyr Ser Pro Asn Thr Ala Tyr Gly
               20                  25
<210> 847
   <211> 26
   <212> PRT
   <213> Artificial Sequence
<220>
   <223> Ciz1 b-variant peptide comprising an exon 14b/exon 15 junction
<400> 847
   Ile Glu Val Arg Ser Arg Asp Ile Ser Arg Glu Glu Trp Lys Gly Ser
   1               5                   10                  15
   Glu Thr Tyr Ser Pro Asn Thr Ala Tyr Gly
               20                  25
<210> 848
   <211> 25
   <212> PRT
   <213> Artificial Sequence
<220>
   <223> Ciz1 b-variant peptide comprising an exon 14b/exon 15 junction
<400> 848
   Glu Val Arg Ser Arg Asp Ile Ser Arg Glu Glu Trp Lys Gly Ser Glu
   1               5                   10                  15
   Thr Tyr Ser Pro Asn Thr Ala Tyr Gly
               20                  25
<210> 849
   <211> 61
   <212> PRT
   <213> Artificial Sequence
<220>
   <223> Ciz1 b-variant peptide comprising an exon 14b/exon 15 junction
<400> 849
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr Gly Val
       50                  55                  60
<210> 850
   <211> 60
   <212> PRT
   <213> Artificial Sequence
<220>
   <223> Ciz1 b-variant peptide comprising an exon 14b/exon 15 junction
<400> 850
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala Tyr Gly Val
       50                  55                  60
<210> 851
   <211> 59
   <212> PRT
   <213> Artificial Sequence
<220>
   <223> Ciz1 b-variant peptide comprising an exon 14b/exon 15 junction
<400> 851
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser Pro Asn Thr Ala Tyr Gly Val
       50                  55
<210> 852
   <211> 58
   <212> PRT
   <213> Artificial Sequence
<220>
   <223> Ciz1 b-variant peptide comprising an exon 14b/exon 15 junction
<400> 852
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr Ser Pro Asn Thr Ala Tyr Gly Val
       50                  55
<210> 853
   <211> 57
   <212> PRT
   <213> Artificial Sequence
<220>
   <223> Ciz1 b-variant peptide comprising an exon 14b/exon 15 junction
<400> 853
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr Ser Pro Asn Thr Ala Tyr Gly Val
       50                  55
<210> 854
   <211> 56
   <212> PRT
   <213> Artificial Sequence
<220>
   <223> Ciz1 b-variant peptide comprising an exon 14b/exon 15 junction
<400> 854
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu Thr Tyr
           35                  40                  45
   Ser Pro Asn Thr Ala Tyr Gly Val
       50                  55
<210> 855
   <211> 55
   <212> PRT
   <213> Artificial Sequence
<220>
   <223> Ciz1 b-variant peptide comprising an exon 14b/exon 15 junction
<400> 855
   Glu Asp His Phe Ile Thr Val Asp Ala Val Gly Cys Phe Glu Gly Asp
   1 5 10 15
   Glu Glu Glu Glu Glu Asp Asp Glu Asp Glu Glu Glu Ile Glu Val Arg
   20 25 30
   Ser Arg Asp Ile Ser Arg Glu Glu Trp Lys Gly Ser Glu Thr Tyr Ser
   35 40 45
   Pro Asn Thr Ala Tyr Gly Val
   50 55
<210> 856
   <211> 54
   <212> PRT
   <213> Artificial Sequence
<220>
   <223> Ciz1 b-variant peptide comprising an exon 14b/exon 15 junction
<400> 856
   Asp His Phe Ile Thr Val Asp Ala Val Gly Cys Phe Glu Gly Asp Glu
   1 5 10 15
   Glu Glu Glu Glu Asp Asp Glu Asp Glu Glu Glu Ile Glu Val Arg Ser
   20 25 30
   Arg Asp Ile Ser Arg Glu Glu Trp Lys Gly Ser Glu Thr Tyr Ser Pro
   35 40 45
   Asn Thr Ala Tyr Gly Val
   50
<210> 857
   <211> 53
   <212> PRT
   <213> Artificial Sequence
<220>
   <223> Ciz1 b-variant peptide comprising an exon 14b/exon 15 junction
<400> 857
   His Phe Ile Thr Val Asp Ala Val Gly Cys Phe Glu Gly Asp Glu Glu
   1 5 10 15
   Glu Glu Glu Asp Asp Glu Asp Glu Glu Glu Ile Glu Val Arg Ser Arg
   20 25 30
   Asp Ile Ser Arg Glu Glu Trp Lys Gly Ser Glu Thr Tyr Ser Pro Asn
   35 40 45
   Thr Ala Tyr Gly Val
   50
<210> 858
   <211> 52
   <212> PRT
   <213> Artificial Sequence
<220>
   <223> Ciz1 b-variant peptide comprising an exon 14b/exon 15 junction
<400> 858
   Phe Ile Thr Val Asp Ala Val Gly Cys Phe Glu Gly Asp Glu Glu Glu
   1 5 10 15
   Glu Glu Asp Asp Glu Asp Glu Glu Glu Ile Glu Val Arg Ser Arg Asp
   20 25 30
   Ile Ser Arg Glu Glu Trp Lys Gly Ser Glu Thr Tyr Ser Pro Asn Thr
   35 40 45
   Ala Tyr Gly Val
   50
<210> 859
   <211> 51
   <212> PRT
   <213> Artificial Sequence
<220>
   <223> Ciz1 b-variant peptide comprising an exon 14b/exon 15 junction
<400> 859
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr Ala
           35                  40                  45
   Tyr Gly Val
       50
<210> 860
   <211> 50
   <212> PRT
   <213> Artificial Sequence
<220>
   <223> Ciz1 b-variant peptide comprising an exon 14b/exon 15 junction
<400> 860
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn Thr Ala Tyr
           35                  40                  45
   Gly Val
       50
<210> 861
   <211> 49
   <212> PRT
   <213> Artificial Sequence
<220>
   <223> Ciz1 b-variant peptide comprising an exon 14b/exon 15 junction
<400> 861
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala Tyr Gly
           35                  40                  45
   Val
<210> 862
   <211> 48
   <212> PRT
   <213> Artificial Sequence
<220>
   <223> Ciz1 b-variant peptide comprising an exon 14b/exon 15 junction
<400> 862
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro Asn Thr Ala Tyr Gly Val
           35                  40                  45
<210> 863
   <211> 47
   <212> PRT
   <213> Artificial Sequence
<220>
   <223> Ciz1 b-variant peptide comprising an exon 14b/exon 15 junction
<400> 863
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr Tyr Ser Pro Asn Thr Ala Tyr Gly Val
           35                  40                  45
<210> 864
   <211> 46
   <212> PRT
   <213> Artificial Sequence
<220>
   <223> Ciz1 b-variant peptide comprising an exon 14b/exon 15 junction
<400> 864
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro Asn Thr Ala Tyr Gly Val
           35                  40                  45
<210> 865
   <211> 45
   <212> PRT
   <213> Artificial Sequence
<220>
   <223> Ciz1 b-variant peptide comprising an exon 14b/exon 15 junction
<400> 865
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro Asn Thr Ala Tyr Gly Val
           35                  40                  45
<210> 866
   <211> 44
   <212> PRT
   <213> Artificial Sequence
<220>
   <223> Ciz1 b-variant peptide comprising an exon 14b/exon 15 junction
<400> 866
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
   20 25 30
   Ser Glu Thr Tyr Ser Pro Asn Thr Ala Tyr Gly Val
   35 40
<210> 867
   <211> 43
   <212> PRT
   <213> Artificial Sequence
<220>
   <223> Ciz1 b-variant peptide comprising an exon 14b/exon 15 junction
<400> 867
   Phe Glu Gly Asp Glu Glu Glu Glu Glu Asp Asp Glu Asp Glu Glu Glu
   1 5 10 15
   Ile Glu Val Arg Ser Arg Asp Ile Ser Arg Glu Glu Trp Lys Gly Ser
   20 25 30
   Glu Thr Tyr Ser Pro Asn Thr Ala Tyr Gly Val
   35 40
<210> 868
   <211> 42
   <212> PRT
   <213> Artificial Sequence
<220>
   <223> Ciz1 b-variant peptide comprising an exon 14b/exon 15 junction
<400> 868
   Glu Gly Asp Glu Glu Glu Glu Glu Asp Asp Glu Asp Glu Glu Glu Ile
   1 5 10 15
   Glu Val Arg Ser Arg Asp Ile Ser Arg Glu Glu Trp Lys Gly Ser Glu
   20 25 30
   Thr Tyr Ser Pro Asn Thr Ala Tyr Gly Val
   35 40
<210> 869
   <211> 41
   <212> PRT
   <213> Artificial Sequence
<220>
   <223> Ciz1 b-variant peptide comprising an exon 14b/exon 15 junction
<400> 869
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
   Tyr Ser Pro Asn Thr Ala Tyr Gly Val
           35                  40
<210> 870
   <211> 40
   <212> PRT
   <213> Artificial Sequence
<220>
   <223> Ciz1 b-variant peptide comprising an exon 14b/exon 15 junction
<400> 870
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro Asn Thr Ala Tyr Gly Val
           35                  40
<210> 871
   <211> 39
   <212> PRT
   <213> Artificial Sequence
<220>
   <223> Ciz1 b-variant peptide comprising an exon 14b/exon 15 junction
<400> 871
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr Tyr Ser
               20                  25                  30
   Pro Asn Thr Ala Tyr Gly Val
           35
<210> 872
   <211> 38
   <212> PRT
   <213> Artificial Sequence
<220>
   <223> Ciz1 b-variant peptide comprising an exon 14b/exon 15 junction
<400> 872
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr Ala Tyr Gly Val
           35
<210> 873
   <211> 37
   <212> PRT
   <213> Artificial Sequence
<220>
   <223> Ciz1 b-variant peptide comprising an exon 14b/exon 15 junction
<400> 873
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr Ala Tyr Gly Val
           35
<210> 874
   <211> 36
   <212> PRT
   <213> Artificial Sequence
<220>
   <223> Ciz1 b-variant peptide comprising an exon 14b/exon 15 junction
<400> 874
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
   Ala Tyr Gly Val
           35
<210> 875
   <211> 35
   <212> PRT
   <213> Artificial Sequence
<220>
   <223> Ciz1 b-variant peptide comprising an exon 14b/exon 15 junction
<400> 875
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr Ala
               20                  25                  30
   Tyr Gly Val
           35
<210> 876
   <211> 34
   <212> PRT
   <213> Artificial Sequence
<220>
   <223> Ciz1 b-variant peptide comprising an exon 14b/exon 15 junction
<400> 876
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn Thr Ala Tyr
               20                  25                  30
   Gly Val
<210> 877
   <211> 33
   <212> PRT
   <213> Artificial Sequence
<220>
   <223> Ciz1 b-variant peptide comprising an exon 14b/exon 15 junction
<400> 877
   Asp Glu Asp Glu Glu Glu Ile Glu Val Arg Ser Arg Asp Ile Ser Arg
   1               5                   10                  15
   Glu Glu Trp Lys Gly Ser Glu Thr Tyr Ser Pro Asn Thr Ala Tyr Gly
               20                  25                  30
   Val
<210> 878
   <211> 32
   <212> PRT
   <213> Artificial Sequence
<220>
   <223> Ciz1 b-variant peptide comprising an exon 14b/exon 15 junction
<400> 878
   Glu Asp Glu Glu Glu Ile Glu Val Arg Ser Arg Asp Ile Ser Arg Glu
   1               5                   10                  15
   Glu Trp Lys Gly Ser Glu Thr Tyr Ser Pro Asn Thr Ala Tyr Gly Val
               20                  25                  30
<210> 879
   <211> 31
   <212> PRT
   <213> Artificial Sequence
<220>
   <223> Ciz1 b-variant peptide comprising an exon 14b/exon 15 junction
<400> 879
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro Asn Thr Ala Tyr Gly Val
               20                  25                  30
<210> 880
   <211> 30
   <212> PRT
   <213> Artificial Sequence
<220>
   <223> Ciz1 b-variant peptide comprising an exon 14b/exon 15 junction
<400> 880
   Glu Glu Glu Ile Glu Val Arg Ser Arg Asp Ile Ser Arg Glu Glu Trp
   1 5 10 15
   Lys Gly Ser Glu Thr Tyr Ser Pro Asn Thr Ala Tyr Gly Val
   20 25 30
<210> 881
   <211> 29
   <212> PRT
   <213> Artificial Sequence
<220>
   <223> Ciz1 b-variant peptide comprising an exon 14b/exon 15 junction
<400> 881
   Glu Glu Ile Glu Val Arg Ser Arg Asp Ile Ser Arg Glu Glu Trp Lys
   1 5 10 15
   Gly Ser Glu Thr Tyr Ser Pro Asn Thr Ala Tyr Gly Val
   20 25
<210> 882
   <211> 28
   <212> PRT
   <213> Artificial Sequence
<220>
   <223> Ciz1 b-variant peptide comprising an exon 14b/exon 15 junction
<400> 882
   Glu Ile Glu Val Arg Ser Arg Asp Ile Ser Arg Glu Glu Trp Lys Gly
   1 5 10 15
   Ser Glu Thr Tyr Ser Pro Asn Thr Ala Tyr Gly Val
   20 25
<210> 883
   <211> 27
   <212> PRT
   <213> Artificial Sequence
<220>
   <223> Ciz1 b-variant peptide comprising an exon 14b/exon 15 junction
<400> 883
   Ile Glu Val Arg Ser Arg Asp Ile Ser Arg Glu Glu Trp Lys Gly Ser
   1 5 10 15
   Glu Thr Tyr Ser Pro Asn Thr Ala Tyr Gly Val
   20 25
<210> 884
   <211> 26
   <212> PRT
   <213> Artificial Sequence
<220>
   <223> Ciz1 b-variant peptide comprising an exon 14b/exon 15 junction
<400> 884
   Glu Val Arg Ser Arg Asp Ile Ser Arg Glu Glu Trp Lys Gly Ser Glu
   1               5                   10                  15
   Thr Tyr Ser Pro Asn Thr Ala Tyr Gly Val
               20                  25
<210> 885
   <211> 62
   <212> PRT
   <213> Artificial Sequence
<220>
   <223> Ciz1 b-variant peptide comprising an exon 14b/exon 15 junction
<400> 885
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr Gly Val Asp
       50                  55                  60
<210> 886
   <211> 61
   <212> PRT
   <213> Artificial Sequence
<220>
   <223> Ciz1 b-variant peptide comprising an exon 14b/exon 15 junction
<400> 886
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala Tyr Gly Val Asp
       50                  55                  60
<210> 887
   <211> 60
   <212> PRT
   <213> Artificial Sequence
<220>
   <223> Ciz1 b-variant peptide comprising an exon 14b/exon 15 junction
<400> 887
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser Pro Asn Thr Ala Tyr Gly Val Asp
       50                  55                  60
<210> 888
   <211> 59
   <212> PRT
   <213> Artificial Sequence
<220>
   <223> Ciz1 b-variant peptide comprising an exon 14b/exon 15 junction
<400> 888
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr Ser Pro Asn Thr Ala Tyr Gly Val Asp
       50                  55
<210> 889
   <211> 58
   <212> PRT
   <213> Artificial Sequence
<220>
   <223> Ciz1 b-variant peptide comprising an exon 14b/exon 15 junction
<400> 889
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr Ser Pro Asn Thr Ala Tyr Gly Val Asp
       50                  55
<210> 890
   <211> 57
   <212> PRT
   <213> Artificial Sequence
<220>
   <223> Ciz1 b-variant peptide comprising an exon 14b/exon 15 junction
<400> 890
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu Thr Tyr
           35                  40                  45
   Ser Pro Asn Thr Ala Tyr Gly Val Asp
       50                  55
<210> 891
   <211> 56
   <212> PRT
   <213> Artificial Sequence
<220>
   <223> Ciz1 b-variant peptide comprising an exon 14b/exon 15 junction
<400> 891
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu Thr Tyr Ser
           35                  40                  45
   Pro Asn Thr Ala Tyr Gly Val Asp
       50                  55
<210> 892
   <211> 55
   <212> PRT
   <213> Artificial Sequence
<220>
   <223> Ciz1 b-variant peptide comprising an exon 14b/exon 15 junction
<400> 892
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr Ser Pro
           35                  40                  45
   Asn Thr Ala Tyr Gly Val Asp
       50                  55
<210> 893
   <211> 54
   <212> PRT
   <213> Artificial Sequence
<220>
   <223> Ciz1 b-variant peptide comprising an exon 14b/exon 15 junction
<400> 893
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser Pro Asn
           35                  40                  45
   Thr Ala Tyr Gly Val Asp
       50
<210> 894
   <211> 53
   <212> PRT
   <213> Artificial Sequence
<220>
   <223> Ciz1 b-variant peptide comprising an exon 14b/exon 15 junction
<400> 894
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro Asn Thr
           35                  40                  45
   Ala Tyr Gly Val Asp
       50
<210> 895
   <211> 52
   <212> PRT
   <213> Artificial Sequence
<220>
   <223> Ciz1 b-variant peptide comprising an exon 14b/exon 15 junction
<400> 895
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr Ala
           35                  40                  45
   Tyr Gly Val Asp
       50
<210> 896
   <211> 51
   <212> PRT
   <213> Artificial Sequence
<220>
   <223> Ciz1 b-variant peptide comprising an exon 14b/exon 15 junction
<400> 896
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn Thr Ala Tyr
           35                  40                  45
   Gly Val Asp
       50
<210> 897
   <211> 50
   <212> PRT
   <213> Artificial Sequence
<220>
   <223> Ciz1 b-variant peptide comprising an exon 14b/exon 15 junction
<400> 897
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala Tyr Gly
           35                  40                  45
   Val Asp
       50
<210> 898
   <211> 49
   <212> PRT
   <213> Artificial Sequence
<220>
   <223> Ciz1 b-variant peptide comprising an exon 14b/exon 15 junction
<400> 898
   Asp Ala Val Gly Cys Phe Glu Gly Asp Glu Glu Glu Glu Glu Asp Asp
   1 5 10 15
   Glu Asp Glu Glu Glu Ile Glu Val Arg Ser Arg Asp Ile Ser Arg Glu
   20 25 30
   Glu Trp Lys Gly Ser Glu Thr Tyr Ser Pro Asn Thr Ala Tyr Gly Val
   35 40 45
   Asp
<210> 899
   <211> 48
   <212> PRT
   <213> Artificial Sequence
<220>
   <223> Ciz1 b-variant peptide comprising an exon 14b/exon 15 junction
<400> 899
   Ala Val Gly Cys Phe Glu Gly Asp Glu Glu Glu Glu Glu Asp Asp Glu
   1 5 10 15
   Asp Glu Glu Glu Ile Glu Val Arg Ser Arg Asp Ile Ser Arg Glu Glu
   20 25 30
   Trp Lys Gly Ser Glu Thr Tyr Ser Pro Asn Thr Ala Tyr Gly Val Asp
   35 40 45
<210> 900
   <211> 47
   <212> PRT
   <213> Artificial Sequence
<220>
   <223> Ciz1 b-variant peptide comprising an exon 14b/exon 15 junction
<400> 900
   Val Gly Cys Phe Glu Gly Asp Glu Glu Glu Glu Glu Asp Asp Glu Asp
   1 5 10 15
   Glu Glu Glu Ile Glu Val Arg Ser Arg Asp Ile Ser Arg Glu Glu Trp
   20 25 30
   Lys Gly Ser Glu Thr Tyr Ser Pro Asn Thr Ala Tyr Gly Val Asp
   35 40 45
<210> 901
   <211> 46
   <212> PRT
   <213> Artificial Sequence
<220>
   <223> Ciz1 b-variant peptide comprising an exon 14b/exon 15 junction
<400> 901
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro Asn Thr Ala Tyr Gly Val Asp
           35                  40                  45
<210> 902
   <211> 45
   <212> PRT
   <213> Artificial Sequence
<220>
   <223> Ciz1 b-variant peptide comprising an exon 14b/exon 15 junction
<400> 902
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn Thr Ala Tyr Gly Val Asp
           35                  40                  45
<210> 903
   <211> 44
   <212> PRT
   <213> Artificial Sequence
<220>
   <223> Ciz1 b-variant peptide comprising an exon 14b/exon 15 junction
<400> 903
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn Thr Ala Tyr Gly Val Asp
           35                  40
<210> 904
   <211> 43
   <212> PRT
   <213> Artificial Sequence
<220>
   <223> Ciz1 b-variant peptide comprising an exon 14b/exon 15 junction
<400> 904
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn Thr Ala Tyr Gly Val Asp
           35                  40
<210> 905
   <211> 42
   <212> PRT
   <213> Artificial Sequence
<220>
   <223> Ciz1 b-variant peptide comprising an exon 14b/exon 15 junction
<400> 905
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
   Tyr Ser Pro Asn Thr Ala Tyr Gly Val Asp
           35                  40
<210> 906
   <211> 41
   <212> PRT
   <213> Artificial Sequence
<220>
   <223> Ciz1 b-variant peptide comprising an exon 14b/exon 15 junction
<400> 906
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro Asn Thr Ala Tyr Gly Val Asp
           35                  40
<210> 907
   <211> 40
   <212> PRT
   <213> Artificial Sequence
<220>
   <223> Ciz1 b-variant peptide comprising an exon 14b/exon 15 junction
<400> 907
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr Tyr Ser
               20                  25                  30
   Pro Asn Thr Ala Tyr Gly Val Asp
           35                  40
<210> 908
   <211> 39
   <212> PRT
   <213> Artificial Sequence
<220>
   <223> Ciz1 b-variant peptide comprising an exon 14b/exon 15 junction
<400> 908
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr Ala Tyr Gly Val Asp
           35
<210> 909
   <211> 38
   <212> PRT
   <213> Artificial Sequence
<220>
   <223> Ciz1 b-variant peptide comprising an exon 14b/exon 15 junction
<400> 909
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr Ala Tyr Gly Val Asp
           35
<210> 910
   <211> 37
   <212> PRT
   <213> Artificial Sequence
<220>
   <223> Ciz1 b-variant peptide comprising an exon 14b/exon 15 junction
<400> 910
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
   Ala Tyr Gly Val Asp
           35
<210> 911
   <211> 36
   <212> PRT
   <213> Artificial Sequence
<220>
   <223> Ciz1 b-variant peptide comprising an exon 14b/exon 15 junction
<400> 911
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr Ala
               20                  25                  30
   Tyr Gly Val Asp
           35
<210> 912
   <211> 35
   <212> PRT
   <213> Artificial Sequence
<220>
   <223> Ciz1 b-variant peptide comprising an exon 14b/exon 15 junction
<400> 912
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn Thr Ala Tyr
               20                  25                  30
   Gly Val Asp
           35
<210> 913
   <211> 34
   <212> PRT
   <213> Artificial Sequence
<220>
   <223> Ciz1 b-variant peptide comprising an exon 14b/exon 15 junction
<400> 913
   Asp Glu Asp Glu Glu Glu Ile Glu Val Arg Ser Arg Asp Ile Ser Arg
   1               5                   10                  15
   Glu Glu Trp Lys Gly Ser Glu Thr Tyr Ser Pro Asn Thr Ala Tyr Gly
               20                  25                  30
   Val Asp
<210> 914
   <211> 33
   <212> PRT
   <213> Artificial Sequence
<220>
   <223> Ciz1 b-variant peptide comprising an exon 14b/exon 15 junction
<400> 914
   Glu Asp Glu Glu Glu Ile Glu Val Arg Ser Arg Asp Ile Ser Arg Glu
   1 5 10 15
   Glu Trp Lys Gly Ser Glu Thr Tyr Ser Pro Asn Thr Ala Tyr Gly Val
   20 25 30
   Asp
<210> 915
   <211> 32
   <212> PRT
   <213> Artificial Sequence
<220>
   <223> Ciz1 b-variant peptide comprising an exon 14b/exon 15 junction
<400> 915
   Asp Glu Glu Glu Ile Glu Val Arg Ser Arg Asp Ile Ser Arg Glu Glu
   1 5 10 15
   Trp Lys Gly Ser Glu Thr Tyr Ser Pro Asn Thr Ala Tyr Gly Val Asp
   20 25 30
<210> 916
   <211> 31
   <212> PRT
   <213> Artificial Sequence
<220>
   <223> Ciz1 b-variant peptide comprising an exon 14b/exon 15 junction
<400> 916
   Glu Glu Glu Ile Glu Val Arg Ser Arg Asp Ile Ser Arg Glu Glu Trp
   1 5 10 15
   Lys Gly Ser Glu Thr Tyr Ser Pro Asn Thr Ala Tyr Gly Val Asp
   20 25 30
<210> 917
   <211> 30
   <212> PRT
   <213> Artificial Sequence
<220>
   <223> Ciz1 b-variant peptide comprising an exon 14b/exon 15 junction
<400> 917
   Glu Glu Ile Glu Val Arg Ser Arg Asp Ile Ser Arg Glu Glu Trp Lys
   1 5 10 15
   Gly Ser Glu Thr Tyr Ser Pro Asn Thr Ala Tyr Gly Val Asp
   20 25 30
<210> 918
   <211> 29
   <212> PRT
   <213> Artificial Sequence
<220>
   <223> Ciz1 b-variant peptide comprising an exon 14b/exon 15 junction
<400> 918
   Glu Ile Glu Val Arg Ser Arg Asp Ile Ser Arg Glu Glu Trp Lys Gly
   1 5 10 15
   Ser Glu Thr Tyr Ser Pro Asn Thr Ala Tyr Gly Val Asp
   20 25
<210> 919
   <211> 28
   <212> PRT
   <213> Artificial Sequence
<220>
   <223> Ciz1 b-variant peptide comprising an exon 14b/exon 15 junction
<400> 919
   Ile Glu Val Arg Ser Arg Asp Ile Ser Arg Glu Glu Trp Lys Gly Ser
   1 5 10 15
   Glu Thr Tyr Ser Pro Asn Thr Ala Tyr Gly Val Asp
   20 25
<210> 920
   <211> 27
   <212> PRT
   <213> Artificial Sequence
<220>
   <223> Ciz1 b-variant peptide comprising an exon 14b/exon 15 junction
<400> 920
   Glu Val Arg Ser Arg Asp Ile Ser Arg Glu Glu Trp Lys Gly Ser Glu
   1 5 10 15
   Thr Tyr Ser Pro Asn Thr Ala Tyr Gly Val Asp
   20 25
<210> 921
   <211> 63
   <212> PRT
   <213> Artificial Sequence
<220>
   <223> Ciz1 b-variant peptide comprising an exon 14b/exon 15 junction
<400> 921
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr Gly Val Asp Phe
       50                  55                  60
<210> 922
   <211> 62
   <212> PRT
   <213> Artificial Sequence
<220>
   <223> Ciz1 b-variant peptide comprising an exon 14b/exon 15 junction
<400> 922
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala Tyr Gly Val Asp Phe
       50                  55                  60
<210> 923
   <211> 61
   <212> PRT
   <213> Artificial Sequence
<220>
   <223> Ciz1 b-variant peptide comprising an exon 14b/exon 15 junction
<400> 923
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser Pro Asn Thr Ala Tyr Gly Val Asp Phe
       50                  55                  60
<210> 924
   <211> 60
   <212> PRT
   <213> Artificial Sequence
<220>
   <223> Ciz1 b-variant peptide comprising an exon 14b/exon 15 junction
<400> 924
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr Ser Pro Asn Thr Ala Tyr Gly Val Asp Phe
       50                  55                  60
<210> 925
   <211> 59
   <212> PRT
   <213> Artificial Sequence
<220>
   <223> Ciz1 b-variant peptide comprising an exon 14b/exon 15 junction
<400> 925
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr Ser Pro Asn Thr Ala Tyr Gly Val Asp Phe
       50                  55
<210> 926
   <211> 58
   <212> PRT
   <213> Artificial Sequence
<220>
   <223> Ciz1 b-variant peptide comprising an exon 14b/exon 15 junction
<400> 926
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu Thr Tyr
           35                  40                  45
   Ser Pro Asn Thr Ala Tyr Gly Val Asp Phe
       50                  55
<210> 927
   <211> 57
   <212> PRT
   <213> Artificial Sequence
<220>
   <223> Ciz1 b-variant peptide comprising an exon 14b/exon 15 junction
<400> 927
   Glu Asp His Phe Ile Thr Val Asp Ala Val Gly Cys Phe Glu Gly Asp
   1               5                   10                  15
   Glu Glu Glu Glu Glu Asp Asp Glu Asp Glu Glu Glu Ile Glu Val Arg
               20                  25                  30
   Ser Arg Asp Ile Ser Arg Glu Glu Trp Lys Gly Ser Glu Thr Tyr Ser
           35                  40                  45
   Pro Asn Thr Ala Tyr Gly Val Asp Phe
       50                  55
<210> 928
   <211> 56
   <212> PRT
   <213> Artificial Sequence
<220>
   <223> Ciz1 b-variant peptide comprising an exon 14b/exon 15 junction
<400> 928
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr Ser Pro
           35                  40                  45
   Asn Thr Ala Tyr Gly Val Asp Phe
       50                  55
<210> 929
   <211> 55
   <212> PRT
   <213> Artificial Sequence
<220>
   <223> Ciz1 b-variant peptide comprising an exon 14b/exon 15 junction
<400> 929
   His Phe Ile Thr Val Asp Ala Val Gly Cys Phe Glu Gly Asp Glu Glu
   1 5 10 15
   Glu Glu Glu Asp Asp Glu Asp Glu Glu Glu Ile Glu Val Arg Ser Arg
   20 25 30
   Asp Ile Ser Arg Glu Glu Trp Lys Gly Ser Glu Thr Tyr Ser Pro Asn
   35 40 45
   Thr Ala Tyr Gly Val Asp Phe
   50 55
<210> 930
   <211> 54
   <212> PRT
   <213> Artificial Sequence
<220>
   <223> Ciz1 b-variant peptide comprising an exon 14b/exon 15 junction
<400> 930
   Phe Ile Thr Val Asp Ala Val Gly Cys Phe Glu Gly Asp Glu Glu Glu
   1 5 10 15
   Glu Glu Asp Asp Glu Asp Glu Glu Glu Ile Glu Val Arg Ser Arg Asp
   20 25 30
   Ile Ser Arg Glu Glu Trp Lys Gly Ser Glu Thr Tyr Ser Pro Asn Thr
   35 40 45
   Ala Tyr Gly Val Asp Phe
   50
<210> 931
   <211> 53
   <212> PRT
   <213> Artificial Sequence
<220>
   <223> Ciz1 b-variant peptide comprising an exon 14b/exon 15 junction
<400> 931
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr Ala
           35                  40                  45
   Tyr Gly Val Asp Phe
       50
<210> 932
   <211> 52
   <212> PRT
   <213> Artificial Sequence
<220>
   <223> Ciz1 b-variant peptide comprising an exon 14b/exon 15 junction
<400> 932
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn Thr Ala Tyr
           35                  40                  45
   Gly Val Asp Phe
       50
<210> 933
   <211> 51
   <212> PRT
   <213> Artificial Sequence
<220>
   <223> Ciz1 b-variant peptide comprising an exon 14b/exon 15 junction
<400> 933
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala Tyr Gly
           35                  40                  45
   Val Asp Phe
       50
<210> 934
   <211> 50
   <212> PRT
   <213> Artificial Sequence
<220>
   <223> Ciz1 b-variant peptide comprising an exon 14b/exon 15 junction
<400> 934
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro Asn Thr Ala Tyr Gly Val
           35                  40                  45
   Asp Phe
       50
<210> 935
   <211> 49
   <212> PRT
   <213> Artificial Sequence
<220>
   <223> Ciz1 b-variant peptide comprising an exon 14b/exon 15 junction
<400> 935
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
   20 25 30
   Trp Lys Gly Ser Glu Thr Tyr Ser Pro Asn Thr Ala Tyr Gly Val Asp
   35 40 45
   Phe
<210> 936
   <211> 48
   <212> PRT
   <213> Artificial Sequence
<220>
   <223> Ciz1 b-variant peptide comprising an exon 14b/exon 15 junction
<400> 936
   Val Gly Cys Phe Glu Gly Asp Glu Glu Glu Glu Glu Asp Asp Glu Asp
   1 5 10 15
   Glu Glu Glu Ile Glu Val Arg Ser Arg Asp Ile Ser Arg Glu Glu Trp
   20 25 30
   Lys Gly Ser Glu Thr Tyr Ser Pro Asn Thr Ala Tyr Gly Val Asp Phe
   35 40 45
<210> 937
   <211> 47
   <212> PRT
   <213> Artificial Sequence
<220>
   <223> Ciz1 b-variant peptide comprising an exon 14b/exon 15 junction
<400> 937
   Gly Cys Phe Glu Gly Asp Glu Glu Glu Glu Glu Asp Asp Glu Asp Glu
   1 5 10 15
   Glu Glu Ile Glu Val Arg Ser Arg Asp Ile Ser Arg Glu Glu Trp Lys
   20 25 30
   Gly Ser Glu Thr Tyr Ser Pro Asn Thr Ala Tyr Gly Val Asp Phe
   35 40 45
<210> 938
   <211> 46
   <212> PRT
   <213> Artificial Sequence
<220>
   <223> Ciz1 b-variant peptide comprising an exon 14b/exon 15 junction
<400> 938
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn Thr Ala Tyr Gly Val Asp Phe
           35                  40                  45
<210> 939
   <211> 45
   <212> PRT
   <213> Artificial Sequence
<220>
   <223> Ciz1 b-variant peptide comprising an exon 14b/exon 15 junction
<400> 939
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn Thr Ala Tyr Gly Val Asp Phe
           35                  40                  45
<210> 940
   <211> 44
   <212> PRT
   <213> Artificial Sequence
<220>
   <223> Ciz1 b-variant peptide comprising an exon 14b/exon 15 junction
<400> 940
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn Thr Ala Tyr Gly Val Asp Phe
           35                  40
<210> 941
   <211> 43
   <212> PRT
   <213> Artificial Sequence
<220>
   <223> Ciz1 b-variant peptide comprising an exon 14b/exon 15 junction
<400> 941
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
   Tyr Ser Pro Asn Thr Ala Tyr Gly Val Asp Phe
           35                  40
<210> 942
   <211> 42
   <212> PRT
   <213> Artificial Sequence
<220>
   <223> Ciz1 b-variant peptide comprising an exon 14b/exon 15 junction
<400> 942
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro Asn Thr Ala Tyr Gly Val Asp Phe
           35                  40
<210> 943
   <211> 41
   <212> PRT
   <213> Artificial Sequence
<220>
   <223> Ciz1 b-variant peptide comprising an exon 14b/exon 15 junction
<400> 943
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr Tyr Ser
               20                  25                  30
   Pro Asn Thr Ala Tyr Gly Val Asp Phe
           35                  40
<210> 944
   <211> 40
   <212> PRT
   <213> Artificial Sequence
<220>
   <223> Ciz1 b-variant peptide comprising an exon 14b/exon 15 junction
<400> 944
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr Ala Tyr Gly Val Asp Phe
           35                  40
<210> 945
   <211> 39
   <212> PRT
   <213> Artificial Sequence
<220>
   <223> Ciz1 b-variant peptide comprising an exon 14b/exon 15 junction
<400> 945
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr Ala Tyr Gly Val Asp Phe
           35
<210> 946
   <211> 38
   <212> PRT
   <213> Artificial Sequence
<220>
   <223> Ciz1 b-variant peptide comprising an exon 14b/exon 15 junction
<400> 946
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
   Ala Tyr Gly Val Asp Phe
           35
<210> 947
   <211> 37
   <212> PRT
   <213> Artificial Sequence
<220>
   <223> Ciz1 b-variant peptide comprising an exon 14b/exon 15 junction
<400> 947
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr Ala
               20                  25                  30
   Tyr Gly Val Asp Phe
           35
<210> 948
   <211> 36
   <212> PRT
   <213> Artificial Sequence
<220>
   <223> Ciz1 b-variant peptide comprising an exon 14b/exon 15 junction
<400> 948
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn Thr Ala Tyr
               20                  25                  30
   Gly Val Asp Phe
           35
<210> 949
   <211> 35
   <212> PRT
   <213> Artificial Sequence
<220>
   <223> Ciz1 b-variant peptide comprising an exon 14b/exon 15 junction
<400> 949
   Asp Glu Asp Glu Glu Glu Ile Glu Val Arg Ser Arg Asp Ile Ser Arg
   1 5 10 15
   Glu Glu Trp Lys Gly Ser Glu Thr Tyr Ser Pro Asn Thr Ala Tyr Gly
   20 25 30
   Val Asp Phe
   35
<210> 950
   <211> 34
   <212> PRT
   <213> Artificial Sequence
<220>
   <223> Ciz1 b-variant peptide comprising an exon 14b/exon 15 junction
<400> 950
   Glu Asp Glu Glu Glu Ile Glu Val Arg Ser Arg Asp Ile Ser Arg Glu
   1 5 10 15
   Glu Trp Lys Gly Ser Glu Thr Tyr Ser Pro Asn Thr Ala Tyr Gly Val
   20 25 30
   Asp Phe
<210> 951
   <211> 33
   <212> PRT
   <213> Artificial Sequence
<220>
   <223> Ciz1 b-variant peptide comprising an exon 14b/exon 15 junction
<400> 951
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro Asn Thr Ala Tyr Gly Val Asp
               20                  25                  30
   Phe
<210> 952
   <211> 32
   <212> PRT
   <213> Artificial Sequence
<220>
   <223> Ciz1 b-variant peptide comprising an exon 14b/exon 15 junction
<400> 952
   Glu Glu Glu Ile Glu Val Arg Ser Arg Asp Ile Ser Arg Glu Glu Trp
   1               5                   10                  15
   Lys Gly Ser Glu Thr Tyr Ser Pro Asn Thr Ala Tyr Gly Val Asp Phe
               20                  25                  30
<210> 953
   <211> 31
   <212> PRT
   <213> Artificial Sequence
<220>
   <223> Ciz1 b-variant peptide comprising an exon 14b/exon 15 junction
<400> 953
   Glu Glu Ile Glu Val Arg Ser Arg Asp Ile Ser Arg Glu Glu Trp Lys
   1               5                   10                  15
   Gly Ser Glu Thr Tyr Ser Pro Asn Thr Ala Tyr Gly Val Asp Phe
               20                  25                  30
<210> 954
   <211> 30
   <212> PRT
   <213> Artificial Sequence
<220>
   <223> Ciz1 b-variant peptide comprising an exon 14b/exon 15 junction
<400> 954
   Glu Ile Glu Val Arg Ser Arg Asp Ile Ser Arg Glu Glu Trp Lys Gly
   1               5                   10                  15
   Ser Glu Thr Tyr Ser Pro Asn Thr Ala Tyr Gly Val Asp Phe
               20                  25                  30
<210> 955
   <211> 29
   <212> PRT
   <213> Artificial Sequence
<220>
   <223> Ciz1 b-variant peptide comprising an exon 14b/exon 15 junction
<400> 955
   Ile Glu Val Arg Ser Arg Asp Ile Ser Arg Glu Glu Trp Lys Gly Ser
   1               5                   10                  15
   Glu Thr Tyr Ser Pro Asn Thr Ala Tyr Gly Val Asp Phe
               20                  25
<210> 956
   <211> 28
   <212> PRT
   <213> Artificial Sequence
<220>
   <223> Ciz1 b-variant peptide comprising an exon 14b/exon 15 junction
<400> 956
   Glu Val Arg Ser Arg Asp Ile Ser Arg Glu Glu Trp Lys Gly Ser Glu
   1               5                   10                  15
   Thr Tyr Ser Pro Asn Thr Ala Tyr Gly Val Asp Phe
               20                  25
<210> 957
   <211> 64
   <212> PRT
   <213> Artificial Sequence
<220>
   <223> Ciz1 b-variant peptide comprising an exon 14b/exon 15 junction
<400> 957
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr Gly Val Asp Phe Leu
       50                  55                  60
<210> 958
   <211> 63
   <212> PRT
   <213> Artificial Sequence
<220>
   <223> Ciz1 b-variant peptide comprising an exon 14b/exon 15 junction
<400> 958
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala Tyr Gly Val Asp Phe Leu
       50                  55                  60
<210> 959
   <211> 62
   <212> PRT
   <213> Artificial Sequence
<220>
   <223> Ciz1 b-variant peptide comprising an exon 14b/exon 15 junction
<400> 959
   Ala Gly Gln Asp Glu Asp His Phe Ile Thr Val Asp Ala Val Gly Cys
   1 5 10 15
   Phe Glu Gly Asp Glu Glu Glu Glu Glu Asp Asp Glu Asp Glu Glu Glu
   20 25 30
   Ile Glu Val Arg Ser Arg Asp Ile Ser Arg Glu Glu Trp Lys Gly Ser
   35 40 45
   Glu Thr Tyr Ser Pro Asn Thr Ala Tyr Gly Val Asp Phe Leu
   50 55 60
<210> 960
   <211> 61
   <212> PRT
   <213> Artificial Sequence
<220>
   <223> Ciz1 b-variant peptide comprising an exon 14b/exon 15 junction
<400> 960
   Gly Gln Asp Glu Asp His Phe Ile Thr Val Asp Ala Val Gly Cys Phe
   1 5 10 15
   Glu Gly Asp Glu Glu Glu Glu Glu Asp Asp Glu Asp Glu Glu Glu Ile
   20 25 30
   Glu Val Arg Ser Arg Asp Ile Ser Arg Glu Glu Trp Lys Gly Ser Glu
   35 40 45
   Thr Tyr Ser Pro Asn Thr Ala Tyr Gly Val Asp Phe Leu
   50 55 60
<210> 961
   <211> 60
   <212> PRT
   <213> Artificial Sequence
<220>
   <223> Ciz1 b-variant peptide comprising an exon 14b/exon 15 junction
<400> 961
   Gln Asp Glu Asp His Phe Ile Thr Val Asp Ala Val Gly Cys Phe Glu
   1 5 10 15
   Gly Asp Glu Glu Glu Glu Glu Asp Asp Glu Asp Glu Glu Glu Ile Glu
   20 25 30
   Val Arg Ser Arg Asp Ile Ser Arg Glu Glu Trp Lys Gly Ser Glu Thr
   35 40 45
   Tyr Ser Pro Asn Thr Ala Tyr Gly Val Asp Phe Leu
   50 55 60
<210> 962
   <211> 59
   <212> PRT
   <213> Artificial Sequence
<220>
   <223> Ciz1 b-variant peptide comprising an exon 14b/exon 15 junction
<400> 962
   Asp Glu Asp His Phe Ile Thr Val Asp Ala Val Gly Cys Phe Glu Gly
   1 5 10 15
   Asp Glu Glu Glu Glu Glu Asp Asp Glu Asp Glu Glu Glu Ile Glu Val
   20 25 30
   Arg Ser Arg Asp Ile Ser Arg Glu Glu Trp Lys Gly Ser Glu Thr Tyr
   35 40 45
   Ser Pro Asn Thr Ala Tyr Gly Val Asp Phe Leu
   50 55
<210> 963
   <211> 58
   <212> PRT
   <213> Artificial Sequence
<220>
   <223> Ciz1 b-variant peptide comprising an exon 14b/exon 15 junction
<400> 963
   Glu Asp His Phe Ile Thr Val Asp Ala Val Gly Cys Phe Glu Gly Asp
   1 5 10 15
   Glu Glu Glu Glu Glu Asp Asp Glu Asp Glu Glu Glu Ile Glu Val Arg
   20 25 30
   Ser Arg Asp Ile Ser Arg Glu Glu Trp Lys Gly Ser Glu Thr Tyr Ser
   35 40 45
   Pro Asn Thr Ala Tyr Gly Val Asp Phe Leu
   50 55
<210> 964
   <211> 57
   <212> PRT
   <213> Artificial Sequence
<220>
   <223> Ciz1 b-variant peptide comprising an exon 14b/exon 15 junction
<400> 964
   Asp His Phe Ile Thr Val Asp Ala Val Gly Cys Phe Glu Gly Asp Glu
   1 5 10 15
   Glu Glu Glu Glu Asp Asp Glu Asp Glu Glu Glu Ile Glu Val Arg Ser
   20 25 30
   Arg Asp Ile Ser Arg Glu Glu Trp Lys Gly Ser Glu Thr Tyr Ser Pro
   35 40 45
   Asn Thr Ala Tyr Gly Val Asp Phe Leu
   50 55
<210> 965
   <211> 56
   <212> PRT
   <213> Artificial Sequence
<220>
   <223> Ciz1 b-variant peptide comprising an exon 14b/exon 15 junction
<400> 965
   His Phe Ile Thr Val Asp Ala Val Gly Cys Phe Glu Gly Asp Glu Glu
   1 5 10 15
   Glu Glu Glu Asp Asp Glu Asp Glu Glu Glu Ile Glu Val Arg Ser Arg
   20 25 30
   Asp Ile Ser Arg Glu Glu Trp Lys Gly Ser Glu Thr Tyr Ser Pro Asn
   35 40 45
   Thr Ala Tyr Gly Val Asp Phe Leu
   50 55
<210> 966
   <211> 55
   <212> PRT
   <213> Artificial Sequence
<220>
   <223> Ciz1 b-variant peptide comprising an exon 14b/exon 15 junction
<400> 966
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro Asn Thr
           35                  40                  45
   Ala Tyr Gly Val Asp Phe Leu
       50                  55
<210> 967
   <211> 54
   <212> PRT
   <213> Artificial Sequence
<220>
   <223> Ciz1 b-variant peptide comprising an exon 14b/exon 15 junction
<400> 967
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr Ala
           35                  40                  45
   Tyr Gly Val Asp Phe Leu
       50
<210> 968
   <211> 53
   <212> PRT
   <213> Artificial Sequence
<220>
   <223> Ciz1 b-variant peptide comprising an exon 14b/exon 15 junction
<400> 968
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn Thr Ala Tyr
           35                  40                  45
   Gly Val Asp Phe Leu
       50
<210> 969
   <211> 52
   <212> PRT
   <213> Artificial Sequence
<220>
   <223> Ciz1 b-variant peptide comprising an exon 14b/exon 15 junction
<400> 969
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala Tyr Gly
           35                  40                  45
   Val Asp Phe Leu
       50
<210> 970
   <211> 51
   <212> PRT
   <213> Artificial Sequence
<220>
   <223> Ciz1 b-variant peptide comprising an exon 14b/exon 15 junction
<400> 970
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro Asn Thr Ala Tyr Gly Val
           35                  40                  45
   Asp Phe Leu
       50
<210> 971
   <211> 50
   <212> PRT
   <213> Artificial Sequence
<220>
   <223> Ciz1 b-variant peptide comprising an exon 14b/exon 15 junction
<400> 971
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr Tyr Ser Pro Asn Thr Ala Tyr Gly Val Asp
           35                  40                  45
   Phe Leu
       50
<210> 972
   <211> 49
   <212> PRT
   <213> Artificial Sequence
<220>
   <223> Ciz1 b-variant peptide comprising an exon 14b/exon 15 junction
<400> 972
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro Asn Thr Ala Tyr Gly Val Asp Phe
           35                  40                  45
   Leu
<210> 973
   <211> 48
   <212> PRT
   <213> Artificial Sequence
<220>
   <223> Ciz1 b-variant peptide comprising an exon 14b/exon 15 junction
<400> 973
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro Asn Thr Ala Tyr Gly Val Asp Phe Leu
           35                  40                  45
<210> 974
   <211> 47
   <212> PRT
   <213> Artificial Sequence
<220>
   <223> Ciz1 b-variant peptide comprising an exon 14b/exon 15 junction
<400> 974
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn Thr Ala Tyr Gly Val Asp Phe Leu
           35                  40                  45
<210> 975
   <211> 46
   <212> PRT
   <213> Artificial Sequence
<220>
   <223> Ciz1 b-variant peptide comprising an exon 14b/exon 15 junction
<400> 975
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn Thr Ala Tyr Gly Val Asp Phe Leu
           35                  40                  45
<210> 976
   <211> 45
   <212> PRT
   <213> Artificial Sequence
<220>
   <223> Ciz1 b-variant peptide comprising an exon 14b/exon 15 junction
<400> 976
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn Thr Ala Tyr Gly Val Asp Phe Leu
           35                  40                  45
<210> 977
   <211> 44
   <212> PRT
   <213> Artificial Sequence
<220>
   <223> Ciz1 b-variant peptide comprising an exon 14b/exon 15 junction
<400> 977
   Gly Asp Glu Glu Glu Glu Glu Asp Asp Glu Asp Glu Glu Glu Ile Glu
   1 5 10 15
   Val Arg Ser Arg Asp Ile Ser Arg Glu Glu Trp Lys Gly Ser Glu Thr
   20 25 30
   Tyr Ser Pro Asn Thr Ala Tyr Gly Val Asp Phe Leu
   35 40
<210> 978
   <211> 43
   <212> PRT
   <213> Artificial Sequence
<220>
   <223> Ciz1 b-variant peptide comprising an exon 14b/exon 15 junction
<400> 978
   Asp Glu Glu Glu Glu Glu Asp Asp Glu Asp Glu Glu Glu Ile Glu Val
   1 5 10 15
   Arg Ser Arg Asp Ile Ser Arg Glu Glu Trp Lys Gly Ser Glu Thr Tyr
   20 25 30
   Ser Pro Asn Thr Ala Tyr Gly Val Asp Phe Leu
   35 40
<210> 979
   <211> 42
   <212> PRT
   <213> Artificial Sequence
<220>
   <223> Ciz1 b-variant peptide comprising an exon 14b/exon 15 junction
<400> 979
   Glu Glu Glu Glu Glu Asp Asp Glu Asp Glu Glu Glu Ile Glu Val Arg
   1 5 10 15
   Ser Arg Asp Ile Ser Arg Glu Glu Trp Lys Gly Ser Glu Thr Tyr Ser
   20 25 30
   Pro Asn Thr Ala Tyr Gly Val Asp Phe Leu
   35 40
<210> 980
   <211> 41
   <212> PRT
   <213> Artificial Sequence
<220>
   <223> Ciz1 b-variant peptide comprising an exon 14b/exon 15 junction
<400> 980
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr Ala Tyr Gly Val Asp Phe Leu
           35                  40
<210> 981
   <211> 40
   <212> PRT
   <213> Artificial Sequence
<220>
   <223> Ciz1 b-variant peptide comprising an exon 14b/exon 15 junction
<400> 981
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr Ala Tyr Gly Val Asp Phe Leu
           35                  40
<210> 982
   <211> 39
   <212> PRT
   <213> Artificial Sequence
<220>
   <223> Ciz1 b-variant peptide comprising an exon 14b/exon 15 junction
<400> 982
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
   Ala Tyr Gly Val Asp Phe Leu
           35
<210> 983
   <211> 38
   <212> PRT
   <213> Artificial Sequence
<220>
   <223> Ciz1 b-variant peptide comprising an exon 14b/exon 15 junction
<400> 983
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr Ala
               20                  25                  30
   Tyr Gly Val Asp Phe Leu
           35
<210> 984
   <211> 37
   <212> PRT
   <213> Artificial Sequence
<220>
   <223> Ciz1 b-variant peptide comprising an exon 14b/exon 15 junction
<400> 984
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn Thr Ala Tyr
               20                  25                  30
   Gly Val Asp Phe Leu
           35
<210> 985
   <211> 36
   <212> PRT
   <213> Artificial Sequence
<220>
   <223> Ciz1 b-variant peptide comprising an exon 14b/exon 15 junction
<400> 985
   Asp Glu Asp Glu Glu Glu Ile Glu Val Arg Ser Arg Asp Ile Ser Arg
   1 5 10 15
   Glu Glu Trp Lys Gly Ser Glu Thr Tyr Ser Pro Asn Thr Ala Tyr Gly
   20 25 30
   Val Asp Phe Leu
   35
<210> 986
   <211> 35
   <212> PRT
   <213> Artificial Sequence
<220>
   <223> Ciz1 b-variant peptide comprising an exon 14b/exon 15 junction
<400> 986
   Glu Asp Glu Glu Glu Ile Glu Val Arg Ser Arg Asp Ile Ser Arg Glu
   1 5 10 15
   Glu Trp Lys Gly Ser Glu Thr Tyr Ser Pro Asn Thr Ala Tyr Gly Val
   20 25 30
   Asp Phe Leu
   35
<210> 987
   <211> 34
   <212> PRT
   <213> Artificial Sequence
<220>
   <223> Ciz1 b-variant peptide comprising an exon 14b/exon 15 junction
<400> 987
   Asp Glu Glu Glu Ile Glu Val Arg Ser Arg Asp Ile Ser Arg Glu Glu
   1 5 10 15
   Trp Lys Gly Ser Glu Thr Tyr Ser Pro Asn Thr Ala Tyr Gly Val Asp
   20 25 30
   Phe Leu
<210> 988
   <211> 33
   <212> PRT
   <213> Artificial Sequence
<220>
   <223> Ciz1 b-variant peptide comprising an exon 14b/exon 15 junction
<400> 988
   Glu Glu Glu Ile Glu Val Arg Ser Arg Asp Ile Ser Arg Glu Glu Trp
   1               5                   10                  15
   Lys Gly Ser Glu Thr Tyr Ser Pro Asn Thr Ala Tyr Gly Val Asp Phe
               20                  25                  30
   Leu
<210> 989
   <211> 32
   <212> PRT
   <213> Artificial Sequence
<220>
   <223> Ciz1 b-variant peptide comprising an exon 14b/exon 15 junction
<400> 989
   Glu Glu Ile Glu Val Arg Ser Arg Asp Ile Ser Arg Glu Glu Trp Lys
   1               5                   10                  15
   Gly Ser Glu Thr Tyr Ser Pro Asn Thr Ala Tyr Gly Val Asp Phe Leu
               20                  25                  30
<210> 990
   <211> 31
   <212> PRT
   <213> Artificial Sequence
<220>
   <223> Ciz1 b-variant peptide comprising an exon 14b/exon 15 junction
<400> 990
   Glu Ile Glu Val Arg Ser Arg Asp Ile Ser Arg Glu Glu Trp Lys Gly
   1               5                   10                  15
   Ser Glu Thr Tyr Ser Pro Asn Thr Ala Tyr Gly Val Asp Phe Leu
               20                  25                  30
<210> 991
   <211> 30
   <212> PRT
   <213> Artificial Sequence
<220>
   <223> Ciz1 b-variant peptide comprising an exon 14b/exon 15 junction
<400> 991
   Ile Glu Val Arg Ser Arg Asp Ile Ser Arg Glu Glu Trp Lys Gly Ser
   1               5                   10                  15
   Glu Thr Tyr Ser Pro Asn Thr Ala Tyr Gly Val Asp Phe Leu
               20                  25                  30
<210> 992
   <211> 29
   <212> PRT
   <213> Artificial Sequence
<220>
   <223> Ciz1 b-variant peptide comprising an exon 14b/exon 15 junction
<400> 992
   Glu Val Arg Ser Arg Asp Ile Ser Arg Glu Glu Trp Lys Gly Ser Glu
   1               5                   10                  15
   Thr Tyr Ser Pro Asn Thr Ala Tyr Gly Val Asp Phe Leu
               20                  25
<210> 993
   <211> 65
   <212> PRT
   <213> Artificial Sequence
<220>
   <223> Ciz1 b-variant peptide comprising an exon 14b/exon 15 junction
<400> 993
   Glu Ile Ala Gly Gln Asp Glu Asp His Phe Ile Thr Val Asp Ala Val
   1               5                   10                  15
   Gly Cys Phe Glu Gly Asp Glu Glu Glu Glu Glu Asp Asp Glu Asp Glu
               20                  25                  30
   Glu Glu Ile Glu Val Arg Ser Arg Asp Ile Ser Arg Glu Glu Trp Lys
           35                  40                  45
   Gly Ser Glu Thr Tyr Ser Pro Asn Thr Ala Tyr Gly Val Asp Phe Leu
       50                  55                  60
   Val
   65
<210> 994
   <211> 64
   <212> PRT
   <213> Artificial Sequence
<220>
   <223> Ciz1 b-variant peptide comprising an exon 14b/exon 15 junction
<400> 994
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala Tyr Gly Val Asp Phe Leu Val
       50                  55                  60
<210> 995
   <211> 63
   <212> PRT
   <213> Artificial Sequence
<220>
   <223> Ciz1 b-variant peptide comprising an exon 14b/exon 15 junction
<400> 995
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser Pro Asn Thr Ala Tyr Gly Val Asp Phe Leu Val
       50                  55                  60
<210> 996
   <211> 62
   <212> PRT
   <213> Artificial Sequence
<220>
   <223> Ciz1 b-variant peptide comprising an exon 14b/exon 15 junction
<400> 996
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr Ser Pro Asn Thr Ala Tyr Gly Val Asp Phe Leu Val
       50                  55                  60
<210> 997
   <211> 61
   <212> PRT
   <213> Artificial Sequence
<220>
   <223> Ciz1 b-variant peptide comprising an exon 14b/exon 15 junction
<400> 997
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr Ser Pro Asn Thr Ala Tyr Gly Val Asp Phe Leu Val
       50                  55                  60
<210> 998
   <211> 60
   <212> PRT
   <213> Artificial Sequence
<220>
   <223> Ciz1 b-variant peptide comprising an exon 14b/exon 15 junction
<400> 998
   Asp Glu Asp His Phe Ile Thr Val Asp Ala Val Gly Cys Phe Glu Gly
   1 5 10 15
   Asp Glu Glu Glu Glu Glu Asp Asp Glu Asp Glu Glu Glu Ile Glu Val
   20 25 30
   Arg Ser Arg Asp Ile Ser Arg Glu Glu Trp Lys Gly Ser Glu Thr Tyr
   35 40 45
   Ser Pro Asn Thr Ala Tyr Gly Val Asp Phe Leu Val
   50 55 60
<210> 999
   <211> 59
   <212> PRT
   <213> Artificial Sequence
<220>
   <223> Ciz1 b-variant peptide comprising an exon 14b/exon 15 junction
<400> 999
   Glu Asp His Phe Ile Thr Val Asp Ala Val Gly Cys Phe Glu Gly Asp
   1 5 10 15
   Glu Glu Glu Glu Glu Asp Asp Glu Asp Glu Glu Glu Ile Glu Val Arg
   20 25 30
   Ser Arg Asp Ile Ser Arg Glu Glu Trp Lys Gly Ser Glu Thr Tyr Ser
   35 40 45
   Pro Asn Thr Ala Tyr Gly Val Asp Phe Leu Val
   50 55
<210> 1000
   <211> 58
   <212> PRT
   <213> Artificial Sequence
<220>
   <223> Ciz1 b-variant peptide comprising an exon 14b/exon 15 junction
<400> 1000
   Asp His Phe Ile Thr Val Asp Ala Val Gly Cys Phe Glu Gly Asp Glu
   1               5                   10                  15
   Glu Glu Glu Glu Asp Asp Glu Asp Glu Glu Glu Ile Glu Val Arg Ser
               20                  25                  30
   Arg Asp Ile Ser Arg Glu Glu Trp Lys Gly Ser Glu Thr Tyr Ser Pro
           35                  40                  45
   Asn Thr Ala Tyr Gly Val Asp Phe Leu Val
       50                  55
<210> 1001
   <211> 57
   <212> PRT
   <213> Artificial Sequence
<220>
   <223> Ciz1 b-variant peptide comprising an exon 14b/exon 15 junction
<400> 1001
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser Pro Asn
           35                  40                  45
   Thr Ala Tyr Gly Val Asp Phe Leu Val
       50                  55
<210> 1002
   <211> 56
   <212> PRT
   <213> Artificial Sequence
<220>
   <223> Ciz1 b-variant peptide comprising an exon 14b/exon 15 junction
<400> 1002
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro Asn Thr
           35                  40                  45
   Ala Tyr Gly Val Asp Phe Leu Val
       50                  55
<210> 1003
   <211> 55
   <212> PRT
   <213> Artificial Sequence
<220>
   <223> Ciz1 b-variant peptide comprising an exon 14b/exon 15 junction
<400> 1003
   Ile Thr Val Asp Ala Val Gly Cys Phe Glu Gly Asp Glu Glu Glu Glu
   1               5                   10                  15
   Glu Asp Asp Glu Asp Glu Glu Glu Ile Glu Val Arg Ser Arg Asp Ile
               20                  25                  30
   Ser Arg Glu Glu Trp Lys Gly Ser Glu Thr Tyr Ser Pro Asn Thr Ala
           35                  40                  45
   Tyr Gly Val Asp Phe Leu Val
       50                  55
<210> 1004
   <211> 54
   <212> PRT
   <213> Artificial Sequence
<220>
   <223> Ciz1 b-variant peptide comprising an exon 14b/exon 15 junction
<400> 1004
   Thr Val Asp Ala Val Gly Cys Phe Glu Gly Asp Glu Glu Glu Glu Glu
   1               5                   10                  15
   Asp Asp Glu Asp Glu Glu Glu Ile Glu Val Arg Ser Arg Asp Ile Ser
               20                  25                  30
   Arg Glu Glu Trp Lys Gly Ser Glu Thr Tyr Ser Pro Asn Thr Ala Tyr
           35                  40                  45
   Gly Val Asp Phe Leu Val
       50
<210> 1005
   <211> 53
   <212> PRT
   <213> Artificial Sequence
<220>
   <223> Ciz1 b-variant peptide comprising an exon 14b/exon 15 junction
<400> 1005
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala Tyr Gly
           35                  40                  45
   Val Asp Phe Leu Val
       50
<210> 1006
   <211> 52
   <212> PRT
   <213> Artificial Sequence
<220>
   <223> Ciz1 b-variant peptide comprising an exon 14b/exon 15 junction
<400> 1006
   Asp Ala Val Gly Cys Phe Glu Gly Asp Glu Glu Glu Glu Glu Asp Asp
   1 5 10 15
   Glu Asp Glu Glu Glu Ile Glu Val Arg Ser Arg Asp Ile Ser Arg Glu
   20 25 30
   Glu Trp Lys Gly Ser Glu Thr Tyr Ser Pro Asn Thr Ala Tyr Gly Val
   35 40 45
   Asp Phe Leu Val
   50
<210> 1007
   <211> 51
   <212> PRT
   <213> Artificial Sequence
<220>
   <223> Ciz1 b-variant peptide comprising an exon 14b/exon 15 junction
<400> 1007
   Ala Val Gly Cys Phe Glu Gly Asp Glu Glu Glu Glu Glu Asp Asp Glu
   1 5 10 15
   Asp Glu Glu Glu Ile Glu Val Arg Ser Arg Asp Ile Ser Arg Glu Glu
   20 25 30
   Trp Lys Gly Ser Glu Thr Tyr Ser Pro Asn Thr Ala Tyr Gly Val Asp
   35 40 45
   Phe Leu Val
   50
<210> 1008
   <211> 50
   <212> PRT
   <213> Artificial Sequence
<220>
   <223> Ciz1 b-variant peptide comprising an exon 14b/exon 15 junction
<400> 1008
   Val Gly Cys Phe Glu Gly Asp Glu Glu Glu Glu Glu Asp Asp Glu Asp
   1 5 10 15
   Glu Glu Glu Ile Glu Val Arg Ser Arg Asp Ile Ser Arg Glu Glu Trp
   20 25 30
   Lys Gly Ser Glu Thr Tyr Ser Pro Asn Thr Ala Tyr Gly Val Asp Phe
   35 40 45
   Leu Val
   50
<210> 1009
   <211> 49
   <212> PRT
   <213> Artificial Sequence
<220>
   <223> Ciz1 b-variant peptide comprising an exon 14b/exon 15 junction
<400> 1009
   Gly Cys Phe Glu Gly Asp Glu Glu Glu Glu Glu Asp Asp Glu Asp Glu
   1 5 10 15
   Glu Glu Ile Glu Val Arg Ser Arg Asp Ile Ser Arg Glu Glu Trp Lys
   20 25 30
   Gly Ser Glu Thr Tyr Ser Pro Asn Thr Ala Tyr Gly Val Asp Phe Leu
   35 40 45
   Val
<210> 1010
   <211> 48
   <212> PRT
   <213> Artificial Sequence
<220>
   <223> Ciz1 b-variant peptide comprising an exon 14b/exon 15 junction
<400> 1010
   Cys Phe Glu Gly Asp Glu Glu Glu Glu Glu Asp Asp Glu Asp Glu Glu
   1 5 10 15
   Glu Ile Glu Val Arg Ser Arg Asp Ile Ser Arg Glu Glu Trp Lys Gly
   20 25 30
   Ser Glu Thr Tyr Ser Pro Asn Thr Ala Tyr Gly Val Asp Phe Leu Val
   35 40 45
<210> 1011
   <211> 47
   <212> PRT
   <213> Artificial Sequence
<220>
   <223> Ciz1 b-variant peptide comprising an exon 14b/exon 15 junction
<400> 1011
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn Thr Ala Tyr Gly Val Asp Phe Leu Val
           35                  40                  45
<210> 1012
   <211> 46
   <212> PRT
   <213> Artificial Sequence
<220>
   <223> Ciz1 b-variant peptide comprising an exon 14b/exon 15 junction
<400> 1012
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn Thr Ala Tyr Gly Val Asp Phe Leu Val
           35                  40                  45
<210> 1013
   <211> 45
   <212> PRT
   <213> Artificial Sequence
<220>
   <223> Ciz1 b-variant peptide comprising an exon 14b/exon 15 junction
<400> 1013
   Gly Asp Glu Glu Glu Glu Glu Asp Asp Glu Asp Glu Glu Glu Ile Glu
   1 5 10 15
   Val Arg Ser Arg Asp Ile Ser Arg Glu Glu Trp Lys Gly Ser Glu Thr
   20 25 30
   Tyr Ser Pro Asn Thr Ala Tyr Gly Val Asp Phe Leu Val
   35 40 45
<210> 1014
   <211> 44
   <212> PRT
   <213> Artificial Sequence
<220>
   <223> Ciz1 b-variant peptide comprising an exon 14b/exon 15 junction
<400> 1014
   Asp Glu Glu Glu Glu Glu Asp Asp Glu Asp Glu Glu Glu Ile Glu Val
   1 5 10 15
   Arg Ser Arg Asp Ile Ser Arg Glu Glu Trp Lys Gly Ser Glu Thr Tyr
   20 25 30
   Ser Pro Asn Thr Ala Tyr Gly Val Asp Phe Leu Val
   35 40
<210> 1015
   <211> 43
   <212> PRT
   <213> Artificial Sequence
<220>
   <223> Ciz1 b-variant peptide comprising an exon 14b/exon 15 junction
<400> 1015
   Glu Glu Glu Glu Glu Asp Asp Glu Asp Glu Glu Glu Ile Glu Val Arg
   1 5 10 15
   Ser Arg Asp Ile Ser Arg Glu Glu Trp Lys Gly Ser Glu Thr Tyr Ser
   20 25 30
   Pro Asn Thr Ala Tyr Gly Val Asp Phe Leu Val
   35 40
<210> 1016
   <211> 42
   <212> PRT
   <213> Artificial Sequence
<220>
   <223> Ciz1 b-variant peptide comprising an exon 14b/exon 15 junction
<400> 1016
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr Ala Tyr Gly Val Asp Phe Leu Val
           35                  40
<210> 1017
   <211> 41
   <212> PRT
   <213> Artificial Sequence
<220>
   <223> Ciz1 b-variant peptide comprising an exon 14b/exon 15 junction
<400> 1017
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr Ala Tyr Gly Val Asp Phe Leu Val
           35                  40
<210> 1018
   <211> 40
   <212> PRT
   <213> Artificial Sequence
<220>
   <223> Ciz1 b-variant peptide comprising an exon 14b/exon 15 junction
<400> 1018
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
   Ala Tyr Gly Val Asp Phe Leu Val
           35                  40
<210> 1019
   <211> 39
   <212> PRT
   <213> Artificial Sequence
<220>
   <223> Ciz1 b-variant peptide comprising an exon 14b/exon 15 junction
<400> 1019
   Glu Asp Asp Glu Asp Glu Glu Glu Ile Glu Val Arg Ser Arg Asp Ile
   1               5                   10                  15
   Ser Arg Glu Glu Trp Lys Gly Ser Glu Thr Tyr Ser Pro Asn Thr Ala
               20                  25                  30
   Tyr Gly Val Asp Phe Leu Val
           35
<210> 1020
   <211> 38
   <212> PRT
   <213> Artificial Sequence
<220>
   <223> Ciz1 b-variant peptide comprising an exon 14b/exon 15 junction
<400> 1020
   Asp Asp Glu Asp Glu Glu Glu Ile Glu Val Arg Ser Arg Asp Ile Ser
   1               5                   10                  15
   Arg Glu Glu Trp Lys Gly Ser Glu Thr Tyr Ser Pro Asn Thr Ala Tyr
               20                  25                  30
   Gly Val Asp Phe Leu Val
           35
<210> 1021
   <211> 37
   <212> PRT
   <213> Artificial Sequence
<220>
   <223> Ciz1 b-variant peptide comprising an exon 14b/exon 15 junction
<400> 1021
   Asp Glu Asp Glu Glu Glu Ile Glu Val Arg Ser Arg Asp Ile Ser Arg
   1 5 10 15
   Glu Glu Trp Lys Gly Ser Glu Thr Tyr Ser Pro Asn Thr Ala Tyr Gly
   20 25 30
   Val Asp Phe Leu Val
   35
<210> 1022
   <211> 36
   <212> PRT
   <213> Artificial Sequence
<220>
   <223> Ciz1 b-variant peptide comprising an exon 14b/exon 15 junction
<400> 1022
   Glu Asp Glu Glu Glu Ile Glu Val Arg Ser Arg Asp Ile Ser Arg Glu
   1 5 10 15
   Glu Trp Lys Gly Ser Glu Thr Tyr Ser Pro Asn Thr Ala Tyr Gly Val
   20 25 30
   Asp Phe Leu Val
   35
<210> 1023
   <211> 35
   <212> PRT
   <213> Artificial Sequence
<220>
   <223> Ciz1 b-variant peptide comprising an exon 14b/exon 15 junction
<400> 1023
   Asp Glu Glu Glu Ile Glu Val Arg Ser Arg Asp Ile Ser Arg Glu Glu
   1 5 10 15
   Trp Lys Gly Ser Glu Thr Tyr Ser Pro Asn Thr Ala Tyr Gly Val Asp
   20 25 30
   Phe Leu Val
   35
<210> 1024
   <211> 34
   <212> PRT
   <213> Artificial Sequence
<220>
   <223> Ciz1 b-variant peptide comprising an exon 14b/exon 15 junction
<400> 1024
   Glu Glu Glu Ile Glu Val Arg Ser Arg Asp Ile Ser Arg Glu Glu Trp
   1 5 10 15
   Lys Gly Ser Glu Thr Tyr Ser Pro Asn Thr Ala Tyr Gly Val Asp Phe
   20 25 30
   Leu Val
<210> 1025
   <211> 33
   <212> PRT
   <213> Artificial Sequence
<220>
   <223> Ciz1 b-variant peptide comprising an exon 14b/exon 15 junction
<400> 1025
   Glu Glu Ile Glu Val Arg Ser Arg Asp Ile Ser Arg Glu Glu Trp Lys
   1 5 10 15
   Gly Ser Glu Thr Tyr Ser Pro Asn Thr Ala Tyr Gly Val Asp Phe Leu
   20 25 30
   Val
<210> 1026
   <211> 32
   <212> PRT
   <213> Artificial Sequence
<220>
   <223> Ciz1 b-variant peptide comprising an exon 14b/exon 15 junction
<400> 1026
   Glu Ile Glu Val Arg Ser Arg Asp Ile Ser Arg Glu Glu Trp Lys Gly
   1 5 10 15
   Ser Glu Thr Tyr Ser Pro Asn Thr Ala Tyr Gly Val Asp Phe Leu Val
   20 25 30
<210> 1027
   <211> 31
   <212> PRT
   <213> Artificial Sequence
<220>
   <223> Ciz1 b-variant peptide comprising an exon 14b/exon 15 junction
<400> 1027
   Ile Glu Val Arg Ser Arg Asp Ile Ser Arg Glu Glu Trp Lys Gly Ser
   1               5                   10                  15
   Glu Thr Tyr Ser Pro Asn Thr Ala Tyr Gly Val Asp Phe Leu Val
               20                  25                  30
<210> 1028
   <211> 30
   <212> PRT
   <213> Artificial Sequence
<220>
   <223> Ciz1 b-variant peptide comprising an exon 14b/exon 15 junction
<400> 1028
   Glu Val Arg Ser Arg Asp Ile Ser Arg Glu Glu Trp Lys Gly Ser Glu
   1               5                   10                  15
   Thr Tyr Ser Pro Asn Thr Ala Tyr Gly Val Asp Phe Leu Val
               20                  25                  30
<210> 1029
   <211> 65
   <212> PRT
   <213> Artificial Sequence
<220>
   <223> Ciz1 b-variant peptide comprising an exon 14b/exon 15 junction
<400> 1029
   Ile Ala Gly Gln Asp Glu Asp His Phe Ile Thr Val Asp Ala Val Gly
   1               5                   10                  15
   Cys Phe Glu Gly Asp Glu Glu Glu Glu Glu Asp Asp Glu Asp Glu Glu
               20                  25                  30
   Glu Ile Glu Val Arg Ser Arg Asp Ile Ser Arg Glu Glu Trp Lys Gly
           35                  40                  45
   Ser Glu Thr Tyr Ser Pro Asn Thr Ala Tyr Gly Val Asp Phe Leu Val
       50                  55                  60
   Pro
   65
<210> 1030
   <211> 64
   <212> PRT
   <213> Artificial Sequence
<220>
   <223> Ciz1 b-variant peptide comprising an exon 14b/exon 15 junction
<400> 1030
   Ala Gly Gln Asp Glu Asp His Phe Ile Thr Val Asp Ala Val Gly Cys
   1               5                   10                  15
   Phe Glu Gly Asp Glu Glu Glu Glu Glu Asp Asp Glu Asp Glu Glu Glu
               20                  25                  30
   Ile Glu Val Arg Ser Arg Asp Ile Ser Arg Glu Glu Trp Lys Gly Ser
           35                  40                  45
   Glu Thr Tyr Ser Pro Asn Thr Ala Tyr Gly Val Asp Phe Leu Val Pro
       50                  55                  60
<210> 1031
   <211> 63
   <212> PRT
   <213> Artificial Sequence
<220>
   <223> Ciz1 b-variant peptide comprising an exon 14b/exon 15 junction
<400> 1031
   Gly Gln Asp Glu Asp His Phe Ile Thr Val Asp Ala Val Gly Cys Phe
   1               5                   10                  15
   Glu Gly Asp Glu Glu Glu Glu Glu Asp Asp Glu Asp Glu Glu Glu Ile
               20                  25                  30
   Glu Val Arg Ser Arg Asp Ile Ser Arg Glu Glu Trp Lys Gly Ser Glu
           35                  40                  45
   Thr Tyr Ser Pro Asn Thr Ala Tyr Gly Val Asp Phe Leu Val Pro
       50                  55                  60
<210> 1032
   <211> 62
   <212> PRT
   <213> Artificial Sequence
<220>
   <223> Ciz1 b-variant peptide comprising an exon 14b/exon 15 junction
<400> 1032
   Gln Asp Glu Asp His Phe Ile Thr Val Asp Ala Val Gly Cys Phe Glu
   1               5                   10                  15
   Gly Asp Glu Glu Glu Glu Glu Asp Asp Glu Asp Glu Glu Glu Ile Glu
               20                  25                  30
   Val Arg Ser Arg Asp Ile Ser Arg Glu Glu Trp Lys Gly Ser Glu Thr
           35                  40                  45
   Tyr Ser Pro Asn Thr Ala Tyr Gly Val Asp Phe Leu Val Pro
       50                  55                  60
<210> 1033
   <211> 61
   <212> PRT
   <213> Artificial Sequence
<220>
   <223> Ciz1 b-variant peptide comprising an exon 14b/exon 15 junction
<400> 1033
   Asp Glu Asp His Phe Ile Thr Val Asp Ala Val Gly Cys Phe Glu Gly
   1               5                   10                  15
   Asp Glu Glu Glu Glu Glu Asp Asp Glu Asp Glu Glu Glu Ile Glu Val
               20                  25                  30
   Arg Ser Arg Asp Ile Ser Arg Glu Glu Trp Lys Gly Ser Glu Thr Tyr
           35                  40                  45
   Ser Pro Asn Thr Ala Tyr Gly Val Asp Phe Leu Val Pro
       50                  55                  60
<210> 1034
   <211> 60
   <212> PRT
   <213> Artificial Sequence
<220>
   <223> Ciz1 b-variant peptide comprising an exon 14b/exon 15 junction
<400> 1034
   Glu Asp His Phe Ile Thr Val Asp Ala Val Gly Cys Phe Glu Gly Asp
   1 5 10 15
   Glu Glu Glu Glu Glu Asp Asp Glu Asp Glu Glu Glu Ile Glu Val Arg
   20 25 30
   Ser Arg Asp Ile Ser Arg Glu Glu Trp Lys Gly Ser Glu Thr Tyr Ser
   35 40 45
   Pro Asn Thr Ala Tyr Gly Val Asp Phe Leu Val Pro
   50 55 60
<210> 1035
   <211> 59
   <212> PRT
   <213> Artificial Sequence
<220>
   <223> Ciz1 b-variant peptide comprising an exon 14b/exon 15 junction
<400> 1035
   Asp His Phe Ile Thr Val Asp Ala Val Gly Cys Phe Glu Gly Asp Glu
   1 5 10 15
   Glu Glu Glu Glu Asp Asp Glu Asp Glu Glu Glu Ile Glu Val Arg Ser
   20 25 30
   Arg Asp Ile Ser Arg Glu Glu Trp Lys Gly Ser Glu Thr Tyr Ser Pro
   35 40 45
   Asn Thr Ala Tyr Gly Val Asp Phe Leu Val Pro
   50 55
<210> 1036
   <211> 58
   <212> PRT
   <213> Artificial Sequence
<220>
   <223> Ciz1 b-variant peptide comprising an exon 14b/exon 15 junction
<400> 1036
   His Phe Ile Thr Val Asp Ala Val Gly Cys Phe Glu Gly Asp Glu Glu
   1               5                   10                  15
   Glu Glu Glu Asp Asp Glu Asp Glu Glu Glu Ile Glu Val Arg Ser Arg
               20                  25                  30
   Asp Ile Ser Arg Glu Glu Trp Lys Gly Ser Glu Thr Tyr Ser Pro Asn
           35                  40                  45
   Thr Ala Tyr Gly Val Asp Phe Leu Val Pro
       50                  55
<210> 1037
   <211> 57
   <212> PRT
   <213> Artificial Sequence
<220>
   <223> Ciz1 b-variant peptide comprising an exon 14b/exon 15 junction
<400> 1037
   Phe Ile Thr Val Asp Ala Val Gly Cys Phe Glu Gly Asp Glu Glu Glu
   1               5                   10                  15
   Glu Glu Asp Asp Glu Asp Glu Glu Glu Ile Glu Val Arg Ser Arg Asp
               20                  25                  30
   Ile Ser Arg Glu Glu Trp Lys Gly Ser Glu Thr Tyr Ser Pro Asn Thr
           35                  40                  45
   Ala Tyr Gly Val Asp Phe Leu Val Pro
       50                  55
<210> 1038
   <211> 56
   <212> PRT
   <213> Artificial Sequence
<220>
   <223> Ciz1 b-variant peptide comprising an exon 14b/exon 15 junction
<400> 1038
   Ile Thr Val Asp Ala Val Gly Cys Phe Glu Gly Asp Glu Glu Glu Glu
   1 5 10 15
   Glu Asp Asp Glu Asp Glu Glu Glu Ile Glu Val Arg Ser Arg Asp Ile
   20 25 30
   Ser Arg Glu Glu Trp Lys Gly Ser Glu Thr Tyr Ser Pro Asn Thr Ala
   35 40 45
   Tyr Gly Val Asp Phe Leu Val Pro
   50 55
<210> 1039
   <211> 55
   <212> PRT
   <213> Artificial Sequence
<220>
   <223> Ciz1 b-variant peptide comprising an exon 14b/exon 15 junction
<400> 1039
   Thr Val Asp Ala Val Gly Cys Phe Glu Gly Asp Glu Glu Glu Glu Glu
   1 5 10 15
   Asp Asp Glu Asp Glu Glu Glu Ile Glu Val Arg Ser Arg Asp Ile Ser
   20 25 30
   Arg Glu Glu Trp Lys Gly Ser Glu Thr Tyr Ser Pro Asn Thr Ala Tyr
   35 40 45
   Gly Val Asp Phe Leu Val Pro
   50 55
<210> 1040
   <211> 54
   <212> PRT
   <213> Artificial Sequence
<220>
   <223> Ciz1 b-variant peptide comprising an exon 14b/exon 15 junction
<400> 1040
   Val Asp Ala Val Gly Cys Phe Glu Gly Asp Glu Glu Glu Glu Glu Asp
   1               5                   10                  15
   Asp Glu Asp Glu Glu Glu Ile Glu Val Arg Ser Arg Asp Ile Ser Arg
               20                  25                  30
   Glu Glu Trp Lys Gly Ser Glu Thr Tyr Ser Pro Asn Thr Ala Tyr Gly
   35                  40                  45
   Val Asp Phe Leu Val Pro
       50
<210> 1041
   <211> 53
   <212> PRT
   <213> Artificial Sequence
<220>
   <223> Ciz1 b-variant peptide comprising an exon 14b/exon 15 junction
<400> 1041
   Asp Ala Val Gly Cys Phe Glu Gly Asp Glu Glu Glu Glu Glu Asp Asp
   1               5                   10                  15
   Glu Asp Glu Glu Glu Ile Glu Val Arg Ser Arg Asp Ile Ser Arg Glu
               20                  25                  30
   Glu Trp Lys Gly Ser Glu Thr Tyr Ser Pro Asn Thr Ala Tyr Gly Val
           35                  40                  45
   Asp Phe Leu Val Pro
       50
<210> 1042
   <211> 52
   <212> PRT
   <213> Artificial Sequence
<220>
   <223> Ciz1 b-variant peptide comprising an exon 14b/exon 15 junction
<400> 1042
   Ala Val Gly Cys Phe Glu Gly Asp Glu Glu Glu Glu Glu Asp Asp Glu
   1               5                   10                  15
   Asp Glu Glu Glu Ile Glu Val Arg Ser Arg Asp Ile Ser Arg Glu Glu
               20                  25                  30
   Trp Lys Gly Ser Glu Thr Tyr Ser Pro Asn Thr Ala Tyr Gly Val Asp
           35                  40                  45
   Phe Leu Val Pro
       50
<210> 1043
   <211> 51
   <212> PRT
   <213> Artificial Sequence
<220>
   <223> Ciz1 b-variant peptide comprising an exon 14b/exon 15 junction
<400> 1043
   Val Gly Cys Phe Glu Gly Asp Glu Glu Glu Glu Glu Asp Asp Glu Asp
   1               5                   10                  15
   Glu Glu Glu Ile Glu Val Arg Ser Arg Asp Ile Ser Arg Glu Glu Trp
               20                  25                  30
   Lys Gly Ser Glu Thr Tyr Ser Pro Asn Thr Ala Tyr Gly Val Asp Phe
           35                  40                  45
   Leu Val Pro
       50
<210> 1044
   <211> 50
   <212> PRT
   <213> Artificial Sequence
<220>
   <223> Ciz1 b-variant peptide comprising an exon 14b/exon 15 junction
<400> 1044
   Gly Cys Phe Glu Gly Asp Glu Glu Glu Glu Glu Asp Asp Glu Asp Glu
   1               5                   10                  15
   Glu Glu Ile Glu Val Arg Ser Arg Asp Ile Ser Arg Glu Glu Trp Lys
               20                  25                  30
   Gly Ser Glu Thr Tyr Ser Pro Asn Thr Ala Tyr Gly Val Asp Phe Leu
           35                  40                  45
   Val Pro
       50
<210> 1045
   <211> 49
   <212> PRT
   <213> Artificial Sequence
<220>
   <223> Ciz1 b-variant peptide comprising an exon 14b/exon 15 junction
<400> 1045
   Cys Phe Glu Gly Asp Glu Glu Glu Glu Glu Asp Asp Glu Asp Glu Glu
   1               5                   10                  15
   Glu Ile Glu Val Arg Ser Arg Asp Ile Ser Arg Glu Glu Trp Lys Gly
               20                  25                  30
   Ser Glu Thr Tyr Ser Pro Asn Thr Ala Tyr Gly Val Asp Phe Leu Val
           35                  40                  45
   Pro
<210> 1046
   <211> 48
   <212> PRT
   <213> Artificial Sequence
<220>
   <223> Ciz1 b-variant peptide comprising an exon 14b/exon 15 junction
<400> 1046
   Phe Glu Gly Asp Glu Glu Glu Glu Glu Asp Asp Glu Asp Glu Glu Glu
   1               5                   10                  15
   Ile Glu Val Arg Ser Arg Asp Ile Ser Arg Glu Glu Trp Lys Gly Ser
               20                  25                  30
   Glu Thr Tyr Ser Pro Asn Thr Ala Tyr Gly Val Asp Phe Leu Val Pro
           35                  40                  45
<210> 1047
   <211> 47
   <212> PRT
   <213> Artificial Sequence
<220>
   <223> Ciz1 b-variant peptide comprising an exon 14b/exon 15 junction
<400> 1047
   Glu Gly Asp Glu Glu Glu Glu Glu Asp Asp Glu Asp Glu Glu Glu Ile
   1               5                   10                  15
   Glu Val Arg Ser Arg Asp Ile Ser Arg Glu Glu Trp Lys Gly Ser Glu
               20                  25                  30
   Thr Tyr Ser Pro Asn Thr Ala Tyr Gly Val Asp Phe Leu Val Pro
           35                  40                  45
<210> 1048
   <211> 46
   <212> PRT
   <213> Artificial Sequence
<220>
   <223> Ciz1 b-variant peptide comprising an exon 14b/exon 15 junction
<400> 1048
   Gly Asp Glu Glu Glu Glu Glu Asp Asp Glu Asp Glu Glu Glu Ile Glu
   1               5                   10                  15
   Val Arg Ser Arg Asp Ile Ser Arg Glu Glu Trp Lys Gly Ser Glu Thr
               20                  25                  30
   Tyr Ser Pro Asn Thr Ala Tyr Gly Val Asp Phe Leu Val Pro
           35                  40                  45
<210> 1049
   <211> 45
   <212> PRT
   <213> Artificial Sequence
<220>
   <223> Ciz1 b-variant peptide comprising an exon 14b/exon 15 junction
<400> 1049
   Asp Glu Glu Glu Glu Glu Asp Asp Glu Asp Glu Glu Glu Ile Glu Val
   1               5                   10                  15
   Arg Ser Arg Asp Ile Ser Arg Glu Glu Trp Lys Gly Ser Glu Thr Tyr
               20                  25                  30
   Ser Pro Asn Thr Ala Tyr Gly Val Asp Phe Leu Val Pro
           35                  40                  45
<210> 1050
   <211> 44
   <212> PRT
   <213> Artificial Sequence
<220>
   <223> Ciz1 b-variant peptide comprising an exon 14b/exon 15 junction
<400> 1050
   Glu Glu Glu Glu Glu Asp Asp Glu Asp Glu Glu Glu Ile Glu Val Arg
   1               5                   10                  15
   Ser Arg Asp Ile Ser Arg Glu Glu Trp Lys Gly Ser Glu Thr Tyr Ser
               20                  25                  30
   Pro Asn Thr Ala Tyr Gly Val Asp Phe Leu Val Pro
           35                  40
<210> 1051
   <211> 43
   <212> PRT
   <213> Artificial Sequence
<220>
   <223> Ciz1 b-variant peptide comprising an exon 14b/exon 15 junction
<400> 1051
   Glu Glu Glu Glu Asp Asp Glu Asp Glu Glu Glu Ile Glu Val Arg Ser
   1               5                   10                  15
   Arg Asp Ile Ser Arg Glu Glu Trp Lys Gly Ser Glu Thr Tyr Ser Pro
               20                  25                  30
   Asn Thr Ala Tyr Gly Val Asp Phe Leu Val Pro
           35                  40
<210> 1052
   <211> 42
   <212> PRT
   <213> Artificial Sequence
<220>
   <223> Ciz1 b-variant peptide comprising an exon 14b/exon 15 junction
<400> 1052
   Glu Glu Glu Asp Asp Glu Asp Glu Glu Glu Ile Glu Val Arg Ser Arg
   1               5                   10                  15
   Asp Ile Ser Arg Glu Glu Trp Lys Gly Ser Glu Thr Tyr Ser Pro Asn
               20                  25                  30
   Thr Ala Tyr Gly Val Asp Phe Leu Val Pro
           35                  40
<210> 1053
   <211> 41
   <212> PRT
   <213> Artificial Sequence
<220>
   <223> Ciz1 b-variant peptide comprising an exon 14b/exon 15 junction
<400> 1053
   Glu Glu Asp Asp Glu Asp Glu Glu Glu Ile Glu Val Arg Ser Arg Asp
   1               5                   10                  15
   Ile Ser Arg Glu Glu Trp Lys Gly Ser Glu Thr Tyr Ser Pro Asn Thr
               20                  25                  30
   Ala Tyr Gly Val Asp Phe Leu Val Pro
           35                  40
<210> 1054
   <211> 40
   <212> PRT
   <213> Artificial Sequence
<220>
   <223> Ciz1 b-variant peptide comprising an exon 14b/exon 15 junction
<400> 1054
   Glu Asp Asp Glu Asp Glu Glu Glu Ile Glu Val Arg Ser Arg Asp Ile
   1 5 10 15
   Ser Arg Glu Glu Trp Lys Gly Ser Glu Thr Tyr Ser Pro Asn Thr Ala
   20 25 30
   Tyr Gly Val Asp Phe Leu Val Pro
   35 40
<210> 1055
   <211> 39
   <212> PRT
   <213> Artificial Sequence
<220>
   <223> Ciz1 b-variant peptide comprising an exon 14b/exon 15 junction
<400> 1055
   Asp Asp Glu Asp Glu Glu Glu Ile Glu Val Arg Ser Arg Asp Ile Ser
   1 5 10 15
   Arg Glu Glu Trp Lys Gly Ser Glu Thr Tyr Ser Pro Asn Thr Ala Tyr
   20 25 30
   Gly Val Asp Phe Leu Val Pro
   35
<210> 1056
   <211> 38
   <212> PRT
   <213> Artificial Sequence
<220>
   <223> Ciz1 b-variant peptide comprising an exon 14b/exon 15 junction
<400> 1056
   Asp Glu Asp Glu Glu Glu Ile Glu Val Arg Ser Arg Asp Ile Ser Arg
   1 5 10 15
   Glu Glu Trp Lys Gly Ser Glu Thr Tyr Ser Pro Asn Thr Ala Tyr Gly
   20 25 30
   Val Asp Phe Leu Val Pro
   35
<210> 1057
   <211> 37
   <212> PRT
   <213> Artificial Sequence
<220>
   <223> Ciz1 b-variant peptide comprising an exon 14b/exon 15 junction
<400> 1057
   Glu Asp Glu Glu Glu Ile Glu Val Arg Ser Arg Asp Ile Ser Arg Glu
   1               5                   10                  15
   Glu Trp Lys Gly Ser Glu Thr Tyr Ser Pro Asn Thr Ala Tyr Gly Val
               20                  25                  30
   Asp Phe Leu Val Pro
           35
<210> 1058
   <211> 36
   <212> PRT
   <213> Artificial Sequence
<220>
   <223> Ciz1 b-variant peptide comprising an exon 14b/exon 15 junction
<400> 1058
   Asp Glu Glu Glu Ile Glu Val Arg Ser Arg Asp Ile Ser Arg Glu Glu
   1               5                   10                  15
   Trp Lys Gly Ser Glu Thr Tyr Ser Pro Asn Thr Ala Tyr Gly Val Asp
               20                  25                  30
   Phe Leu Val Pro
           35
<210> 1059
   <211> 35
   <212> PRT
   <213> Artificial Sequence
<220>
   <223> Ciz1 b-variant peptide comprising an exon 14b/exon 15 junction
<400> 1059
   Glu Glu Glu Ile Glu Val Arg Ser Arg Asp Ile Ser Arg Glu Glu Trp
   1               5                   10                  15
   Lys Gly Ser Glu Thr Tyr Ser Pro Asn Thr Ala Tyr Gly Val Asp Phe
               20                  25                  30
   Leu Val Pro
           35
<210> 1060
   <211> 34
   <212> PRT
   <213> Artificial Sequence
<220>
   <223> Ciz1 b-variant peptide comprising an exon 14b/exon 15 junction
<400> 1060
   Glu Glu Ile Glu Val Arg Ser Arg Asp Ile Ser Arg Glu Glu Trp Lys
   1               5                   10                  15
   Gly Ser Glu Thr Tyr Ser Pro Asn Thr Ala Tyr Gly Val Asp Phe Leu
               20                  25                  30
   Val Pro
<210> 1061
   <211> 33
   <212> PRT
   <213> Artificial Sequence
<220>
   <223> Ciz1 b-variant peptide comprising an exon 14b/exon 15 junction
<400> 1061
   Glu Ile Glu Val Arg Ser Arg Asp Ile Ser Arg Glu Glu Trp Lys Gly
   1               5                   10                  15
   Ser Glu Thr Tyr Ser Pro Asn Thr Ala Tyr Gly Val Asp Phe Leu Val
               20                  25                  30
   Pro
<210> 1062
   <211> 32
   <212> PRT
   <213> Artificial Sequence
<220>
   <223> Ciz1 b-variant peptide comprising an exon 14b/exon 15 junction
<400> 1062
   Ile Glu Val Arg Ser Arg Asp Ile Ser Arg Glu Glu Trp Lys Gly Ser
   1               5                   10                  15
   Glu Thr Tyr Ser Pro Asn Thr Ala Tyr Gly Val Asp Phe Leu Val Pro
               20                  25                  30
<210> 1063
   <211> 31
   <212> PRT
   <213> Artificial Sequence
<220>
   <223> Ciz1 b-variant peptide comprising an exon 14b/exon 15 junction
<400> 1063
   Glu Val Arg Ser Arg Asp Ile Ser Arg Glu Glu Trp Lys Gly Ser Glu
   1               5                   10                  15
   Thr Tyr Ser Pro Asn Thr Ala Tyr Gly Val Asp Phe Leu Val Pro
               20                  25                  30

## Description

### FIELD OF THE INVENTION

The present invention relates to assays and methods useful for the detection of Cip1-interacting zinc finger protein (Ciz1) in a sample. In particular, the present invention relates to the use of such assays and methods for the diagnosis of cancer.

### BACKGROUND OF THE INVENTION

Cip1-interacting zinc finger protein 1 (Ciz1) (as exemplified by NCBI Reference Sequence: NM_001131016.1) is required for cell proliferation. Ciz1 localises to nuclear matrix bound foci that form sites of DNA replication during early S phase and promotes the initiation of DNA replication in association with cell cycle regulators including cyclin A / cyclin dependent kinase (CDK)2, cyclin E/CDK2 and p21cip1. In the context of transcription, *CIZ1* is an oestrogen responsive gene that is itself a positive cofactor of the oestrogen receptor (ER), capable of enhancing the recruitment of ER to target chromatin. Ciz1 is alternatively spliced to produce conserved isoforms in mouse and man. Normal Ciz1 protein comprises at least two defined functional domains, a 'replication' domain and an 'immobilisation' domain.

Alternative splicing of Ciz1 exon 14 to generate a Ciz1 b-variant has been determined in various cancers, including small cell lung cancer (SCLC), non-small cell lung cancer (NSCLC), lymphomas, thyroid, kidney and liver cancer. Excess expression of either the replication or immobilisation domain of Ciz1 has also been demonstrated in cancers, for example in NSCLC, breast, colon, kidney, liver, bladder and thyroid cancers. A correlation of domain expression with the stage of the cancer has also been determined (see WO 2012/078208).

Higgins, G., et al., 2012. Proceedings of the National Academy of Sciences, 109(45), pp.E3128-E3135 discloses that variant Ciz1 is a strong candidate for a cancer-specific single marker capable of identifying early-stage lung cancer within at-risk groups without resort to invasive procedures.

The present invention addresses the continued need to develop diagnostic tests and treatments that improve the survival rates of patients suffering from cancers such as lung cancer through novel biomarkers and targets.

### SUMMARY OF THE INVENTION

The present inventors have determined that Ciz1 b-variant exists in a complex with fibrinogen. This has allowed the inventors to develop a novel assay for detecting the presence of Ciz1 b-variant in a subject.

Furthermore, the present inventors have determined that Ciz1 b-variant may exist as peptide fragments. Furthermore, the present inventors have determined that Ciz1 b-variant and/or fibrinogen/Ciz1 b-variant complexes may exist in the exosomal compartment of a blood sample.

These findings instruct the design of assays for the detection of Ciz1 b-variant peptides, either when alone or when in complex with fibrinogen.

In particular, the finding that Ciz1 b-variant is complexed with fibrinogen has enabled the provision of a detection assay, in particular a diagnostic assay, which utilises the capture of fibrinogen or Ciz1 b-variant as an initial step in order to isolate the fibrinogen/Ciz1 b-variant complex. The assay may involve the use a fibrinogen capture agent to isolate the fibrinogen/Ciz1 b-variant complex followed by the use of Ciz1 b-variant detection agent to detect the Ciz1 b-variant which may be within said isolated complex. Alternatively, the assay may involve the use a Ciz1 b-variant capture agent to isolate the fibrinogen/Ciz1 b-variant complex followed by the use of fibrinogen detection agent to detect the fibrinogen which may be within said isolated complex.

Thus, in a first aspect the present invention relates to the use of a fibrinogen capture agent or a fibrinogen detection agent in an assay to detect a Ciz1 b-variant.

As used herein, a capture agent refers to an agent which is used to isolate (i.e. enrich) the fibrinogen/Ciz1 b-variant complex from a sample. For example, the sample may be a sample which has been isolated from a subject. In particular, the sample may be a blood sample (e.g. a plasma sample) which has been isolated from a subject.

As used herein, a detection agent refers to an agent which is used to bind the fibrinogen/Ciz1 b-variant complex after it has been isolated (i.e. enriched) from a sample using a capture agent. As such, the detection agent is typically used after a capture step to determine the presence of the isolated (i.e. enriched) fibrinogen/Ciz1 b-variant complex.

Suitably, the assay may be an enzyme-linked immunosorbent assay.

The fibrinogen capture agent or fibrinogen detection agent may be an antibody or a fragment thereof, or Ciz1 b-variant peptide which specifically binds fibrinogen.

In a preferred embodiment, the fibrinogen capture agent or fibrinogen detection agent is an antibody or a fragment thereof which specifically binds fibrinogen. In another preferred embodiment, the fibrinogen capture agent is a Ciz1 b-variant peptide which specifically binds fibrinogen.

The fibrinogen may be fibrinogen alpha chain.

The Ciz1 b-variant may be detected in a sample from a subject, for example a blood, urine, saliva or bronchoalveolar lavage sample.

In a preferred embodiment the sample is a blood sample from a subject. In a particularly preferred embodiment the sample is a plasma sample.

The Ciz1 b-variant may be detected by a method which comprises the following steps: a) capturing a fibrinogen/Ciz1 b-variant complex using a fibrinogen capture agent; and b) detecting Ciz1 b-variant using a Ciz1 b-variant detection agent; or a) capturing a fibrinogen/Ciz1 b-variant complex using a Ciz1 b-variant capture agent; and b) detecting fibrinogen using a fibrinogen detection agent.

The Ciz1 b-variant capture agent may be an antibody or a fragment thereof, which specifically binds Ciz1 b-variant.

The Ciz1 b-variant capture agent may be an antibody or a fragment thereof which specifically binds Ciz1 b-variant.

In a further aspect the present invention relates to a method of diagnosing cancer in a subject which comprises detecting a Ciz1 b-variant peptide in a sample from the subject, wherein the presence of Ciz1 b-variant peptide in the sample indicates that the subject has cancer and wherein the method comprises the step of capturing a fibrinogen/Ciz1 b-variant peptide complex from the sample.

The method may comprise the steps of capturing a fibrinogen/Ciz1 b-variant complex using a fibrinogen capture agent; and detecting Ciz1 b-variant using a Ciz1 b-variant detection agent; or capturing a fibrinogen/Ciz1 b-variant complex using a Ciz1 b-variant capture agent; and detecting fibrinogen using a fibrinogen detection agent.

Suitably, the step of detecting the Ciz1 b-variant peptide or fibrinogen may employ antibody-based arrays, enzyme linked immunosorbent assays (ELISA), radioimmuno-assay (RIA), western blotting or mass spectrometry.

The method may further comprise the step of releasing a fibrinogen/Ciz1 b-variant peptide complex from an exosomal component of the sample.

The fibrinogen/Ciz1 b-variant peptide complex may be released from the exosomal component by detergent treatment, such as SDS treatment.

The method may further comprise the step of enriching the exosomal fraction from the sample.

For example, the step of enriching the exosomal fraction may comprise ultracentrifugation, ultrafiltration, continuous flow electrophoresis, chromatography or cross-flow ultrafiltration.

The cancer may be selected from lung, lymphoma, kidney, breast, liver, bladder, ovarian or thyroid cancer.

In particular, the cancer may be lung cancer.

Described herein is a method for treating a subject with cancer comprising administering to the subject a cancer therapeutic; wherein the subject has been identified as having cancer by the method of the invention.

Described herein is an anticancer lung cancer drug for use in treating lung cancer wherein the subject has been identified as having lung cancer by the method of the invention.

In a further aspect the present invention provides the use of a detergent to release a fibrinogen/Ciz1 b-variant complex from an exosomal compartment of a sample.

In another aspect the present invention relates to a method for detecting a Ciz1 b-variant in a sample which comprises the step of releasing a fibrinogen/Ciz1 b-variant complex from an exosomal compartment of the sample by treating the sample with detergent and detecting if the Ciz1 b-variant is present in the sample. The method may further comprise the step of enriching the exosomal fraction from the sample prior to releasing the Ciz1 b-variant.

The step of enriching the exosomal fraction may comprise ultracentrifugation, ultrafiltration, continuous flow electrophoresis, chromatography or cross-flow ultrafiltration.

In a further aspect the present invention provides a kit comprising an agent which specifically binds fibrinogen and an agent which specifically binds Ciz1 b-variant.

The fibrinogen may fibrinogen alpha chain.

The agent which specifically binds fibrinogen and/or the agent which specifically binds Ciz1 b-variant may be an antibody or a fragment thereof.

The agent which specifically binds to fibrinogen may be a Ciz1 b-variant peptide.

According to any of the above aspects of the present invention, the Ciz1 b-variant may be a Ciz1 b-variant peptide shown as SEQ ID NO: 11 or a fragment thereof which comprises EVR as shown in positions 36 to 38 of SEQ ID NO: 11,

### DESCRIPTION OF THE DRAWINGS

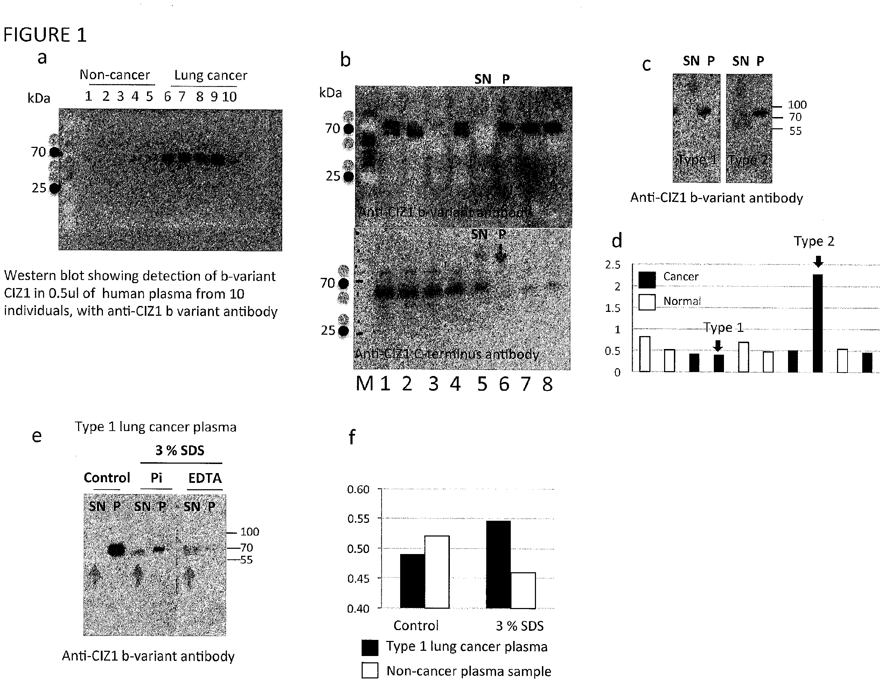

**Figure** 1 - a) Ciz1 b-variant epitope-containing species, detected with splice junction-selective anti-Ciz1 rabbit polyclonal antibody 043. 043 was generated and validated as described for antibody 2B (Higgins et al.; (2012); PNAS; Nov 6;109(45):E3128-35) and is capable of detection of Ciz1 b-variant by western blot in plasma from lung cancer patients. Western blots were performed and show Ciz1 b-variant in 0.5ul of representative samples from 5 patients with lung carcinomas (various stages) and five from individuals with no disease. b) Separation of plasma from lung cancer patients into the exosomal compartment and soluble compartment illustrated here using Invitrogen Total Exosome Isolation Kit (Catalog Number 4484450) used as recommended by the manufacturer. Fractions were separated by SDS-PAGE, and probed with 043 to reveal Ciz1 b-variant, or with 'generic' Ciz1 antibodies that recognizes an epitope elsewhere in the protein (data shown here is generated with NB100-74624 (Nov4), Novus Biologicals that detects the C-terminus of Ciz1). Lane 1, untreated plasma (0.5ul), lane 2 after low speed clearing spin (0.5ul), lane 3 after high speed clearing spin (0.5ul), lane 4 after addition of exosome reagent (0.5ul equivalent), lane 5 high speed spin supernatant (SN, 0.5ul equivalent), lane 6 high speed spin pellet (0.5ul equivalent), lane 7 high speed spin pellet (P, 1ul equivalent), lane 8 high speed spin pellet (2ul equivalent). Red arrows indicate key lanes, showing different partitioning of Ciz1 epitopes. M, molecular weight marker. c) Comparison of type 1 plasma samples in which Ciz1 b-variant is exclusively in the exosome fraction, and rare patients who display a type 2 pattern in which a fraction of Ciz1 b-variant is in the soluble fraction. d) Quantitative immuno-analysis generated using a prototype sandwich ELISA (without treating samples with detergent) showing increased signal in the type 2 plasma sample illustrated in c. e) Partitioning of Ciz1 b-variant in naïve type 1 plasma, and after treatment with 3% SDS (tested in the presence of protease inhibitor, PI, or 10mM EDTA), revealing a shift of epitope into the soluble fraction. Blue arrows indicate soluble fraction after application of exosome isolation protocol. MW markers are shown to the right. SN = supernatant, P = pellet (exosome). f) Enhanced cancer-specific signal in SDS-treated type 1 plasma in ELISA immunoassay.

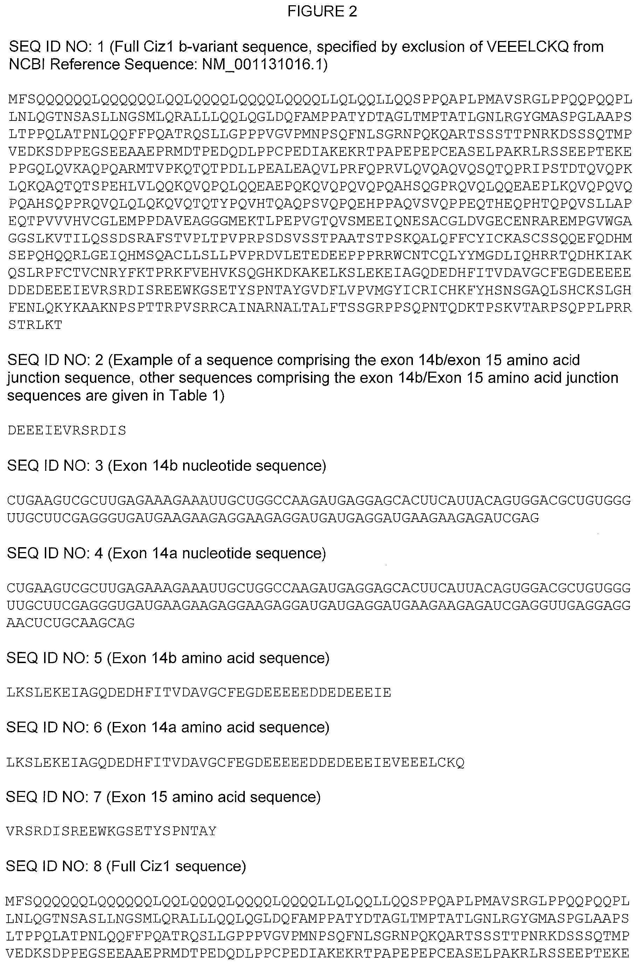

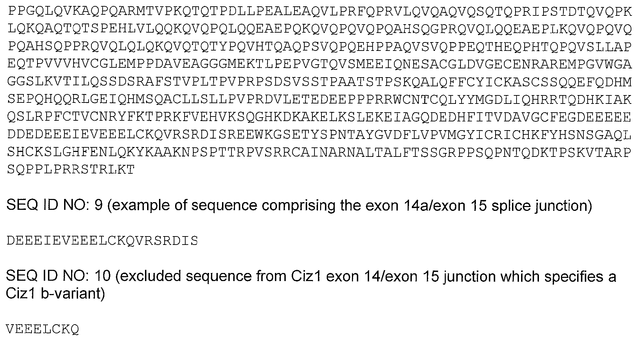

**Figure 2** **-** Ciz1 Sequences

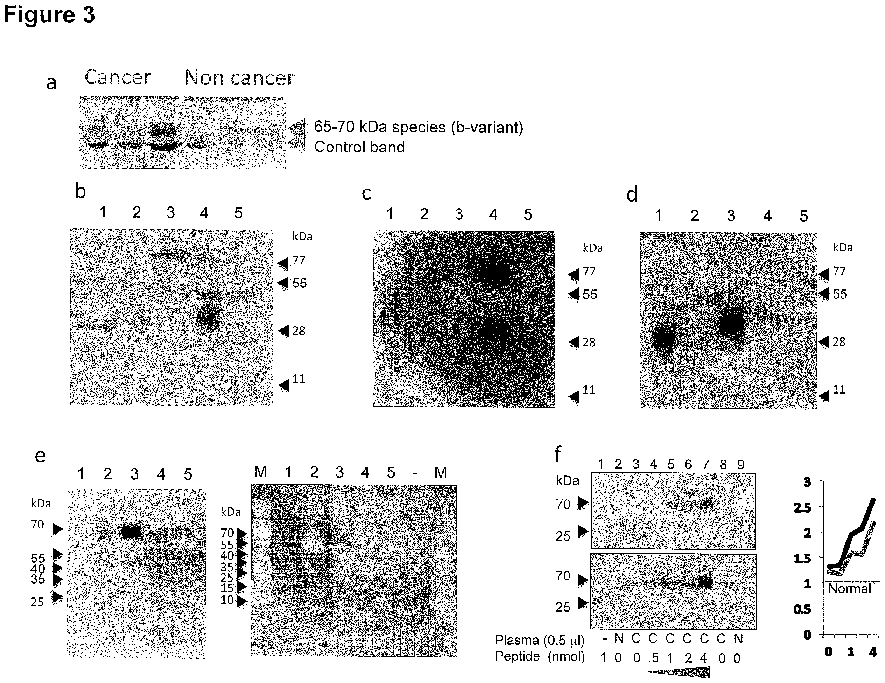

**Figure 3** - Ciz1 b-variant ∼65-70kDa species is a Ciz1 b-variant peptide stably bound to a carrier protein, which may augment antigenicity of Ciz1 b-variant peptide. a) Western blot illustrating cancer-selective epitope recognised by anti-Ciz1 b-variant antibody 2B, as described previously (Higgins *et al;* as above). b) Reconstitution of a Ciz1 b-variant epitope from synthetic b-variant peptide (50 nmols of EIAGQDEDHFITVDAVGCFEGDEEEEEDDEDEEEIEVRSRDISREEWKGSETYSPNTAYGV DFLV - SEQ ID NO: 12) mixed with 0.5ul of non-cancer plasma. Free peptide migrates with mobility indicative of a MW 3-4x greater than predicted (∼30 kDa instead of 7.6 kDa), indicated by blue arrow. When incubated in plasma mobility decreases, antigenicity increases, and a new species is created at ∼80kDa, indicated by red arrow. c) Similar results were generated with a separate anti-Ciz1 b-variant monoclonal antibody. d) For reference, a duplicate blot is probed with a monoclonal antibody that is selective for the exon 14a version of the peptide (EIAGQDEDHFITVDAVGCFEGDEEEEEDDEDEEEIEVEEELCKQRSRDISREEWKGSETYS PNTAYGVDFLV - SEQ ID NO: 13), showing detection of the peptide in lanes 1 and 3. Key: Lane 1, WT peptide only. Lane 2, B-var peptide only. Lane 3, WT peptide + normal plasma. Lane 4, B-var peptide + normal plasma. Lane 5, normal plasma only. Results show that normal plasma acquires Ciz1 b-variant signal at ∼80kDa upon addition of b-var peptide, but not a-var peptide. e) Similar results were obtained using short b-variant peptide (DEEEIEVRSRDIS - SEQ ID NO: 2), when combined with either cancer or non-cancer plasma. Western blot shows results with anti-Ciz1 b-variant monoclonal antibody (left) or anti-Ciz1 b-variant polyclonal antibody 043 (right). Key: Lane 1, B-var peptide only. Lane 2, B-var peptide + normal plasma. Lane 3, B-var peptide + lung cancer plasma. Lane 4, Normal plasma alone. Lane 5, Lung cancer plasma alone. Note that mobility of new signal is similar to endogenous signal in cancer patient plasma. F) Western blot showing titration of short peptide (DEEEIEVRSRDIS - SEQ ID NO: 2) into cancer patient plasma, detected with anti-Ciz1 b-variant monoclonal antibody (upper) or anti-Ciz1 b-variant polyclonal antibody 043 (lower). Graph shows quantification of results by densitometry, with signal in normal plasma (N) set at 1. Results show that carrier protein is not limiting up to 4nmol peptide/0.5ul plasma. Overall, results show that endogenous b-var65 species, detected by anti Ciz1 b-variant antibodies, is likely not one single 65 kDa Ciz1 b-variant species, but a stable composite of Ciz1 b-variant fragment and carrier protein with MW close to 65kDa.

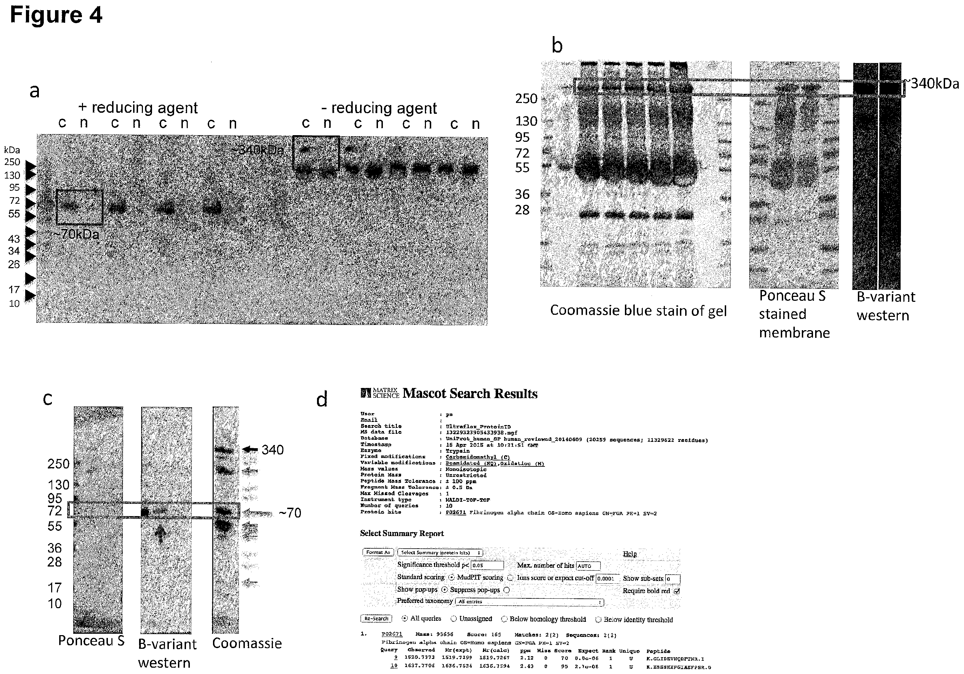

**Figure 4** - Isolation using a two-dimensional gel approach shows that the carrier protein is Fibrinogen. a) Western blot of cancer (C) and non-cancer (N) plasma samples after incubation in 2% SDS sample buffer with and without 200mM b-mercaptoethanol. In the presence of reducing agent, a cancer-specific signal at ∼65kDa is produced with anti-b variant antibodies, while in the absence of reducing agent cancer-specific signal is retarded to ∼340kDa. b) First dimension gel (without reducing agent) is used to isolate Ciz1 b-variant epitope-containing ∼340kDa band. c) Band is further resolved after incubation in reducing agent to reveal constitutive polypeptides and migration of Ciz1 b-variant epitope with a ∼70 kDa component. The band was excised for mass spectrometry. d) MASCOT assigned one significant identity based on two separate peptide masses after trypsin digest. Identity: Fibrinogen alpha chain.

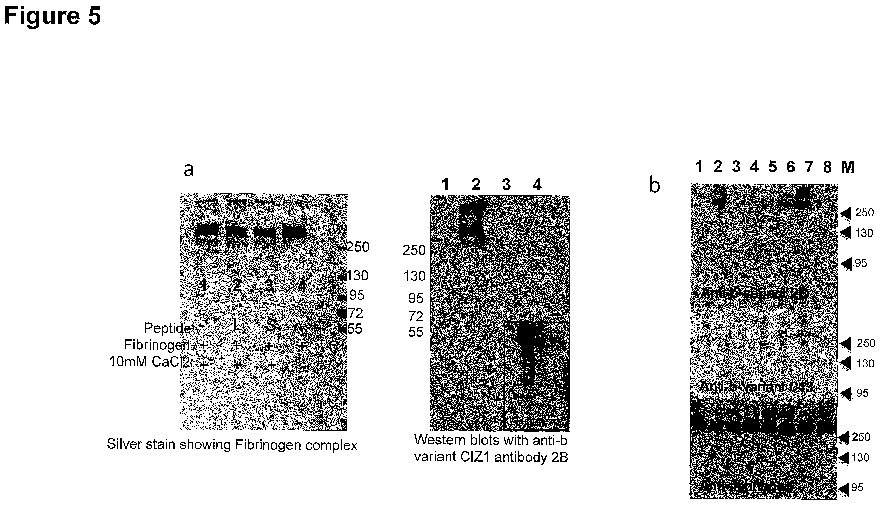

**Figure 5** - Reconstitution of Ciz1 b-variant epitope from purified fibrinogen and synthetic peptides. a) Silver stained gel (left) showing 6 nmols of purified fibrinogen complex (Sigma Cat. F3879), after electrophoresis in the absence of reducing agent (in 2% SDS loading buffer). Peptides are included in the experiment (100 pmols/lane) but do not stain well with silver due to their amino-acid content. Western blots of parallel gels are shown on the right, probed with two anti-b variant antibodies. L, Long Ciz1 b-variant peptide EIAGQDEDHFITVDAVGCFEGDEEEEEDDEDEEEIEVRSRDISREEWKGSETYSPNTAYGV DFLV (SEQ ID NO: 12). S, Short Ciz1 b-variant peptide DEEEIEVRSRDIS (SEQ ID NO: 2). b) Similar experiment comparing a range of Ciz1 b-variant and a-variant peptides for their ability to form epitope that is reactive to the same antibodies, when in complex with fibrinogen. Key: 1, no additions. 2, Long Ciz1 b-variant peptide EIAGQDEDHFITVDAVGCFEGDEEEEEDDEDEEEIEVRSRDISREEWKGSETYSPNTAYGV DFLV (SEQ ID NO: 12). 3, WT long peptide EIAGQDEDHFITVDAVGCFEGDEEEEEDDEDEEEIEVEEELCKQRSRDISREEWKGSETYS PNTAYGVDFLV (SEQ ID NO: 13). 4, no additions. 5, Short Ciz1 b-variant DEEEIEVRSRDIS (SEQ ID NO: 2). 6, C-DEEγIEVRSRDIS-coNH2 (SEQ ID NO: 14). 7, C-DEyEIEVRSRDIS-coNH2 (SEQ ID NO: 23). 8, C-DyyylyVRSRDIS-coNH2 (SEQ ID NO: 15).

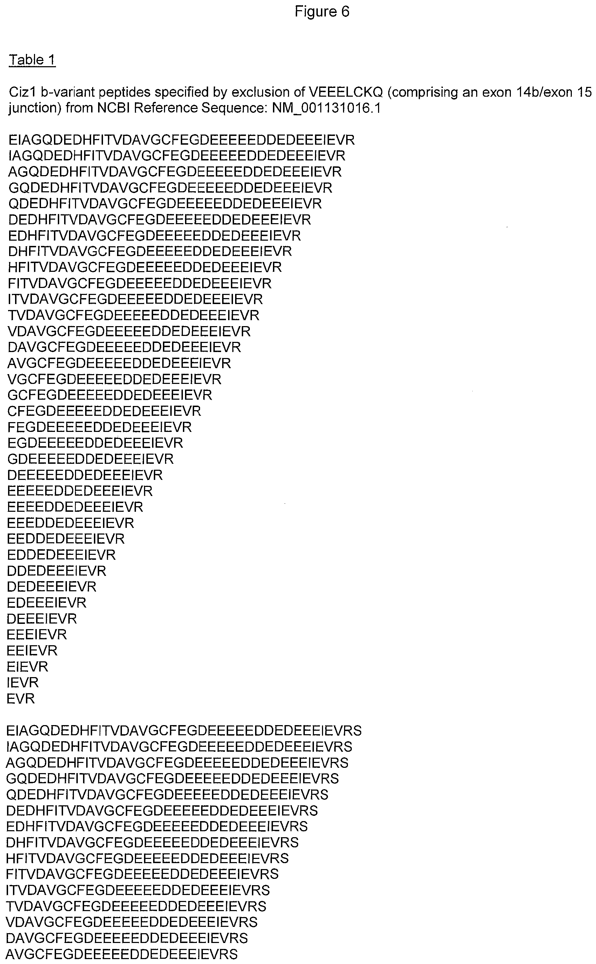

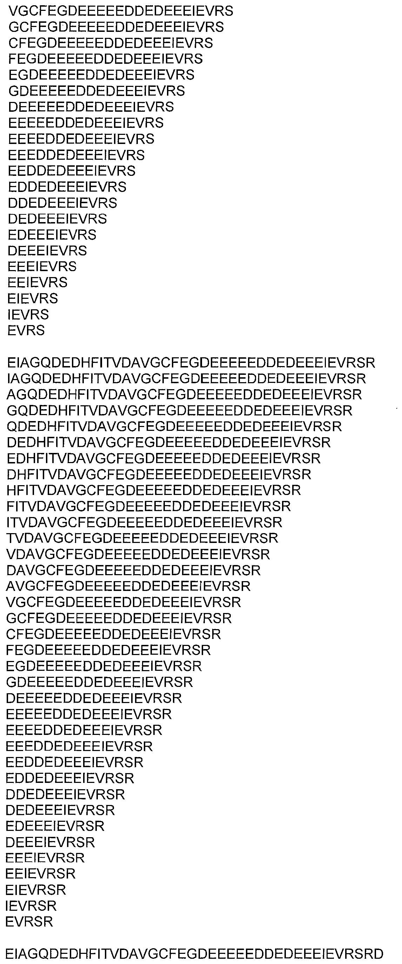

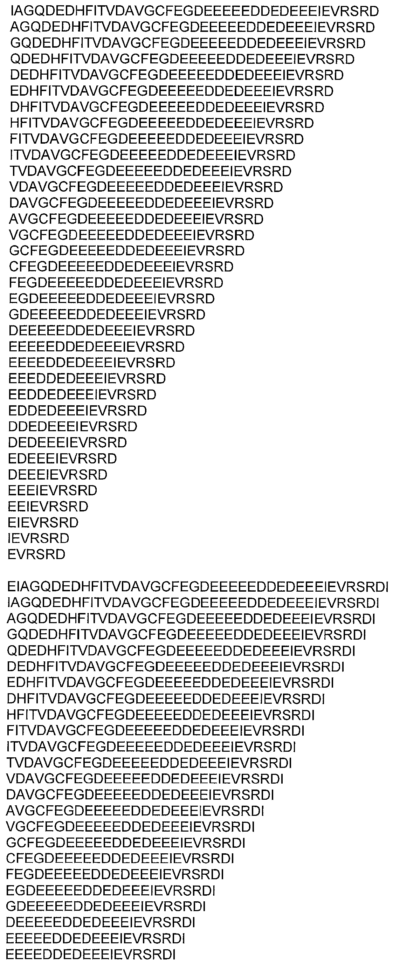

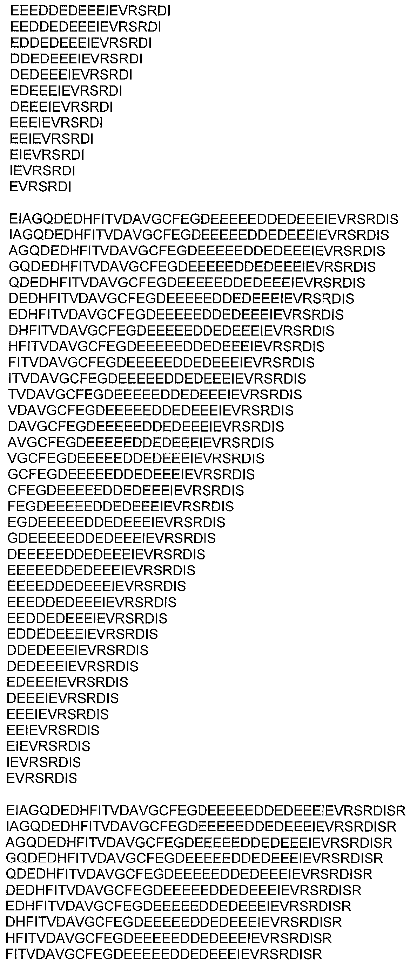

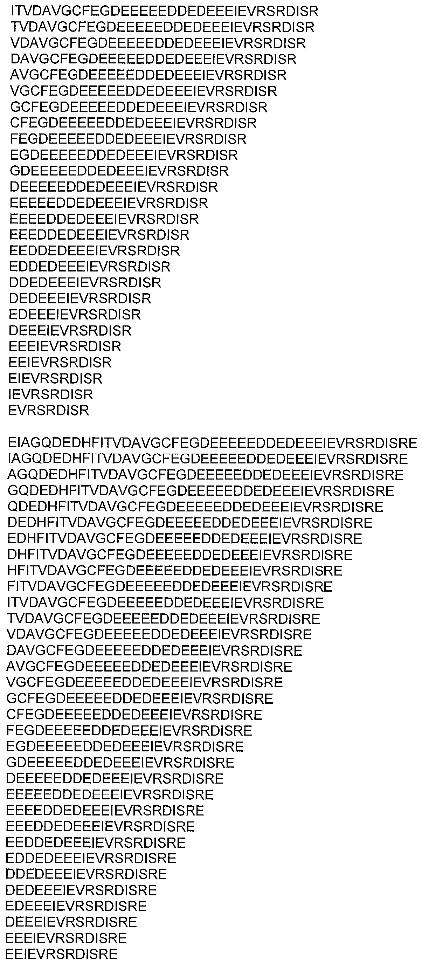

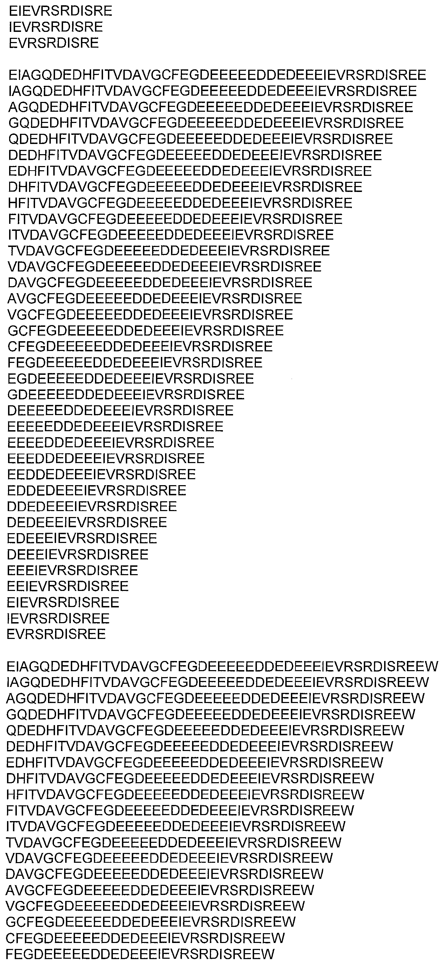

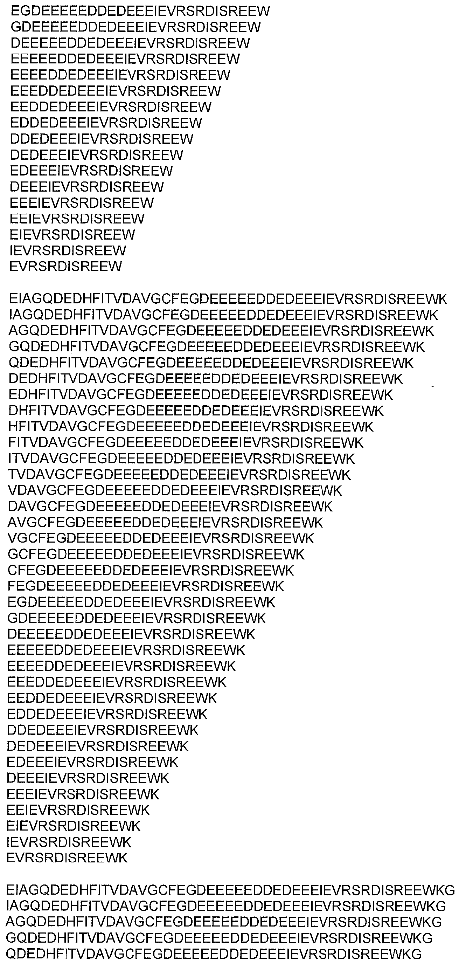

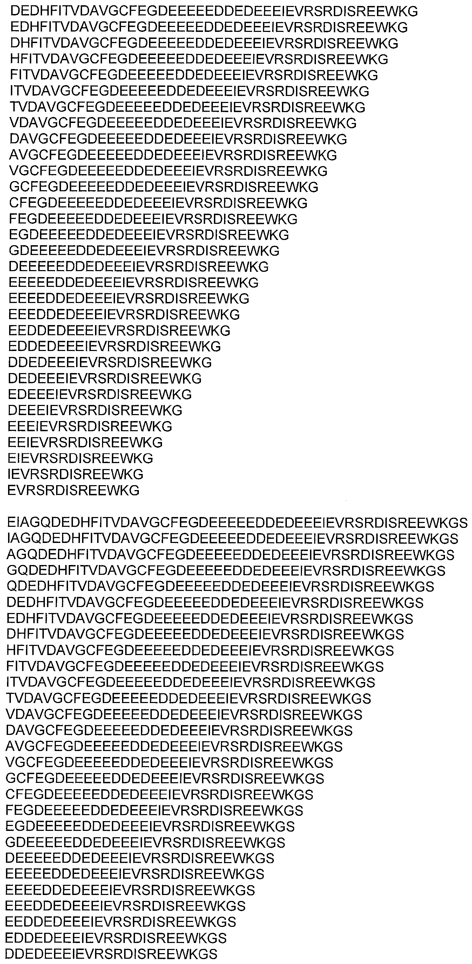

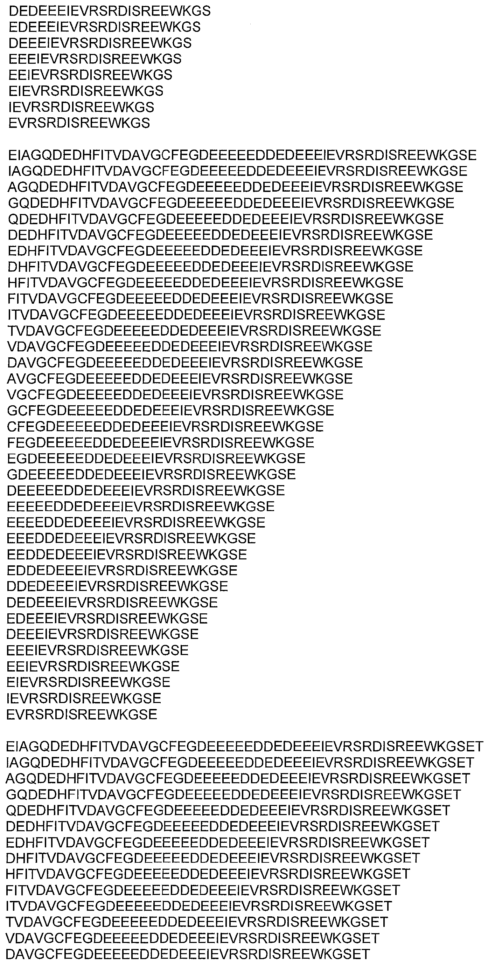

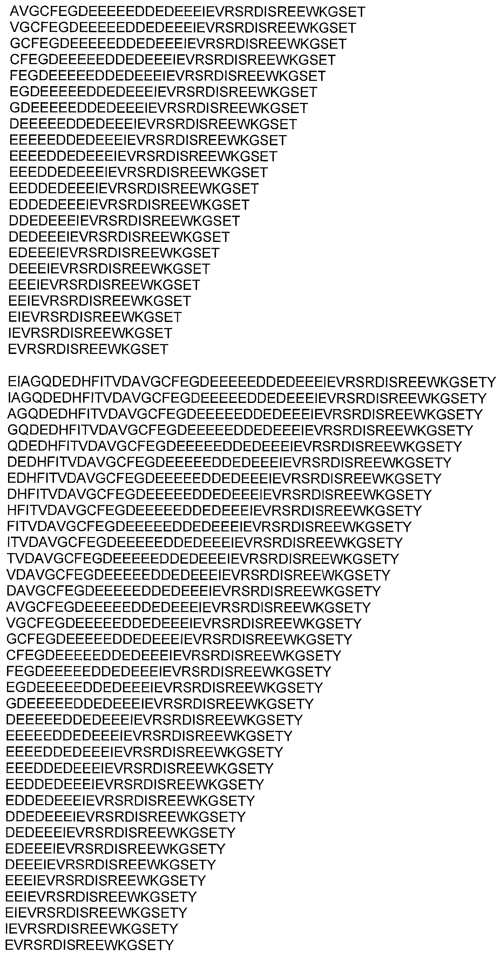

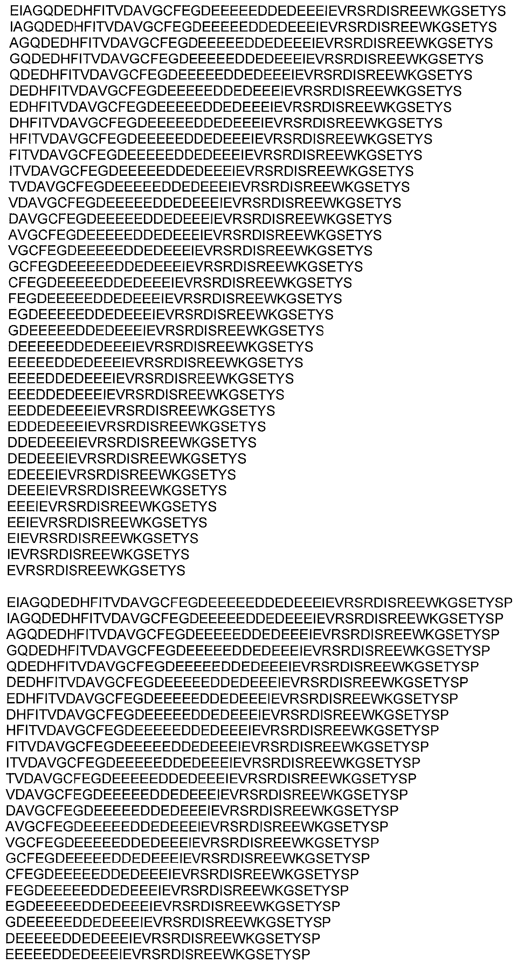

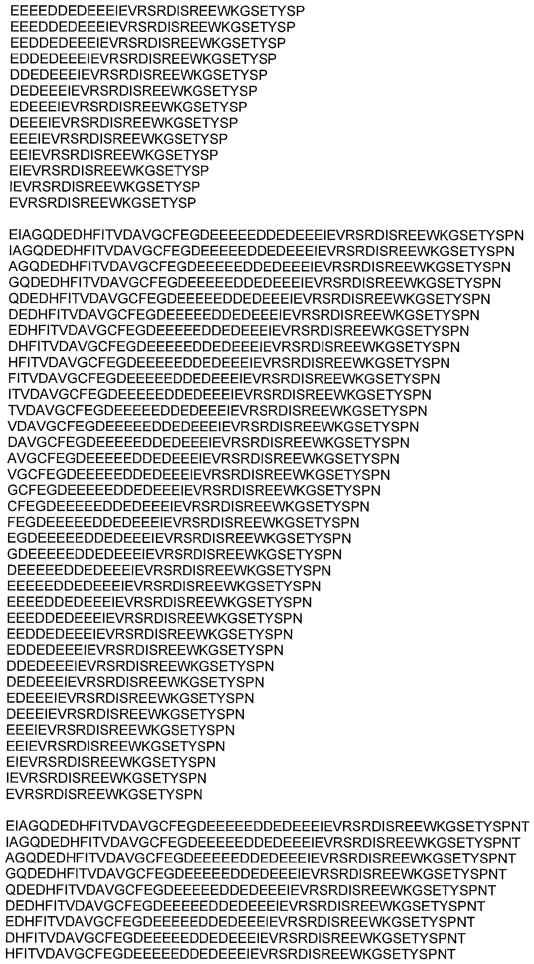

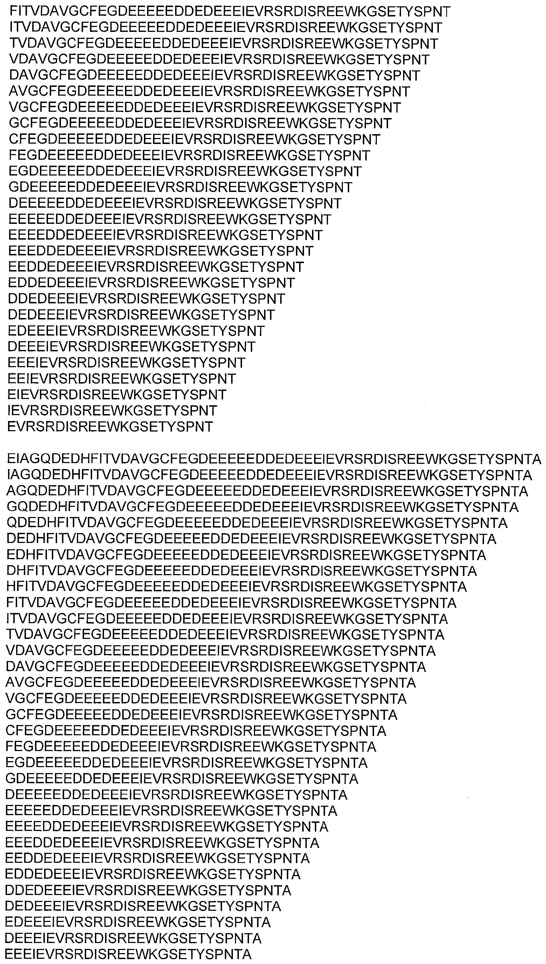

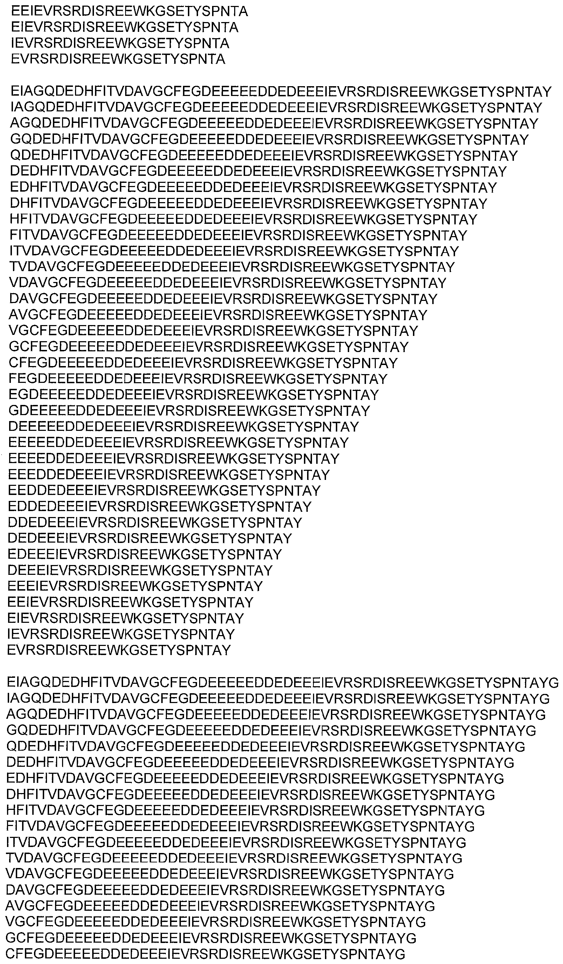

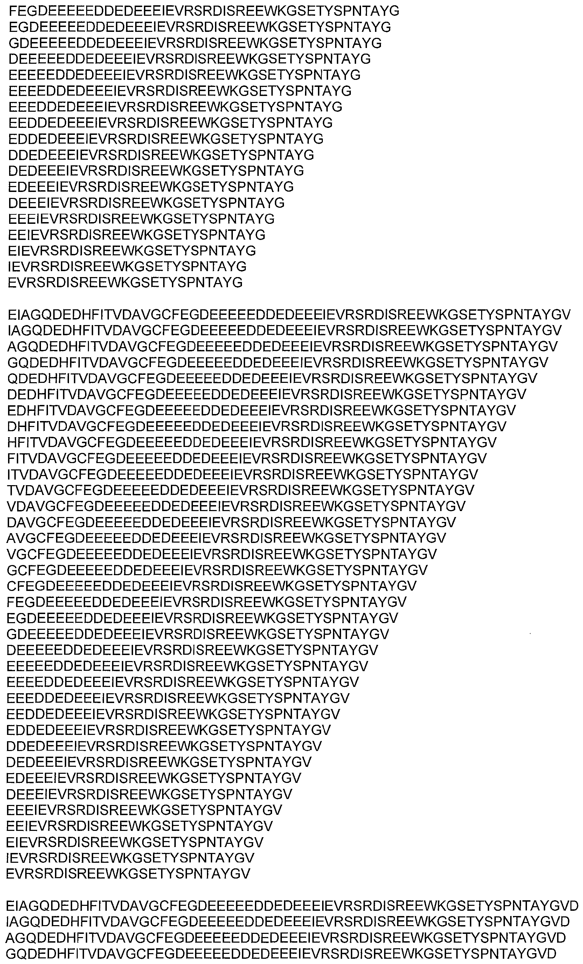

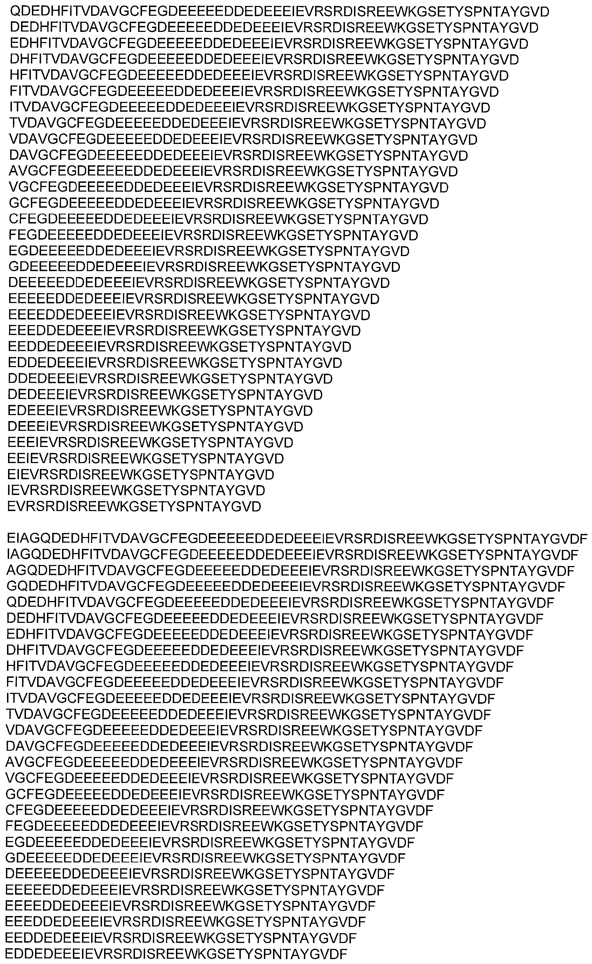

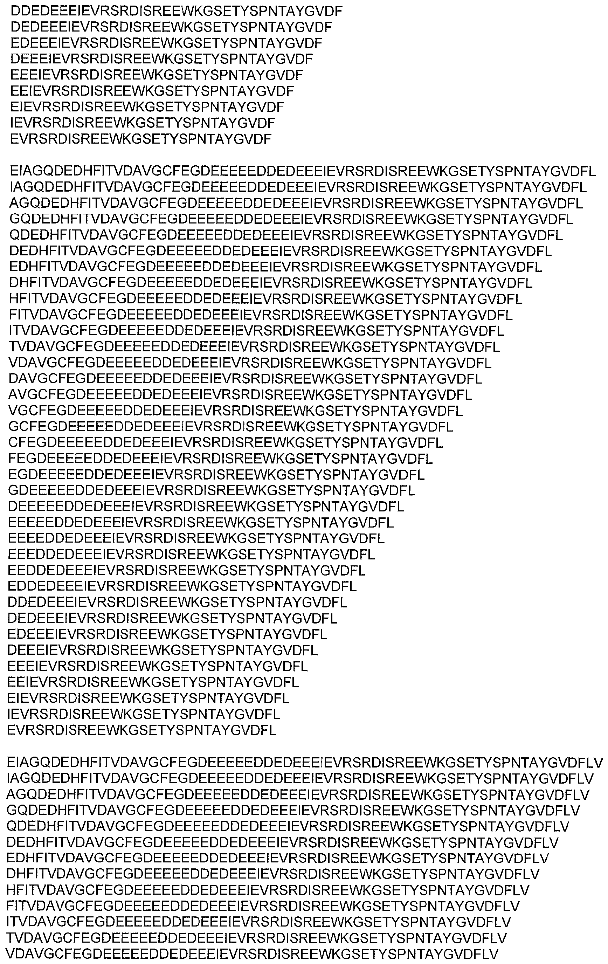

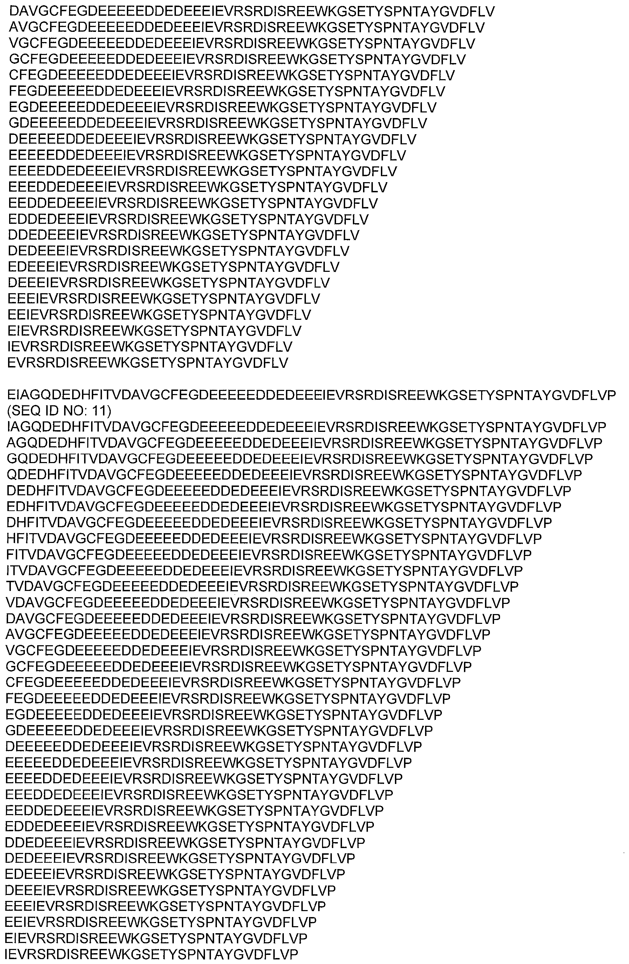

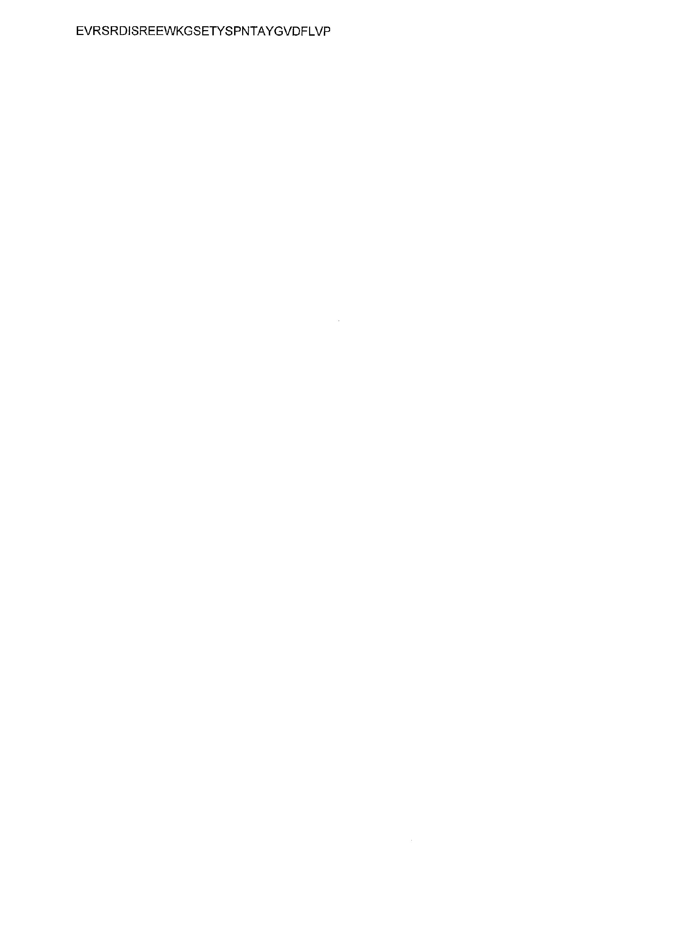

**Figure 6** - Table 1:Ciz1 b-variant peptides comprising an exon 14b/exon 15 junction (specified by exclusion of VEEELCKQ - SEQ ID NO: 10) from NCBI Reference Sequence: NM_001131016.1.

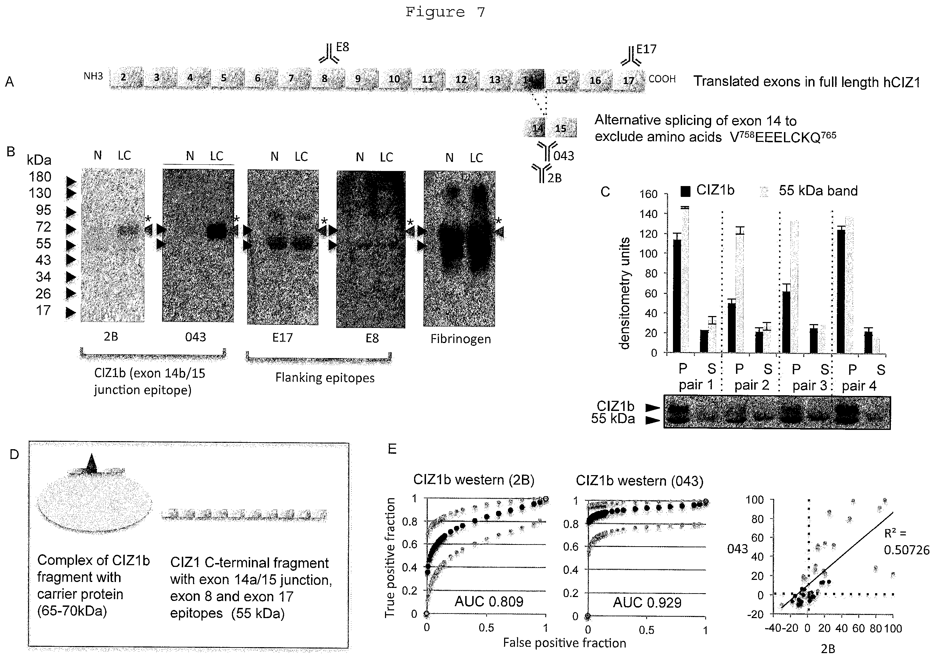

**Figure 7** - Exon junction specific antibodies detect CIZ1b in lung cancer patient plasma. A) Map of CIZ1 translated exons showing alternative splicing of exon 14 to yield 14b (CIZ1b) by exclusion of the indicated 8 amino-acids (positions refer to NCBI Reference Sequence: NM_012127.2). Also shown are the location of epitopes detected with antibodies used in B. B) Western blot of representative plasma samples from a patient with NSCLC (LC) and normal control (N) after denaturation for SDS-PAGE, showing CIZ1b band at 65-70kDa in LC (red arrow, marked "*") detected by CIZ1b antibodies 2B and 043, and a 'generic' CIZ1 antibody-reactive band at 55kDa in both N and LC, detected via epitopes in exon 8 and exon 17. Also indicated is fibrinogen alpha chain. C) Comparison of CIZ1b levels in paired plasma (P) and serum (S) samples from four individuals analysed with CIZ1b antibody 2B. Histogram shows quantified band intensities for the 65-70kDa and 55kDa entities, derived from triplicate samples, with standard deviation. Note that serum lacks the 65-70kDa CIZ1b band. D) Model showing the simplest interpretation of the data in B, though extensive alternative splicing and likely secondary modification mean that the exact identity of the 55kDa entity in plasma is unverified. E) Receiver operating characteristic (ROC) curves showing area under the curve (AUC) values for 2B and 043 western blot results on lung cancer test set A. Dot plot shows correlation between the two date sets (LC samples are orange). Thresholds (black dotted lines) are set at the mean of non-cancer samples in the set, plus one SD. These yield sensitivity/specificity estimates for this set of 20 lung cancer and 20 non-cancer samples of: 2B, 85/67.5; 043, 90/77.5. Using an arbitrary threshold specified by both antibodies these are 90/87.5.

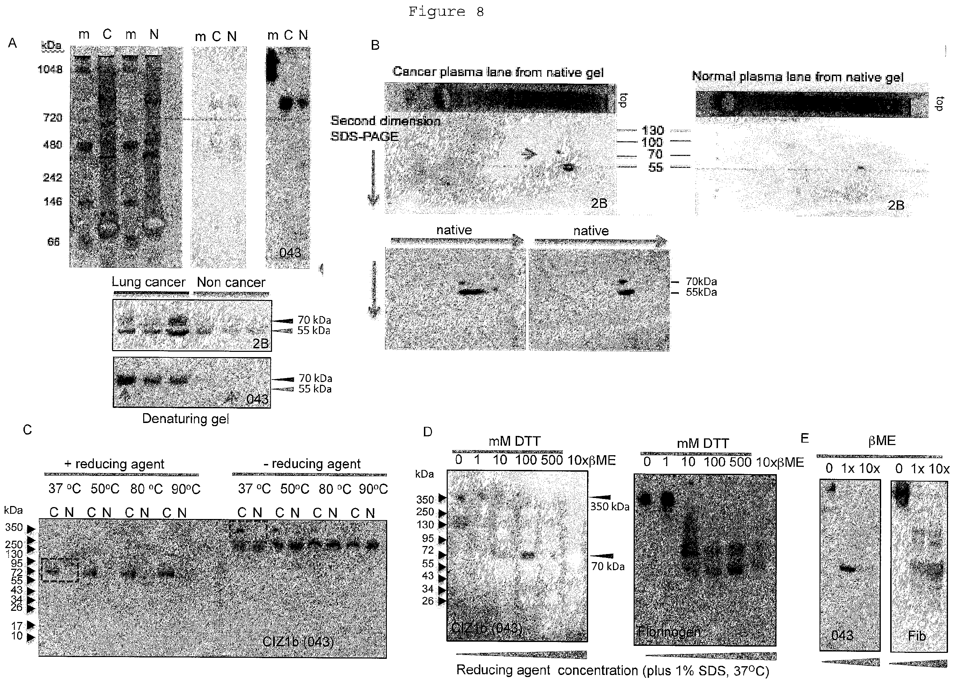

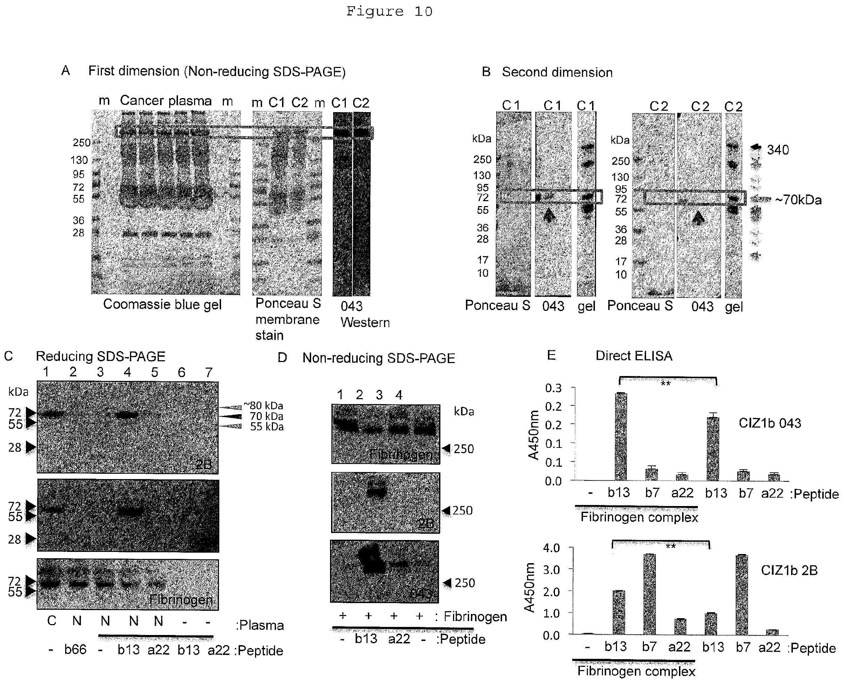

**Figure 8** - Effect of denaturation on CIZ1b epitope. A) Left, Coomassie blue stained gel of a representative lung cancer (C) and normal (N) plasma sample (2ul), separated under fully native conditions (in the absence of SDS or reducing agent). m indicates marker lanes. Middle, parallel gel after transfer to nitrocellulose stained with Ponceau S. Right, the same membrane probed with CIZ1b antibody 043. Lower panel shows the same two samples (red arrows) separated by denaturing SDS-PAGE, also probed with 043. B) Native gel first dimension was soaked in 4x SDS-PAGE loading buffer for 30 minutes without heating, and further separated by size (second dimension). Western blot reveals one band at 55kDa that is reactive with antibody 2B in the non-cancer sample, and two bands (55 and 70 kDa) in the cancer sample. Two additional cancer plasma samples, treated in the same way are shown below. C) Migration of 043-reactive bands in a representative cancer (C) and non-cancer (N) plasma sample through a non-denaturing gel, after prior incubation at the indicated temperatures, in the presence of 2% SDS, with and without reducing agent (200mM β-mercaptoethanol, βME) as indicated. Note the shift of cancer-specific band (red box) from relative mobility of approximately 340 kDa in the absence of reducing agent, to ∼70 kDa in the presence of reducing agent (accompanied by complete loss of the 'generic' band that was detected in all samples in the absence of reducing agent). D) Plasma from a lung cancer patient showing 043-reactive band after incubation with 1% SDS at 37°C for 30 mins, with the indicated concentrations of DTT, or a 10-fold excess of βME. The reactive band is indicated as it shifts from 340 kDa to 70 kDa, and is then lost under maximally reducing conditions. The behaviour of fibrinogen in the same samples is shown for comparison, detected with antibody F8512. E) Synthetic CIZ1b epitope generated by combining CIZ1b peptide (b66, Table 2) with normal (non-cancer) plasma (see Fig. 10), behaves similarly to endogenous epitope, dissociating from within a high molecular weight complex, to a ∼70kDa species, and then disappearing under maximally reducing conditions. Fibrinogen is detected with antibody AF4786. βME concentrations refer to multiples of the standard concentration used in SDS-PAGE of 200 mM.

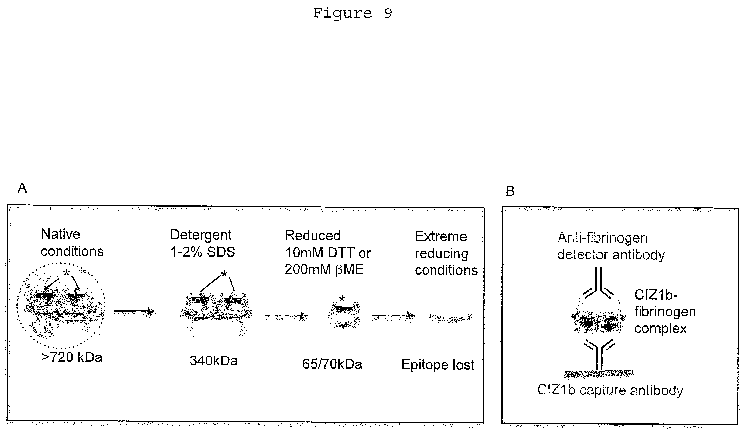

**Figure 9** - Deconstruction of CIZ1b biomarker and immunoassay design A) Schematic of results shown in Fig.8, illustrating the complexity of the native complex in which CIZ1b epitope (red bar, marked "*") resides in plasma, and the disrupting effect of detergent and reducing agents. Under native conditions CIZ1b is part of a complex in excess of 720 kDa, most likely encompassed within lipid vesicles. SDS shifts the epitope to 340kDa, and reducing agents shift it further to the mobility typically observed in standard SDS-PAGE gels (65-70kDa). In the presence of excess reducing agent the western blot epitope is lost for both 2B and 043 CIZ1b antibodies. B) Schematic of sandwich ELISA format based on information summarised in A.
**Figure 10** - Reconstitution of CIZ1b biomarker from purified components A) Two independent lung cancer patient plasmas were separated after incubation with 1% SDS (no reducing agent), in order to isolate the 340kDa band from stained gels (left). Band identity was verified by ponceau S stain followed by Western blot with anti-CIZ1b antibody 043 of a parallel gel (right). B) Gel slices were soaked in 2% SDS-PAGE loading buffer (with 200 mM βME) and separated through a denaturing gel to recover the 043-reactive species at 70kDa. Note that sample loaded as a gel slice is slightly retardation compared to markers which are loaded in solution. Coomassie blue staining revealed 5 dominant bands including one at 70 kDa, which was isolated and digested with either trypsin (cancer plasma 1, C1) or AspN (cancer plasma 2, C2). C) Western blot of normal human plasma (non-cancer, N) showing reconstitution of CIZ1b epitope via complex formation between a carrier protein in plasma and synthetic CIZ1b peptides of the indicated lengths, but not equivalent CIZ1a peptide (Table 2). Note that free peptides migrate in reducing SDS-PAGE with a relative mobility 3 times expected (-21 kDa instead of 7.6kDa for b66, ∼5kDa instead of 1.6 for b13). None are recognized by CIZ1b antibodies in western blot unless complexed with carrier protein. For CIZ1b66 peptide a new reactive species is created at ∼80kDa, while CIZ1b13 peptide increases antigenicity at the same mobility as the endogenous epitope in lung cancer plasmas (C, lane 1). D) Western blot of non-reducing SDS-PAGE gel showing purified fibrinogen (6nmols) in all lanes (upper), with and without prior incubation with 100 pmols of the indicated peptides (Table 2), probed with CIZ1b antibodies as indicated. Under non-reducing conditions fibrinogen migrates with apparent molecular weight of ∼340kDa and this co-migrates with CIZ1b signal. E) Direct ELISA showing mean A450nm (triplicate analysis with SEM) generated by CIZ1b antibodies 2B or 043 as indicated, using molar equivalents of preformed peptide/fibrinogen complex as analyte. ** T test p<0.05.

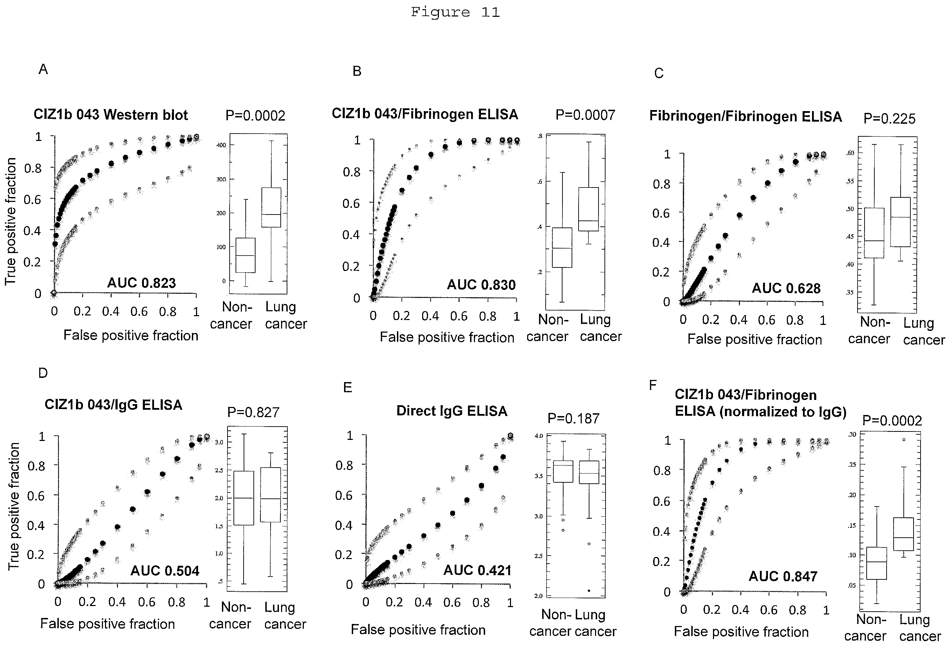

**Figure 11** - Immunoassay validation using lung cancer plasma and controls (n=39 non-cancer, 13 lung cancer). A) CIZ1b western blot with antibody 043. B) Sandwich ELISA format for CIZ1b antigen in plasma, using 043 capture antibody and anti-fibrinogen detector antibody. C) Quantitative detection of fibrinogen in the same samples using paired fibrinogen antibodies D) Substitution of anti-human IgG for anti-fibrinogen in a sandwich format similar to that shown in B. E) Detection of IgG in plasma by direct ELISA. F) Normalization of the data in C against the data in G. In all cases, receiver operating characteristic curves (ROC) indicate the relationship between true positive and false positive fraction (black points) and 95% confidence interval of the fitted ROC curve (grey points), and area under the curve (AUC) values in bold. Box and whisker plots display minimum and maximum, lower, median, and upper quartile, and outliers.

### DETAILED DESCRIPTION OF THE INVENTION

### CIZ1

Cip1-interacting zinc finger protein (Ciz1) is a protein that in humans is encoded by the *CIZ1* gene.

Ciz1 promotes initiation of mammalian DNA replication, where it helps coordinate the sequential functions of cyclin E- and A-dependent protein kinases (Coverley et al; J Cell Sci. 2005; 118(Pt 1):101-112). It interacts directly with cyclins E and A, CDK2, and cyclin-dependent kinase inhibitor p21 and also plays an indirect role in DNA replication by modulating the expression of genes, including cyclin D, that influence cell proliferation (den Hollander et al; Cancer Res. 2006;66(22):11021-11029). Ciz1 is often attached to the salt- and nuclease-resistant protein component of the nucleus referred to as the "nuclear matrix" and resides within foci that partially colocalize with sites of DNA replication (Ainscough; J Cell Sci. 2007;120(Pt 1):115-124), implicating Ciz1 in the spatial organization of DNA replication.

Various splice variants of Ciz1 are known in the art. For example, as described in WO 2004/051269 and Rahman et al. (BMC Cancer; 2010; 10:482).

The alternative splicing of Ciz1 exon 14 to generate a Ciz1 b-variant isoform has previously been demonstrated in various cancers ((WO 2012/017208) and (Higgins et al.; PNAS; Nov 6;109(45):E3128-35)).

As described herein, a Ciz1 b-variant isoform is derived from a Ciz1 mRNA transcript which comprises a variant of exon 14 referred to herein as exon 14b (SEQ ID NO: 3). Ciz1 exon 14b lacks 24 nucleotides at the 3' end as compared to full length exon 14, referred to as exon 14a (SEQ ID NO: 4). Ciz1 transcripts expressing exon 14b rather than exon 14a (a-variant) are referred to herein as Ciz1 b-variant, CIZ1b or simply b-variant. The corresponding amino acid sequences of Ciz1 exon 14b and 14a are shown as SEQ ID NO: 5 (LKSLEKEIAGQDEDHFITVDAVGCFEGDEEEEEDDEDEEEIE) and SEQ ID NO: 6 (LKSLEKEIAGQDEDHFITVDAVGCFEGDEEEEEDDEDEEEIEVEEELCKQ) respectively (see Figure 2).

The sequence spanning a splice junction of exon 14b and exon 15 is therefore different to the sequence spanning a splice junction of exon 14a and exon 15. In particular, the junction of exon 14b and exon 15 lacks the sequence VEEELCKQ (SEQ ID NO: 10) which is present at the junction between exon 14a and exon 15 in Ciz1 a-variant proteins.

The present inventors have determined that Ciz1 b-variant peptides exist in a complex with fibrinogen. In particular, it has been shown that Ciz1 b-variant peptides which span an exon 14b/exon 15 junction are able to bind to fibrinogen. In contrast, Ciz1 a-variant peptides are not able to bind to fibrinogen.

Accordingly, as described herein, a Ciz1 b-variant peptide refers to a peptide which can be derived from a Ciz1 b-variant protein but not a Ciz1 a-variant protein. In other words, a Ciz1 b-variant peptide refers to a portion of a Ciz1 b-variant protein which is derived from exons 14b and exon 15 of a Ciz1 b-variant protein and therefore lacks the sequence VEEELCKQ (SEQ ID NO: 10) at the splice junction.

In particular, a Ciz1 b-variant peptide comprises an exon 14b/exon 15 splice junction. In other words, a ciz1 b-variant peptide comprises amino acid sequences derived from Ciz1 exon 14b (SEQ ID NO: 5) and Ciz1 exon 15 (SEQ ID NO: 7) and comprises the sequence EVR as shown in positions 36 to 38 of SEQ ID NO: 11.
SEQ ID NO: 11 EIAGQDEDHFITVDAVGCFEGDEEEEEDDEDEEEIEVRSRDISREEWKGSETYSPNTAYGVDFLVP

Notably, Ciz1 b-variant peptides do not contain amino acids which are specific to Ciz1 a-variant proteins and therefore do not comprise the sequence VEEELCKQ (SEQ ID NO: 10).

As such, a Ciz1 b-variant peptide may be the Ciz1 b-variant peptide shown as SEQ ID NO: 11 or a fragment thereof which comprises EVR as shown in positions 36 to 38 of SEQ ID NO: 11.

Suitably, the Ciz1 b-variant peptide may comprise at least 3, at least 5, at least 10, at least 20, at least 30, at least 40, at least 50 or at least 60 contiguous amino acids of SEQ ID NO: 11 and comprise EVR as shown in positions 36 to 38 of SEQ ID NO: 11.

Suitably, the Ciz1 b-variant peptide may comprise or consist of from 3 to 60, 5 to 60, 5 to 50, 10 to 50, 10 to 40, 10 to 30, 15 to 30 or 10 to 20 contiguous amino acids of SEQ ID NO: 11 and comprise EVR as shown in positions 36 to 38 of SEQ ID NO: 11.

Suitably, the Ciz1 b-variant peptide may comprise or consist of 10 to 20 contiguous amino acids of SEQ ID NO: 11 and comprise EVR as shown in positions 36 to 38 of SEQ ID NO: 11.

Suitably, a Ciz1 b-variant peptide may be a peptide shown in Table 1 (see Figure 6).

### FIBRINOGEN

In one aspect, the present invention relates to the use of a fibrinogen capture agent or a fibrinogen detection agent in an assay to detect Ciz1 b-variant.

Described herein is the use of a fibrinogen binding agent in an assay to detect Ciz1 b-variant.

The fibrinogen binding agent may be an antibody or a fragment thereof, which specifically binds fibrinogen.

In particular, the present invention provides the use of an antibody or a fragment thereof, which binds fibrinogen in an assay to detect Ciz1 b-variant.

Fibrinogen is involved in blood clotting, fibrinolysis, cellular and matrix interactions, the inflammatory response, wound healing and neoplasia.

Fibrinogen molecules are elongated 45 nm structures that consist of two outer D domains, each connected by a coiled-coil segment to a central E domain. The molecule is comprised of two sets of three polypeptide chains termed α, β, and γ, which are joined together in the N-terminal E domain by five symmetrical disulfide bridges. Non-symmetrical disulfide bridges form a 'disulfide ring' in this region (Mosesson; 2005; Journal of Thrombosis and Haemostasis; 3(8); 1894-1940).

Polymerisation of fibrinogen molecules results in the formation of an insoluble fibrin matrix. Conversion of fibrinogen to fibrin is triggered by thrombin, which cleaves fibrinopeptides A and B from α and β chains, and thus exposes the N-terminal polymerization sites responsible for the formation of the soft clot. The soft clot is converted into the hard clot by factor XIIIA which catalyzes the epsilon-(gamma-glutamyl)lysine cross-linking between γ chains (stronger) and between α chains (weaker) of different monomers.

The α-chain consists of 610, the β-chain 461, and the major γ-chain form, γA, 411 residues. Each fibrinogen α-chain contains an N-terminal fibrinopeptide A (FPA) sequence, cleavage of which by thrombin initiates fibrin assembly by exposing a polymerization site termed EA. One portion of EA is at the N-terminus of the fibrin α-chain comprising residues 17-20 gly-pro-arg-val (GPRV), and another portion is located in the fibrin β-chain between residues 15 and 42. Each EA-site combines with a constitutive complementary-binding pocket (Da) in the D domain of neighbouring molecules that is located between γ337 and γ379. The initial EA:Da associations cause fibrin molecules to align in a staggered overlapping end-to-middle domain arrangement to form double-stranded twisting fibrils. Fibrils also undergo lateral associations to create multi-stranded fibres (Mosesson; as above).

An example fibrinogen α protein is the human fibrinogen α protein having the UniProtKB accession number P02671. This exemplified sequence is 866 amino acids in length of which amino acids 1 to 19 form a signal peptide.

An example fibrinogen β protein is the human fibrinogen β protein having the UniProtKB accession number P02675. This exemplified sequence is 491 amino acids in length of which amino acids 1 to 30 form a signal peptide.

An example fibrinogen γ protein is the human fibrinogen γ protein having the UniProtKB accession number P02679. This exemplified sequence is 453 amino acids in length of which amino acids 1 to 26 form a signal peptide.

The term 'fibrinogen' may refer to a complex comprising two α chains, two β chains and two γ chains or a sub-complex thereof (e.g. which does not include all the chains of the full fibrinogen complex).

The present inventors have determined that Ciz1 b-variant peptides are capable of binding to the fibrinogen α chain. As such, in particular embodiments the present assays and methods may involve the capture or detection of the fibrinogen α chain. For example the present assays and methods may involve the detection of fibrinogen (e.g. fibrinogen α chain) following the capture of Ciz1 b-variant in the fibrinogen/Ciz1 b-variant complex.

A 'fibrinogen/Ciz1 b-variant complex' as referred to herein may comprise other peptides and/or proteins aside from the fibrinogen and/or Ciz1 b-variant peptide.

As used herein, a capture agent refers to an agent which is used to isolate (i.e. enrich) the fibrinogen/Ciz1 b-variant complex from a sample. For example, the sample may be a sample which has been isolated from a subject. In particular, the sample may be a blood sample (e.g. a plasma sample) which has been isolated from a subject.

A 'fibrinogen capture agent' refers to an entity which is capable of binding to a fibrinogen/Ciz1 b-variant complex. Preferably, a 'fibrinogen capture agent' is an entity which is capable of specifically binding to fibrinogen and is used to isolate (i.e. enrich) the fibrinogen/Ciz1 b-variant complex from a sample. Such specific binding may enable the isolation/enrichment of fibrinogen from a sample. In particular embodiments, the fibrinogen capture agent may be capable of specifically binding to a fibrinogen α chain.

The fibrinogen capture agent may be an antibody or a fragment thereof, or Ciz1 b-variant peptide which specifically binds fibrinogen.

As used herein, a detection agent refers to an agent which is used to bind the fibrinogen/Ciz1 b-variant complex after it has been isolated (i.e. enriched) from a sample using a capture agent. As such, the detection agent is typically used after a capture step to determine the presence of the isolated (i.e. enriched) fibrinogen/Ciz1 b-variant complex.

A 'fibrinogen detection agent' refers to an entity which is capable of binding to a fibrinogen. Preferably, a 'fibrinogen detection agent' is an entity which is capable of specifically binding to fibrinogen. Such specific binding enables the detection of fibrinogen following the capture of Ciz1 b-variant in the fibrinogen/Ciz1 b-variant complex. In particular embodiments, the fibrinogen capture agent or fibrinogen detection agent may be capable of specifically binding to a fibrinogen α chain.

The fibrinogen detection agent may be an antibody or a fragment thereof, which specifically binds fibrinogen.

The fibrinogen detection agent may be labelled with an entity which enables it to be detected in an assay as described herein. For example, the fibrinogen detection agent may be labelled with a fluorescent entity or an enzyme-substrate label as described herein.

### ANTIBODY

An antibody or a fragment thereof, refers to any portion of an antibody which retains the ability to bind to the same antigen target as the parental antibody - e.g. a fibrinogen antibody or a fragment thereof is able to bind to fibrinogen.

Fibrinogen antibodies are known in the art. For example, sheep anti-fibrinogen pAb (AF4786 R&D systems), ab58207 (Abcam); Anti-Fibrinogen, clone 85D4 (Cat. No. F9902; Sigma Aldrich); and Fibrinogen Antibody (MFB-HB) (Cat. No. MA1-35371; Life Technologies).

Fibrinogen α chain antibodies are also known in the art. For example, EPR2918 (ab108616; Abcam); Fibrinogen Antibody (5C5) (LF-MA0108; Pierce); and EPR2919 (TA307697; Origene).

The present invention also encompasses methods which comprise the step of detecting Ciz1 b-variant or capturing Ciz1 b-variant/fibrinogen complex by contacting the Ciz1 b-variant with an antibody or fragment thereof that binds to the Ciz1 b-variant. An antibody which specifically binds a Ciz1 b-variant peptide may be generated as previously described for antibody 2B (Higgins et al; 2012; PNAS; Nov 6;109(45):E3128-35).

The antibody may be a chimeric antibody. Chimeric antibodies may be produced by transplanting antibody variable domains from one species (for example, a mouse) onto antibody constant domains from another species (for example a human).

The antibody may be a full-length, classical antibody. For example the antibody may be an IgG, IgM or IgA molecule.

The antibody may be a functional antibody fragment. Specific antibody fragments include, but are not limited to, (i) the Fab fragment consisting of VL, VH, CL and CH1 domains, (ii) the Fd fragment consisting of the VH and CH1 domains, (iii) the Fv fragment consisting of the VL and VH domains of a single antibody, (iv) the dAb fragment, which consists of a single variable domain, (v) isolated CDR regions, (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site, (viii) bispecific single chain Fv dimers, and (ix) "diabodies" or "triabodies", multivalent or multispecific fragments constructed by gene fusion. The antibody fragments may be modified. For example, the molecules may be stabilized by the incorporation of disulphide bridges linking the VH and VL domains.

The antibody described herein may be a multispecific antibody, and notably a bispecific antibody, also sometimes referred to as "diabodies". These are antibodies that bind to two (or more) different antigens. Diabodies can be manufactured in a variety of ways known in the art, e.g., prepared chemically or from hybrid hybridomas. The antibody may be a minibody. Minibodies are minimized antibody-like proteins comprising a scFv joined to a CH3 domain. In some cases, the scFv can be joined to the Fc region, and may include some or all of the hinge region.

The antibody may be a domain antibody (also referred to as a single-domain antibody or nanobody). This is an antibody fragment containing a single monomeric single variable antibody domain. Examples of single-domain antibodies include, but are not limited to, VHH fragments originally found in camelids and VNAR fragments originally found in cartilaginous fishes. Single-domain antibodies may also be generated by splitting the dimeric variable domains from common IgG molecules into monomers.

The antibody may be a synthetic antibody (also referred to as an antibody mimetic). Antibody mimetics include, but are not limited to, Affibodies, DARPins, Anticalins, Avimers, Versabodies and Duocalins.

### APTAMERS

Aptamers that specifically recognize fibrinogen or Ciz1 b-variant may be synthesized using standard nucleic acid synthesis techniques or selected from a large random sequence pool, for example using the Systematic Evolution of Ligands by Exponential Enrichment (SELEX) technique.

Aptamers can be single strand DNA or RNA sequences that fold in a unique 3D structure having a combination of stems, loops, quadruplexes, pseudoknots, bulges, or hairpins. The molecular recognition of aptamers results from intermolecular interactions such as the stacking of aromatic rings, electrostatic and van der Waals interactions, or hydrogen bonding with a target compound. In addition, the specific interaction between an aptamer and its target is complemented through an induced fit mechanism, which requires the aptamer to adopt a unique folded structure to its target. Aptamers can be modified to be linked with labeling molecules such as dyes, or immobilized on the surface of beads or substrates for different applications.

Aptamers may be paired with nanotechnology, microarray, microfluidics, mass spectrometry and other technologies for quantification in a given sample.

Typically, a fibrinogen capture agent or a Ciz1 b-variant capture agent will be immobilised on a support, such as an array, or be captured on a solid support, such as beads. A sample to be tested is then incubated with the support comprising the capture agent such that Ciz1 b-variant/fibrinogen complex can be isolated/enriched from the sample. Supports (eg. solid supports) can be made of a variety of materials - such as glass, silica, plastic, nylon or nitrocellulose. When attached to a solid support it is preferably rigid and have a planar surface.

The fibrinogen capture agent or Ciz1 b-variant capture agent may comprise a moiety that can be captured on a solid surface, such as a biotin moiety which can be captured by streptavidin beads. In such embodiments, capture to a support can occur follow incubation of the capture agent with a sample.

### DETECTING CIZ1 b-VARIANT OR CAPTURING A CIZ1 b-VARIANT

Suitably, the Ciz1 b-variant may be a Ciz1 b-variant peptide as described herein.

A Ciz1 b-variant may be detected using a variety of suitable methods and techniques known in the art.

The method may comprise the following steps: a) capturing a fibrinogen/Ciz1 b-variant complex using a fibrinogen capture agent; and b) detecting Ciz1 b-variant using a Ciz1 b-variant detection agent.

The method may comprise the following steps: a) capturing a fibrinogen/Ciz1 b-variant complex using a Ciz1 b-variant capture agent; and b) detecting fibrinogen using a fibrinogen detection agent.

As used herein, a 'Ciz1 b-variant capture agent' refers to an entity which is capable of binding to a Ciz1 b-variant and is used to isolate (i.e. enrich) the fibrinogen/Ciz1 b-variant complex from a sample. Preferably, a 'Ciz1 b-variant capture agent' is an entity which is capable of specifically binding to Ciz1 b-variant. Such specific binding enables the isolation/enrichment of the fibrinogen/Ciz1 b-variant complex from a sample.

The Ciz1 b-variant capture agent may be an antibody or a fragment thereof, or Ciz1 b-variant peptide which specifically binds fibrinogen.

In particular embodiments, a Ciz1 b-variant/fibrinogen complex may be captured using an antibody or fragment thereof that binds to the Ciz1 b-variant peptide. An antibody which specifically binds a Ciz1 b-variant peptide may be generated as previously described for antibody 2B (Higgins et al; 2012; PNAS; Nov 6;109(45):E3128-35).

A 'Ciz1 b-variant detection agent' refers to an entity which is capable of binding to a Ciz1 b-variant and is used to bind the fibrinogen/Ciz1 b-variant complex after it has been isolated (i.e. enriched) from a sample using a capture agent. Preferably, a 'Ciz1 b-variant detection agent' is an entity which is capable of specifically binding to Ciz1 b-variant. Such specific binding enables the detection of Ciz1 b-variant following the capture of the fibrinogen/Ciz1 b-variant complex.

The Ciz1 b-variant detection agent may be an antibody or a fragment thereof, which specifically binds Ciz1 b-variant.

The Ciz1 b-variant detection agent may be labelled with an entity which enables it to be detected in an assay as described herein. For example, the Ciz1 b-variant detection agent may be labelled with a fluorescent entity or an enzyme-substrate label as described herein.

For example, methods for detecting Ciz1 b-variant may employ antibody-based arrays, enzyme linked immunosorbent assays (ELISA), radioimmuno-assay (RIA), western blotting or mass spectrometry.

An ELISA may be performed according to general methods which are known in the art. For example, the ELISA may be a sandwich or competitive ELISA.

A sandwich ELISA may comprise the following steps:
- a surface (i.e. a microtitre plate well) is prepared to which a known quantity of capture agent is bound (e.g. a Ciz1 b-variant capture agent or a fibrinogen capture agent);
- any nonspecific binding sites on the surface are blocked;
- the sample comprising the Ciz1 b-variant is applied to the plate;
- the plate is washed to remove unbound antigen;
- a primary antibody which is capable of specifically binding the fibrinogen or Ciz1 b-variant is added, and binds to antigen;
- an enzyme-linked secondary antibody is applied as a detection antibody that bind specifically to the antibody Fc region;
- the plate is washed to remove the unbound antibody-enzyme conjugates, before a chemical is added to be converted by the enzyme into a colour or fluorescent or electrochemical signal;
- the absorbency or fluorescence or electrochemical signal of the plate wells is measured to determine the presence and quantity of antigen.

In particular embodiments, the present methods comprise the step of capturing a fibrinogen/Ciz1 b-variant complex using a fibrinogen capture agent or a Ciz1 b-variant capture agent as described herein.

In one embodiment the present methods comprise a sandwich ELISA which comprises the steps of capturing a fibrinogen/Ciz1 b-variant complex using a Ciz1 b-variant capture agent as described herein and detecting the fibrinogen/Ciz1 b-variant complex using a fibrinogen detection agent as described herein.

A competitive ELISA may comprise the following steps:
- a labelled antibody which specifically binds the Ciz1 b-variant is incubated in the presence of a sample comprising the Ciz1 b-variant;
- the bound antibody/antigen complexes are then added to an antigen-coated well;
- the plate is washed, so unbound antibody is removed;
- a secondary antibody, specific to the primary antibody, and coupled to an enzyme is added;
- a substrate for the enzyme is added, and the reaction of the enzyme with its substrate elicits a chromogenic or fluorescent signal;
- the reaction is stopped to prevent eventual saturation of the signal.

Various enzyme-substrate labels are available, e.g. as disclosed in US 4,275,149. The enzyme generally catalyses a chemical alteration of the chromogenic substrate that can be detected. For example, the enzyme may catalyse a colour change in a substrate, or may alter the fluorescence or chemiluminescence of the substrate. Examples of enzymatic labels include peroxidase such as horseradish peroxidase (HRPO), alkaline phosphatase, beta-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are well known.

Detecting a Ciz1 b-variant peptide may comprise contacting said Ciz1 b-variant peptide with an antibody or fragment thereof that binds to the Ciz1 b-variant peptide. An antibody which specifically binds a Ciz1 b-variant peptide may be generated as previously described for antibody 2B (Higgins et al; 2012; PNAS; Nov 6;109(45):E3128-35).

In some embodiments the b-variant specific antibody may bind to an epitope comprising the EVR sequence shown in positions 36 to 38 of SEQ ID NO: 11.

In some embodiments, the antibody specifically binds to an amino acid sequence comprising DEEEIEVRSRDIS (SEQ ID NO: 2) but does not specifically bind to an amino acid sequence comprising DEEEIEVEEELCKQVRSRDIS (SEQ ID NO: 9).

However, in some embodiments, the sequence bound by a b-variant specific antibody may be present in both Ciz1 exon 14a (SEQ ID NO: 6) and Ciz1 exon 14b (SEQ ID NO: 5). In such embodiments, the b-variant specific antibody may be unable to bind to Ciz1 a-variant because the presence of the VEEELCKQ sequence (SEQ ID NO: 10) prevents the antibody binding. In other embodiments, the antibodies may bind to sequences present in both Ciz1 exon 14a and Ciz1 exon 14b, because the sequences are presented in a b-variant specific conformation or structure.

In some embodiments, the b-variant specific antibody may bind to an epitope formed by a Ciz1 b-variant peptide/fibrinogen complex.

Without being bound by theory, the binding of Ciz1 b-variant to fibrinogen may expose epitopes which comprise amino acid sequences which are common between Ciz1 exon 14a (SEQ ID NO: 6) and Ciz1 exon 14b (SEQ ID NO: 5) in a conformation which is unique to the fibrinogen/Ciz1 b-variant. Such conformational epitopes will therefore not be present in Ciz1 α-variant.

### SAMPLE

In certain embodiments, the present invention utilises a sample isolated from a subject. This sample may be referred to as a 'test sample'. Thus the present methods are typically practiced outside of the human or animal body, e.g. on a sample that was previously obtained from the subject to be tested.

The sample may be a blood, urine, saliva or bronchoalveolar lavage sample.

In a preferred embodiment the sample is a blood sample. Suitably, the blood sample may be a whole blood sample. Suitably, the blood sample may be a blood fraction, for example a plasma sample.

Techniques for collecting blood samples and separating blood fractions are well known in the art. For instance, vena blood samples can be collected from patients using a needle and deposited into plastic tubes. The collection tubes may, for example, contain anticoagulants, spray-coated silica, or a polymer gel for separation. Plasma can be separated by centrifugation at 1300 RCF for 10 min at room temperature and stored in small plastic tubes at-80°C.

In a preferred embodiment the sample is a blood sample, in particular a plasma sample. In a preferred embodiment the blood sample is treated with anticoagulants (e.g. heparin) following collection.

### DETERGENT

In one embodiment, the present methods may comprise treating the sample with a detergent in order to release the Ciz1 b-variant and/or a fibrinogen/Ciz1 b-variant complex.

Examples of detergent include, but are not limited to, sodium dodecyl sulfate (SDS) and polysorbate 20. For example, the sample may be treated with about 1 to 5%, preferably 1 to 2% SDS.

Suitably, the present methods may further comprise treating the sample with a reducing agent in order to further purify the Ciz1 b-variant and/or a fibrinogen/Ciz1 b-variant complex. Examples of suitable reducing agents include, but are not limited to, Dithiothreitol (DTT) - which may be used at a concentration of about 10 mM DTT, for example.

### EXOSOMES

The present methods may comprise the step of releasing Ciz1 b-variant and/or a fibrinogen/Ciz1 b-variant complex from the exosomal compartment of a sample. Suitably, the present methods may comprise the step of releasing Ciz1 b-variant peptides from the exosomal compartment of a sample.

Exosomes are nanometer-sized (30-100nm) vesicles found in many biological fluids, such as urine, blood (in both plasma and serum), ascites, and cerebrospinal fluid. They originate as internal vesicles of multi-vesicular bodies (MVBs) in cells and were first described as products of circulating blood cells, such as erythrocytes and lymphocytes.

Exosomes are surrounded by a phospholipid membrane containing relatively high levels of cholesterol, sphingomyelin, and ceramide and containing detergent-resistant membrane domains (lipid rafts) (Mathivanan et al., 2010; J Proteomics 73:1907-1920).

Exosomes are characterized by the presence of proteins involved in membrane transport and fusion, such as Rab, GTPases, annexins, and flotillin, components of the endosomal sorting complex required for transport (ESCRT) complex such as Alix, tumor susceptibility gene 101 (TSG101), heat shock proteins (HSPs), integrins, and tetraspanins, including CD63, CD81, and CD82 (van der Pol *et al.;* 2012; 64(3); 676-705).

Releasing Ciz1 b-variant and/or a fibrinogen/Ciz1 b-variant complex from an exosomal compartment refers to disrupting the structural integrity of the exosome such that Ciz1 b-variant epitopes which were previously concealed within the internal structure of the exosomes are exposed for binding.

Suitably, releasing Ciz1 b-variant and/or a fibrinogen/Ciz1 b-variant complex from an exosomal compartment may refer to treating a sample with a detergent. Examples of detergent include, but are not limited to, sodium dodecyl sulfate (SDS) and polysorbate 20.

In one embodiment, the sample may be treated with a detergent at a concentration which is suitable to release Ciz1 b-variant and/or a fibrinogen/Ciz1 b-variant complex from an exosomal compartment. For example, the sample may be treated with about 1 to 5%, preferably 1 to 2% SDS.

### EXOSOME ENRICHMENT

The present methods may involve enriching an exosomal fraction from a sample.

As used herein, enriching an exosomal fraction is synonymous to purifying or isolating an exosomal fraction.

Exosomes may be purified by methods known in the art such as ultracentrifugation (Pisitkun et al (2004) PNAS 101:13368-13373) or ultrafiltration (Cheruvanky et al (2007) Am. J. Physiol. Renal Physiol. 292:F1657-F1661). Methods are also known involving continuous flow electrophoresis and chromatography procedures which may precede centrifugation (Taylor and Gercel-Taylor (2005) Br J Cancer 92:305-311). Cross-flow ultrafiltration may also be used as part of the exosome purification method (Lamparski et al (2002) J Immunol. Methods 270:211-226).

Commercial kits for the isolation of exosomes are also available, for example the Total Exosome Isolation Kit provided by Invitrogen.

Exosomes can be isolated from a multitude of cell line and body fluids by combining differential centrifugation, membrane filtration, concentration, rate zonal centrifugation and immunocapture, exosomes (Simpson et al.; Proteomics, 8 (2008), pp. 4083-4099).

Exosomes can be detected and/or purified on the basis of their expression of exosomal markers such as tumour susceptibility gene (TSG101), aqua-porin-2 (AQP2), neuron-specific enolase (NES), annexin V, podocalyxin (PODXL) and CD9. For example, exosomes may be captured on to a microplate, for example by immunoaffinity capture.

Characterization of isolated exosomes is typically performed using electron microscopy, FACS, LC-MS/MS, Western blotting or ELISA (Simpson *et al.;* as above) and (van Niel et al; J Biochem (Tokyo), 140 (2006), pp. 13-21).

### CANCER

In one aspect, the present invention relates to a method of diagnosing cancer in a subject.

As used herein, the term "cancer" or "cancerous" refers to cells having the capacity for autonomous growth, i.e., an abnormal state or condition characterized by rapidly proliferating cell growth. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting, for example, lung, breast, thyroid, lymphoid, gastrointestinal, reproductive and genito-urinary tract, as well as adenocarcinomas which include malignancies such as most colon cancers, renal-cell carcinoma, prostate cancer and/or testicular tumours, non-small cell carcinoma of the lung, cancer of the small intestine and cancer of the esophagus.

Suitably, the cancer may be lung cancer, lymphoma, kidney cancer, breast cancer, liver cancer, bladder cancer, ovarian or thyroid cancer.

In one embodiment, the cancer is lung cancer. The lung cancer may be small cell lung cancer. The lung cancer may be non-small cell lung cancer.

### SUBJECT

The subject may be a human or animal subject. Preferably, the subject is a human.

The subject may have or be at risk of cancer, as described herein. 'At risk of cancer' refers to a subject who is not showing any symptoms of the disease. The subject may have a predisposition for, or be thought to be at risk of developing, cancer.

### TREATING

Described herein is a method for treating a subject with cancer comprising administering to the subject a cancer therapeutic; wherein the subject has been identified as having cancer by a method of the present invention.

Described here is an anticancer drug for use in treating cancer wherein the subject has been identified as having cancer by a method of the present invention. Described herein is an anticancer lung cancer drug for use in treating lung cancer wherein the subject has been identified as having lung cancer by a method of the present invention.

'Treating' or 'to treat' refers to the therapeutic use of a cancer therapeutic or anticancer agent. Herein the cancer therapeutic or anticancer agent may be administered to a subject having an existing disease or condition in order to lessen, reduce or improve at least one symptom associated with the disease and/or to slow down, reduce or block the progression of the disease.

### ANTICANCER DRUG

An 'anticancer drug' is synonymous with a cancer therapeutic and refers to an entity which may be used to treat cancer.

The anti-cancer drug may be a cytokine or haematopoietic factor including, but not limited to, IL-1, IL-2, IL-4, IL-5, IL-13, IL-6, CSF-1, M-CSF, GM-CSF, IFNα, IFNβ, IFNγ, IL-10, IL-12, VEGF, bone morphogenic proteins, FGFs, TNF and TGFβ.

The anticancer drug may be a chemotherapeutic agent, which may be a cytotoxic drug. A chemotherapeutic agent contemplated includes, without limitation, alkylating agents, nitrosoureas, ethylenimines/methylmelamine, alkyl sulfonates, antimetabolites, pyrimidine analogs, epipodophylotoxins, enzymes such as L-asparaginase; biological response modifiers such as IFNα, IL-2, G-CSF and GM-CSF; platinium coordination complexes such as cisplatin and carboplatin, anthracenediones, substituted urea such as hydroxyurea, methylhydrazine derivatives including N-methylhydrazine (MIH) and procarbazine, adrenocortical suppressants such as mitotane (o,p'-DDD) and aminoglutethimide; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide.

The anticancer drug may be a siRNA or shRNA.

Suitably, the anticancer drug may be an anticancer lung cancer drug. An anticancer lung cancer drug refers to an entity which may be used to treat lung cancer.

### KIT

In one aspect, the present invention provides a kit comprising an agent which specifically binds fibrinogen and an agent which specifically binds Ciz1 b-variant.

The agent which specifically binds fibrinogen may be a fibrinogen capture agent as described herein.

The agent which specifically binds Ciz1 b-variant may be a Ciz1 b-variant detection agent as described herein.

The terms "comprising", "comprises" and "comprised of as used herein are synonymous with "including", "includes" or "containing", "contains", and are inclusive or open-ended and do not exclude additional, non-recited members, elements or method steps. The terms "comprising", "comprises" and "comprised of also include the term "consisting of.

The invention will now be further described by way of Examples, which are meant to serve to assist one of ordinary skill in the art in carrying out the invention and are not intended in any way to limit the scope of the invention.

### EXAMPLES

### Example 1 - Enrichment of Ciz1 b-variant in an exosomal fraction from a blood sample

The antibody 043 was generated and validated as described previously for antibody 2B (Higgins et al; PNAS 2012; Nov 6;109(45):E3128-35)). The antibody was capable of detecting of Ciz-1 b-variant polypeptide by western blot in plasma from lung cancer patients (see Figure 1a).

Plasma from lung cancer patients was separated into the exosomal compartment and soluble compartment using Invitrogen Total Exosome Isolation Kit (Catalog Number 4484450) used as recommended by the manufacturer. Fractions were separated by SDS-PAGE and probed with 043 antibody or with 'generic' CIZ1 antibodies that recognize an epitope elsewhere in the protein (data shown in Figure 1b is generated with NB100-74624 (Nov4), Novus Biologicals).

The Ciz1 b-variant epitope containing species migrated with relative mobility of ∼70kDa. The Ciz1 b-variant epitope containing species co-fractionated with the exosomal fraction in lung cancer patients (see Figure 1b, red and blue arrows indicate lanes showing different partitioning of Ciz1 isoforms). In rare patients, the Ciz1 b-variant epitope containing species partly partitions with the non-exosomal fraction (type 2, Fig. 1c). Plasma from these patients gave a positive signal in Ciz1 b-variant sandwich ELISA without any pre-treatment to dissolve exosomes. For patients whose Ciz1 b-variant epitope containing species is wholly in the exosome fraction, the Ciz1 b-variant epitope may be accessed by treating plasma (type 1) with detergent prior to assay (Fig. 1e,f).

### Example 2 - Reconstitution of epitope from synthetic b-variant peptides and fibrinogen

The antibody 2B (Fig. 3a) and 043 (Fig. 1a) recognise Ciz1 b-variant in the region of the unique junction between exon 14b and 15. They do not react with a species with relative mobility of ∼70kDa in normal plasma (Fig. 3B, C), but do weakly react with synthetic b-variant peptide (Fig. 3B, C). However when combined, synthetic b-variant peptides and normal plasma generate a new, highly reactive species of ∼80kDa. Similar results are obtained with a synthetic b-variant peptide of a different length (Fig. 3e), quantitatively generating epitope (Fig. 3f).

Mobility of endogenous b-variant epitope in plasma from cancer patients is sensitive to reducing agent, migrating at -340 kDa in the absence of DTT and at ∼70kDa in the presence of DTT (Fig. 4a). Excision of the 340kDa complex (Fig. 4b), followed by further separation into constituent polypeptides upon reduction shifts the epitope to ∼70kDa (Fig. 4c). Excision and elution of this band identified fibrinogen alpha chain by mass spectrometry (Fig. 4d).

Incubation of purified fibrinogen complex (Fig. 5a) with synthetic b-variant peptides generates a b-variant epitope with mobility similar to the fibrinogen complex (Fig. 5a). Comparison with a-variant peptides, or carboxylated derivatives of b-variant peptides, or with different lengths of b-variant peptides showed that i) reconstitution of epitope is specific to b-variant containing peptides, ii) carboxylation of certain E residues near the splice junction affects reconstitution of epitope, iii) b-variant junction containing peptides of different lengths may constitute the epitope that naturally exists in human plasma.

### Example 3 - Characterisation of Ciz-1 b-variant as a biomarker

Analysis of CIZ1b was carried out with anti-peptide rabbit polyclonal antibody 2B, which was raised against a short unique peptide spanning the alternatively spliced 14b-15 junction (Fig.7A), with sequence DEEEIEVRSRDIS (SEQ ID NO: 2 - junction indicated by underlined valine). The strategy for generation, validation and purification was described previously (Higgins *et al.,* as above). The western blot reactivity profile for 2B includes a 65-70kDa entity in SDS-PAGE of plasma from lung cancer patients, plus a 55kDa entity in plasma from all people (Fig.7B). The cancer-specific 65-70kDa species is the CIZ1b biomarker that is referred to here and by Higgins *et al.* In contrast to plasma, this protein could not be detected in serum samples from the same lung cancer patients (Fig.7C), suggesting that CIZ1b biomarker is sequestered in coagulated blood. Notably the 55kDa generic band co-migrates with CIZ1 spec7), identifying the 55kDa species as a variant or proteolytic fragment of full-length hCIZ1 (which has a predicted full length MW of 99kDa, NCBI Reference Sequence: NM_012127.2). The width of the bands detected via exon 8 and exon 17 is different, possibly because the exon 8 epitope lies within an alternatively spiced region, while detection via exon 17 would reveal species with or without alternative splicing at exon 8. Thus at least one form of the CIZ1 protein is present in the circulatory system in cancer and non-cancer plasma samples alike, suggesting it to be part of normal physiology.

Exon 17 and exon 8 epitopes are not present in the 65-70kDa species recognised by CIZ1 b antibody 2B in cancer patients, which means that sequences both upstream and downstream of the CIZ1b epitope are missing from this protein. The data suggest that the 65-70kDa species is either a complicated CIZ1 splice variant, or an SDS-stable complex between a CIZ1b fragment of CIZ1 and a carrier protein (Fig.7D).

A further set of rabbit antibodies was generated using the same immunisation protocol as for 2B (see Higgins *et al.,* as above). This yielded antibody 043, which cleanly and uniquely reacts with the 65-70kDa band in cancer patients (Fig.7B). Thus 043 provides a detection tool with applications in other assay formats, including ELISA. The lack of recognition of the 55kDa species suggests less contribution of junction flanking sequences to its epitope compared to 2B, and suggests that the 55kDa species does not contain the CIZ1 b junction.

Direct comparison of the ability of these two antibodies to discriminate lung cancer patients by western blot shows them both to be discriminatory, with a high degree of correlation (Fig.7E), though notably they do not share exactly the same profile across plasma test set. Thus, there is some variation in their epitopes but a common ability to discriminate patients with lung cancer from those without. There is also a small advantage in integrating their output to maximise selectivity.

### Example 4 - Stability of the Ciz-1 b-variant biomarker

The data support the assertion that CIZ1b is a potent circulating biomarker for lung cancer with significant potential for application in clinical practise. To realise this potential it must be robust enough to withstand variations in sample processing and collection regimes that occur in busy hospitals. Therefore, the stability of the 65-70kDa cancer-selective CIZ1b species in plasma and whole blood was tested, and compared with the 55kDa species, using antibody 2B. The data show stability in isolated plasma over at least 6 hours when incubated at 37°C, with gradual decay in both species thereafter. Similarly there was relatively little change during an hour at temperatures up to 50°C, and stability over at least 3 freeze-thaw cycles. Furthermore, when whole blood was left on the bench for up to 24 hours prior to fractionation there was no loss of signal, all of which indicate it to be a robust biomarker suitable for application in a range of clinical settings.

### Example 5 - Dissociation of the Ciz-1 b-variant biomarker complex

Under native conditions both 2B and 043 detect a complex in excess of 720kDa that co-migrates with an abundant protein in plasma (Fig. 8A), but there is very little discrimination between cancer and non-cancer plasmas. Upon further separation through a second dimension under denaturing conditions the cancer-specific 65-70kDa species is revealed and (for 2B) separated from the 55kDa generic band (Fig.8B). Thus cancer-specific detection of CIZ1b in a completely native immunoassay format is unlikely even with 043. The three major denaturing influences (heat, SDS, reducing agent) that reveal cancer-specific signal and were evaluated for their effect on the mobility and discrimination of CIZ1b by 043. This showed that 1% SDS shifts the major reactive species from >720kDa in native gel, to approximately 340kDa (Fig.8C, right), revealing cancer-specific signal, as well as an unspecific band at ∼200kDa in both cancer and non-cancer samples. Increasing the incubation temperature has a detrimental effect on the cancer-specific species, with maximum differential achieved at 37°C. Inclusion of reducing agent further shifted the mobility of the cancer specific signal to the expected position of 65-70 kDa (Fig.8C, left).

Notably, higher concentrations of reducing agent resulted in loss of CIZ1b signal (Fig.8D,E). Taken together these data suggest that the 65-70kDa entity is itself a complex that is resistant to standard SDSPAGE denaturing conditions (10 mM DTT) but not more aggressive treatments (500mM DTT), and that it is normally present within a higher order complex of 340kDa, which is itself released by SDS from a still bigger complex in excess of 720kDa (illustrated in Fig.9A). In fact, fractionation of plasma to enrich for the exosomal compartment differentially partitioned the 55kDa species (detected via exon 17) and 65-70kDa species (detected via 043), with the 65-70kDa species detected exclusively in the pellet, which is a complex fraction that includes exosomes.

### Example 6 - Identification of the Ciz-1 b-variant biomarker complex

These observations enable a two-step gel-purification strategy for the 65-70kDa CIZ1b band. First, the 340kDa species was isolated from two independent lung cancer patient plasmas (Fig.10A), followed by further separation in the presence of reducing agent (Fig. 10B). The 65-70kDa band corresponding to the cancer-specific western blot signal was excised and digested with either trypsin or Asp-N endoprotease, for identification by mass spectrometry (MS). For both enzymatic digestions and two patients, Mascot database searching returned a single significant identification (P<0.05), corresponding to Uniprot P02671; human fibrinogen alpha chain. The endogenous fibrinogen molecule is a hexameric glycoprotein with a pre-cleavage formula mass of 326kDa and relative mobility of ∼340kDa, comprised of two sets of three different chains (α, β, and y) of 67, 51 and 45kDa respectively.

Notably, CIZ1b was not identified in the 65-70kDa band after digestion with either enzyme. To determine whether a diagnostic CIZ1b fragment could be observed by MS if present, a short synthetic CIZ1b peptide (b13) and long CIZ1b peptide (b66, Table 2) were digested with Asp-N, which generates the diagnostic fragment DEEEIEVRSR (SEQ ID NO: 16) by cutting either side of the CIZ1b exon junction. The peptide was positively identified post digestion of b13 and b66 by MALDI-MS/MS, though at low intensity relative to other peptides in the digest. Further analysis was performed by LC-MS/MS using both data dependent acquisition (DDA) and targeted selection reaction monitoring (SRM), which focused the analysis to cycle through fragmentation of the expected 2+ and 3+ ions of DEEEIEVRSR (SEQ ID NO: 16), excluding all other precursors, to maximize specificity and sensitivity for this analyte. DEEEIEVRSR (SEQ ID NO: 16) was identified in both the DDA and SRM analyses, indicating that the diagnostic peptide is observable by MS when excised from chemically synthesized peptide (SRM chromatogram).

However, when the same peptide was spiked into normal human plasma to reconstitute CIZ1b, the diagnostic DEEEIEVRSR (SEQ ID NO: 16) peptide was not detected in the excised 65-70kDa band. Lack of identification could reflect: i) secondary modifications that alter the expected mass of the CIZ1 b diagnostic peptide or impair enzymatic digestion, ii) the more abundant proteins in the sample, primarily fibrinogen alpha chain, causing ion suppression or iii) absolute abundance falling below the limit of detection for the instrumentation after the losses incurred from the PAGE and digestion steps. It should be noted that the peptide is highly acidic, which is likely to reduce ionization efficiency in positive-mode MS, so that even with SRM a small drop in abundance could fall below the limit of detection. It was concluded if the spiked peptide could not be reliably detected at physiologically relevant levels it was unlikely to be possible to detect endogenous CIZ1b, and so followed alternative approaches were followed.

**Table 2**

| **Designati on** | **Sequence** |
|---|---|
| Short CIZ1b (b13) | CDEEEIEVRSRDIS-NH2 (SEQ ID NO: 22) |
| Super short CIZ1B (b7) | CEIEVRSR-NH2 (SEQ ID NO: 17) |
| Short CIZ1a (a22) | CDEEEIEVEEELCKQVRSRDISR-NH2 (SEQ ID NO: 18) |
| Long CIZ1b peptide (b66) | |
| Long CIZ1a peptide (a74) | |
| GLA3 | CDEEγIEVRSRDIS-NH2 (SEQ ID NO: 14) |
| GLA4 | CDEγEIEVRSRDIS-NH2 (SEQ ID NO: 23) |
| GLA6 | CDγγγIγVRSRDIS-NH2 (SEQ ID NO: 15) |
| Exon 17 | C-TSSGRPPSQPNTQDKTPSK (SEQ ID NO: 20) |
| | C-TARPSQPPLPRRSTRLKT (SEQ ID NO: 21) |

The 8 amino-acids spliced out of CIZ1b are underlined in non-alternatively spliced sequences, and the junction created by their absence is indicated in CIZ1b sequences by a V. Where indicated peptides contain an additional N-terminal cysteine residue, and amidation of the C-terminus. Carboxylated glutamic acid residues are indicated by γ.

### Example 7 - Epitope reconstitution of the Ciz-1 b-variant biomarker complex

In order to confirm that the 65-70kDa cancer-specific epitope does indeed contain CIZ1b sequences, an epitope reconstitution strategy was adopted. To achieve this, a pair of synthetic peptides spanning the exon14/15 junction (CIZ1b13, and CIZ1a22 which includes the alternatively spliced intervening sequence, Table 2) were incubated with normal human plasma (Fig.10C), and the output compared to lung cancer patient plasma. The CIZ1b peptide, but not CIZ1a, generated 043 and 2B-reactive epitope of comparable mobility to the endogenous epitope (65 70kDa), which is far in excess of the relative molecular mass of free peptide and not present in either of the individual components of the reaction (peptide lanes 6, 7, or plasma lane 3). This suggests that CIZ1b peptide forms an SDS/DTT-resistant complex with a carrier protein in human plasma, recreating the CIZ1b biomarker recognised by 2B and 043. Since no mobility difference between endogenous (cancer plasma lane 1) and reconstituted epitope was detected, the data also imply that the CIZ1b fragment contributing to the 65-70kDa cancer-specific species is very short, in the order of 10-20 amino acids in length. Furthermore, individual molecules may not necessarily have uniform molecular boundaries, possibly accounting for the often-diffuse nature of the band.

A molar equivalent of the much longer CIZ1b peptide (b66, Table 2) reconstituted reactive epitope less efficiently, and was detectable only by 2B (Fig.10C lane 2, and at higher concentration). With this peptide the reconstituted signal migrated with greater relative molecular mass, at ∼80kDa, consistent with the increased size of the contributing peptide. Importantly reconstituted CIZ1b epitope, like endogenous epitope, resists standard denaturing SDS-PAGE conditions (10 mM DTT, 2% SDS, 90°C), but is dissociated by a more aggressive reducing environment. Taken together the data suggest that, *in vivo,* the cancer specific epitope is composed of a relatively short fragment of CIZ1 encompassing the exon14b/15 junction, mounted on fibrinogen alpha chain, which itself makes up most of the mass of the 65- 70kDa species. To further confirm this, epitope reconstitution experiments were performed with purified human fibrinogen (Fig.10D). For both antibodies, reactive epitope was generated at the mobility of fibrinogen complex (340kDa in non-reducing gels) and the specificity of the signal was confirmed by lack of reactivity with equivalent CIZ1a peptide. Direct ELISA using human fibrinogen complexed with molar equivalents of CIZ1a and CIZ1b peptides (Fig.10E), confirmed i) the specificity of epitope reconstitution with peptide b13 over a22, ii) negligible signal with fibrinogen alone, and iii) greater reactivity when peptide is mounted on fibrinogen. Moreover, inclusion of the very short CIZ1b peptide b7 (Table 2) revealed differences between 2B and 043; 2B recognises b7 while 043 does not in a manner that is unrelated to the presence of fibrinogen. The existence of CIZ1b epitope in a complex with fibrinogen is consistent with the earlier observations that the biomarker is depleted from serum samples isolated from clotted blood (Fig.7C).

### Example 8 - CIZ1b immunoassay

Based on knowledge of the composition of CIZ1b biomarker, a sandwich immunoassay format capable of quantitative detection in partially denatured plasma was designed. Antigen capture with CIZ1b antibody 043 from 5 ul of plasma and detection via fibrinogen alpha chain, generated a cancer-specific signal. Specifically, results by western blot with 043 generated ROC AUC 0.823 (P=0.0002) and by ELISA with 043 /anti-fibrinogen of 0.830 (P=0.0007, Fig.11A,B). Thus, the two methods generate very similar outcomes, and the data is significantly correlated.

This data set, which represents patients with a wide range of conditions presenting at York Hospital respiratory medicine clinics, was also used to evaluate the contribution of the CIZ1b component of the assay, since fibrinogen alone has been reported to have some potential as a biomarker for lung cancer (Allin et al.; 2016; Int J Cancer 139(7):1493-1500). Quantitative detection of fibrinogen in the linear range returned some discriminatory capability across this set, though relatively poor ROC AUC of 0.628 (p=0.225). This is consistent with western blot evaluation of fibrinogen, which indicated little difference between lung cancer patients and those without malignant disease. Thus capture of CIZ1b is the element that contributes the majority of the cancer selectivity to this assay. The specificity of the configuration was further tested by analysing the sample set after capture with 043, but substituting anti-fibrinogen detector antibody with anti-human IgG (Fig. 11D). Results indicate no discrimination between patients and control plasmas, confirming that anti-fibrinogen is an informative detector reagent that specifically detects a cancer-selective complex retrieved via CIZ1b. Finally, measurement of total IgG levels across set B by direct ELISA (Fig. 11E) was used to normalize the output generated by 043/fibrinogen, leading to a marginal improvement in discrimination and ROC AUC 0.845 (p=0.0002, Fig.11F).

The CIZ1b-fibrinogen alpha chain complex ELISA was performed as a sandwich ELISA after partial denaturation of 5 ul of plasma in 100 ul of 0.5% tween20, 1% SDS in PBS, supplemented with 0.5mM PMSF (protease inhibitors) and incubation at 20°C for 30 mins with repeated vortexing. Plates (Nunc Maxisorb 442404) were coated with 1ug of 043 CIZ1b pAb protein A fraction, overnight at 4°C in 100ul of 50 mM carbonate pH 9.6, then blocked for two hours at room temperature with 300 ul of filtered 1% BSA in PBS. After three washes in 300 ul of 0.05% tween20 in PBS, analyte was added for 2 hours at room temperature with gentle shaking. After fives washes, captured complex was detected with sheep anti-fibrinogen pAb (AF4786 R&D systems) followed by anti-sheep HRP (Jackson ImmunoResearch 213-032-177), and results developed using 100 ul Sure Blue (KPL 520001) with detection at A450nm using a FLUOstar OPTIMA plate reader (BMG Labtech).

For measurement of fibrinogen capture antibody was chicken IgY (1ug/well, Genway 15-288-22856), and analyte concentration was reduced five orders of magnitude by serial dilution. For measurement of human IgG in direct ELISA, 0.5 ul of plasma was coated directly onto plates in 100ul of 50 mM carbonate buffer pH 9.6, blocked, washed and detected with anti-IgG (Jackson Immuno Research 709-035-149). For direct ELISA of synthetic analyte, 0.5ug purified fibrinogen (Sigma F3879) complexed with 100pmol peptide was coated onto plates in 100ul of 50 mM carbonate pH 9.6 as above, probe with 043 or 2B and detected with anti-rabbit HRP (Jackson ImmunoResearch).

Thus, the present sandwich ELISA format is suitable for high-throughput application on hospital platforms, and can reliably quantify circulating CIZ1b biomarker, and can be used to identify patients with early stage lung cancer.

Various modifications and variations of the described methods and system of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. Use of a fibrinogen capture agent or a fibrinogen detection agent in an assay to detect the Ciz1 b-variant.

2. The use according to claim 1 wherein the assay is an enzyme-linked immunosorbent assay.

3. The use according to claim 1 or 2 wherein the fibrinogen capture agent or fibrinogen detection agent is an antibody or a fragment thereof, or Ciz1 b-variant peptide which specifically binds fibrinogen, wherein the Ciz1 b-variant peptide comprises the sequence shown in SEQ ID NO: 11 or a fragment thereof which comprises EVR motif as shown in positions 36 to 38 of SEQ ID NO: 11.

4. The use according to claim 1 or 2 wherein the fibrinogen capture agent or a fibrinogen detection agent is an antibody or a fragment thereof which specifically binds fibrinogen or wherein the fibrinogen capture agent is a Ciz1 b-variant peptide which specifically binds fibrinogen, wherein the Ciz1 b-variant peptide comprises the sequence shown in SEQ ID NO: 11 or a fragment thereof which comprises EVR motif as shown in positions 36 to 38 of SEQ ID NO: 11.

5. The use according to any preceding claim wherein the fibrinogen is fibrinogen alpha chain.

6. The use according to any preceding claim wherein the Ciz1 b-variant is detected by a method which comprises the following steps:
a) capturing a fibrinogen/Ciz1 b-variant peptide complex using a fibrinogen capture agent; and
b) detecting Ciz1 b-variant peptide using a Ciz1 b-variant peptide detection agent; or
a) capturing a fibrinogen/Ciz1 b-variant peptide complex using a Ciz1 b-variant peptide capture agent; and
b) detecting fibrinogen using a fibrinogen detection agent.

7. A method of diagnosing cancer in a subject which comprises detecting a Ciz1 b-variant peptide in a sample from the subject, wherein the presence of Ciz1 b-variant peptide in the sample indicates that the subject has cancer and wherein the method comprises the step of capturing a fibrinogen/Ciz1 b-variant peptide complex from the sample, wherein the Ciz1 b-variant peptide comprises the sequence shown in SEQ ID NO: 11 or a fragment thereof which comprises EVR motif as shown in positions 36 to 38 of SEQ ID NO: 11.

8. A method according to claim 7 which comprises the following steps:
a) capturing a fibrinogen/Ciz1 b-variant peptide complex using a fibrinogen capture agent; and
b) detecting Ciz1 b-variant peptide using a Ciz1 b-variant peptide detection agent; or
a) capturing a fibrinogen/Ciz1 b-variant peptide complex using a Ciz1 b-variant peptide capture agent; and
b) detecting fibrinogen using a fibrinogen detection agent.

9. The method according to claim 8 wherein the Ciz1 b-variant peptide capture agent is an antibody or a fragment thereof which specifically binds Ciz1 b-variant peptide.

10. The method according to any of claims 7 to 9 wherein the step of detecting the Ciz1 b-variant peptide or fibrinogen employs antibody-based arrays, enzyme linked immunosorbent assays (ELISA), radioimmuno-assay (RIA), western blotting or mass spectrometry.

11. The method according to any of claims 7 to 10, further comprising the step of releasing a fibrinogen/Ciz1 b-variant peptide complex from an exosomal compartment of the sample, optionally wherein the fibrinogen/Ciz1 b-variant peptide complex is released from the exosomal compartment by detergent treatment.

12. The method according to claim 11, further comprising the step of enriching the exosomal fraction from the sample, optionally wherein the step of enriching the exosomal fraction comprises ultracentrifugation, ultrafiltration, continuous flow electrophoresis, chromatography, precipitation or cross-flow ultrafiltration.

13. The method according to any of claims 8 to 16 wherein the cancer is selected from lung, lymphoma, kidney, breast, liver, bladder, ovarian or thyroid cancer, preferably wherein the cancer is lung cancer.

14. Use of a detergent to release a fibrinogen/Ciz1 b-variant peptide complex from an exosomal compartment of a sample, wherein the Ciz1 b-variant peptide comprises the sequence shown in SEQ ID NO: 11 or a fragment thereof which comprises EVR motif as shown in positions 36 to 38 of SEQ ID NO: 11.

15. A method for detecting a Ciz1 b-variant peptide in a sample which comprises the step of releasing a fibrinogen/Ciz1 b-variant peptide complex from an exosomal compartment of the sample by treating the sample with detergent and detecting if the Ciz1 b-variant peptide is present in the sample, wherein the Ciz1 b-variant peptide comprises the sequence shown in SEQ ID NO: 11 or a fragment thereof which comprises EVR motif as shown in positions 36 to 38 of SEQ ID NO: 11.

16. A kit comprising an agent which specifically binds fibrinogen and an agent which specifically binds Ciz1 b-variant peptide, wherein the Ciz1 b-variant peptide comprises the sequence shown in SEQ ID NO: 11 or a fragment thereof which comprises EVR motif as shown in positions 36 to 38 of SEQ ID NO: 11.

17. A kit according to claim 16 wherein the fibrinogen is fibrinogen alpha chain.

18. A kit according to claim 16 or 17 wherein the agent which specifically binds fibrinogen and/or the agent which specifically binds Ciz1 b-variant peptide is an antibody or a fragment thereof.

## Patentansprüche

1. Verwendung eines Fibrinogen-Einfangmittels oder eines Fibrinogen-Nachweismittels in einem Assay zum Nachweis der Ciz1 b-Variante.

2. Verwendung nach Anspruch 1, wobei der Assay ein Enzyme-linked Immunosorbent Assay ist.

3. Verwendung nach Anspruch 1 oder 2, wobei das Fibrinogen-Einfangmittel oder das Fibrinogen-Nachweismittel ein Antikörper oder ein Fragment davon oder ein Ciz1 b-Variantenpeptid, das spezifisch Fibrinogen bindet, ist, wobei das Ciz1 b-Variantenpeptid die in SEQ ID NO: 11 gezeigte Sequenz oder ein Fragment davon umfasst, das das EVR-Motiv umfasst, wie in den Positionen 36 bis 38 von SEQ ID NO: 11 gezeigt.

4. Verwendung nach Anspruch 1 oder 2, wobei das Fibrinogen-Einfangmittel oder ein Fibrinogen-Nachweismittel ein Antikörper oder ein Fragment davon ist, das spezifisch Fibrinogen bindet, oder wobei das Fibrinogen-Einfangmittel ein Ciz1 b-Variantenpeptid ist, das spezifisch Fibrinogen bindet, wobei das Ciz1 b-Variantenpeptid die in SEQ ID NO: 11 gezeigte Sequenz oder ein Fragment davon umfasst, das das EVR-Motiv umfasst, wie in den Positionen 36 bis 38 von SEQ ID NO: 11 gezeigt.

5. Verwendung nach einem der vorangehenden Ansprüche, wobei das Fibrinogen Fibrinogen-alpha-Kette ist.

6. Verwendung nach einem der vorangehenden Ansprüche, wobei die Ciz1 b-Variante durch ein Verfahren nachgewiesen wird, das die folgenden Schritte umfasst:
a) Einfangen eines Fibrinogen/ Ciz1 b-Variantenpeptidkomplexes unter Verwendung eines Fibrinogen-Einfangmittels; und
b) Nachweis des Ciz1 b-Variantenpeptids unter Verwendung eines Ciz1 b-Variantenpeptid-Nachweismittels; oder
a) Einfangen eines Fibrinogen/ Ciz1 b-Variantenpeptidkomplexes unter Verwendung eines Ciz1 b-Variantenpeptid-Einfangmittels; und
b) Nachweisen von Fibrinogen unter Verwendung eines Fibrinogen-Nachweismittels.

7. Verfahren zur Diagnose von Krebs in einem Subjekt, umfassend das Nachweisen eines Ciz1 b-Variantenpeptids in einer Probe des Subjekts, wobei das Vorliegen des Ciz1 b-Variantenpeptids in der Probe anzeigt, dass das Subjekt Krebs hat, und wobei das Verfahren den Schritt des Einfangens eines Fibrinogen/ Ciz1 b-Variantenpeptidkomplexes aus der Probe umfasst, wobei das Ciz1 b-Variantenpeptid die in SEQ ID NO: 11 gezeigte Sequenz oder ein Fragment davon umfasst, das das EVR-Motiv umfasst, wie in den Positionen 36 bis 38 von SEQ ID NO: 11 gezeigt.

8. Verfahren nach Anspruch 7, das die folgenden Schritte umfasst:
a) Einfangen eines Fibrinogen/ Ciz1 b-Variantenpeptidkomplexes unter Verwendung eines Fibrinogen-Einfangmittels; und
b) Nachweisen des Ciz1 b-Variantenpeptids unter Verwendung eines Ciz1 b-Variantenpeptid-Nachweismittels; oder
a) Einfangen eines Fibrinogen/ Ciz1 b-Variantenpeptidkomplexes unter Verwendung eines Ciz1 b-Variantenpeptid-Einfangmittels; und
b) Nachweisen von Fibrinogen unter Verwendung eines Fibrinogen-Nachweismittels.

9. Verfahren nach Anspruch 8, wobei das Ciz1 b-Variantenpeptid-Einfangmittel ein Antikörper oder ein Fragment davon ist, das spezifisch das Ciz1 b-Variantenpeptid bindet.

10. Verfahren nach einem der Ansprüche 7 bis 9, wobei der Schritt des Nachweises des Ciz1 b-Variantenpeptids oder Fibrinogens Antikörper-basierte Arrays, Enzyme-linked Immunosorbent Assays (ELISA), Radioimmun-Assays (RIA), Western Blotting oder Massenspektrometrie einsetzt.

11. Verfahren nach einem der Ansprüche 7 bis 10, ferner umfassend den Schritt der Freisetzung des Fibrinogen/ Ciz1 b-Variantenpeptidkomplexes aus einem exosomalen Kompartiment der Probe, wobei der Fibrinogen/ Ciz1 b-Variantenpeptidkomplex gegebenenfalls durch Detergensbehandlung aus dem exosomalen Kompartiment freigesetzt wird.

12. Verfahren nach Anspruch 11, ferner umfassend den Schritt des Anreicherns der exosomalen Fraktion aus der Probe, wobei der Schritt des Anreicherns der exosomalen Fraktion gegebenenfalls Ultrazentrifugation, Ultrafiltration, kontinuierliche Durchflusselektrophorese, Chromatographie, Präzipitation oder Tangentialfluss-Ultrafiltration umfasst.

13. Verfahren nach einem der Ansprüche 8 bis 16, wobei der Krebs ausgewählt ist aus Lungen-, Lymphom-, Nieren-, Brust-, Leber-, Blasen-, Eierstock- oder Schilddrüsenkrebs, wobei der Krebs vorzugsweise Lungenkrebs ist.

14. Verwendung eines Detergens zur Freisetzung eines Fibrinogen/ Ciz1 b-Variantenpeptidkomplexes aus einem exosomalen Kompartiment einer Probe, wobei das Ciz1 b-Variantenpeptid die in SEQ ID NO: 11 gezeigte Sequenz oder ein Fragment davon umfasst, das das EVR-Motiv umfasst, wie in den Positionen 36 bis 38 von SEQ ID NO: 11 gezeigt.

15. Verfahren zum Nachweis eines Ciz1 b-Variantenpeptids in einer Probe, das den Schritt des Freisetzens eines Fibrinogen/ Ciz1 b-Variantenpeptidkomplexes aus einem exosomalen Kompartiment der Probe durch Behandeln der Probe mit einem Detergens und Nachweisen, ob das Ciz1 b-Variantenpeptid in der Probe vorhanden ist, umfasst, wobei das Ciz1 b-Variantenpeptid die in SEQ ID NO: 11 gezeigte Sequenz oder ein Fragment davon umfasst, das ein EVR-Motiv umfasst, wie in den Positionen 36 bis 38 von SEQ ID NO: 11 gezeigt.

16. Kit, umfassend ein Mittel, das spezifisch Fibrinogen bindet, und ein Mittel, das spezifisch Ciz1 b-Variantenpeptid bindet, wobei das Ciz1 b-Variantenpeptid die in SEQ ID NO: 11 gezeigte Sequenz oder ein Fragment davon umfasst, das ein EVR-Motiv umfasst, wie in den Positionen 36 bis 38 von SEQ ID NO: 11 gezeigt.

17. Kit nach Anspruch 16, wobei das Fibrinogen die Fibrinogen-alpha-Kette ist.

18. Kit nach Anspruch 16 oder 17, wobei das Mittel, das spezifisch Fibrinogen bindet, und/oder das Mittel, das spezifisch Ciz1 b-Variantenpeptid bindet, ein Antikörper oder ein Fragment davon ist.

## Revendications

1. Utilisation d'un agent de capture de fibrinogène ou d'un agent de détection de fibrinogène dans un essai pour détecter le variant b de Ciz1.

2. Utilisation selon la revendication 1 dans laquelle l'essai est un essai avec immunosorbant lié à une enzyme.

3. Utilisation selon la revendication 1 ou 2 dans laquelle l'agent de capture de fibrinogène ou l'agent de détection de fibrinogène est un anticorps ou un fragment de celui-ci, ou un peptide de variant b de Ciz1 qui se lie spécifiquement au fibrinogène, dans laquelle le peptide de variant b de Ciz1 comprend la séquence décrite dans SEQ ID NO : 11 ou un fragment de celle-ci qui comprend un motif EVR comme décrit aux positions 36 à 38 de SEQ ID NO : 11.

4. Utilisation selon la revendication 1 ou 2 dans laquelle l'agent de capture de fibrinogène ou un agent de détection de fibrinogène est un anticorps ou un fragment de celui-ci qui se lie spécifiquement au fibrinogène ou dans laquelle l'agent de capture de fibrinogène est un peptide de variant b de Ciz1 qui se lie spécifiquement au fibrinogène, dans laquelle le peptide de variant b de Ciz1 comprend la séquence décrite dans SEQ ID NO : 11 ou un fragment de celle-ci qui comprend un motif EVR comme décrit aux positions 36 à 38 de SEQ ID NO : 11.

5. Utilisation selon l'une quelconque des revendications précédentes dans laquelle le fibrinogène est la chaîne alpha de fibrinogène.

6. Utilisation selon l'une quelconque des revendications précédentes, dans laquelle le variant b de Ciz1 est détecté par un procédé qui comprend les étapes suivantes :
a) capture d'un complexe fibrinogène/peptide de variant b de Ciz1 au moyen d'un agent de capture de fibrinogène ; et
b) détection du peptide de variant b de Ciz1 au moyen d'un agent de détection de peptide de variant b de Ciz1 détection ; ou
a) capture d'un complexe fibrinogène/peptide de variant b de Ciz1 au moyen d'un agent de capture de peptide de variant b de Ciz1 ; et
b) détection du fibrinogène au moyen d'un agent de détection de fibrinogène.

7. Procédé de diagnostic du cancer chez un sujet qui comprend la détection d'un peptide de variant b de Ciz1 dans un échantillon du sujet, dans lequel la présence de peptide de variant b de Ciz1 dans l'échantillon indique que le sujet a un cancer et dans lequel le procédé comprend l'étape de capture d'un complexe fibrinogène/peptide de variant b de Ciz1 à partir de l'échantillon, dans lequel le peptide de variant b de Ciz1 comprend la séquence décrite dans SEQ ID NO : 11 ou un fragment de celle-ci qui comprend un motif EVR comme décrit aux positions 36 à 38 de SEQ ID NO : 11.

8. Procédé selon la revendication 7 qui comprend les étapes suivantes :
a) capture d'un complexe fibrinogène/peptide de variant b de Ciz1 au moyen d'un agent de capture de fibrinogène ; et
b) détection du peptide de variant b de Ciz1 au moyen d'un agent de détection de peptide de variant b de Ciz1 ; ou
a) capture d'un complexe fibrinogène/peptide de variant b de Ciz1 au moyen d'un agent de capture de peptide de variant b de Ciz1 ; et
b) détection du fibrinogène au moyen d'un agent de détection de fibrinogène.

9. Procédé selon la revendication 8, dans lequel l'agent de capture de peptide de variant b de Ciz1 est un anticorps ou un fragment de celui-ci qui se lie spécifiquement au peptide de variant b de Ciz1.

10. Procédé selon l'une quelconque des revendications 7 à 9 dans lequel l'étape de détection du peptide de variant b de Ciz1 ou du fibrinogène utilise des essais à base d'anticorps, des essais avec immunosorbant lié à une enzyme (ELISA), un radioimmunoessai (RIA), un transfert western ou la spectrométrie de masse.

11. Procédé selon l'une quelconque des revendications 7 à 10, comprenant en outre l'étape de libération d'un complexe fibrinogène/peptide de variant b de Ciz1 depuis un compartiment exosomal de l'échantillon, facultativement dans lequel le complexe fibrinogène/peptide de variant b de Ciz1 est libéré depuis le compartiment exosomal par traitement par détergent.

12. Procédé selon la revendication 11, comprenant en outre l'étape d'enrichissement de la fraction exosomale de l'échantillon, facultativement dans lequel l'étape d'enrichissement de la fraction exosomale comprend l'ultracentrifugation, l'ultrafiltration, l'électrophorèse à flux continu, la chromatographie, la précipitation ou l'ultrafiltration à flux latéral.

13. Procédé selon l'une quelconque des revendications 8 à 16, dans lequel le cancer est choisi parmi un cancer du poumon, un lymphome, un cancer du rein, du sein, du foie, de la vessie, des ovaires ou de la thyroïde, de préférence dans lequel le cancer est un cancer du poumon.

14. Utilisation d'un détergent pour libérer un complexe fibrinogène/peptide de variant b de Ciz1 d'un compartiment exosomal d'un échantillon, dans laquelle le peptide de variant b de Ciz1 comprend la séquence décrite dans SEQ ID NO : 11 ou un fragment de celle-ci qui comprend un motif EVR comme décrit aux positions 36 à 38 de SEQ ID NO : 11.

15. Procédé de détection d'un peptide de variant b de Ciz1 dans un échantillon qui comprend l'étape de libération d'un complexe fibrinogène/peptide de variant b de Ciz1 d'un compartiment exosomal de l'échantillon par traitement de l'échantillon avec un détergent et détection du fait que le peptide de variant b de Ciz1 est présent ou non dans l'échantillon, dans lequel le peptide de variant b de Ciz1 comprend la séquence décrite dans SEQ ID NO : 11 ou un fragment de celui-ci qui comprend un motif EVR comme décrit aux positions 36 à 38 de SEQ ID NO : 11.

16. Kit comprenant un agent qui se lie spécifiquement au fibrinogène et un agent qui se lie spécifiquement au peptide de variant b de Ciz1, dans lequel le peptide de variant b de Ciz1 comprend la séquence décrite dans SEQ ID NO : 11 ou un fragment de celle-ci qui comprend un motif EVR comme décrit aux positions 36 à 38 de SEQ ID NO : 11.

17. Kit selon la revendication 16 dans lequel le fibrinogène est la chaîne alpha de fibrinogène.

18. Kit selon la revendication 16 ou 17, dans lequel l'agent qui se lie spécifiquement au fibrinogène et/ou l'agent qui se lie spécifiquement peptide de variant b de Ciz1 est un anticorps ou un fragment de celui-ci.
